# EUROPEAN PATENT SPECIFICATION

(11) **EP 3 952 890 B1**
(45) Date of publication and mention of the grant of the patent: **21.01.2026**
(21) Application number: 20788660.7
(22) Date of filing: 07.04.2020
(51) Int. Cl.: A61K 35/17, C07K 14/54, A61K 38/00, C07K 14/55, C07K 16/28, A61K 45/06

(54) **METHODS AND COMPOSITIONS FOR PROGRAMMING T CELL DIFFERENTIATION AND ENHANCING T CELL PROLIFERATION**
VERFAHREN UND ZUSAMMENSETZUNGEN ZUR PROGRAMMIERUNG VON T-ZELL-DIFFERENZIERUNG UND ZUR STEIGERUNG DER T-ZELL-PROLIFERATION
PROCÉDÉS ET COMPOSITIONS DE PROGRAMMATION DE LA DIFFÉRENCIATION DE LYMPHOCYTES T ET D'AMÉLIORATION DE LA PROLIFÉRATION DE LYMPHOCYTES T

(30) Priority: 08.04.2019 WO PCT/US2019/026378
(43) Date of publication of application: 16.02.2022
(73) Proprietor: The Trustees of The University of Pennsylvania, Philadelphia, PA 19104 (US)
(72) Inventor: POWELL, Daniel J., Bala Cynwyd, Pennsylvania 19004 (US); SMOLE, Anze, Philadelphia, Pennsylvania 19146 (US)
(74) Representative: Grünecker Patent- und Rechtsanwälte PartG mbB
(86) International application number: PCT/US2020/027076
(87) International publication number: WO 2020/210235

(56) References cited:
- US-A1- 2018 044 424
- US-A1- 2018 258 394
- KLEBANOFF C A ET AL: "Inhibition of AKT signaling uncouples T cell differentiation from expansion for receptor-engineered adoptive immunotherapy", JCI INSIGHT, vol. 2, no. 23, E95103, 7 December 2017 (2017-12-07), XP055671296, DOI: 10.1172/jci.insight.95103
- SMOLE A ET AL: "Uni-Vect: Antigen-Inducible Immunomodulatory Molecule Expression from a Single Lentiviral Vector Platform to Enhance CAR T Cell Functions", MOLECULAR THERAPY, vol. 27, no. 4, Suppl. 1, 942, 22 April 2019 (2019-04-22), 22nd Annual Meeting of the American Society of Gene and Cell Therapy; Washington DC, USA; 29 April 29- 2 May 2019, pages 433 - 434, XP093034922, ISSN: 1525-0016
- SMOLE A ET AL: "Expression of inducible factors reprograms CAR-T cells for enhanced function and safety", CANCER CELL, vol. 40, no. 12, 12 December 2022 (2022-12-12), pages 1470 - 1487.e7, XP093034950, DOI: 10.1016/j.ccell.2022.11.006
- NEWTON ET AL.: "Maintenance of CD 4 T cell fitness through regulation of Foxo1", NATURE IMMUNOLOGY, vol. 19, no. 8, 2018, pages 838 - 848, XP036553030, DOI: 10.1038/s41590-018-0157-4

## Description

### CROSS-REFERENCE TO RELATED APPLICATION

This application claims priority under 35 U.S.C. §365(b) to International Application PCT/US2019/026378, filed April 8, 2019

### BACKGROUND OF THE INVENTION

Immunotherapy has demonstrated extraordinary clinical responses in treating various blood cancers which recently led to the first FDA-approved chimeric antigen receptor T cell (CAR T) therapy for patients up to 25 years of age with relapsed or refractory acute lymphoblastic leukemia (ALL).

CAR T therapy has thus far demonstrated limited success in the solid tumor setting. This is attributed to various immune cell intrinsic features, such as T cell exhaustion, as well as active cancer immunosuppressive mechanisms, hostile tumor microenvironment, presence of various immunosuppressive cells *(e.g.,* regulatory T cells (T_{REGS}), myeloid-derived suppressor cells (MDSCs)) and apparent lack of unique surface antigens. (Johnson, L. A. & June, C. H. Driving gene-engineered T cell immunotherapy of cancer. Cell Res. 27, 38-58 (2016)). US20 18/044424 A1 relates to compositions and methods for treating cancer by using immune effector cells, engineered to conditionally express an agent which enhances the immune effector response of an immune effect or cell that expresses a Chimeric Antigen Receptor.

T cell-intrinsic dysfunctions limit clinical efficacy of CAR T cells. Specific genetic programs are master regulators of T cell differentiation, expansion, fitness, and anti-tumor responses. These functional features have emerged as indispensable factors in successful CAR T immunotherapy. There remains an unmet need for the development of strategies to outfit T cells with critical quality attributes required for effective therapy.

### SUMMARY

The present invention is defined by the appended claims. In particular, an engineered cell is provided, the engineered cell comprising (a) a nucleic acid encoding a receptor capable of receiving an activating signal and (b) an inducible promoter operably linked to a nucleic acid encoding FOXO1-3A or TCF7, wherein the inducible promoter is induced when the receptor receives an activating signal.

The present invention provides a method of programming differentiation status and/or increasing proliferation of a cell comprising a receptor capable of receiving an activating signal, the method comprising genetically modifying the cell to operably link an inducible promoter to a nucleic acid encoding FOXO1-3A or TCF7, wherein the inducible promoter is induced when the receptor receives an activating signal.

The present invention provides a method of inducing therapeutically relevant cell states associated with improved anti-tumor activity, efficacy, and functionality in a cell comprising a receptor capable of receiving an activating signal is provided, the method comprising genetically modifying the cell to operably link an inducible promoter to a nucleic acid encoding FOXO1-3A or TCF7, wherein the inducible promoter is induced when the receptor receives an activating signal.

The present invention provides a method of generating a cell for use in adoptive cell therapy, wherein the cell comprises a receptor capable of receiving an activating signal, the method comprising genetically modifying the cell to operably link an inducible promoter to a nucleic acid encoding FOXO1-3A or TCF7, wherein the inducible promoter is induced when the receptor receives an activating signal.

The present invention provides a cell comprising a receptor capable of receiving an activating signal for use in a method of treating cancer in a subject in need thereof , wherein the cell comprises an inducible promoter operably linked to a nucleic acid encoding FOXO1-3A or TCF7, and wherein the inducible promoter is induced when the receptor receives an activating signal. In some embodiments, the receptor specifically binds a tumor antigen.

In some embodiments , the cell is a T cell, NK cell, macrophage, or a regulatory T cell. In some embodiments, the receptor is a chimeric antigen receptor (CAR) or a T cell receptor (TCR). In some other embodiments, the CAR comprises an antigen binding domain, a transmembrane domain, and an intracellular signaling domain. In some embodiments, the antigen binding domain specifically binds a tumor antigen. In some other embodiments, the intracellular signaling domain comprises an intracellular domain of a costimulatory molecule selected from: CD27, CD28, 4-1BB(CD137), OX40, CD30, CD40, PD-1, ICOS, lymphocyte function-associated antigen-1 (LFA-1), CD2, CD7, LIGHT, NKG2C, B7-H3, and a ligand that specifically binds with CD83. In some embodiments, the TCR comprises TCR alpha and beta chains, or TCR gamma and delta chains. In some embodiments, the inducible promoter is an NFAT promoter, a synthetic NFAT promoter, a NF-kB promoter, a CD69 promoter, a CD137 promoter, synthetic hypoxia-responsive element (HRE) promoter, or a PD-1 promoter. In some embodiments, the nucleic acid encoding FOXO1-3A encodes the amino acid sequence set forth in SEQ ID NO: 31. In some embodiments, the nucleic acid encoding TCF7 encodes the amino acid sequence set forth in SEQ ID NO: 30.

Also disclosed herein but not part of the invention, is a method of screening and identifying a receptor that specifically binds an antigen, the method comprising
(a) contacting a cell with an antigen, wherein the cell comprises a single viral vector comprising a first polynucleotide comprising a constitutive promoter operably linked to a nucleic acid encoding a candidate receptor or subunit thereof; and a second polynucleotide comprising an inducible promoter operably linked to a nucleic acid encoding a reporter protein; and
(b) determining the level of expression of the reporter protein in the cell;
wherein the candidate receptor is identified as a receptor that specifically binds the antigen if the level of expression of the reporter protein is increased relative to a reference level.

Also disclosed herein, but not part of the invention, is a method of screening and identifying an antigen that specifically binds a receptor, the method comprising
(a) contacting a cell with a candidate antigen, wherein the cell comprises a single viral vector comprising a first polynucleotide comprising a constitutive promoter operably linked to a nucleic acid encoding a receptor or subunit thereof; and a second polynucleotide comprising an inducible promoter operably linked to a nucleic acid encoding a reporter protein; and
(b) determining the level of expression of the reporter protein in the cell;
wherein the candidate antigen is identified as an antigen that specifically binds the receptor if the level of expression of the reporter protein is increased relative to a reference level.

Also disclosed herein, but not part of the invention, is a method of screening and identifying an antigen that specifically binds a receptor, the method comprising
(a) contacting a cell with a candidate antigen, wherein the cell comprises a receptor capable of receiving an activating signal, and wherein the cell comprises a single viral vector comprising a first polynucleotide comprising a constitutive promoter operably linked to a nucleic acid encoding a first reporter protein; and a second polynucleotide comprising an inducible promoter operably linked to a nucleic acid encoding a second reporter protein; and
(b) determining the level of expression of the second reporter protein in the cell; wherein the candidate antigen is identified as an antigen that specifically binds the receptor if the level of expression of the second reporter protein is increased relative to a reference level.

Also disclosed herein, but not part of the invention, is a method of identifying a receptor that specifically binds an antigen, the method comprising
(a) contacting a cell with an antigen, wherein the cell comprises a candidate receptor capable of receiving an activating signal, and wherein the cell comprises a single viral vector comprising a first polynucleotide comprising a constitutive promoter operably linked to a nucleic acid encoding a first reporter protein; and a second polynucleotide comprising an inducible promoter operably linked to a nucleic acid encoding a second reporter protein; and
(b) determining the level of expression of the second reporter protein in the cell;
wherein the candidate receptor is identified as a receptor that specifically binds the antigen if the level of expression of the second reporter protein is increased relative to a reference level.

The receptor may a T cell receptor (TCR) or a chimeric antigen receptor (CAR). The first and/or second reporter protein may be a fluorescent protein. The inducible promoter may be capable of driving expression of the second reporter protein when the cell is activated. The method further may comprise characterizing the specificity and/or functionality such as functional avidity of the receptor. The cell may be a T cell.

### BRIEF DESCRIPTION OF THE DRAWINGS

The following detailed description of preferred embodiments of the invention will be better understood when read in conjunction with the appended drawings. For the purpose of illustrating the invention, there are shown in the drawings embodiments which are presently preferred. It should be understood, however, that the invention is not limited to the precise arrangements and instrumentalities of the embodiments shown in the drawings.

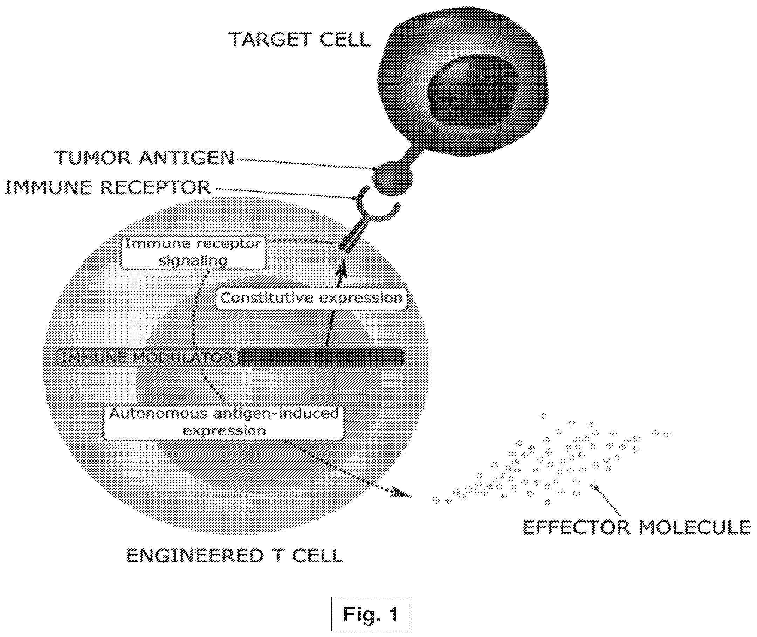

Figure 1 illustrates the concept of an autonomous antigen-induced immune modulator and a constitutive immune receptor expression in a single vector. The immune receptor (CAR or TCR) is expressed constitutively. Ligation with its cognate ligand rewires endogenous NFAT signaling to the expression of an immune modulator molecule from the same lentiviral construct which is integrated as a single module.

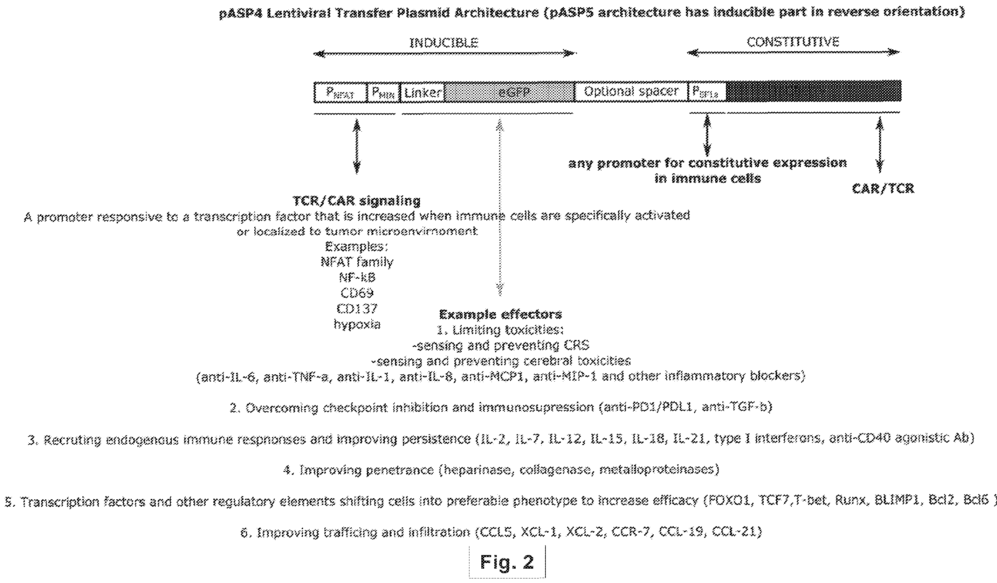

Figure 2 illustrates the design of a lentiviral transfer plasmid construct combining a constitutive portion and an inducible portion in a single lentiviral vector. The constitutive portion enables strong EF-1α promoter (or any other constitutive promoter that enables strong expression in immune cells)-driven expression of a reporter gene mCherry, which may be exchanged for a CAR or TCR of choice in an engineered immune cell. The inducible portion of the construct enables autonomous response to CAR/TCR engagement by a specific antigen or other tumor microenvironment characteristic signaling in engineered immune cells. This was achieved by rewiring endogenous CAR/TCR or other tumor microenvironment characteristic signaling to the expression of the effectors through activation of introduced promoters responsive to transcriptional factor that is increased when immune cells are specifically activated or localized to a given microenvironment, e.g., a tumor microenvironment, including a synthetic NFAT promoter, NF-kB promoter, a CD69 promoter, a CD137 promoter or a synthetic hypoxia-responsive element (HRE) promoter. The inducible portion drives expression of reporter gene eGFP, which may be exchanged by the effector of choice to increase safety and efficacy of immunotherapy.

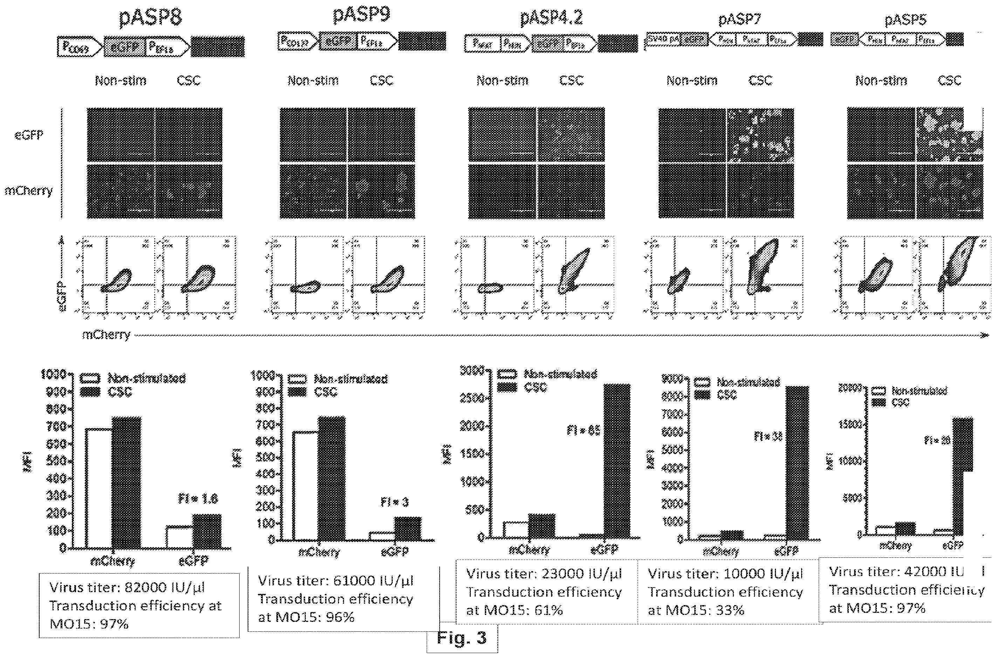

Figure 3 illustrates autonomous NFAT-driven expression of eGFP and constitutive expression of mCherry from a single lentiviral vector in a Jurkat cell line. Jurkat cells were transduced with different lentiviruses (pASP8, pASP9, pASP4.2, pASP7 and pASP5) at a multiplicity of infection (MOI) of 5 (MOI5). 48 h after transduction cells were stimulated with a cell stimulation cocktail (ionomycin and phorbol myristate acetate) and expression of reporter genes was monitored by confocal fluorescence microscopy and flow cytometry 76 h after transduction (24 h after stimulation).

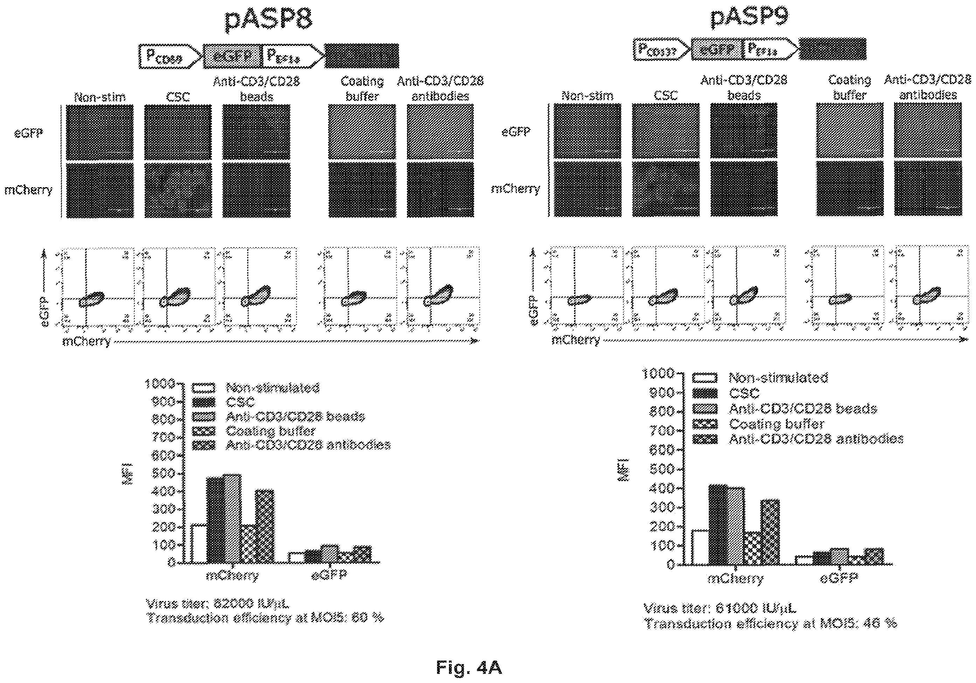

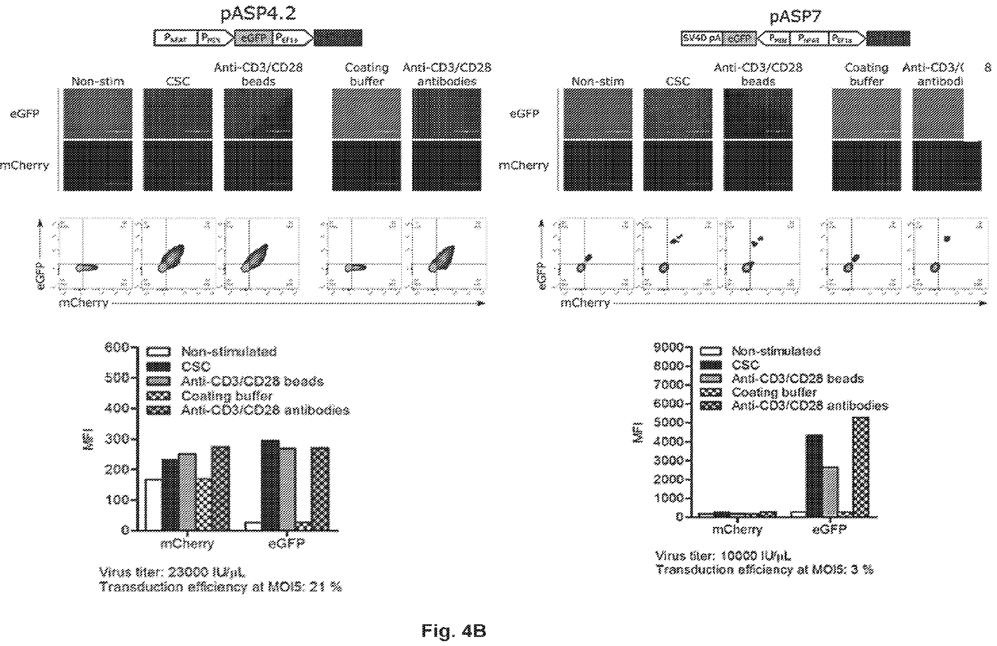

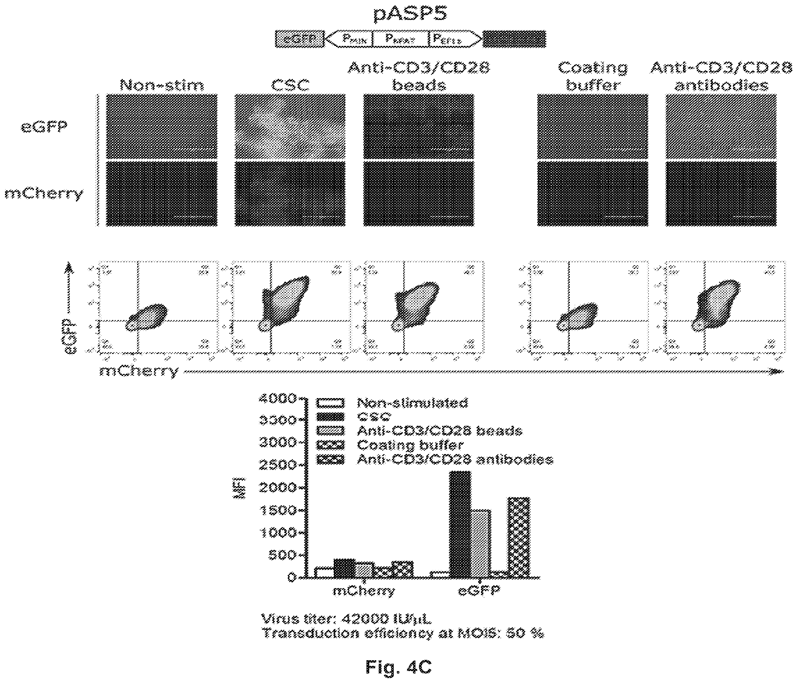

Figures 4A-4C illustrate autonomous NFAT-driven expression of eGFP and constitutive expression of mCherry from a single lentiviral vector in primary human T cells. Primary donor-derived T cells were transduced with different lentiviruses. Figure 4A illustrates that primary donor-derived T cells were transduced with pASP8 or pASP9. After transduction at a multiplicity of infection (MOI) of 5, the cells were expanded according to the standard protocol using anti-CD3/CD28 beads. At day 14 when cells were rested and hence NFAT-signaling was no longer active, the cells were again stimulated with CSC (ionomycin and phorbol myristate acetate), anti-CD3/CD28 beads or anti-CD3/CD28 antibody-coated plates and expression of reporter genes was monitored by confocal fluorescence microscopy and flow cytometry after 24 h. Figure 4B illustrates that primary donor-derived T cells were transduced with pASP4.2 or pASP7. After transduction at a multiplicity of infection (MOI) of 5, the cells were expanded according to the standard protocol using anti-CD3/CD28 beads. At day 14 when cells were rested and hence NFAT-signaling was no longer active, the cells were again stimulated with CSC (ionomycin and phorbol myristate acetate), anti-CD3/CD28 beads or anti-CD3/CD28 antibody-coated plates and expression of reporter genes was monitored by confocal fluorescence microscopy and flow cytometry after 24 h. Figure 4C illustrates that primary donor-derived T cells were transduced with pASP5. After transduction at a multiplicity of infection (MOI) of 5, the cells were expanded according to the standard protocol using anti-CD3/CD28 beads. At day 14 when cells were rested and hence NFAT-signaling was no longer active, the cells were again stimulated with CSC (ionomycin and phorbol myristate acetate), anti-CD3/CD28 beads or anti-CD3/CD28 antibody-coated plates and expression of reporter genes was monitored by confocal fluorescence microscopy and flow cytometry after 24 h.

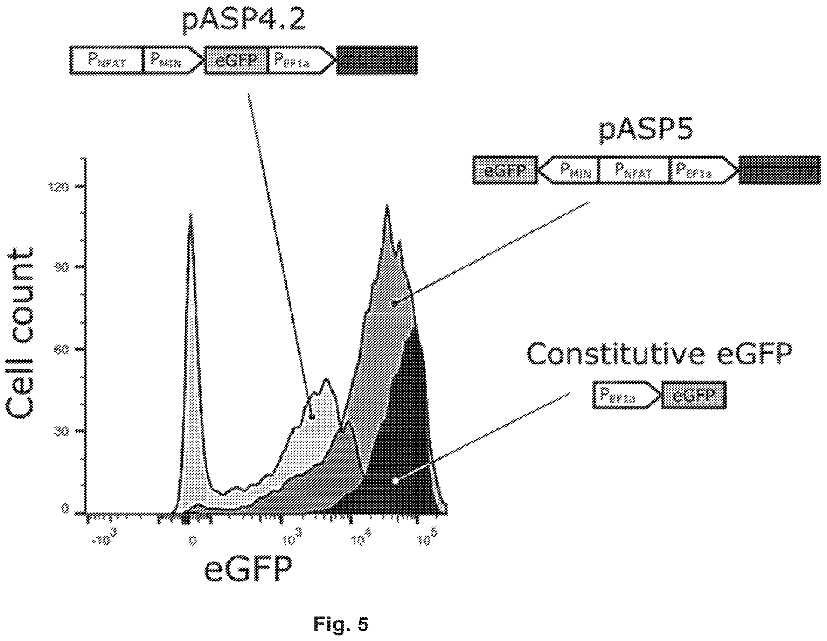

Figure 5 illustrates the capacity of the activated system. The capacity of the inducible activated pASP5 architecture is similar to that of a constitutively expressed system, whereas expression from the pASP4.2 architecture enables lower expression levels. Cells were activated with CSC (ionomycin and phorbol myristate acetate).

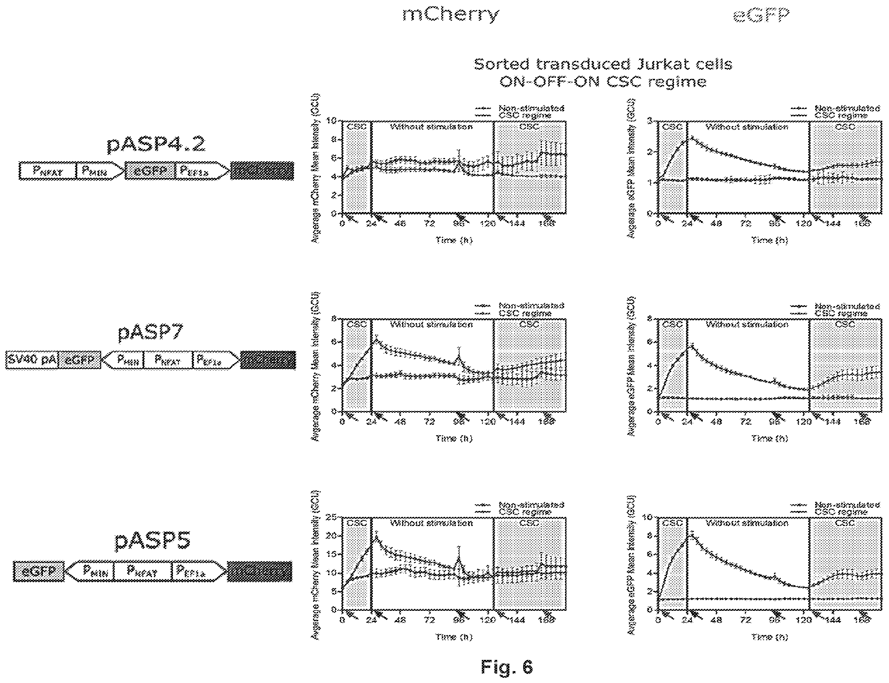

Figure 6 illustrates the reversibility of the system. Transduced Jurkat cells were plated on 96-well plates and monitored in real-time for inducible eGFP and constitutive mCherry expression using the IncuCyte^{®} system. Cells were stimulated with CSC (ionomycin and phorbol myristate acetate) for 24 h to monitor the increase of inducible eGFP expression. After 24 h cells were washed to remove the stimulus and left unstimulated for 120 h, when a drop in eGFP signal was observed. After 120 h they were re-stimulated to show an inducible eGFP response to the second round of stimulation.

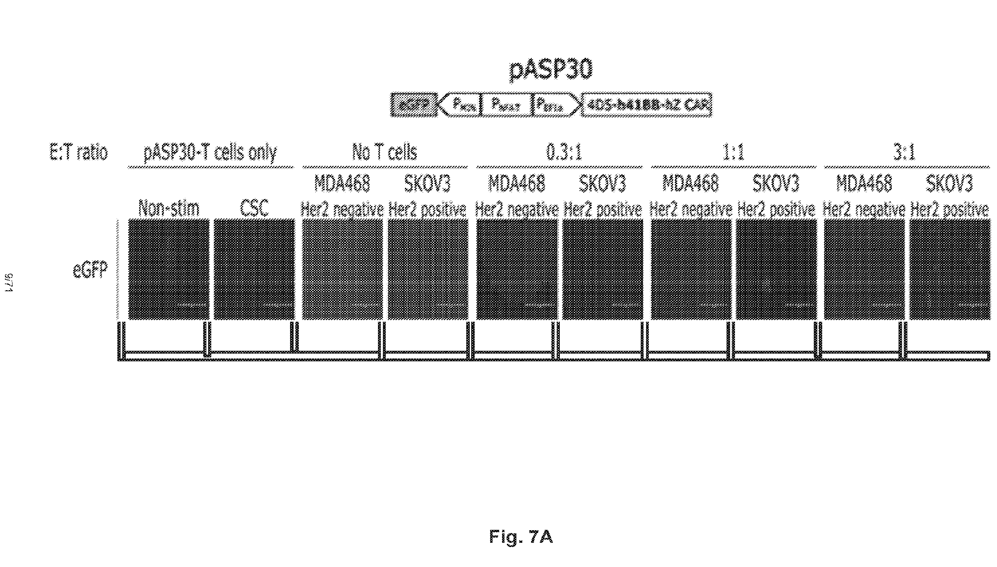

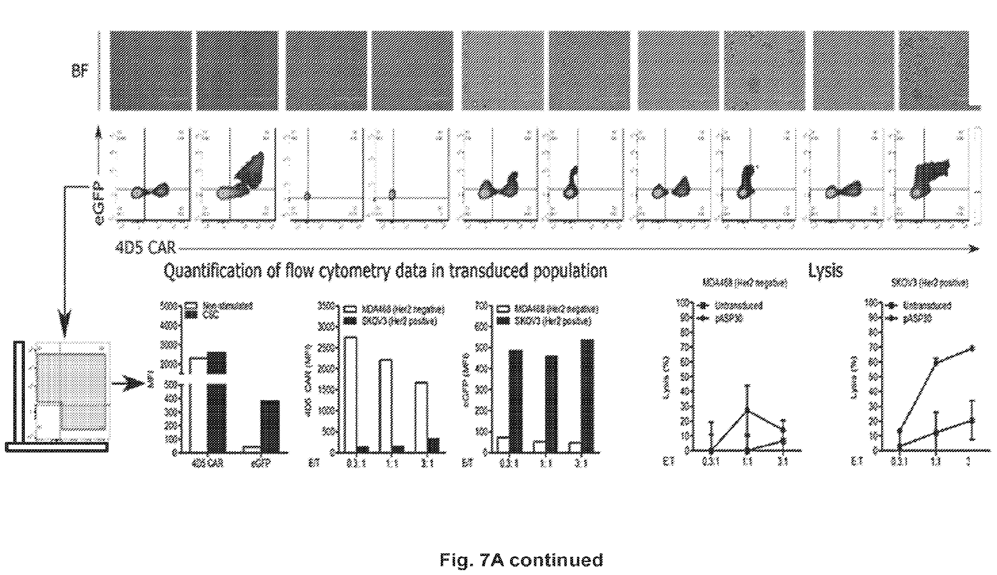

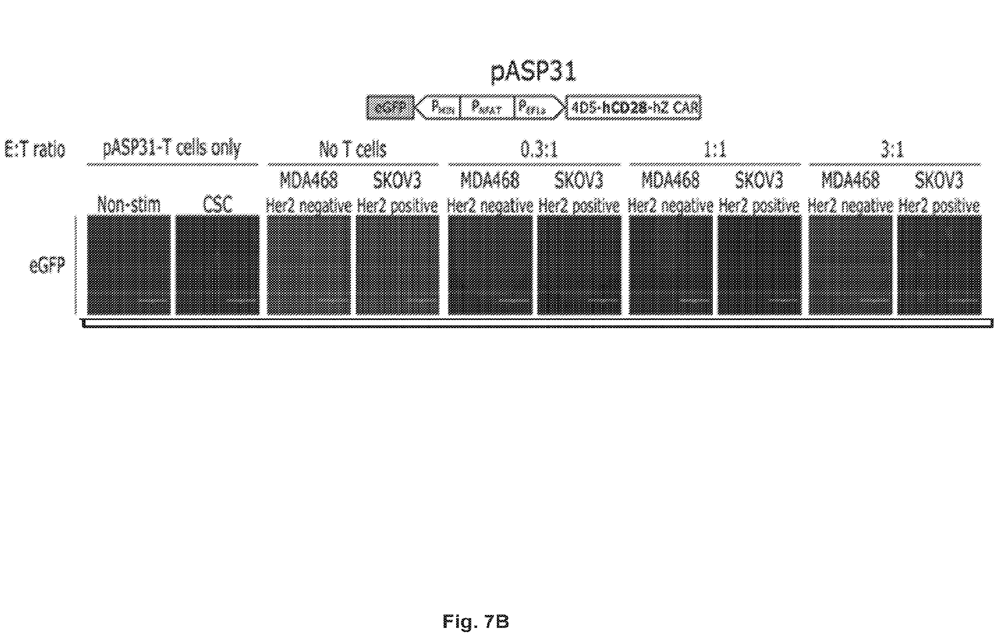

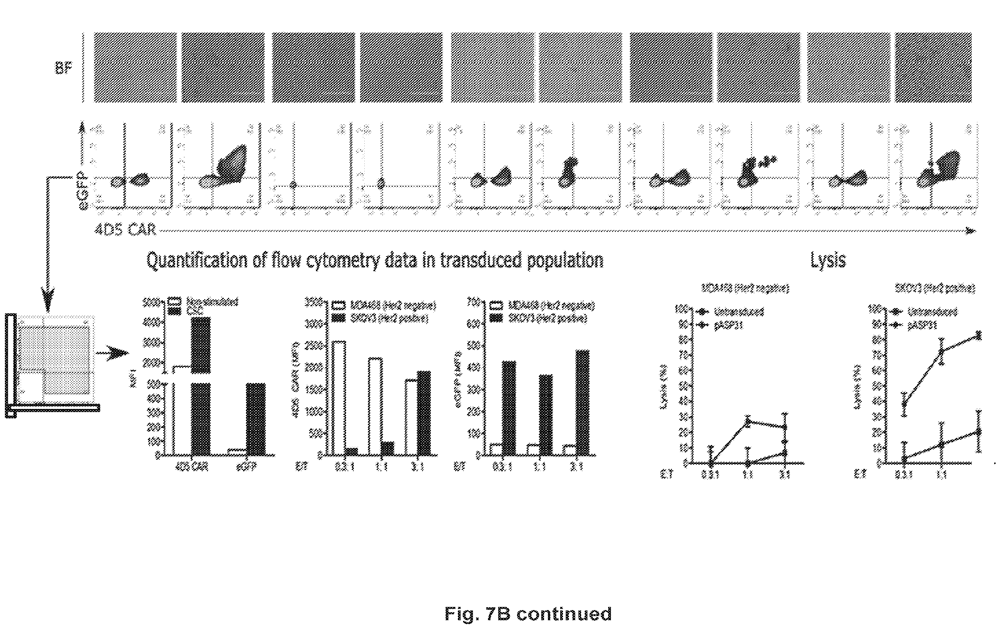

Figures 7A-7B illustrate that designer T cells respond specifically to an antigen-positive adherent cancer cell line. Figure 7A: Primary T cells were activated, transduced with pASP30 (inducible part placed in reverse in combination with a Her2-specific CAR comprising a 4D5 anti-Her2 scFv, a 4-1BB costimulatory domain, and CD3-zeta), expanded and rested according to the standard protocol and then co-cultured with the SKOV3 cancer cell line that expresses high levels of Her2 at various effector to target (E:T) ratios. As a negative control, an MDA468 cell line that does not express significant levels of Her2 was used. As a positive control, cells were non-specifically activated with CSC (ionomycin and phorbol myristate acetate). System performance was monitored using fluorescence microscopy and flow cytometry while killing capacity was determined by a standard luciferase based assay. Figure 7B: Primary T cells were activated, transduced with pASP31 (inducible part placed in reverse in combination with 4D5 anti-Her2 scFv in combination with a CD28 costimulatory domain and CD3-zeta), expanded and rested according to the standard protocol and then co-cultured with the SKOV3 cancer cell line that expresses high levels of Her2 at various effector to target (E:T) ratios. As a negative control an MDA468 cell line that does not express significant levels of Her2 was used. As a positive control, cells were non-specifically activated with CSC (ionomycin and phorbol myristate acetate). System performance was monitored using fluorescence microscopy and flow cytometry while killing capacity was determined by a standard luciferase-based assay.

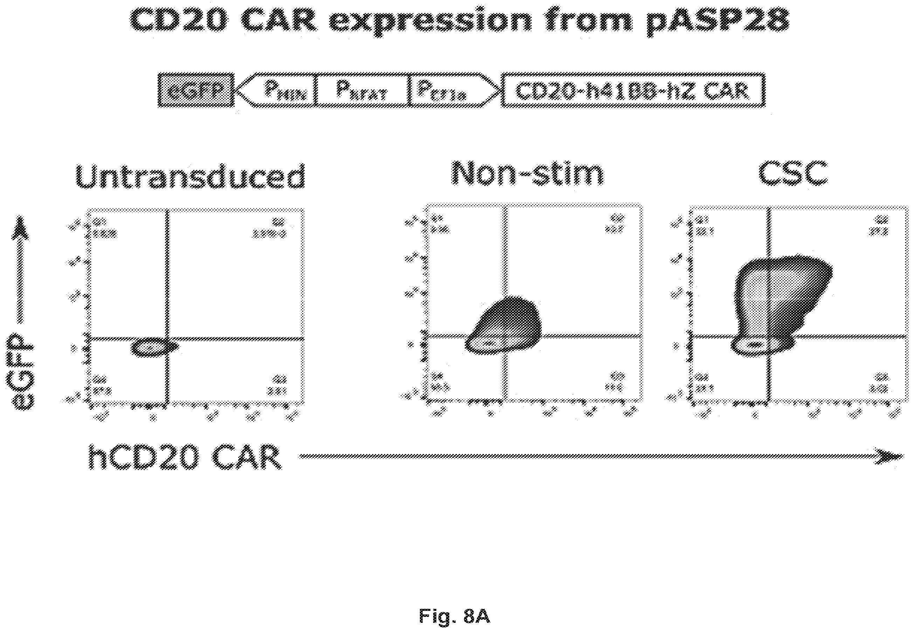

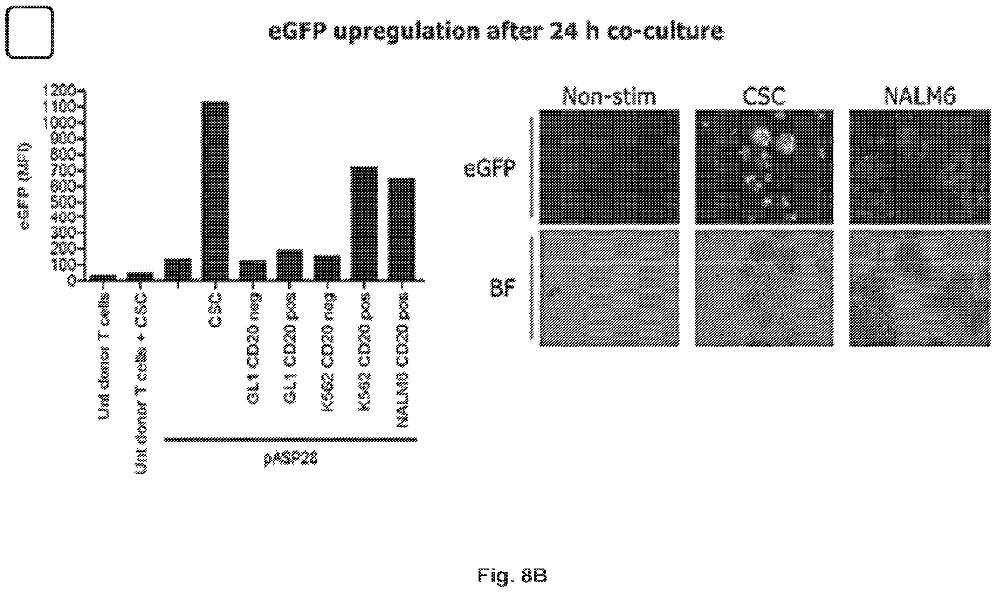

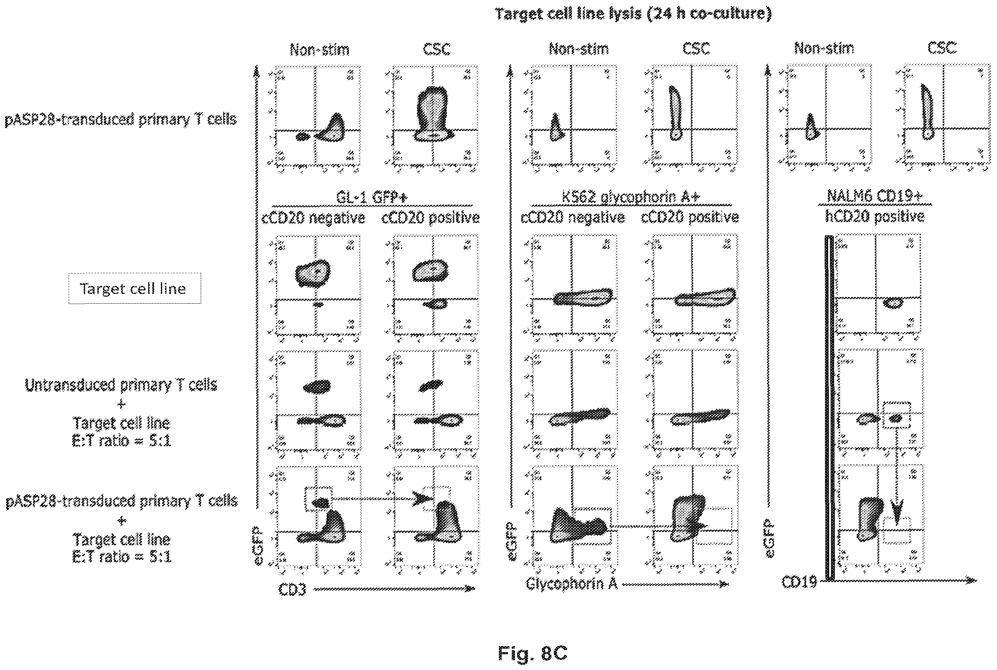

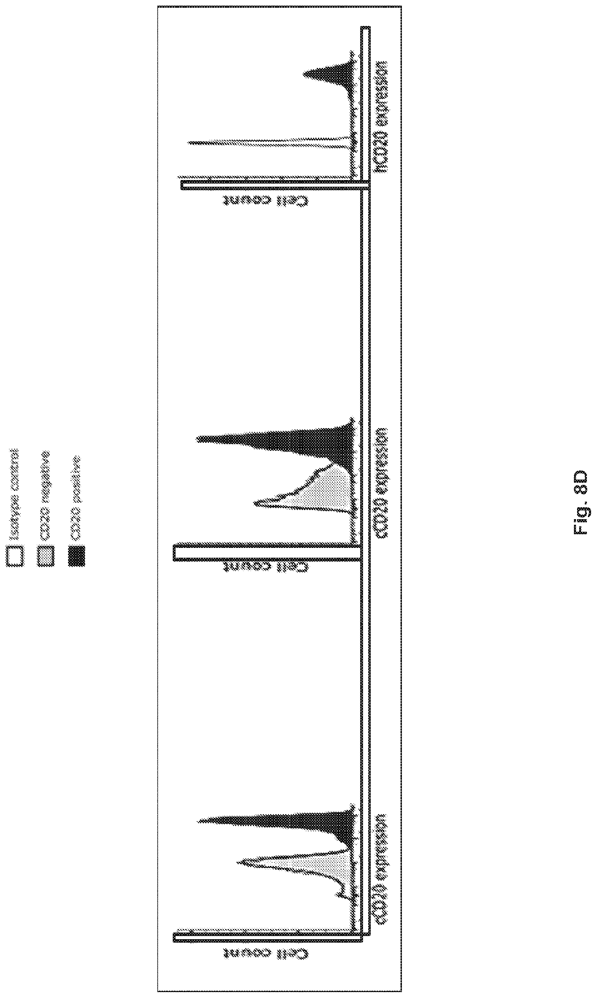

Figures 8A-8D illustrate that designer T cells respond specifically to an antigen-positive suspension cancer cell line. Primary human T cells were activated, transduced with pASP28 (NFAT-inducible eGFP in combination with constitutive anti-CD20 scFv+4-1BB+CD3-zeta CAR), expanded and rested according to the standard protocol. Figure 8A: Anti-CD20 CAR expression and CSC (ionomycin and phorbol myristate acetate) inducible eGFP expression was determined by flow cytometry. Figure 8B: Engineered T cells were co-cultured with CD20 positive or negative cell lines at E:T ratio 5:1 for 24 hours to show inducible and antigen-dependent expression of eGFP as determined by MFI. Figure 8C: Antigen-dependent cell lysis that led to associated eGFP upregulation was demonstrated by flow cytometry where target and effector cells were differentiated as follows: GL-1 cell line was engineered to express GFP and samples were stained with anti-CD3, K562 cell lines were stained for characteristic Glycophorin A expression and NALM6 cell line was stained for CD19 expression. Figure 8D: CD20 target antigen expression.

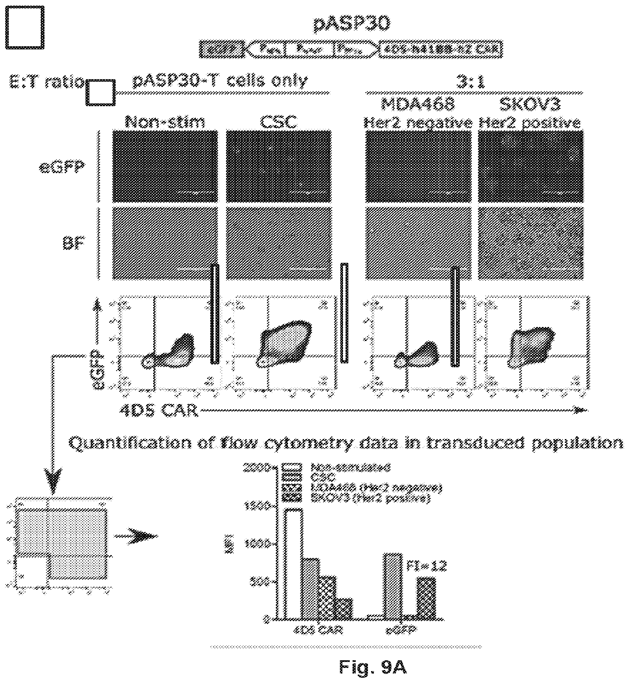

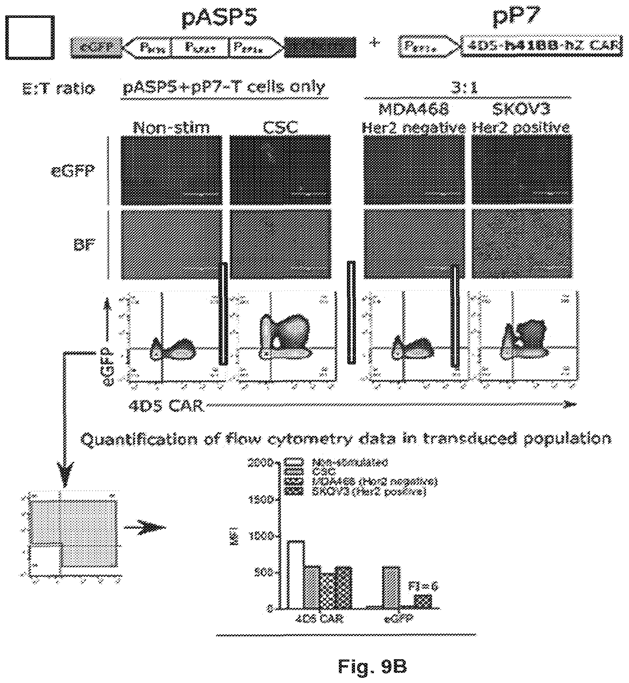

Figures 9A-9B illustrate that the all-in-one lentiviral system has superior functionalities compared to a two-component lentiviral system. Figure 9A: Primary T cells were activated, transduced at MOI3 with pASP30 (NFAT-inducible eGFP in combination with constitutive 4D5 anti-Her2 scFv 4-1BB/CD3-zeta CAR in the same lentiviral construct) in a combination with pP7 (4D5 anti-Her2 scFv 4-1BB/CD3-zeta CAR same as used in pASP30 construct), expanded and rested according to the standard protocol. Cells were then non-specifically activated with CSC (ionomycin and phorbol myristate acetate) or co-cultured with SKOV3 cancer cell line that expresses high levels of Her2 at 3:1 effector to target (E:T) ratio. As a control, the MDA468 cell line that does not express significant levels of Her2 was used. System performance was monitored using fluorescence microscopy and flow cytometry. Figure 9B: Primary T cells were activated, transduced at MOI3 with pASP5 (same construct as pASP30 but with CAR exchanged by mCherry) in a combination with pP7 (4D5 anti-Her2 scFv 4-1BB CAR same as used in pASP30 construct), expanded and rested according to the standard protocol. Cells were then non-specifically activated with CSC (cell stimulation cocktail; ionomycin and phorbol myristate acetate) or co-cultured with SKOV3 cancer cell line that expresses high levels of Her2 at 3:1 effector to target (E:T) ratio. As a control, the MDA468 cell line that does not express significant levels of Her2 was used. System performance was monitored using fluorescence microscopy and flow cytometry.

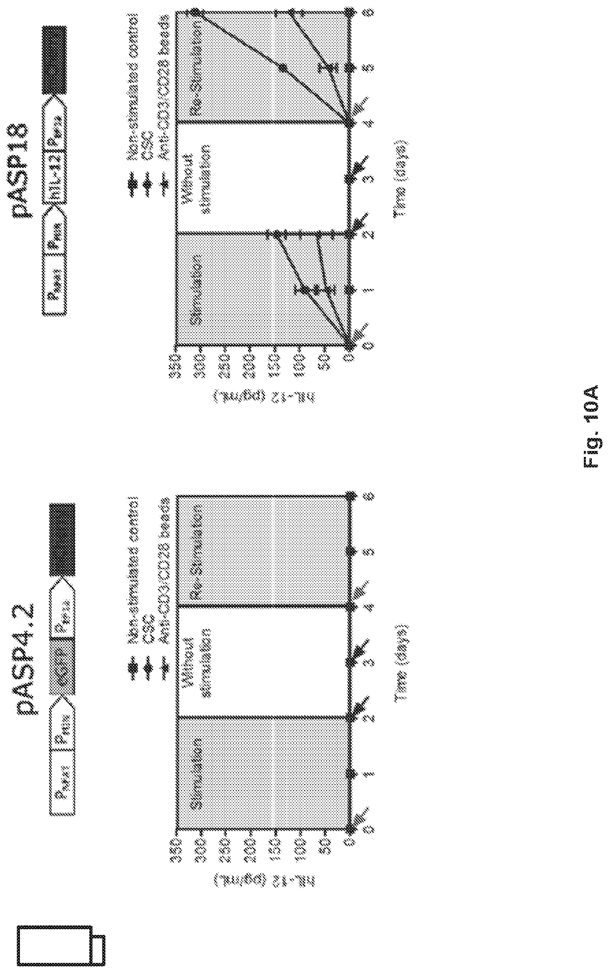

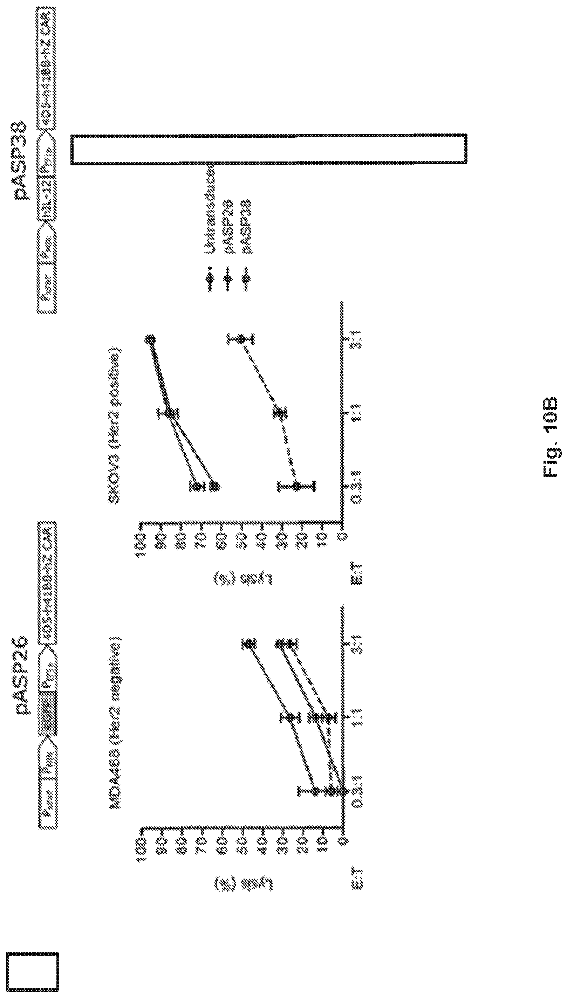

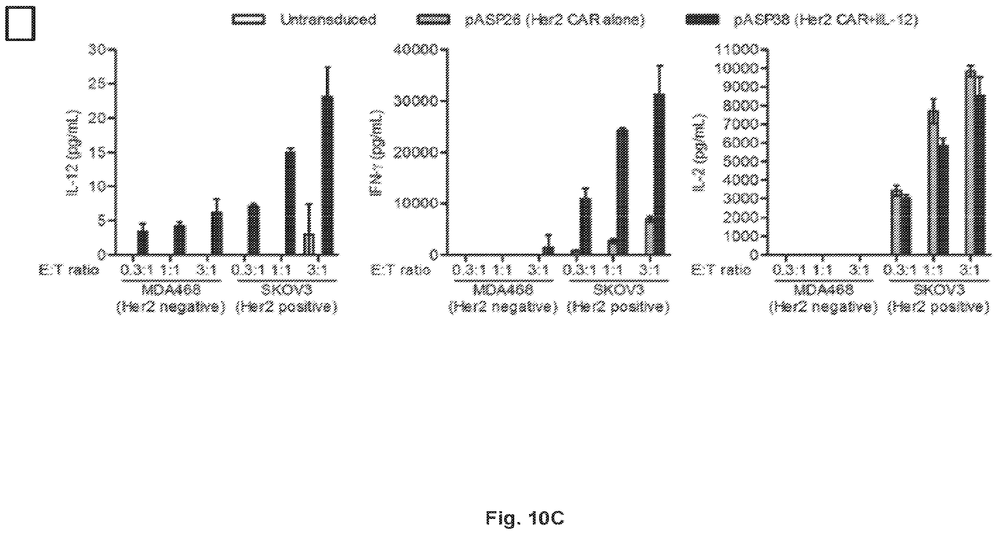

Figures 10A-10C illustrate on demand and reversible expression of human IL-12 (hIL-12) to increase efficacy of engineered primary T cells. Figure 10A: Primary human T cells were activated, transduced with pASP18, expanded and rested according to the standard protocol and then subjected to an ON-OFF-ON activation regime to show expression of hIL-12 and reversibility of the system. Green arrows depict stimulation, black arrows depict washing by media exchange. Figure 10B: pASP38 (NFAT-inducible hIL-12 in combination with constitutive 4D5 anti-Her2 scFv 4-1BB CAR)-transduced primary T cells lysed antigen-positive target cells at comparable levels as control pASP26-transduced cells, where hIL-12 was exchanged by eGFP (pASP26). Figure 10C: Lysis was measured after 24 h co-culture and hIL-12 (left), IFN-γ (middle) and IL-2 (right) levels in supernatants from the 24 h co-culture were measured by ELISA.

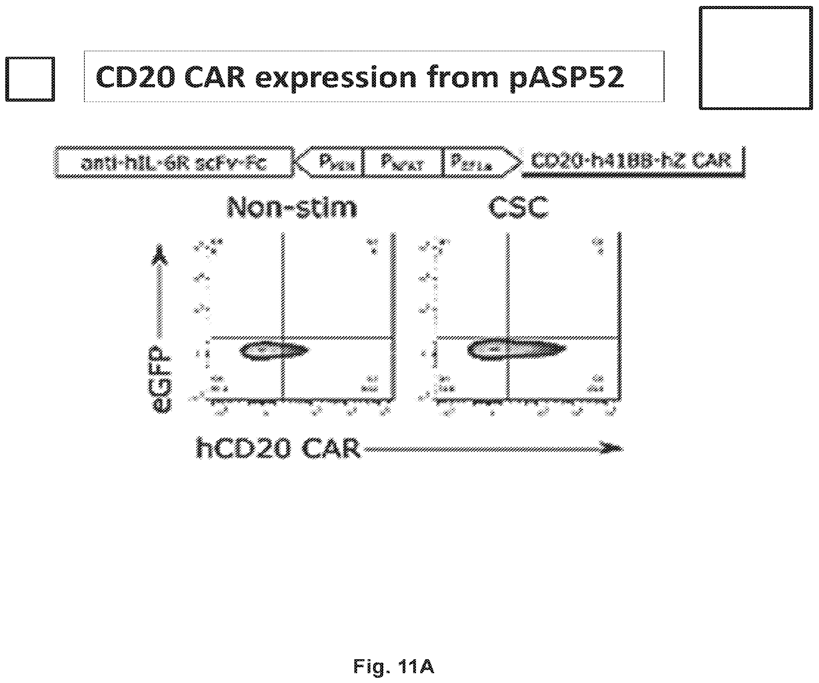

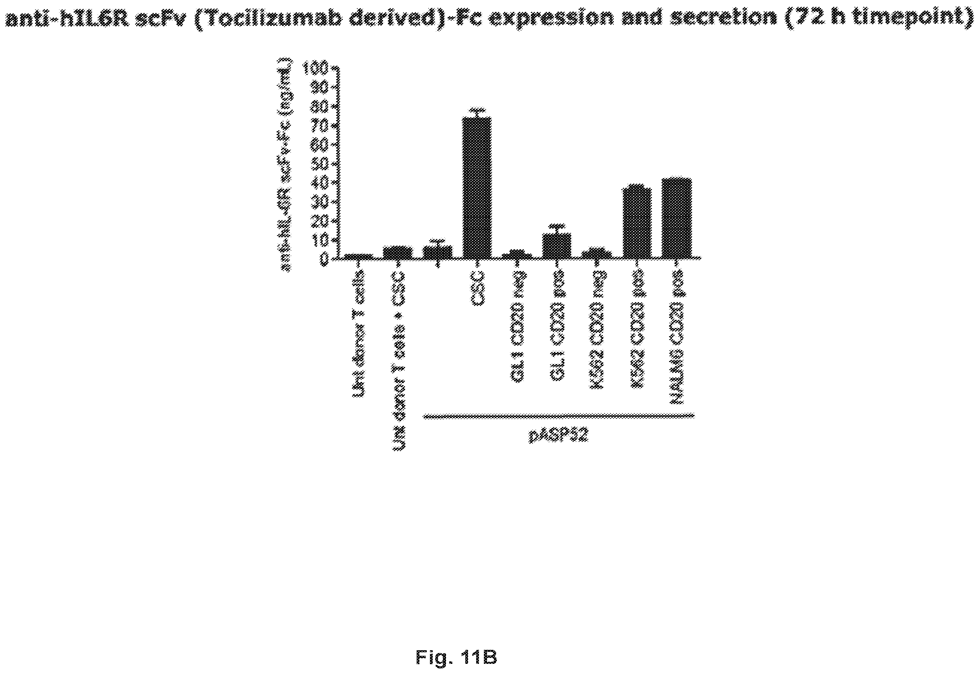

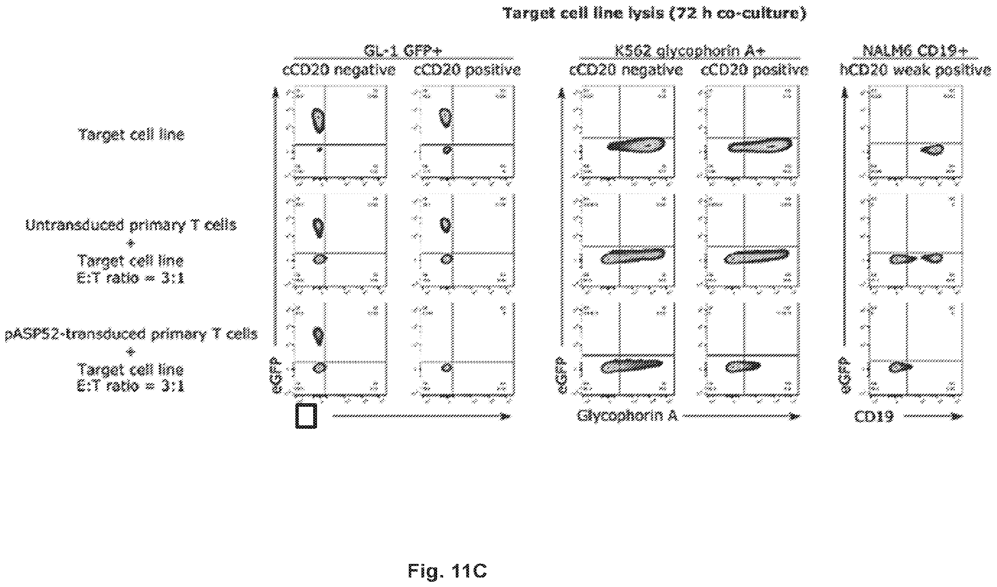

Figures 11A-11C illustrate on demand tailored expression of an IL-6 receptor blocking antibody in primary engineered T cells. Primary human T cells were activated, transduced with pASP52 (NFAT-inducible anti-hIL6R scFV-Fc in combination with constitutive anti-CD20 scFv 4-1BB+CD3-zeta CAR), expanded and rested according to the standard protocol. Figure 11A: Anti-CD20 CAR expression was determined by flow cytometry. Figure 11B: Engineered T cells were co-cultured with CD20 positive or negative cell lines at E:T ratio 3:1 for 72 hours to show inducible and antigen-dependent expression and secretion of hIL-6R blocking antibody as determined by ELISA in the supernatant. Figure 11C: Killing capacity in the same experiment was determined by flow cytometry. As a positive control, cells were non-specifically activated with CSC (ionomycin and phorbol myristate acetate). Target cell lines were differentiated from the effector T cells in the flow panel as follows: the GL-1 cell line was engineered to express GFP, K562 cell lines were stained for characteristic Glycophorin A expression and the NALM6 cell line was stained for CD19 expression.

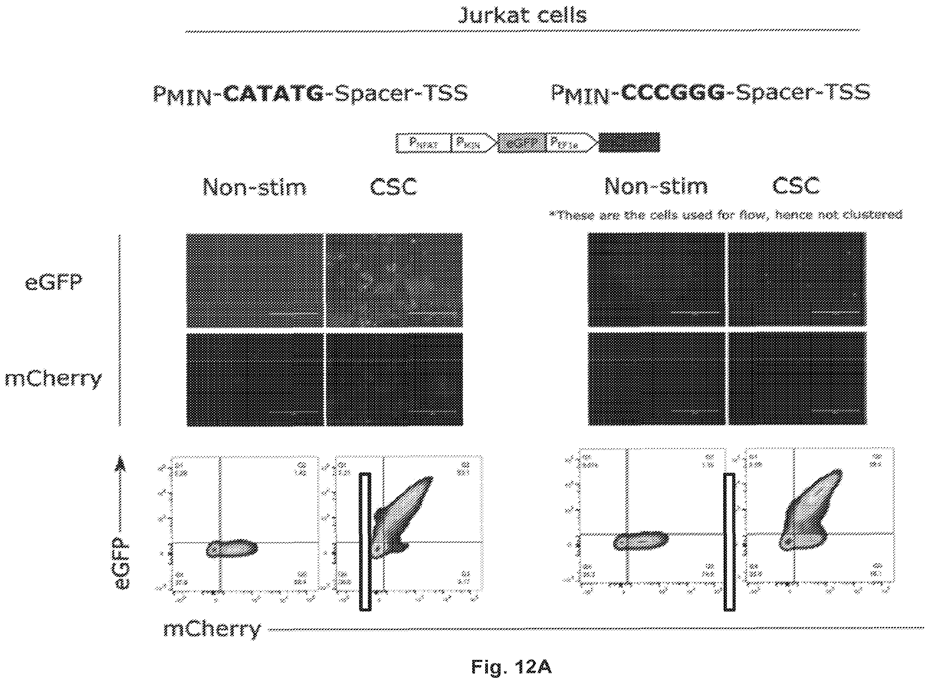

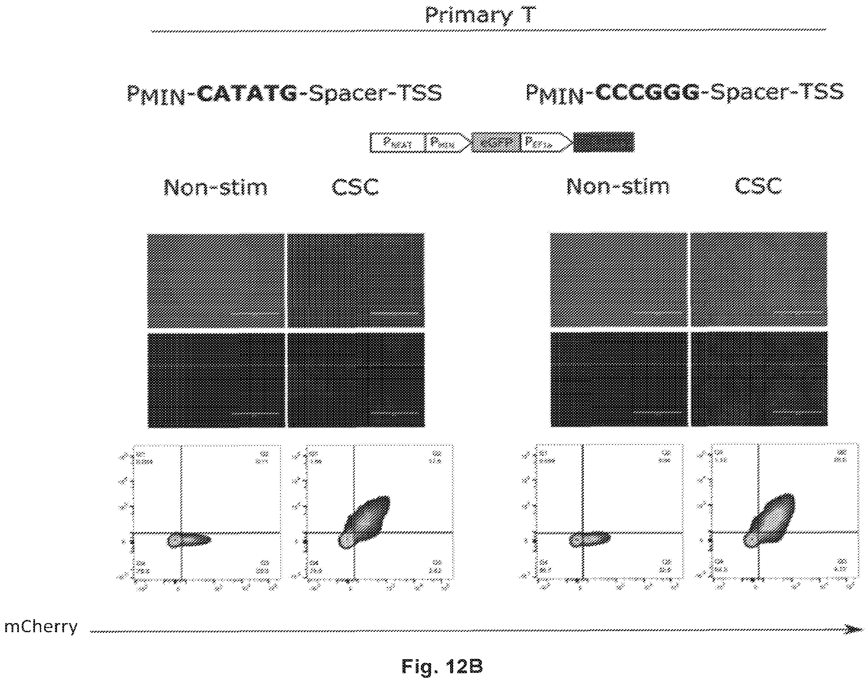

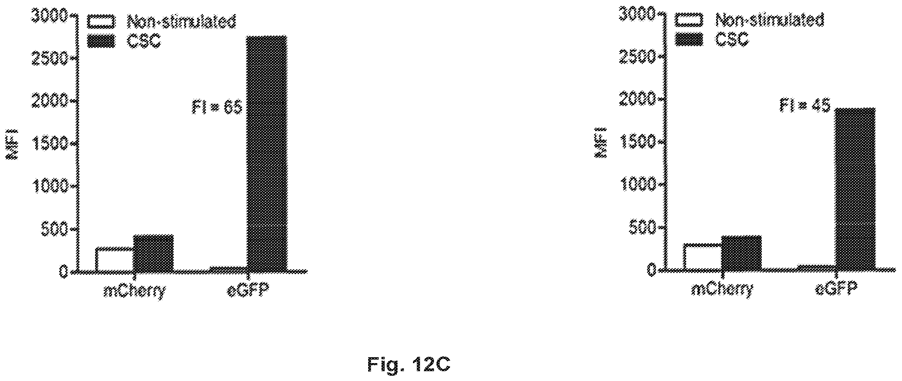

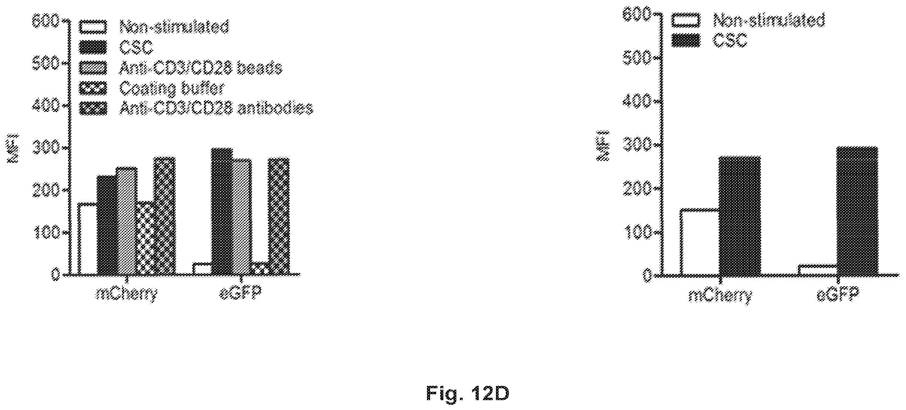

Figures 12A-12D illustrate that the ATG sequence between the minimal promoter and the spacer does not influence systems performance. Jurkat cell line or primary T cells were engineered with two versions of a single-vector system that combines inducible eGFP + constitutive mCherry at MOI5. Vectors differ in the 6 nucleotide sequence between the minimal promoter and the spacer in the inducible module. CATATG 6-nucleotide sequence (SEQ ID NO: 1) and CCCGGG 6-nucleotide sequence (SEQ ID NO: 2) perform similarly in terms transduction efficacy, leakage and maximal activity of the system as determined by flow cytometry and fluorescence microscopy. TSS in the figure means Translational Start Site. Figure 12A: Jurkat cells were transduced with two different lentiviruses described above (Vectors differ in the 6 nucleotide sequence between the minimal promoter and the spacer in the inducible module. Left: CATATG 6-nucleotide sequence (SEQ ID NO: 1) and Right: CCCGGG 6-nucleotide sequence (SEQ ID NO: 2) at a multiplicity of infection (MOI) of 5 (MOI5). 48 h after transduction cells were stimulated with a cell stimulation cocktail (ionomycin and phorbol myristate acetate) and expression of reporter genes was monitored by confocal fluorescence microscopy and flow cytometry 76 h after transduction (24 h after stimulation). Figure 12B: Primary donor-derived T cells were transduced with two different lentiviruses described above (Vectors differ in the 6 nucleotide sequence between the minimal promoter and the spacer in the inducible module. Left: CATATG 6-nucleotide sequence (SEQ ID NO: 1) and Right: CCCGGG 6-nucleotide sequence (SEQ ID NO: 2)) at a multiplicity of infection (MOI) of 5 (MOI5). After transduction, the cells were expanded according to the standard protocol using anti-CD3/CD28 beads. At day 14 when cells were rested and hence NFAT-signaling was no longer active, the cells were again stimulated with CSC (ionomycin and phorbol myristate acetate) and expression of reporter genes was monitored by confocal fluorescence microscopy and flow cytometry after 24 h. Figure 12C: Median fluorescence intensity of eGFP and CAR expression was measured in transduced population of Jurkat cells (described in 12A). Figure 12D: Median fluorescence intensity of eGFP and CAR expression was measured in transduced population of primary human T cells (described in 12A).

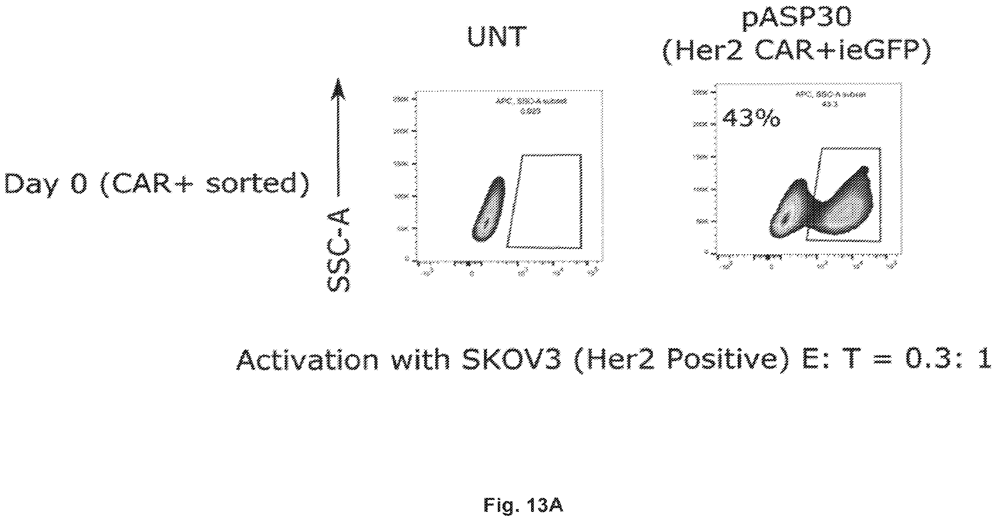

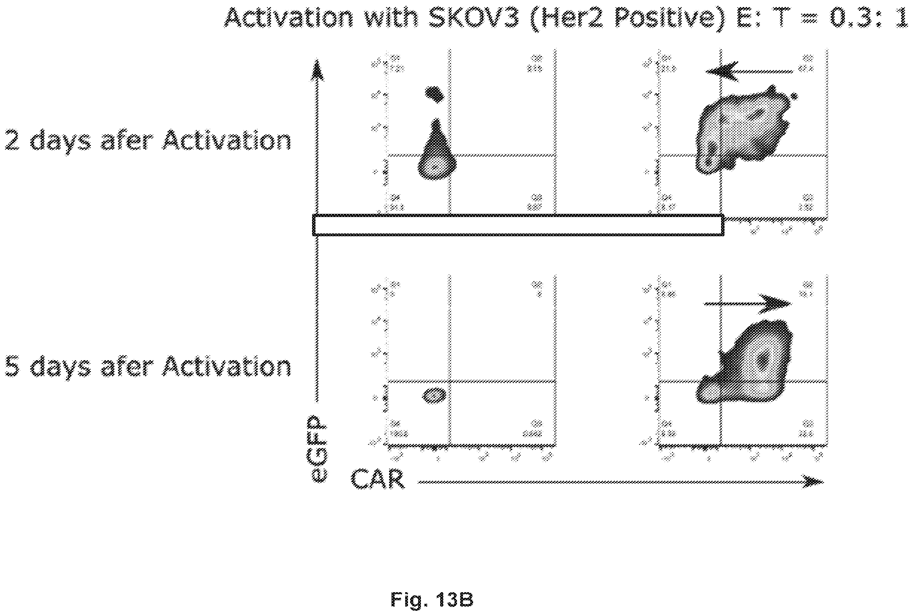

Figures 13A-13B illustrate re-expression of the CAR after its initial target cell-mediated downregulation. Primary T cells were activated, transduced with a single-vector system pASP30 (inducible eGFP + constitutive 4D5 anti-Her2 scFv 4-1BB+CD3-zeta CAR) at MOI5, expanded and rested according to the standard protocol. At the end of the expansion they were sorted for CAR+ cell population (Day 0). Cells were then co-cultured with SKOV3 cancer cell line that expresses high levels of Her2 at 0.3:1 effector to target ratio. Two days later cells were stained for CAR expression and profound downregulation of CAR was observed in cells that recognized target antigen as evidenced by upregulated NFAT inducible eGFP module in this population. Five days after the co-culture was set up, CAR T cells lysed a large majority of SKOV3 target cells which led to the re-expression of CAR. This demonstrated target mediated downregulation and re-expression of CAR on the cell surface.

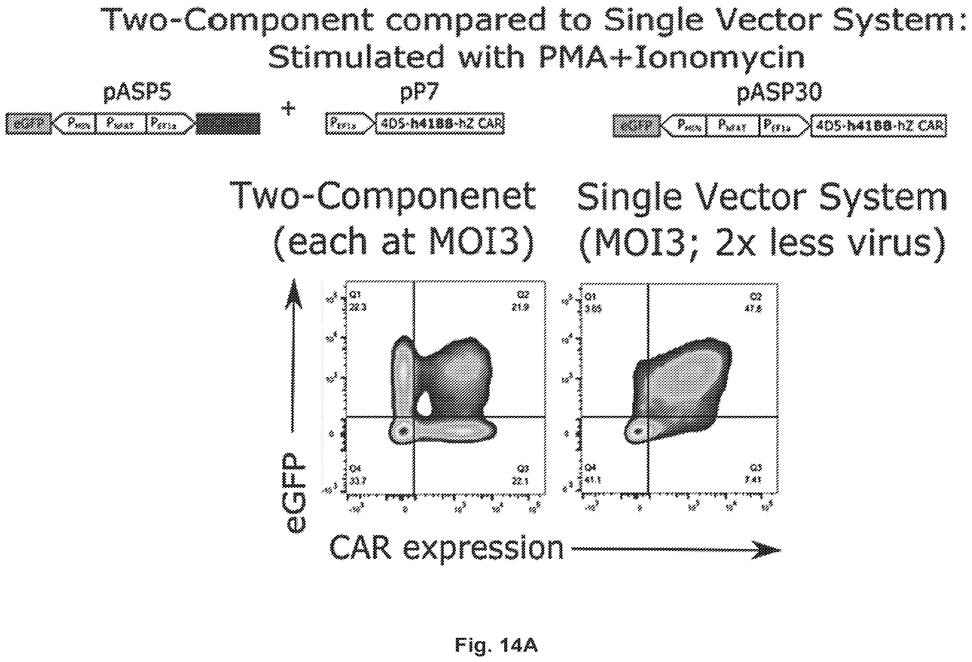

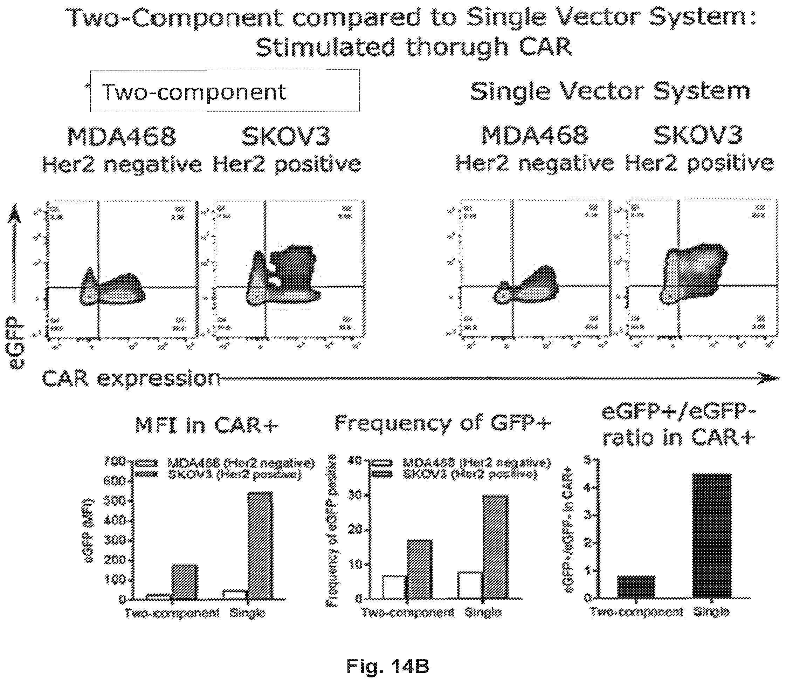

Figures 14A-14B illustrate that the single lentiviral system has superior functionalities compared to a two-component lentiviral system. Figure 14A shows primary T cells were activated, transduced with a combination of two-component pASP5 (inducible eGFP + constitutive mCherry) and pP7 (4D5 anti-Her2 scFv 4-1BB+CD3-zeta CAR) each at MOI3 or with single-vector system pASP30 (inducible eGFP + constitutive 4D5 anti-Her2 scFv 4-1BB+CD3-zeta CAR) alone at MOI3, expanded and rested according to the standard protocol. Cells were then non-specifically activated with ionomycin and phorbol myristate acetate. System performance was monitored using fluorescence microscopy and flow cytometry. As expected, in the two-component system (left) there are 3 populations of transduced CAR T cells (each at approximately 30 % frequency in regards to transduced population): constitutive CAR module positive only, inducible eGFP module positive only (activated because of nonspecific stimulation with PMA and ionomycin) and double positive. Only double positive population is functional and would need to be sorted out before therapeutic implementations. In contrast, the single-vector system (right) confers homogenous population of double positive constitutive CAR and inducible eGFP modules in primary T cells, because both modules are integrated together as a single molecule into the same genomic locus. Figure 14B shows primary T cells were generated as in Figure 14A but activated specifically through co-culture with SKOV3 cancer cell line that expresses high levels of Her2 at 3:1 effector to target (E:T) ratio. The MDA468 cell line that does not express significant levels of Her2 was used as a control. As a consequence of single-construct engineering, in contrast to two-component system, single-vector system provided increased expression of the inducible module in CAR positive population (MFI in eGFP channel, left panel), overall higher eGFP positive frequency of CAR T cells (middle panel) and increased ratio of eGFP positive vs eGFP negative cells in the CAR positive population. Presence of CAR negative and eGFP positive population in the CAR T cells engineered with single-vector system (pASP30) and activated with SKOV3 cells is due to the downregulation of CAR after recognizing target antigen. This phenomena was investigated and demonstrated in Figures 13A-13B (Re-expression of the CAR after its initial target cell-mediated downregulation) in this document.

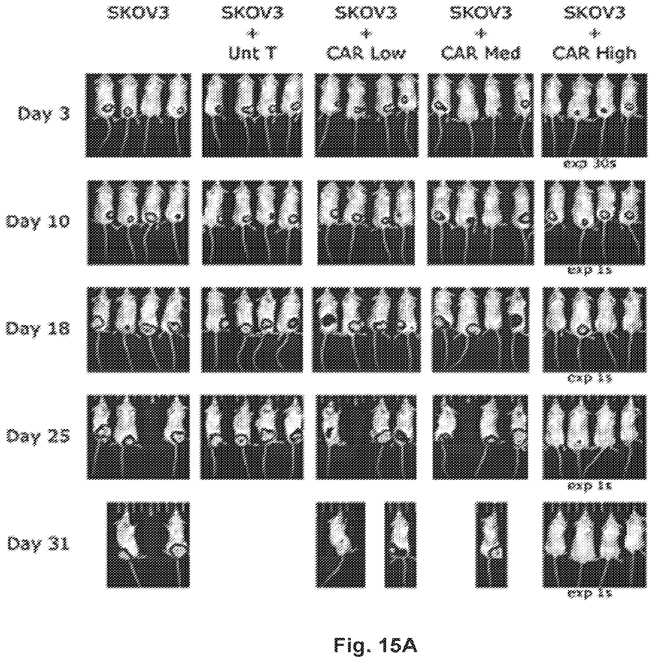

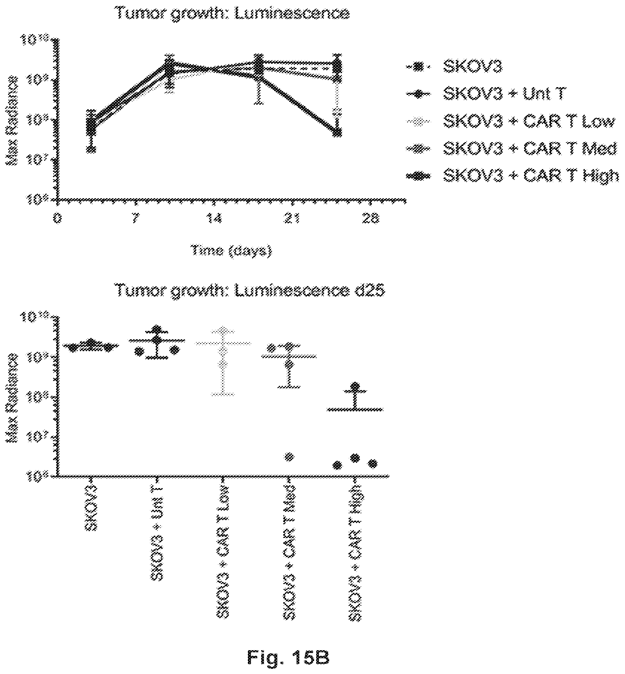

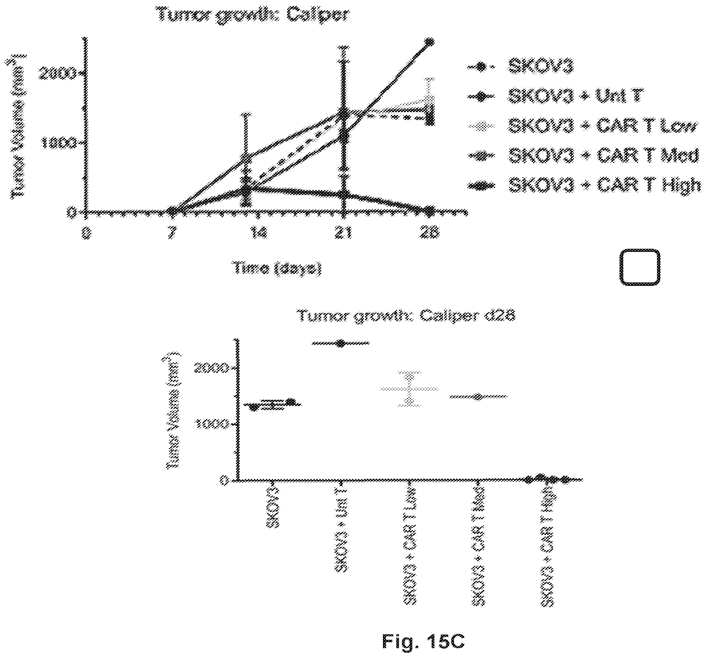

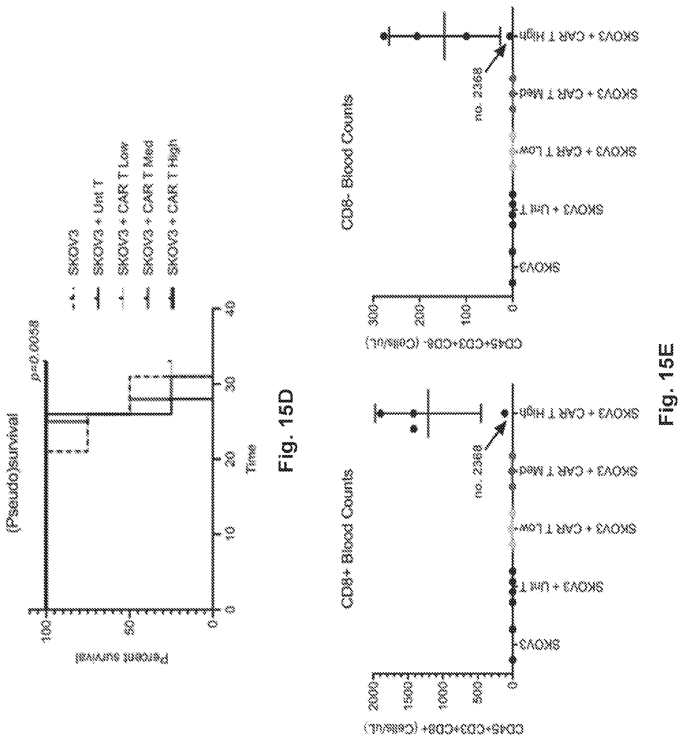

Figures 15A-15E illustrate *in vivo* validation of the single-vector system. Primary T cells were activated, transduced with pASP26 (inducible eGFP + constitutive 4D5 anti-Her2 scFv 4-1BB+CD3-zeta CAR; forward orientation) at MOI5, expanded and rested according to the standard protocol. Engineered primary T cells were injected intravenously (i.v.) at 3 different amounts (0.15/0.3/1 million of CAR+ cells per mouse) into NSG mice bearing subcutaneous (s.c.) xenograft SKOV3 tumors. Figure 15A shows that 1 million of SKOV3 cells (engineered to express luciferase) were injected s.c. 4 days prior to the administration of CAR T cells to establish tumors. 1 million of CAR+ primary T cells completely controlled tumor growth as evidenced by no detectible tumor measured by imaging. Figure 15B shows quantification of luciferase activity (upper panel: average of groups and lower panel: individual mice on day 25), Figure 15C shows caliper measurement of tumor growth (upper panel: average of groups and lower panel: individual mice on day 28). Figure 15D shows survival. Figure 15E shows blood counts of human primary T cells in mice correlated with tumor control where only in highest dose of CAR+ T cells persistence was observed.

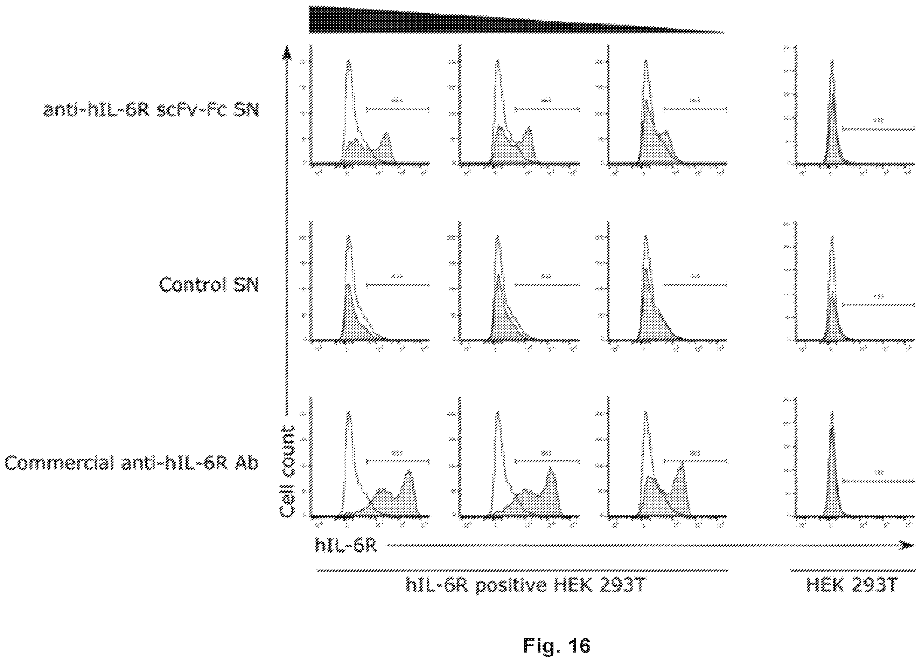

Figure 16 illustrates that engineered anti-hIL-6R scFv-Fc is secreted from activated engineered primary T cells and binds cell surface hIL-6Ra. Primary T cells were activated, transduced with pASP52 (inducible anti-hIL-6R-Myc tag + constitutive anti-CD20 scFv 4-1BB+CD3-zeta CAR; back-to-back orientation) or control pP2 construct (constitutive anti-CD20 scFv 4-1BB+CD3-zeta CAR) at MOI5, expanded and rested. Engineered T cells were co-cultured with CD20 positive cell line NALM6 at 3: 1 ratio for 72 h to induce antigen-dependent expression and secretion of hIL-6R blocking antibody. Supernatant from these cocultures was added to the HEK 293T cells engineered to express hIL-6Ra or control parental cells (with no detectable IL-6Ra expression) and incubated for 1 h. Binding anti-hIL-6R scFv-Fc-Myc tag was detected by secondary stain against Myc tag. Only supernatant from anti-hIL-6R scFv-Fc producing, but not control engineered T cells stained target IL-6Ra expressing cells in a dose dependent manner. The same supernatant did not stain parental HEK 293 T cells demonstrating specific binding. Commercial recombinant anti-hIL-6R antibody was used as a positive control. SN means supernatant.

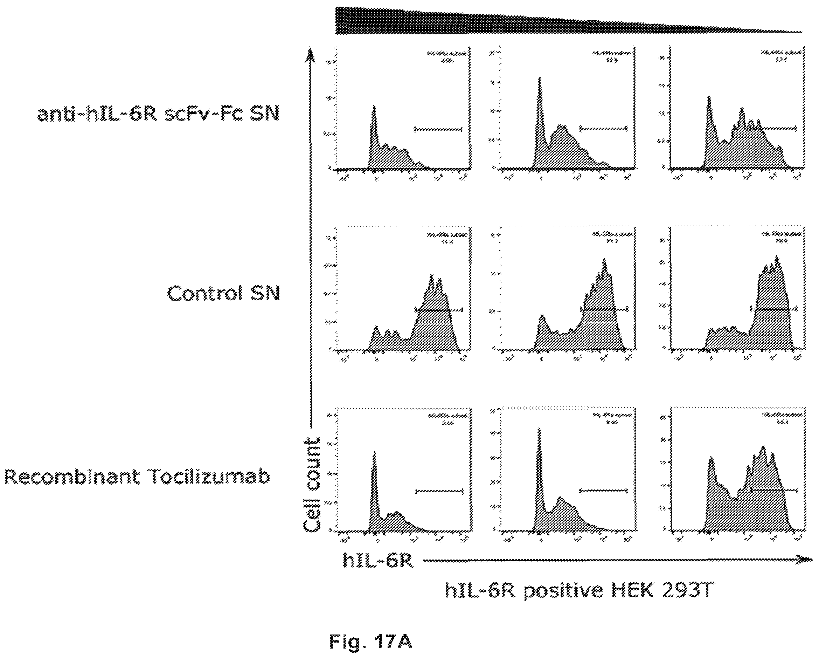

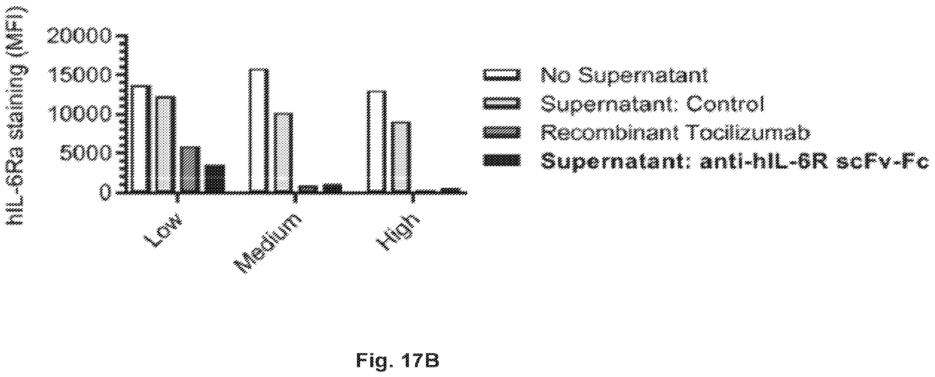

Figures 17A-17B are a series of graphs. Figure 17A illustrates that secreted anti-hIL-6R scFv-Fc blocks hIL-6Ra surface staining by recombinant anti-hIL-6Ra Ab. Figure 17B shows quantification of decreased hIL-6Ra surface staining by anti-IL-6Ra antibody. Designed anti-hIL-6R scFv-Fc was constitutively expressed in HEK 293T cells and secreted in the media. Supernatant from these cells was added to the HEK 293T cells engineered to express hIL-6Ra and incubated 16 h. Cells were then stained with recombinant commercial anti-hIL-6Ra antibody and binding of this antibody was monitored by flow cytometry (histogram in upper panel). MFI of hIL-6Ra staining was measured (lower graph). Only supernatant from anti-hIL-6R scFv-Fc producing, but not control cells blocked staining of target IL-6Ra expressing cells in a dose dependent manner. Clinically used Tocilizumab was used as a positive control. SN means supernatant.

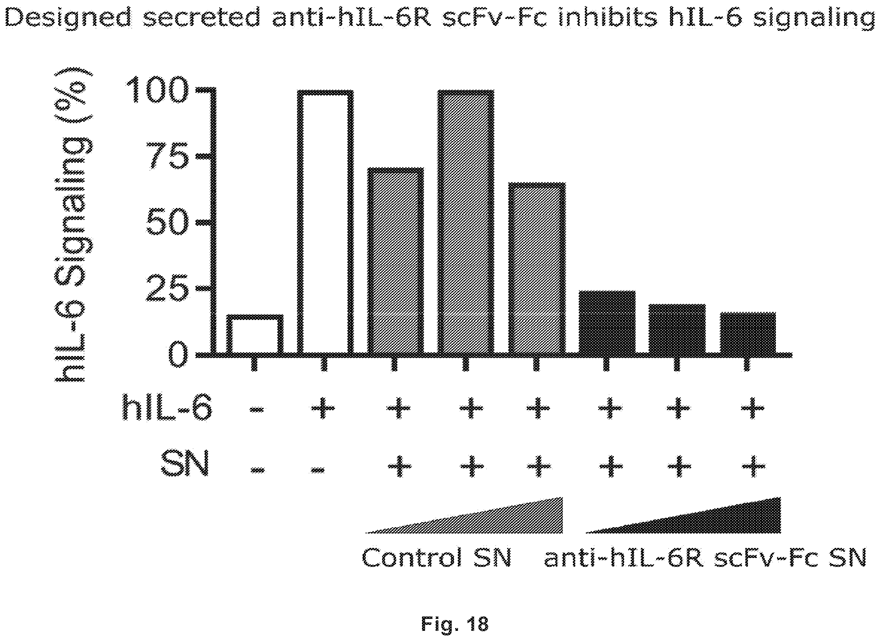

Figure 18 illustrates that secreted anti-hIL-6R scFv-Fc inhibits hIL-6 signaling. To further demonstrate biological activity of secreted engineered human IL-6Ra-blocking antibody, we investigated whether its binding to human IL-6Ra expressed on the cell surface would inhibit IL-6 signaling. Only supernatant from anti-IL6R scFv-Fc producing HEK 293T cells, but not control cells secreting irrelevant antibody, inhibited IL-6 signaling monitored through activity of STAT3 reporter in HEK 293T cells engineered with pASP59 (STAT3-eGFP + hIL-6Ra).

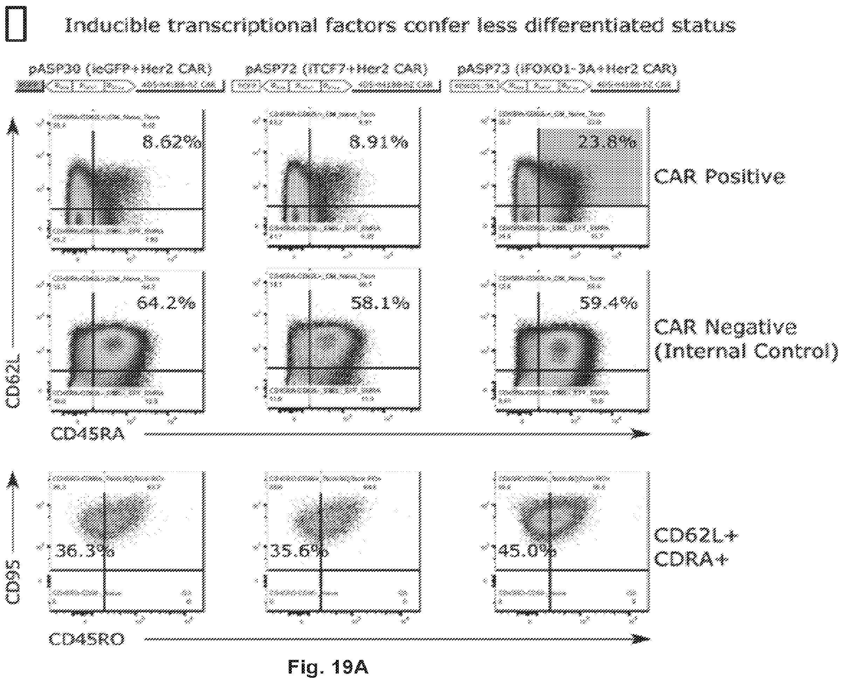

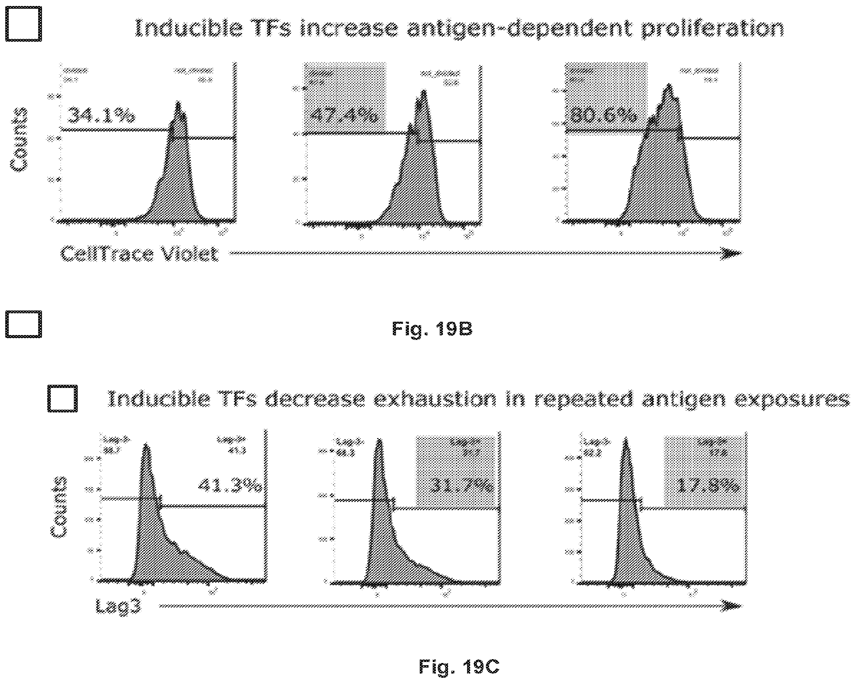

Figures 19A-19C illustrate that antigen-inducible transcriptional factors TCF-7 and FOXO1-3A mediate less differentiated status and improved antigen-dependent proliferation in CAR T cells. Primary T cells were activated, transduced with pASP30 (inducible eGFP + constitutive 4D5 anti-Her2 scFv 4-1BB+CD3-zeta CAR; back-to-back orientation), pASP72 (inducible hTCF-7 + constitutive 4D5 anti-Her2 scFv 4-1BB+CD3-zeta CAR; back-to-back orientation) or pASP73 (inducible human FOXO1-3A + constitutive 4D5 anti-Her2 scFv 4-1BB+CD3-zeta CAR; back-to-back orientation) at MOI5, expanded and rested according to the standard protocol. Figure 19A shows that engineered cells were subjected to 2 repeated rounds of target cells exposure and their phenotype was determined 3 days after last stimulation using multicolor flow cytometry. Figure 19B shows that engineered cells were sorted for CAR positive populations, subjected to 2 repeated rounds of target cells exposure, rested 5 days, stained with CellTrace Violet and re-exposed to Her2 positive target cells for 5 days. Proliferation was measured by flow cytometry. Figure 19C shows that engineered cells were subjected to 3 repeated rounds of target cells exposure and 6 days after last exposure, Lag3 expression was determined by flow cytometry.

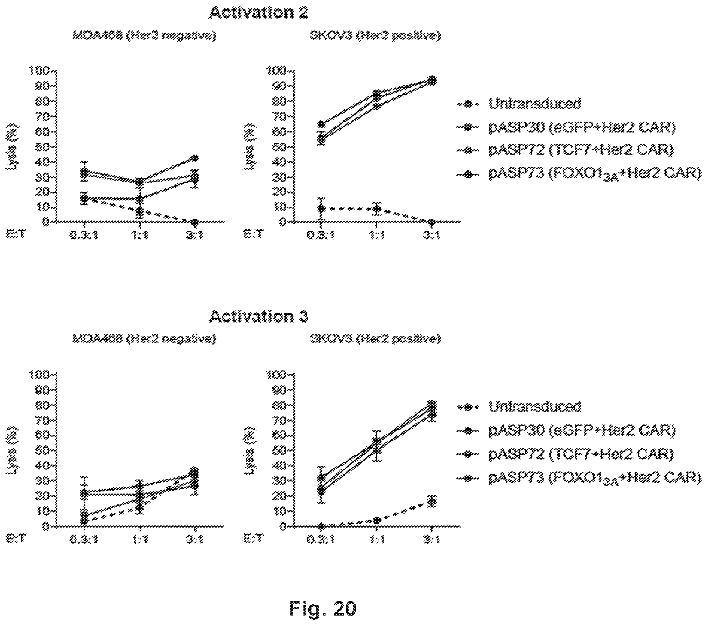

Figure 20 illustrates that CAR T cells engineered with antigen inducible expression of transcriptional factors TCF-7 and FOXO1-3A retain capacity to lyse tumor cells. Primary T cells were activated, transduced with pASP30 (inducible eGFP + constitutive 4D5 anti-Her2 scFv 4-1BB+CD3-zeta CAR; back-to-back orientation), pASP72 (inducible hTCF-7 + constitutive 4D5 anti-Her2 scFv 4-1BB+CD3-zeta CAR; back-to-back orientation) or pASP73 (inducible human FOXO1-3A + constitutive 4D5 anti-Her2 scFv 4-1BB+CD3-zeta CAR; back-to-back orientation) at MOI5, expanded and rested according to the standard protocol. Lysis capacity of Her2 positive cell line SKOV3 was determined after 2 and 3 rounds of antigen exposure. The Her2 negative MDA468 cell line was used as a control.

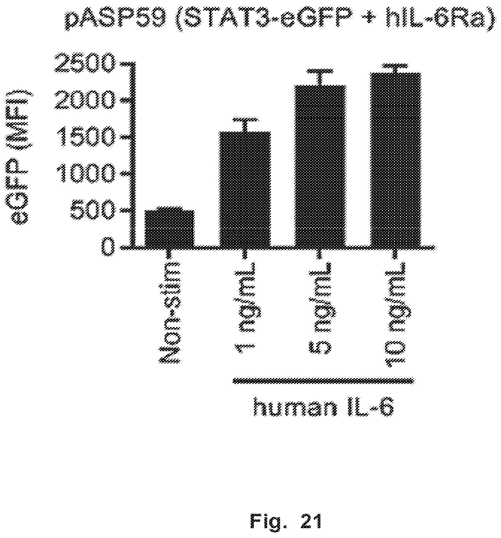

Figure 21 illustrates adaptation of the single-vector system to sense and respond to IL-6. CAR was exchanged with IL-6Ra and NFAT-sensing promoter was exchanged with STAT3-sensing promoter in the single-vector system to generate pASP59 (STAT3-eGFP + hIL-6Ra). HEK 293 T cells were transduced with pASP59 and stimulated with increasing amounts of human IL-6. Specific and dose dependent responses were observed, demonstrating that modular design of the single-vector system where elements (receptors and promoters) can be easily exchanged to detect customized signals and respond by a relevant therapeutic output.

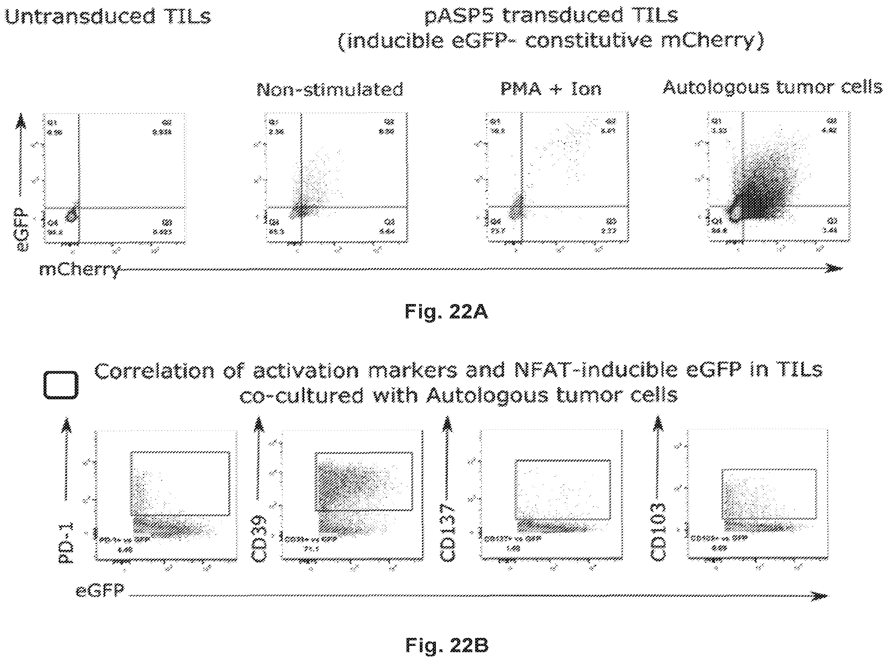

Figures 22A-22B illustrate use of the single-vector system to screen for antigen specific immune cells. Figure 22A shows patient derived Tumor-Infiltrating Lymphocytes (TILs) were successfully transduced with pASP5 (inducible eGFP + constitutive mCherry). Inducible module was upregulated when engineered TILs were stimulated with ionomycin and phorbol myristate acetate. Co-culture of engineered TILs with matching autologous tumor sample led to increased signature of inducible eGFP. Figure 22B shows engineered TILs, co-cultured with autologous tumor cells, were stained with standard activation markers used in determining antigen-specific immune cells. Correlation between these markers and NFAT-inducible eGFP was investigated.

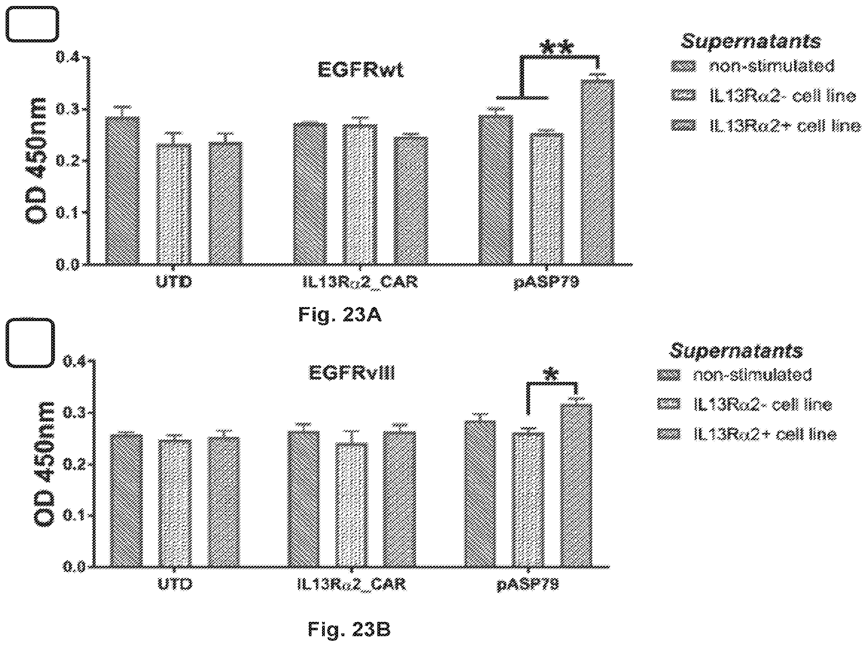

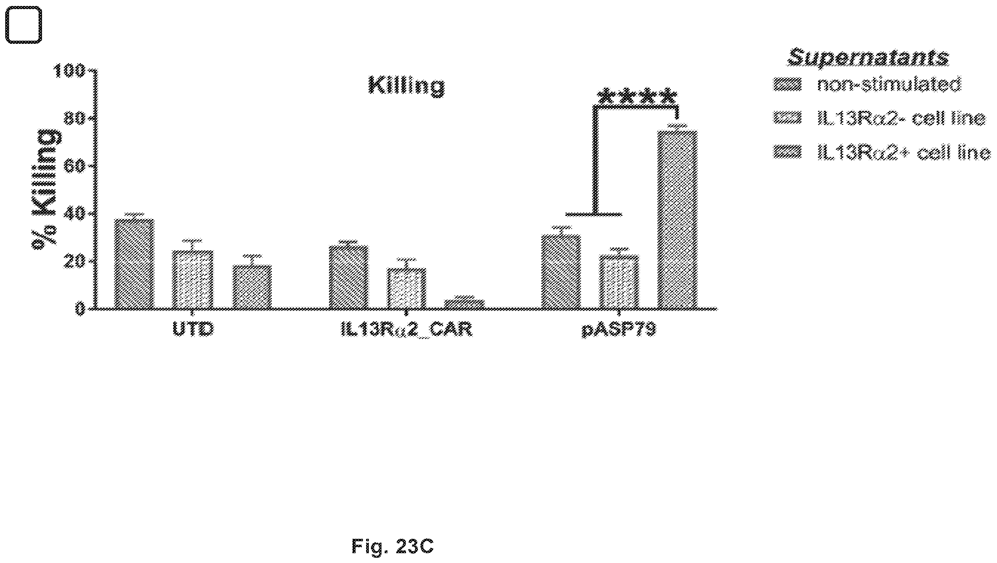

Figures 23A-23C illustrate IL13Rα2-targeting CAR-inducible secretion of functional bispecific T cell engager pASP79 (BiTE). Primary T cells engineered with single-vector IL13Rα2-targeting CAR and inducible BiTE (pASP79; the BiTE targeting EGFR wild type(EGFRwt) and EGFR variant III (EGFRvIII) proteins) were cultured in RPMI1640 medium, IL13Rα2- or IL13Rα2+ glioma stem cell lines for two days. Supernatant was collected and used in ELISA-based detection of BiTE and in a bioluminescence killing assay. Figures 23A-23B are graphs showing ELISA results. Plates were coated with EGFRwt or EGFRvIII proteins, which are the targets of BiTEs in this study. Direct ELISA was used to detect the induced secretion of BiTEs. Figure 23C is a graph showing ELISA results. Un-transduced T cells were co-cultured with an EGFRvIII+ glioma stem cell line in the collected supernatant. The ratio of T cells to tumor cells was is 5:1. 12 hours later, the bioluminescent signal was determined. One way ANOVA was used to detect the statistical differences between each group. **P*<0.05, ***P*<0.01,*****P*<0.0001*.*

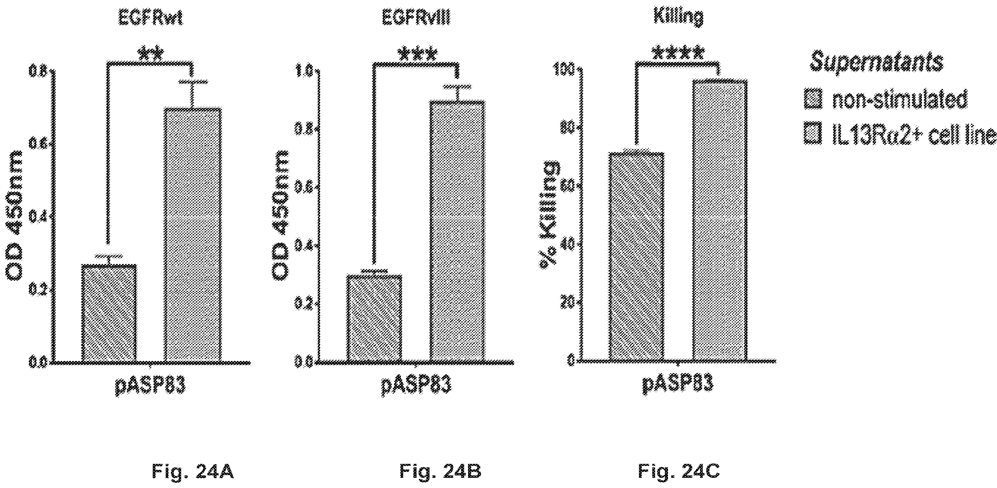

Figures 24A-24C illustrate IL13Rα2-targeting CAR-inducible secretion of functional bispecific T cell engager pASP83 (BiTE). Primary T cells engineered with single-vector IL13Rα2-targeting CAR and inducible BiTE (pASP83; the BiTE targeting EGFR wild type(EGFRwt) and EGFR variant III (EGFRvIII) proteins) were cultured in RPMI1640 medium or IL13Rα2+ glioma stem cell lines for two days. Supernatant was collected and used in ELISA based detection of BiTE and in a bioluminescence killing assay. Figures 24A-24B are graphs showing ELISA results. Plates were coated with EGFRwt or EGFRvIII proteins, which are the targets of BiTEs in this study. Direct ELISA was used to detect the inducible secretion of BiTEs. Figure 24C is a graph showing ELISA results. Un-transduced T cells were co-cultured with an EGFRvIII+ glioma stem cell line in the collected supernatant. The ratio of T cells to tumor cells was is 5:1. 12 hours later, the bioluminescent signal was determined. One way ANOVA was used to detect the statistical differences between each group. Thus, pASP83 CAR T cells secrete biologically active EGFR-specific BiTEs in response to antigen activation, and the secreted BiTEs can redirect the activity of untransduced T cells against EGFR-expressing cancer cells. **P<0.01,****P<0.0001.

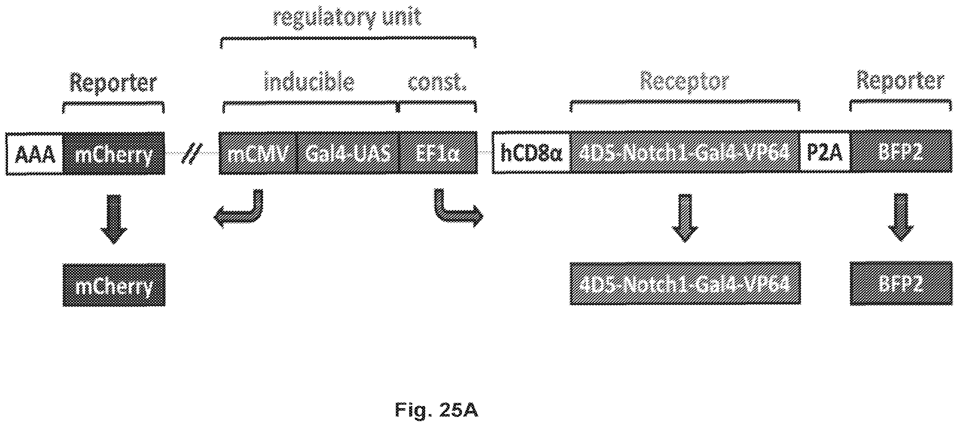

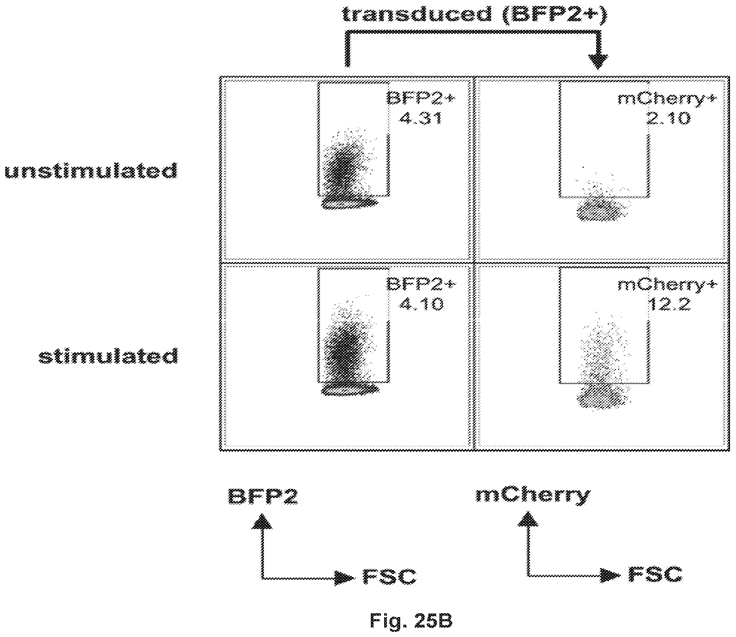

Figures 25A-25B illustrate the synNotch single-vector system. Figure 25A is a schematic of the synNotch single-vector system. The constitutive EF1alpha promoter leads to the continuous expression of a HER2-specific synNotch receptor (4D5-Notch1-Gal4-VP64) and a blue fluorescent BFP2 reporter protein. Binding of the synNotch receptor to its target antigen HER2 induces Notch1 cleavage which releases the transcription factor Gal4-VP64 from the receptor. Gal4-VP64 binds to the Gal4 enhancer sequence (Gal4-UAS) thereby activating the minimal CMV promoter (mCMV) and leading to the production of a red fluorescent mCherry reporter protein. "AAA" represents a synthetic poly-A sequence and hCD8alpha indicates a secretion sequence. P2A is facilitating the co-expression of receptor and BFP2 reporter proteins. Figure 25B shows experimental validation of the synNotch single-vector system. Representative flow-cytometry plots show expression of BFP2 and mCherry in primary human T cells transduced with the synNotch single-vector construct. Transduced T cells were cultured in the absence (unstimulated) or presence (stimulated) of HER2-expressing SKOV3 tumor cells for 24h and analyzed with a LSRFortessa flow-cytometer (BD Biosciences).

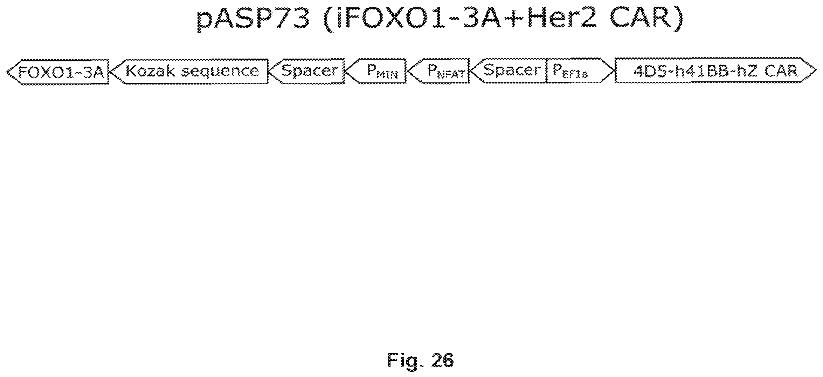

Figure 26 illustrates pASP73 (inducible human FOXO1-3A + constitutive 4D5 anti-Her2 scFv 4-1BB+CD3-zeta CAR; back-to-back orientation).

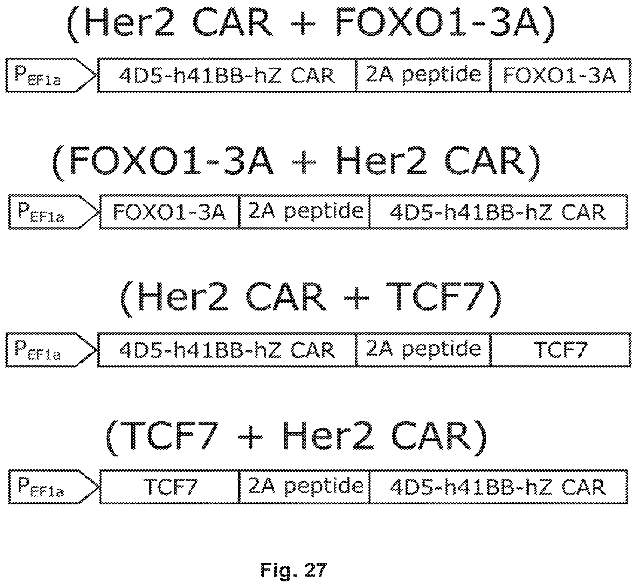

Figure 27 illustrates constructs that express a CAR and a transcription factor under control of a single constitutive promoter. The constructs include a 2A peptide spacer between the CAR and the transcription factor.

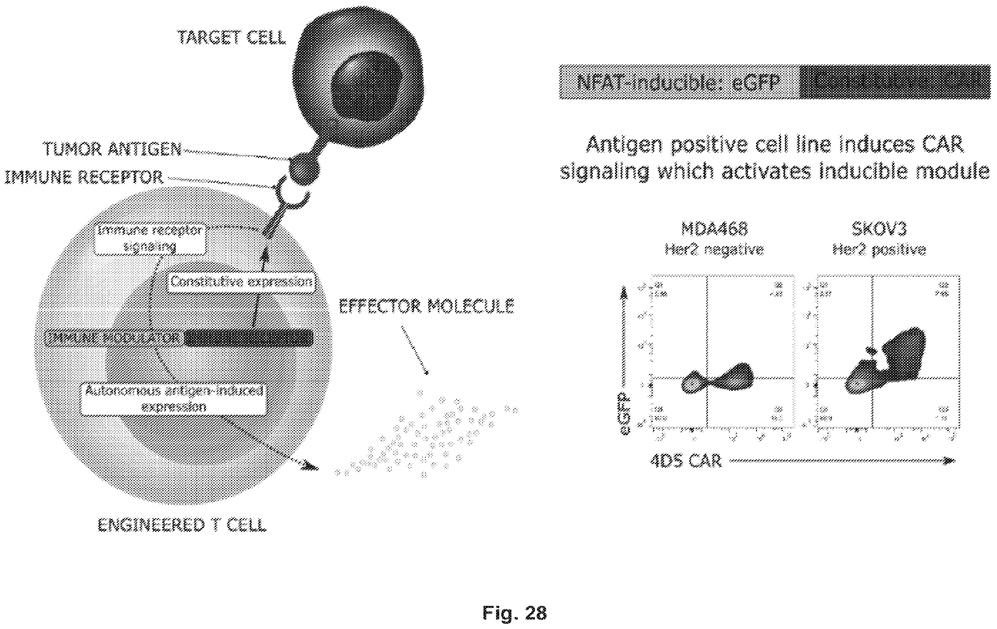

Figure 28 illustrates the concept and a proof-of-principle of the Uni-Vect single vector system in primary T cells. Left: Upon the recognition of cognate antigen by a constitutively expressed immune receptor (CAR or TCR) endogenous NFAT signaling is rewired for the expression of an immune modulator from the same lentiviral construct. Right: A Her2-targeting CAR activates the inducible system (GFP shown) exclusively when in co-culture with Her2 positive cancer cell line.

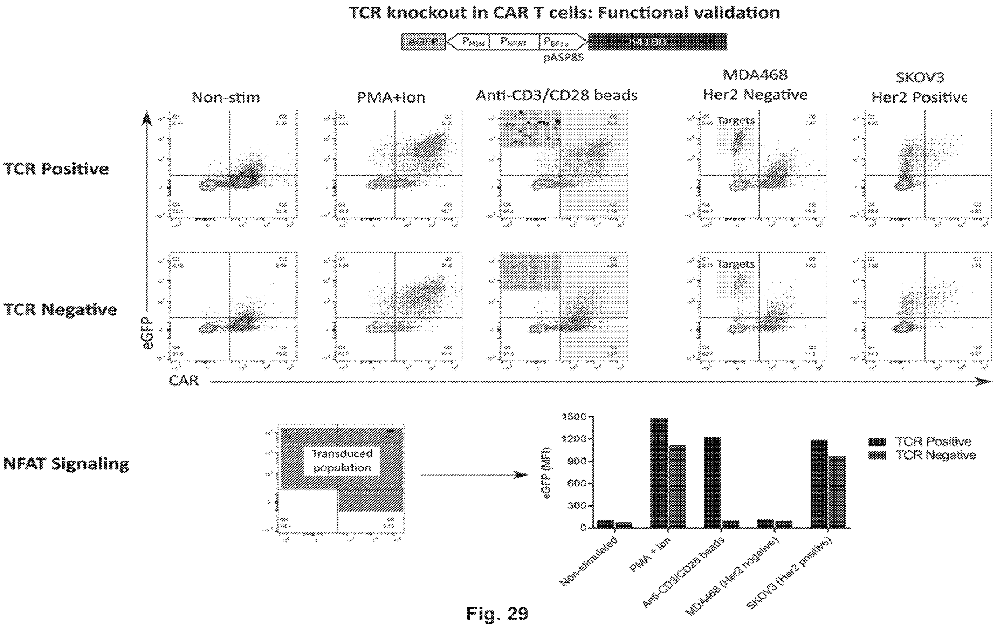

Figure 29 shows that knocking out endogenous TCR renders CAR signaling an exclusive activator of the system. Using CRISPR/Cas9 technology and sgRNAs targeting TCRα and TCRβ, endogenous TCR was knocked-out with approximately 90% efficiency. Subsequently, cells were activated and transduced with the single lentiviral construct pASP85 that combines NFAT-inducible eGFP and constitutive Her2-targeting CAR expression. Edited CAR T cell products were purified by a negative selection where the remaining CD3 positive cells were removed. Activation of generated CAR T cell products was validated by monitoring a built in NFAT signaling reporter system (inducible module of the Uni-Vect). Activation of TCR through anti-CD3/CD28 beads occurred only in TCR positive cells while it was lost in edited and selected TCR negative cells demonstrating a successful knock-out of endogenous TCR. Importantly, knocking-out endogenous TCR did not impair CAR mediated signaling as demonstrated by a specific and effective response to Her2 positive cell line SKOV3.

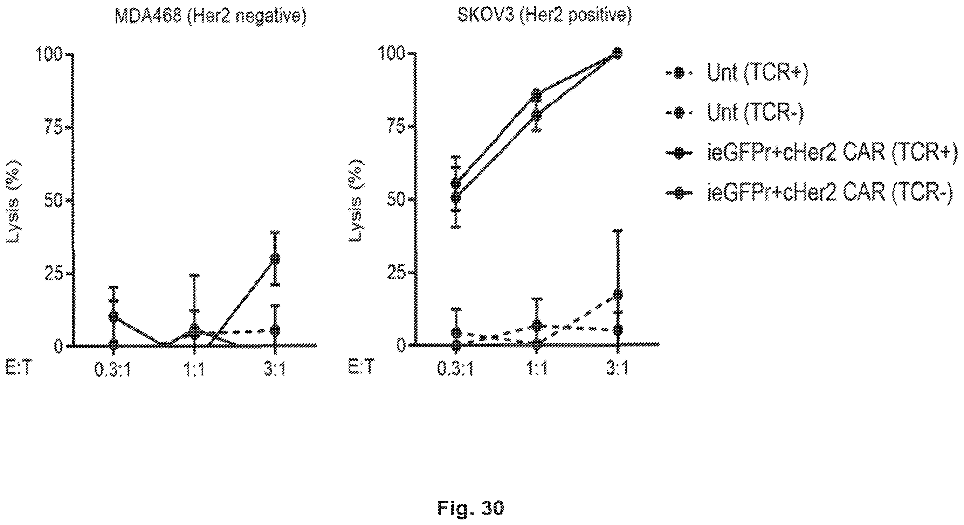

Figure 30: TCR knockout does not impair specific target cell lysis mediated by a CAR. pASP85 engineered primary T cells where endogenous TCR was knocked-out were co-cultured with Her2 positive (SKOV3) and Her2 negative (MDA468) cell lines. No decreased or non-specific lysis was observed in TCR negative compared to TCR positive CAR T cells.

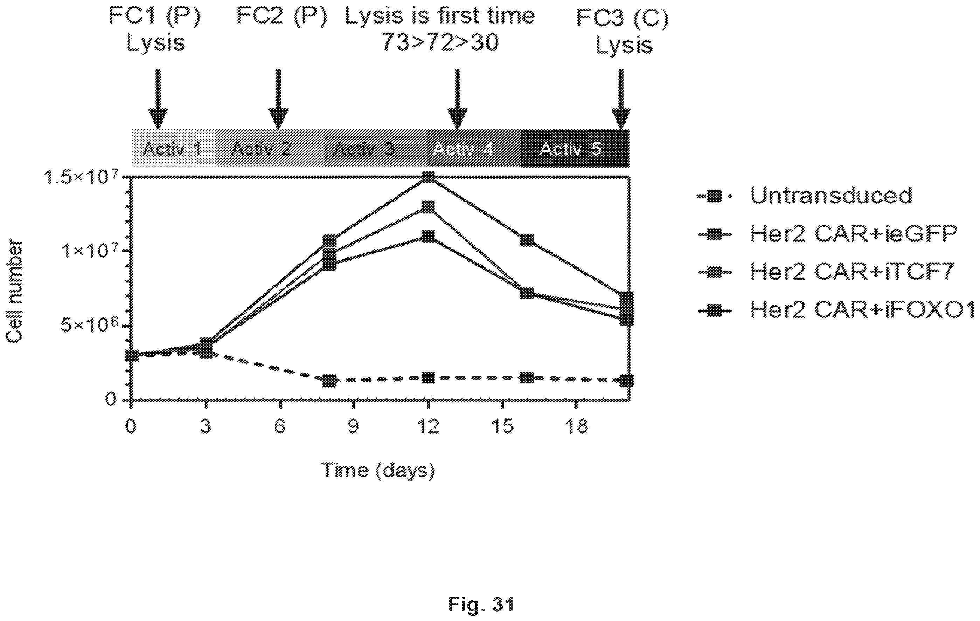

Figure 31: In vitro re-stimulation assay in non-sorted iTF-CAR T cells. Primary T cells were activated, transduced with pASP30 (inducible eGFP + constitutive 4D5 anti-Her2 scFv 4-1BB+CD3-zeta CAR; back-to-back orientation), pASP72 (inducible hTCF7 + constitutive 4D5 anti-Her2 scFv 4-1BB+CD3-zeta CAR; back-to-back orientation) or pASP73 (inducible human FOXO1-3A + constitutive 4D5 anti-Her2 scFv 4-1BB+CD3-zeta CAR; back-to-back orientation) at MOI5, expanded and rested according to the standard protocol. Engineered cells were subjected to 5 repeated rounds of Her2 positive target cells exposure. FC1 (P), FC2 (P) and FC3 (P) depict time when phenotype of iTF-CAR T cells was determined using multicolor flow cytometry. Lysis depicts time when capability to lyse target cells was measured.

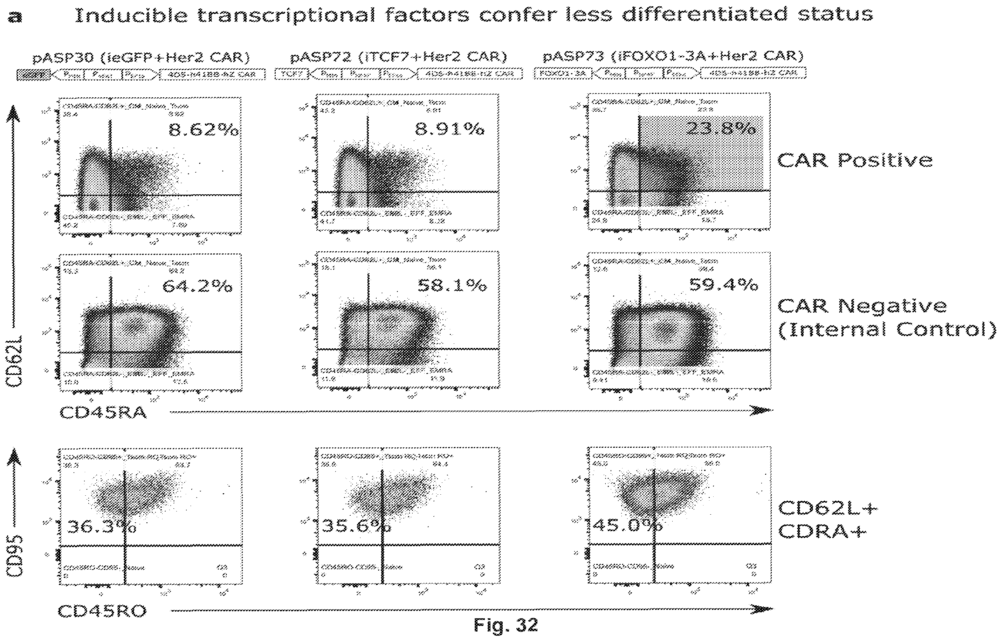

Figure 32 shows that the antigen-inducible expression of transcription factors TCF7 or FOXO1-3A mediates less differentiated state in CAR T cells. Primary T cells were activated, transduced with pASP30 (inducible eGFP + constitutive 4D5 anti-Her2 scFv 4-1BB+CD3-zeta CAR; back-to-back orientation), pASP72 (inducible hTCF7 + constitutive 4D5 anti-Her2 scFv 4-1BB+CD3-zeta CAR; back-to-back orientation) or pASP73 (inducible human FOXO1-3A + constitutive 4D5 anti-Her2 scFv 4-1BB+CD3-zeta CAR; back-to-back orientation) at MOI5, expanded and rested according to the standard protocol. Engineered cells were subjected to 2 repeated rounds of target cells exposure and their phenotype was determined 3 days after last stimulation using multicolor flow cytometry.

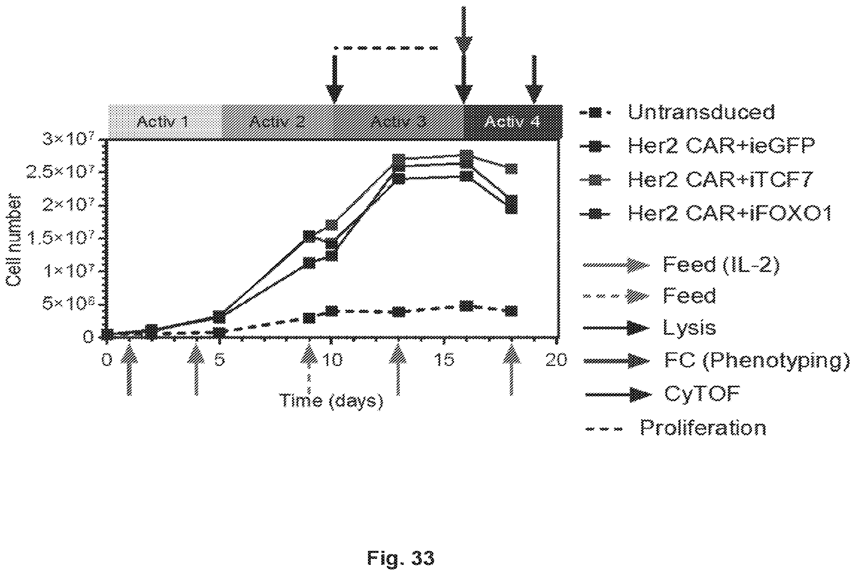

Figure 33: *In vitro* re-stimulation assay in sorted CAR positive iTF-CAR T cells. Primary T cells were activated, transduced with pASP30 (inducible eGFP + constitutive 4D5 anti-Her2 scFv 4-1BB+CD3-zeta CAR; back-to-back orientation), pASP72 (inducible hTCF7 + constitutive 4D5 anti-Her2 scFv 4-1BB+CD3-zeta CAR; back-to-back orientation) or pASP73 (inducible human FOXO1-3A + constitutive 4D5 anti-Her2 scFv 4-1BB+CD3-zeta CAR; back-to-back orientation) at MOI5, expanded and rested according to the standard protocol. Engineered cells were subjected to 4 repeated rounds of Her2 positive target cells exposure. Arrows below the graph depict time when cells were supplemented with media with or without IL-2. Left and middle arrows immediately above the graph represent times when lysis capacity of iTF-CAR T cells was analyzed. The arrow above the middle arrow immediately above the graph represents the time when phenotype of iTF-CAR T cells was determined using multicolor flow cytometry. The right-most arrow immediately above the graph represents the time when iTF-CAR T cells were analyzed by CyTOF mass cytometry. Dotted line above the graph represents the time when a sample of each iTF CAR T cell product and control CAR T cells was used in a parallel co-culture experiment with target cell line to investigate *in vitro* proliferation.

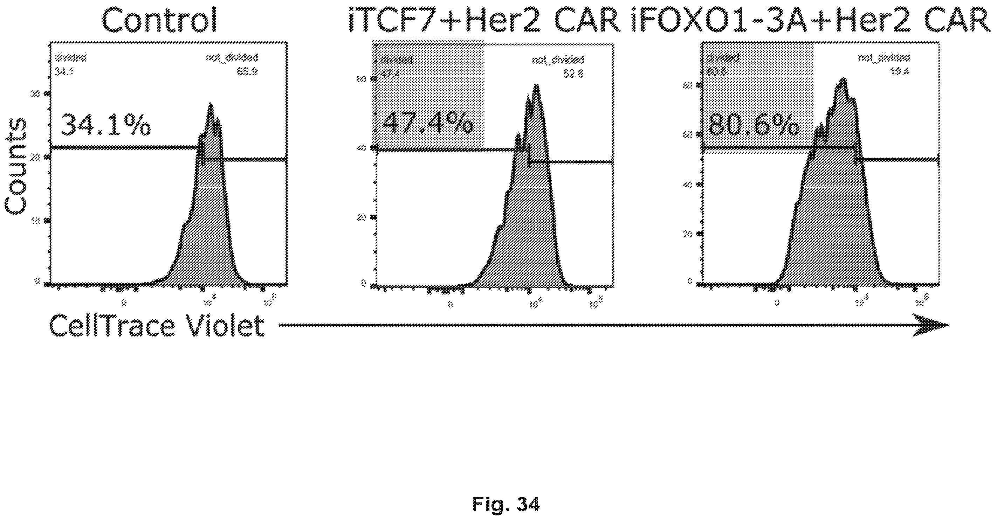

Figure 34 shows that antigen-inducible transcription factors improve *ex vivo* proliferation of CAR T cells. Two independent iTF-CAR T cell products, iTCF7-CAR T and iFOXO1-3A-CAR T, were developed. Primary T cells were activated, transduced with pASP30 (inducible eGFP + constitutive 4D5 anti-Her2 scFv 4-1BB+CD3-zeta CAR; back-to-back orientation), pASP72 (inducible hTCF-7 + constitutive 4D5 anti-Her2 scFv 4-1BB+CD3-zeta CAR; back-to-back orientation) or pASP73 (inducible human FOXO1-3A + constitutive 4D5 anti-Her2 scFv 4-1BB+CD3-zeta CAR; back-to-back orientation) at MOI5, expanded and rested according to the standard protocol. Engineered cells were sorted for CAR positive population and first subjected to 2 repeated rounds of Her2 positive target cells exposure. Next these cells were stained with CellTrace Violet and re-exposed to Her2 positive target cells for 5 days. Proliferation was measured by flow cytometry.

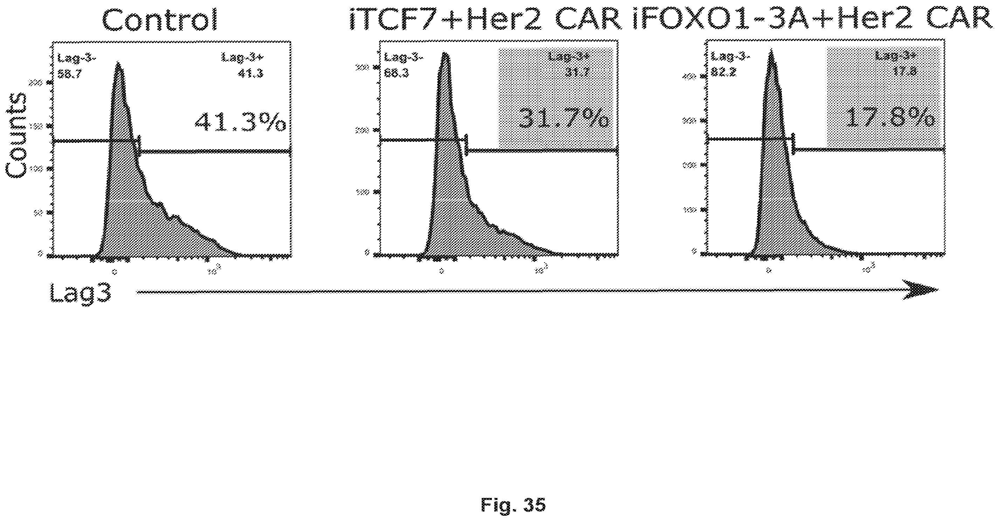

Figure 35 shows that antigen-inducible transcription factors decrease inhibitory marker in CAR T cells. Two independent iTF-CAR T cell products were developed (iTCF7-CAR T and iFOXO1-3A-CAR T). Primary T cells were activated, transduced with pASP30 (inducible eGFP + constitutive 4D5 anti-Her2 scFv 4-1BB+CD3-zeta CAR; back-to-back orientation), pASP72 (inducible hTCF-7 + constitutive 4D5 anti-Her2 scFv 4-1BB+CD3-zeta CAR; back-to-back orientation) or pASP73 (inducible human FOXO1-3A + constitutive 4D5 anti-Her2 scFv 4-1BB+CD3-zeta CAR; back-to-back orientation) at MOI5, expanded and rested according to the standard protocol. Engineered cells were sorted for CAR positive population and subjected to 3 repeated rounds of target cells exposure and 6 days after last exposure, Lag3 expression was determined by flow cytometry.

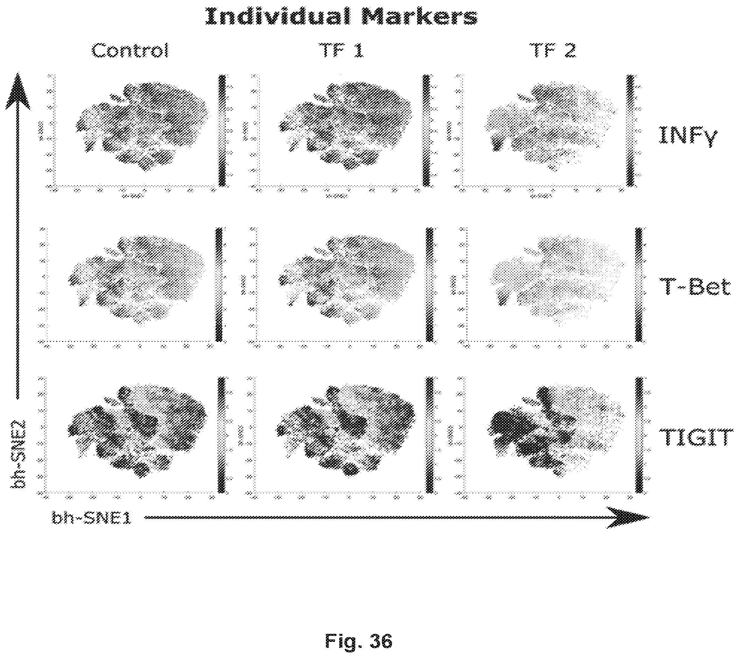

Figure 36: CyTOF analysis reveals markers that favor efficacy in iFOXO1-3A-CAR T cells compared to regular CAR control. Two prototypic iTF-CAR T cellular products (TF1 features TCF7, TF2 features FOXO1-3A) combining inducible transcription factor expression and constitutive Her2 CAR expression were developed. Primary T cells were activated, transduced with pASP30 (inducible eGFP + constitutive 4D5 anti-Her2 scFv 4-1BB+CD3-zeta CAR; back-to-back orientation), pASP72 (inducible hTCF-7 + constitutive 4D5 anti-Her2 scFv 4-1BB+CD3-zeta CAR; back-to-back orientation) or pASP73 (inducible human FOXO1-3A + constitutive 4D5 anti-Her2 scFv 4-1BB+CD3-zeta CAR; back-to-back orientation) at MOI5, expanded and rested according to the standard protocol. Engineered cells were sorted for CAR positive population, subjected to 4 repeated rounds of target cells exposure. Using CyTOF, functionally relevant T cell phenotype markers were analyzed 3 days after last round of stimulation. iFOXO1-3A resulted in a shift in differentiation states and markers that favor activity.

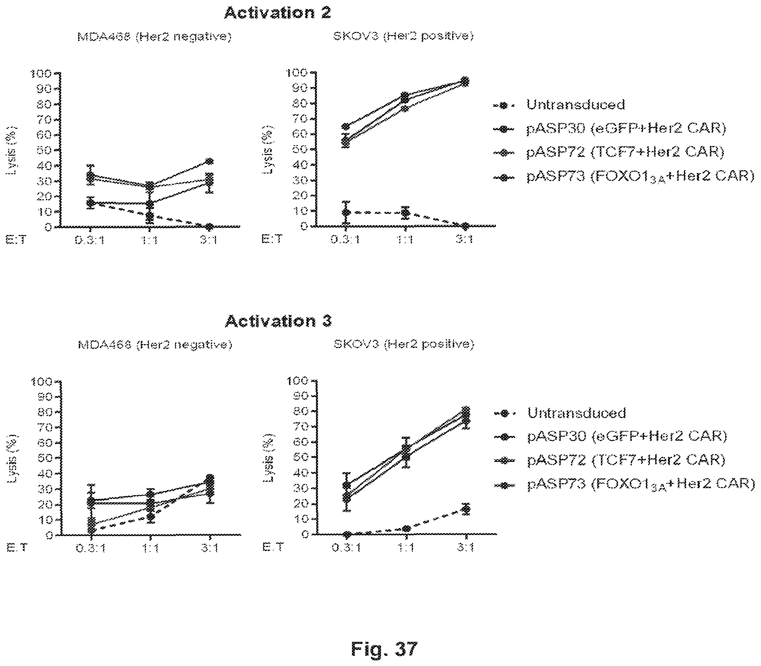

Figure 37: CAR T cells engineered with antigen inducible expression of transcription factors TCF-7 and FOXO1-3A retain capacity to lyse tumor cells. Primary T cells were activated, transduced with pASP30 (inducible eGFP + constitutive 4D5 anti-Her2 scFv 4-1BB+CD3-zeta CAR; back-to-back orientation), pASP72 (inducible hTCF7 + constitutive 4D5 anti-Her2 scFv 4-1BB+CD3-zeta CAR; back-to-back orientation) or pASP73 (inducible human FOXO1-3A + constitutive 4D5 anti-Her2 scFv 4-1BB+CD3-zeta CAR; back-to-back orientation) at MOI5, expanded and rested according to the standard protocol. Lysis capacity of Her2 positive cell line SKOV3 was determined after 2 and 3 rounds of antigen exposure. Her2 negative MDA468 cell line was used as a control.

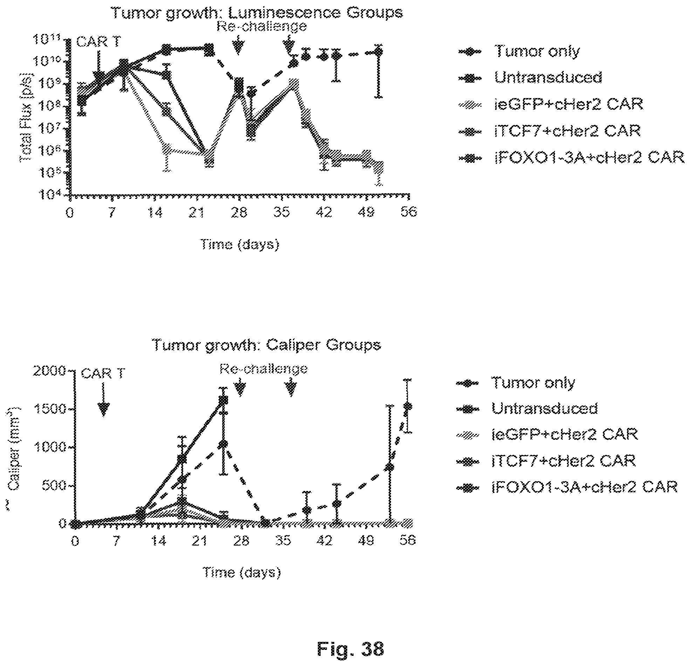

Figure 38: iTF-CAR T eliminate established tumors and prevent tumor growth upon re-challenge. Primary T cells were activated, transduced with pASP30 (inducible eGFP + constitutive 4D5 anti-Her2 scFv 4-1BB+CD3-zeta CAR; back-to-back orientation), pASP72 (inducible hTCF7 + constitutive 4D5 anti-Her2 scFv 4-1BB+CD3-zeta CAR; back-to-back orientation) or pASP73 (inducible human FOXO1-3A + constitutive 4D5 anti-Her2 scFv 4-1BB+CD3-zeta CAR; back-to-back orientation) at MOI5, expanded and rested according to the standard protocol. 1 million of CAR positive iTF-CAR T cells per mouse were injected intraperitoneally into NSG mice bearing subcutaneous (s.c.) xenograft SKOV3 tumors. 1 million of SKOV3 cells (engineered to express luciferase) were injected s.c. 4 days prior to the administration of CAR T cells to establish solid tumor mass. After initial tumor clearance, mice were re-challenged twice with SKOV3 cells, first with 5 million on day 28 and second with 10 million on day 38. At the second re-challenged, second cohort of mice representing tumor only group was established to control for the tumor growth in an absence of CAR T cells. Tumor growth was measured by quantification of luciferase activity in tumor cells (upper panel) and by caliper measurement of tumor growth (lower panel).

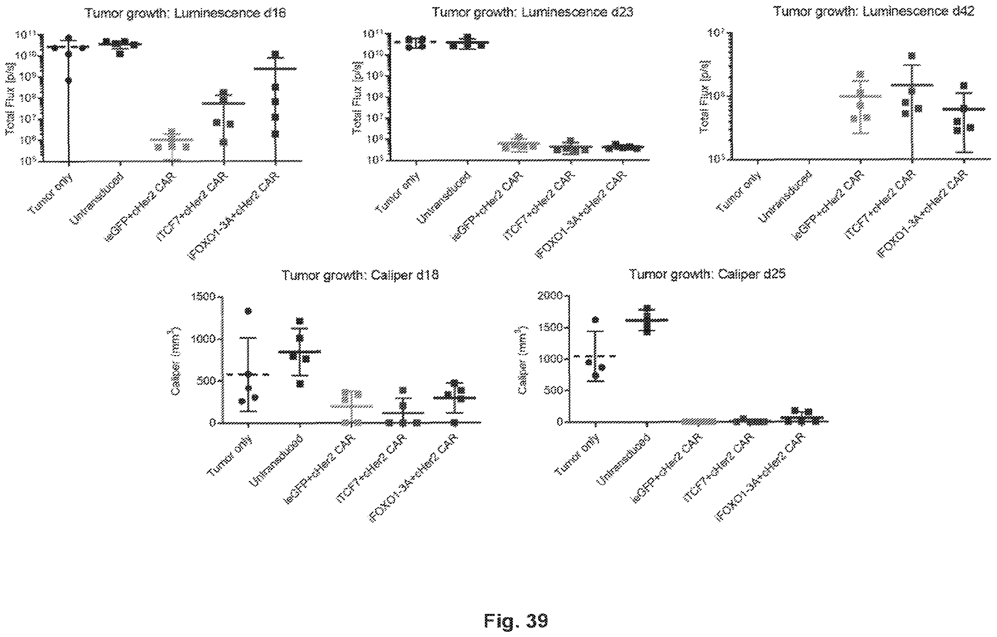

Figure 39: Tumor growth control by iTF-CAR T cells at specific days. Primary T cells were activated, transduced with pASP30 (inducible eGFP + constitutive 4D5 anti-Her2 scFv 4-1BB+CD3-zeta CAR; back-to-back orientation), pASP72 (inducible hTCF7 + constitutive 4D5 anti-Her2 scFv 4-1BB+CD3-zeta CAR; back-to-back orientation) or pASP73 (inducible human FOXO1-3A + constitutive 4D5 anti-Her2 scFv 4-1BB+CD3-zeta CAR; back-to-back orientation) at MOI5, expanded and rested according to the standard protocol. 1 million of CAR positive iTF-CAR T cells per mouse were injected intraperitoneally into NSG mice bearing subcutaneous (s.c.) xenograft SKOV3 tumors. 1 million of SKOV3 cells (engineered to express luciferase) were injected s.c. 4 days prior to the administration of CAR T cells to establish solid tumor mass. After initial tumor clearance, mice were re-challenged twice with SKOV3 cells, first with 5 million on day 28 and second with 10 million on day 38. At the second re-challenged, second cohort of mice representing tumor only group was established to control for tumor growth in an absence of CAR T cells. Tumor growth was measured by quantification of luciferase activity in tumor cells (upper panel) and by caliper measurement of tumor growth (lower panel). Specific days are shown and each data point represents an individual mouse.

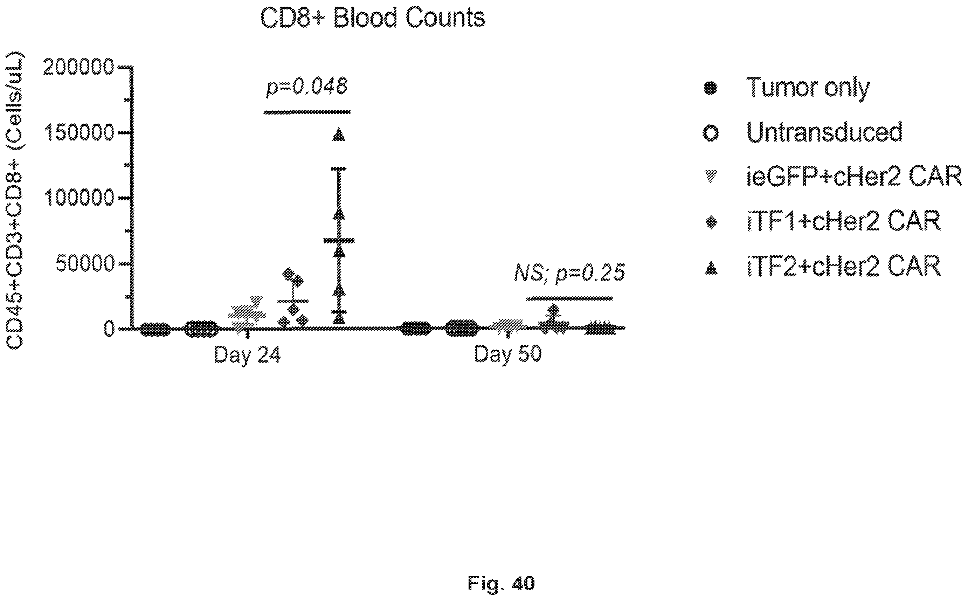

Figure 40: iTF-CAR T cells demonstrate improved *in vivo* expansion and contract to normal levels after tumor is cleared. Primary T cells were activated, transduced with pASP30 (inducible eGFP + constitutive 4D5 anti-Her2 scFv 4-1BB+CD3-zeta CAR; back-to-back orientation, depicted as ieGFP-cHef2 CAR), pASP72 (inducible hTCF7 + constitutive 4D5 anti-Her2 scFv 4-1BB+CD3-zeta CAR; back-to-back orientation, depicted as iTF1-cHer2 CAR) or pASP73 (inducible human FOXO1-3A + constitutive 4D5 anti-Her2 scFv 4-1BB+CD3-zeta CAR; back-to-back orientation depicted as iTF2-cHer2 CAR) at MOI5, expanded and rested according to the standard protocol. 1 million of CAR positive iTF-CAR T cells per mouse were injected intraperitoneally into NSG mice bearing subcutaneous (s.c.) xenograft SKOV3 tumors. 1 million of SKOV3 cells (engineered to express luciferase) were injected s.c. 4 days prior to the administration of CAR T cells to establish solid tumor mass. After initial tumor clearance, mice were re-challenged twice with SKOV3 cells, first with 5 million on day 28 and second with 10 million on day 38. At the second re-challenged, second cohort of mice representing tumor only group was established to control for tumor growth in an absence of CAR T cells. CD8 T cell counts were measured during peak expansion (day 24) and after contraction (day 50). Each data point represents an individual mouse.

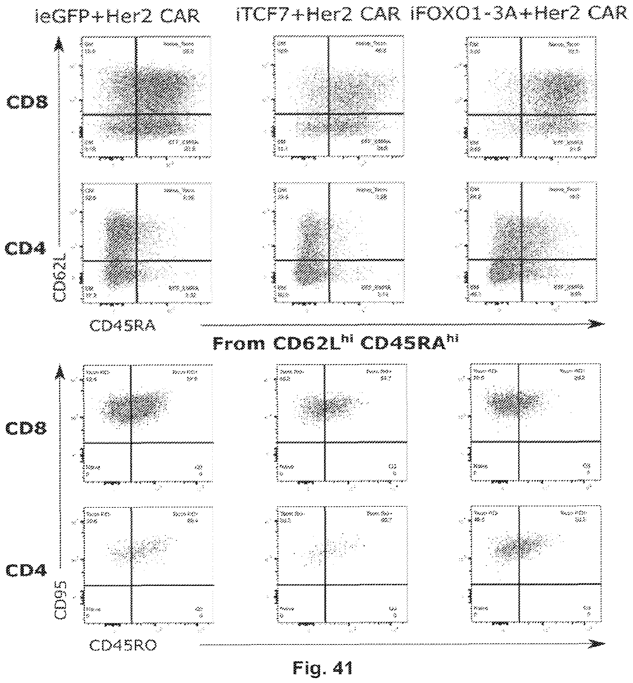

Figure 41: Primary T cells were activated with anti-CD3/CD28 beads, transduced with pASP30 (ieGFP+Her2 CAR), pASP72 (iTCF7+Her2 CAR) or pASP73 (iFOXO1-3A+Her2 CAR) at MOI5, expanded and rested according to the standard protocol. Phenotype was determined at the end of 14 days manufacturing process using multicolor flow cytometry.

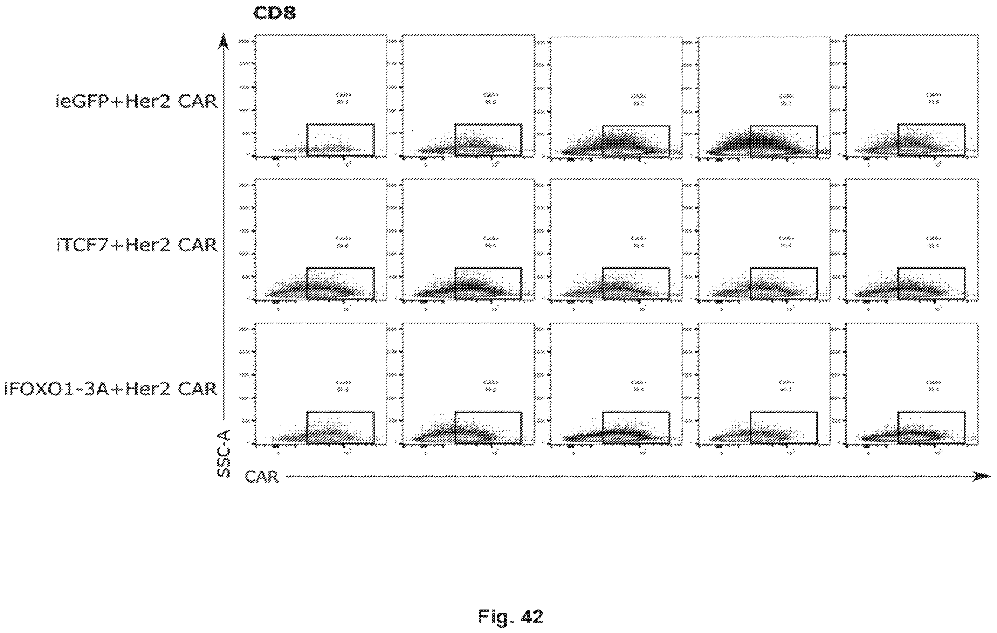

Figure 42: CAR expression in iTF-CAR T cells *in vivo.* Primary T cells were activated with anti-CD3/CD28 beads, transduced with pASP30 (ieGFP+Her2 CAR), pASP72 (iTCF7+Her2 CAR) or pASP73 (iFOXO1-3A+Her2 CAR) at MOI5, expanded and rested according to the standard protocol. 1 million of CAR+ iTF-CAR T cells per mouse were injected intraperitoneally into NSG mice bearing subcutaneous (s.c.) xenograft SKOV3 tumors. 1 million of SKOV3 cells (engineered to express luciferase) were injected s.c. 4 days prior to the administration of CAR T cells to establish solid tumor mass. iTF-CAR T cells from blood were analyzed for CAR expression on day 24 at a peak expansion. Each flow chart represents an individual mouse and bracket represents an estimated CAR gate.

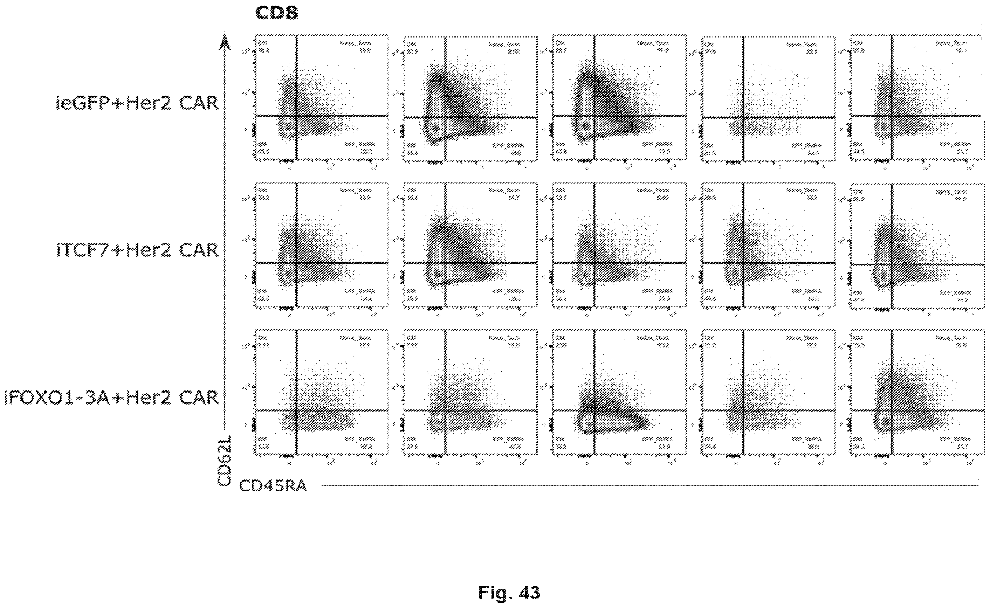

Figure 43: Phenotype of iTF-CAR T cells *in vivo* at peak response. Primary T cells were activated with anti-CD3/CD28 beads, transduced with pASP30 (ieGFP+Her2 CAR), pASP72 (iTCF7+Her2 CAR) or pASP73 (iFOXO1-3A+Her2 CAR) at MOI5, expanded and rested according to the standard protocol. 1 million of CAR+ iTF-CAR T cells per mouse were injected intraperitoneally into NSG mice bearing subcutaneous (s.c.) xenograft SKOV3 tumors. 1 million of SKOV3 cells (engineered to express luciferase) were injected s.c. 4 days prior to the administration of CAR T cells to establish solid tumor mass. Phenotype of iTF-CAR T cells and control cells from blood were analyzed on day 24 at a peak expansion. Each flow chart represents an individual mouse.

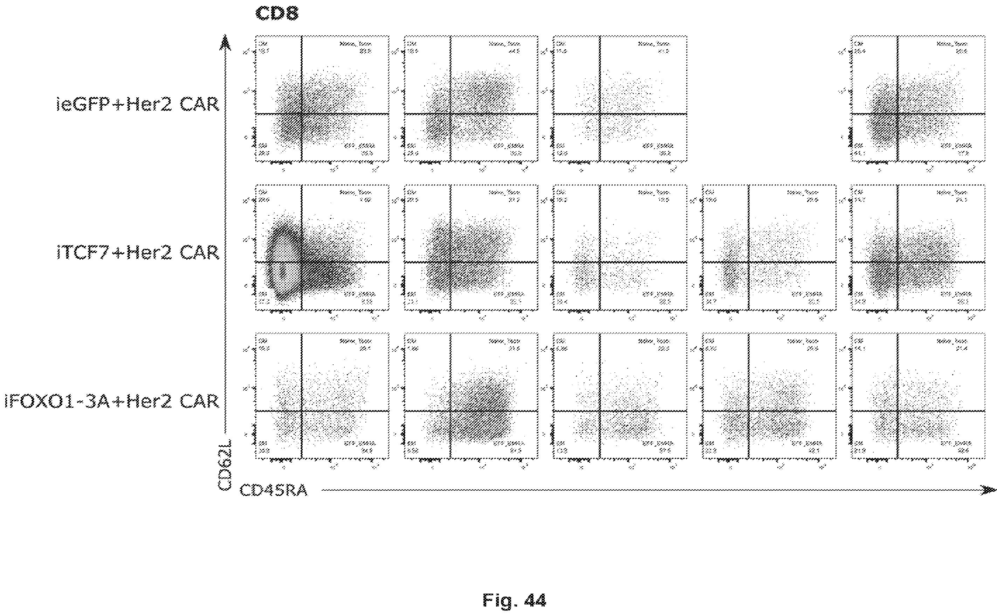

Figure 44: Phenotype of iTF-CAR T cells *in vivo* after three re-challenges with tumor cells. Primary T cells were activated with anti-CD3/CD28 beads, transduced with pASP30 (ieGFP+Her2 CAR), pASP72 (iTCF7+Her2 CAR) or pASP73 (iFOXO1-3A+Her2 CAR) at MOI5, expanded and rested according to the standard protocol. 1 million of CAR+ iTF-CAR T cells per mouse were injected intraperitoneally into NSG mice bearing subcutaneous (s.c.) xenograft SKOV3 tumors. 1 million of SKOV3 cells (engineered to express luciferase) were injected s.c. 4 days prior to the administration of CAR T cells to establish solid tumor mass. Mice were re-challenged with the same tumor twice. Phenotype of iTF-CAR T cells and control cells from blood were analyzed on day 50 after three rounds of tumor exposure and clearance. Each flow chart represents an individual mouse.

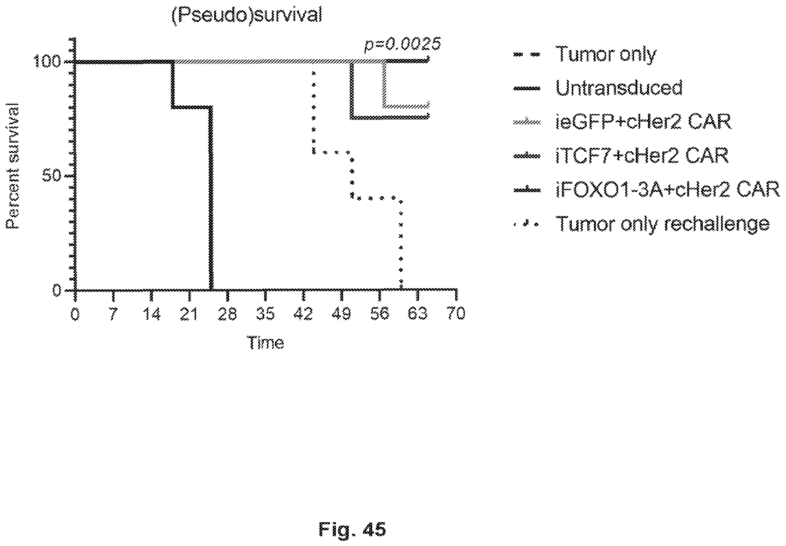

Figure 45: Survival of iTF-CAR T cells *in vivo* after repeated tumor re-challenges. Primary T cells were activated with anti-CD3/CD28 beads, transduced with pASP30 (ieGFP+Her2 CAR), pASP72 (iTCF7+Her2 CAR) or pASP73 (iFOXO1-3A+Her2 CAR) at MOI5, expanded and rested according to the standard protocol. 1 million of CAR+ iTF-CAR T cells per mouse were injected intraperitoneally into NSG mice bearing subcutaneous (s.c.) xenograft SKOV3 tumors. 1 million of SKOV3 cells (engineered to express luciferase) were injected s.c. 4 days prior to the administration of CAR T cells to establish solid tumor mass. Mice were re-challenged with the same tumor twice. Survival of mice was monitored. P value refers to a comparison between iFOXO1-3A and un-transduced groups.

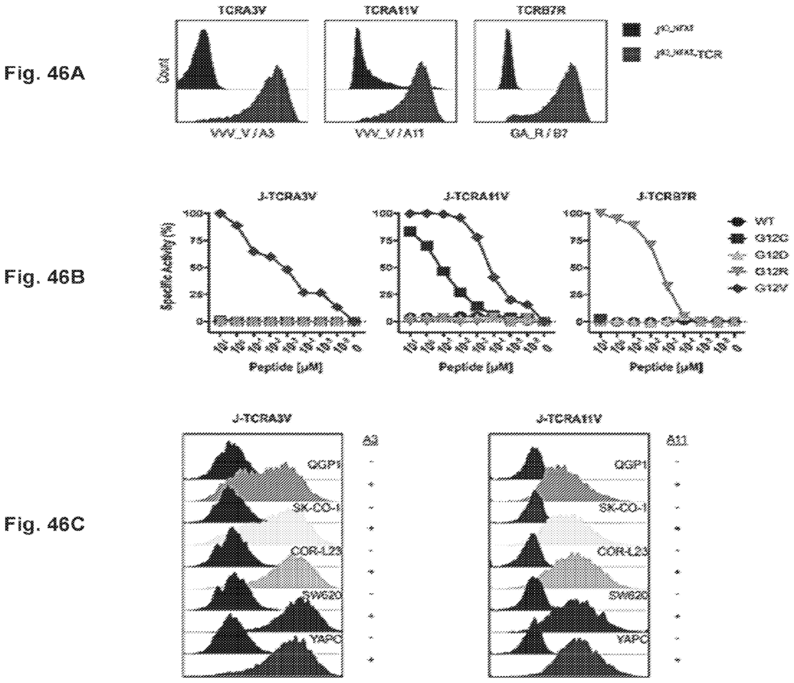

Figures 46A-46C: Validation and functional characterization of mKRAS-specific TCRs derived from healthy donors. Figure 46A: FACS histogram plots demonstrating p/MHC multimer binding following lentiviral transduction of J90_NFAT cells with TCRA3V, TCRA11V and TCRB7R constructs. Figure 46B: J90 NFAT reporter assay to evaluate transgenic TCR functionality. J-TCRA3V, J-TCRA11V and J-TCRB7R cells were cocultured with monoallelic K562 cells lines pulsed with limiting dilutions of WT or mKRAS peptides. TCR signaling is indicated by eGFP expression. TCR avidity was determined by the mean peptide concentrations required to achieve 50% NFAT activation of transgenic TCR J90 NFAT reporter cells. Specific Activity (%) = (%GFPTest - %GFPMin) / (%GFPMax - %GFPMin) x 100. Figure 46C: FACS histogram plots demonstrating eGFP upregulation following 24 hr coculture of J-TCRA3V and J-TCRA11V cells with tumor cell lines harboring endogenous KRAS G12V mutations when modified to express the restricting HLA allele.

### DETAILED DESCRIPTION

### Definitions

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used.

It is also to be understood that the terminology used herein is for the purpose of describing particular embodiments only, and is not intended to be limiting.

The articles "a" and "an" are used herein to refer to one or to more than one (*i.e.,* to at least one) of the grammatical object of the article. By way of example, "an element" means one element or more than one element.

"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

"Activation," as used herein, refers to the state of a T cell that has been sufficiently stimulated to induce detectable cellular proliferation. Activation can also be associated with induced cytokine production, and detectable effector functions. The term "activated T cells" refers to, among other things, T cells that are undergoing cell division.

The term "antibody," as used herein, refers to an immunoglobulin molecule which specifically binds with an antigen. Antibodies can be intact immunoglobulins derived from natural sources or from recombinant sources and can be immunoreactive portions of intact immunoglobulins. Antibodies are typically tetramers of immunoglobulin molecules. The antibodies in the present invention may exist in a variety of forms including, for example, polyclonal antibodies, monoclonal antibodies, Fv, Fab and F(ab)₂, as well as single chain antibodies (scFv) and humanized antibodies (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426).

The term "antibody fragment" refers to a portion of an intact antibody and refers to the antigenic determining variable regions of an intact antibody. Examples of antibody fragments include, but are not limited to, Fab, Fab', F(ab')₂, and Fv fragments, linear antibodies, scFv antibodies, and multispecific antibodies formed from antibody fragments.

An "antibody heavy chain," as used herein, refers to the larger of the two types of polypeptide chains present in all antibody molecules in their naturally occurring conformations.

An "antibody light chain," as used herein, refers to the smaller of the two types of polypeptide chains present in all antibody molecules in their naturally occurring conformations. Kappa (x) and lambda (λ) light chains refer to the two major antibody light chain isotypes.

The term "synthetic antibody" as used herein, refers to an antibody which is generated using recombinant DNA technology, such as, for example, an antibody expressed by a bacteriophage as described herein. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using synthetic DNA or amino acid sequence technology which is available and well known in the art.

The term "antigen" or "Ag" as used herein is defined as a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present invention includes, but is not limited to, the use of partial nucleotide sequences of more than one gene and that these nucleotide sequences are arranged in various combinations to elicit the desired immune response. Moreover, a skilled artisan will understand that an antigen need not be encoded by a "gene" at all. It is readily apparent that an antigen can be generated, synthesized or derived from a biological sample. Such a biological sample can include, but is not limited to a tissue sample, a tumor sample, a cell or a biological fluid.

The term "anti-tumor effect" as used herein, refers to a biological effect observed when treating solid tumors which manifests in a variety of ways, including, for example, by a decrease in tumor volume, a decrease in the number of tumor cells, a decrease in the number of metastases, an increase in life expectancy, or amelioration of various physiological symptoms associated with the cancerous condition. An "anti-tumor effect" can also be manifested by the ability of the peptides, polynucleotides, cells and antibodies of the invention in prevention of the occurrence of tumor in the first place.

The term "auto-antigen" means, in accordance with the present invention, any self-antigen which is recognized by the immune system as being foreign. Auto-antigens comprise, but are not limited to, cellular proteins, phosphoproteins, cellular surface proteins, cellular lipids, nucleic acids, glycoproteins, including cell surface receptors.

The term "autoimmune disease" as used herein is defined as a disorder that results from an autoimmune response. An autoimmune disease is the result of an inappropriate and excessive response to a self-antigen. Examples of autoimmune diseases include but are not limited to, Addision's disease, alopecia areata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, pemphigus vulgaris, psoriasis, rheumatic fever, rheumatoid arthritis, sarcoidosis, scleroderma, Sjogren's syndrome, spondyloarthropathies, thyroiditis, vasculitis, vitiligo, myxedema, pernicious anemia, ulcerative colitis, among others.

As used herein, the term "autologous" refers to any material derived from the same individual to which it is later re-introduced into the individual.

"Allogeneic" refers to a graft or infusion derived from a different animal of the same species.

"Xenogeneic" refers to a graft or infusion derived from an animal of a different species.

The term "cancer" as used herein refers to a disease characterized by the rapid and uncontrolled growth of aberrant cells. Cancer cells can grow locally in its tissue of origin or spread through the bloodstream and lymphatic system to other parts of the body. Examples of various cancers include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like. In certain embodiments, the cancer is medullary thyroid carcinoma.

The term "chimeric antigen receptor" or "CAR," as used herein, refers to a non-MHC-restricted synthetic antigen-specific immune receptor that is engineered to be expressed on an immune effector cell. CARs are commonly used to engineer T cells, subsequently referred to as "CAR T cells". To make a CAR T cell, T cells are removed from a patient and modified to express one or more receptors specific to a particular antigen(s) of interest. CARs are delivered to patients generally as an intravenous infusion through a process referred to as adoptive cell transfer. In some embodiments, the CARs specifically bind a tumor-associated antigen or a tumor-specific antigen, for example. CARs may also comprise an intracellular activation domain, a transmembrane domain and an extracellular domain comprising a tumor-associated antigen binding domain or a tumor-specific antigen binding domain. In some aspects, CARs constructs comprise nucleic acids encoding (1) a single-chain variable fragment (scFv) targeting a clinically-relevant antigen derived from a monoclonal antibody, (2) a transmembrane domain, (3) one or more costimulatory domains, and (4) an ITAM-containing signaling domain such as CD3-zeta. In some embodiments, a CAR can target cancers by redirecting the specificity of a T cell expressing the CAR specific for a tumor-associated antigen or a tumor-specific antigen.

As used herein, the term "conservative sequence modifications" is intended to refer to amino acid modifications that do not significantly affect or alter the binding characteristics of the antibody containing the amino acid sequence. Such conservative modifications include amino acid substitutions, additions and deletions. Modifications can be introduced into an antibody of the invention by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within the CDR regions of an antibody can be replaced with other amino acid residues from the same side chain family and the altered antibody can be tested for the ability to bind antigens using the functional assays described herein.

"Co-stimulatory ligand," as the term is used herein, includes a molecule on an antigen presenting cell (e.g., an aAPC, dendritic cell, B cell, and the like) that specifically binds a cognate co-stimulatory molecule on a T cell, thereby providing a signal which, in addition to the primary signal provided by, for instance, binding of a TCR/CD3 complex with an MHC molecule loaded with peptide, mediates a T cell response, including, but not limited to, proliferation, activation, differentiation, and the like. A co-stimulatory ligand can include, but is not limited to, CD7, B7-1 (CD80), B7-2 (CD86), PD-L1, PD-L2, 4-1BBL, OX40L, inducible costimulatory ligand (ICOS-L), intercellular adhesion molecule (ICAM), CD30L, CD40, CD70, CD83, HLA-G, MICA, MICB, HVEM, lymphotoxin beta receptor, 3/TR6, ILT3, ILT4, HVEM, an agonist or antibody that binds Toll ligand receptor and a ligand that specifically binds with B7-H3. A co-stimulatory ligand also encompasses, inter alia, an antibody that specifically binds with a co-stimulatory molecule present on a T cell, such as, but not limited to, CD27, CD28, 4-1BB, OX40, CD30, CD40, PD-1, ICOS, lymphocyte function-associated antigen-1 (LFA-1), CD2, CD7, LIGHT, NKG2C, B7-H3, and a ligand that specifically binds with CD83.

A "co-stimulatory molecule" refers to the cognate binding partner on a T cell that specifically binds with a co-stimulatory ligand, thereby mediating a co-stimulatory response by the T cell, such as, but not limited to, proliferation. Co-stimulatory molecules include, but are not limited to an MHC class I molecule, BTLA and a Toll ligand receptor.

A "co-stimulatory signal", as used herein, refers to a signal, which in combination with a primary signal, such as TCR/CD3 ligation, leads to T cell proliferation and/or upregulation or downregulation of key molecules.

The term "CRISPR/CAS," "clustered regularly interspaced short palindromic repeats system," or "CRISPR" refers to DNA loci containing short repetitions of base sequences. Each repetition is followed by short segments of spacer DNA from previous exposures to a virus. Bacteria and archaea have evolved adaptive immune defenses termed CRISPR-CRISPR-associated (Cas) systems that use short RNA to direct degradation of foreign nucleic acids. In bacteria, the CRISPR system provides acquired immunity against invading foreign DNA via RNA-guided DNA cleavage.

In the type II CRISPR/Cas system, short segments of foreign DNA, termed "spacers" are integrated within the CRISPR genomic loci are transcribed and processed into short CRISPR RNA (crRNA). These crRNAs anneal to trans-activating crRNAs (tracrRNAs) and direct sequence-specific cleavage and silencing of pathogenic DNA by Cas proteins. Recent work has shown that target recognition by the Cas9 protein requires a "seed" sequence within the crRNA and a conserved dinucleotide-containing protospacer adjacent motif (PAM) sequence upstream of the crRNA-binding region.

To direct Cas9 to cleave sequences of interest, crRNA-tracrRNA fusion transcripts, hereafter referred to as "guide RNAs" or "gRNAs" may be designed, from human U6 polymerase III promoter. CRISPR/Cas mediated genome editing and regulation, highlighted its transformative potential for basic science, cellular engineering and therapeutics.

The term "CRISPRi" refers to a CRISPR system for sequence specific gene repression or inhibition of gene expression at the transcriptional level.

"Effective amount" or "therapeutically effective amount" are used interchangeably herein, each referring to an amount of a compound, formulation, material, or composition, as described herein effective to achieve a particular biological result or provides a therapeutic or prophylactic benefit. Such results may include, but are not limited to, anti-tumor activity as determined by any means suitable in the art.

The term "effector" as used herein refers to a molecule or a protein or fragment thereof that alters, moderates, interferes with or blocks a signaling axis or a transcription modulator or fragment thereof that provides benefit to immune cell function. In some embodiments, effectors comprise effectors for: (1) limiting toxicities (sensing/preventing CRS, sensing/preventing cerebral toxicities); (2) overcoming checkpoint inhibition; (3) recruiting endogenous immune responses and improving persistence; (4) improving penetrance; and/or (5) improving persistence; (6) shifting engineered T cells into a preferred phenotype that provides therapeutic benefit; (7) modulating gene expression that provides benefit to immune cell function. In some embodiments, the effector blocks the IL-6 or another inflammatory signaling axis. The effector may be a cytokine, an interleukin, an interferon, a chemokine, a receptor, a ligand, an antibody or antibody fragment, a bispecific antibody, a checkpoint inhibitor antagonist, an agonist, an enzyme, a regulatory element, a transcription factor, or a DNA binding domain of a transcription factor.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (*i.e.,* rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

As used herein "endogenous" refers to any material from or produced inside an organism, cell, tissue or system.

As used herein, the term "exogenous" refers to any material introduced from or produced outside an organism, cell, tissue or system.

The term "expand" as used herein refers to increasing in number, as in an increase in the number of T cells. In one embodiment, the T cells that are expanded ex vivo increase in number relative to the number originally present in the culture. In another embodiment, the T cells that are expanded ex vivo increase in number relative to other cell types in the culture.

The term "ex vivo" as used herein refers to cells that have been removed from a living organism, (e.g., a human) and propagated outside the organism (e.g., in a culture dish, test tube, or bioreactor).

The term "expression" as used herein refers to the transcription and/or translation of a particular nucleotide sequence driven by an endogenous or exogenous promoter.

"Expression vector" refers to a vector comprising a recombinant polynucleotide comprising expression control sequences. The expression control sequences may be operatively linked to a nucleotide sequence to be expressed. An expression vector comprises sufficient cis-acting elements for expression; other elements for expression can be supplied by the host cell or in an in vitro expression system. Expression vectors include all those known in the art, such as cosmids, plasmids (e.g., naked or contained in liposomes) and viruses (e.g., Sendai viruses, lentiviruses, retroviruses, adenoviruses, and adeno-associated viruses) that incorporate the recombinant polynucleotide.

The term "receptor fusion protein" as used herein refers to a fusion protein that comprises an antigen-binding domain. In some embodiments, the receptor fusion protein comprises an antigen-specific synNotch receptor. In some embodiments, the receptor fusion protein comprises a CAR.

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. For the most part, humanized antibodies are human immunoglobulins (recipient antibody) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies can comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.

"Fully human" refers to an immunoglobulin, such as an antibody, where the whole molecule is of human origin or consists of an amino acid sequence identical to a human form of the antibody.

The term "sequence identity" as used herein refers to the degree of sequence identity between two polymeric molecules particularly between two polypeptide molecules. When two amino acid sequences have the same residues at the same positions; *e.g.,* if a position in each of two polypeptide molecules is occupied by an Arginine, then they are identical at that position. The identity or extent to which two amino acid sequences have the same residues at the same positions in an alignment is often expressed as a percentage. The identity between two amino acid sequences is a direct function of the number of matching or identical positions; *e.g.,* if half (*e.g.,* five positions in a polymer ten amino acids in length) of the positions in two sequences are identical, the two sequences are 50% identical; if 90% of the positions (*e.g.,* 9 of 10), are matched or identical, the two amino acids sequences are 90% identical.

The term "immunoglobulin" or "Ig," as used herein is defined as a class of proteins, which function as antibodies. Antibodies expressed by B cells are sometimes referred to as the BCR (B cell receptor) or antigen receptor. The five members included in this class of proteins are IgA, IgG, IgM, IgD, and IgE. IgA is the primary antibody that is present in body secretions, such as saliva, tears, breast milk, gastrointestinal secretions and mucus secretions of the respiratory and genitourinary tracts. IgG is the most common circulating antibody. IgM is the main immunoglobulin produced in the primary immune response in most subjects. It is the most efficient immunoglobulin in agglutination, complement fixation, and other antibody responses, and is important in defense against bacteria and viruses. IgD is the immunoglobulin that has no known antibody function, but may serve as an antigen receptor. IgE is the immunoglobulin that mediates immediate hypersensitivity by causing release of mediators from mast cells and basophils upon exposure to allergen.

The term "immune response" as used herein is defined as a cellular response to an antigen that occurs when lymphocytes identify antigenic molecules as foreign and induce the formation of antibodies and/or activate lymphocytes to remove the antigen.

"Isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

The term "lentivirus" as used herein refers to viruses in a genus of the Retroviridae family. Lentiviruses are unique among the retroviruses in being able to infect non-dividing cells. In addition, they can deliver a significant amount of genetic information into the DNA of the host cell, so lentivirus-derived vectors are one of the most efficient gene delivery vectors available. HIV, SIV, and FIV are all examples of lentiviruses.

The term "modified" as used herein, refers to a changed state or structure of a molecule or cell of the invention. Molecules may be modified in many ways, including chemically, structurally, and functionally. Cells may be modified through the introduction of nucleic acids. In some embodiments, cells are modified to increase expression of a protein (e.g., FOXO1 or TCF7) relative to an unmodified cell. In some embodiments, cells are modified to decrease nuclear export of a protein (e.g., FOXO1) relative to an unmodified cell.

As used herein, a cell genetically modified to increase expression of a protein "relative to an unmodified cell" means the cell containing the genetic modification exhibits increased expression of the protein when compared to a cell of otherwise the same genetic background but does not contain the genetic modification, under the same conditions. Likewise, as used herein, a cell genetically modified to decrease nuclear export of a protein (e.g., FOXO1) "relative to an unmodified cell" means the cell containing the genetic modification exhibits decreased nuclear export of the protein when compared to a cell of otherwise the same genetic background but does not contain the genetic modification, under the same conditions.

A "mutation" as used herein is a change in a sequence (e.g., an amino acid sequence of a protein) resulting in an alteration from a given reference sequence (which may be, for example, a wildtype amino acid sequence of a protein). In some embodiments, the mutation is a deletion and/or insertion and/or duplication and/or substitution of at least one amino acid. In some embodiments, the mutation produces a change in at least one function and/or activity of a protein.

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

The term "overexpressed" or "overexpression" in reference to a tumor antigen indicates an increased level of expression compared to the level of expression in a normal cell from that tissue or organ. Patients having solid tumors or a hematological malignancy characterized by overexpression of a tumor antigen can be identified by standard assays known in the art.

"Parenteral" administration of an immunogenic composition includes, e.g., subcutaneous (s.c.), intravenous (i.v.), intramuscular (i.m.), or intrasternal injection, or infusion techniques.

The terms "nucleic acid" or "polynucleotide" as used interchangeably herein refer to polymers of nucleotides. Polynucleotides, which can be hydrolyzed into monomeric "nucleotides." The monomeric nucleotides can be hydrolyzed into nucleosides. As used herein, the term "polynucleotides" encompasses, but is not limited to, all nucleic acid sequences which are obtained by any means available in the art, including, without limitation, recombinant means, i.e., the cloning of nucleic acid sequences from a recombinant library or a cell genome, using ordinary cloning technology and PCR, and the like, and by synthetic means.

As used herein, the terms "peptide," "polypeptide," and "protein" are used interchangeably to refer to polymers of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short amino acid polymers, which also commonly are referred to in the art as peptides, oligopeptides and oligomers, for example, and to longer amino acid polymers, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

The term "promoter" as used herein refers to a DNA sequence recognized by the synthetic machinery of the cell, or introduced synthetic machinery, required to initiate the transcription of a specific polynucleotide sequence.

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue-specific manner.

The term "minimal promoter (P_{MIN})" as used herein refers to a TATA-box promoter element upstream of the inducible gene and sequence can be located between the -35 to +35 region with respect to transcription start site (Smale ST (2001) Core promoters: active contributors to combinatorial gene regulation. Genes Dev 15: 2503-2508). Eukaryotic promoters of protein-coding genes have one or more of three conserved sequences in this region (i.e. the TATA-box, initiator region, and downstream promoter element). A minimal promoter enables low basal leakiness in the absence of specific transcriptional activators and high expression when transcription activators are bound upstream of minimal promoter at their specific DNA binding sites. In some embodiments the minimal promoter is derived from the pGL4.23[luc2/minP]; Promega and validated in (Smole, A., Lainšček, D., Bezeljak, U., Horvat, S. & Jerala, R. A Synthetic Mammalian Therapeutic Gene Circuit for Sensing and Suppressing Inflammation. *Mol. Ther.* **25,** 102-119 (2017)). Alternative minimal promoters can be used, such as minimal TATA box promoter, minimal CMV promoter or minimal IL-2 promoter.

A "constitutive" promoter is a nucleotide sequence which, when operably linked with a polynucleotide which encodes or specifies a gene product, causes the gene product to be produced in a cell under most or all physiological conditions of the cell.

An "inducible" promoter is a nucleotide sequence which, when operably linked with a polynucleotide which encodes or specifies a gene product, causes the gene product to be produced in a cell substantially only in the presence or absence of certain conditions such as, for example, when an inducer (e.g., metal ions, alcohol, oxygen, etc.) which corresponds to the promoter is present in the cell.

A "tissue-specific" promoter is a nucleotide sequence which, when operably linked with a polynucleotide encodes or specified by a gene, causes the gene product to be produced in a cell substantially only if the cell is a cell of the tissue type corresponding to the promoter.

A "Sendai virus" refers to a genus of the Paramyxoviridae family. Sendai viruses are negative, single stranded RNA viruses that do not integrate into the host genome or alter the genetic information of the host cell. Sendai viruses have an exceptionally broad host range and are not pathogenic to humans. Used as a recombinant viral vector, Sendai viruses are capable of transient but strong gene expression.

A "signal transduction pathway" refers to the biochemical relationship between a variety of signal transduction molecules that play a role in the transmission of a signal from one portion of a cell to another portion of a cell, or across a cell membrane. The phrase "cell surface receptor" includes molecules and complexes of molecules capable of receiving a signal and transmitting signal across the plasma membrane of a cell.

"Single chain antibodies" refer to antibodies formed by recombinant DNA techniques in which immunoglobulin heavy and light chain fragments are linked to the Fv region via an engineered span of amino acids. Various methods of generating single chain antibodies are known, including those described in U.S. Pat. No. 4,694,778; Bird (1988) Science 242:423-442; Huston et al. (1988) Proc. Natl. Acad. Sci. USA 85:5879-5883; Ward et al. (1989) Nature 334:54454; Skerra et al. (1988) Science 242:1038-1041.

By the term "specifically binds," as used herein with respect to an antibody, is meant an antibody which recognizes a specific antigen, but does not substantially recognize or bind other molecules in a sample. For example, an antibody that specifically binds to an antigen from one species may also bind to that antigen from one or more species. But, such cross-species reactivity does not itself alter the classification of an antibody as specific. In another example, an antibody that specifically binds to an antigen may also bind to different allelic forms of the antigen. However, such cross reactivity does not itself alter the classification of an antibody as specific. In some instances, the terms "specific binding" or "specifically binding," can be used in reference to the interaction of an antibody, a protein, or a peptide with a second chemical species, to mean that the interaction is dependent upon the presence of a particular structure (e.g., an antigenic determinant or epitope) on the chemical species; for example, an antibody recognizes and binds to a specific protein structure rather than to proteins generally. If an antibody is specific for epitope "A", the presence of a molecule containing epitope A (or free, unlabeled A), in a reaction containing labeled "A" and the antibody, will reduce the amount of labeled A bound to the antibody.

By the term "stimulation," is meant a primary response induced by binding of a stimulatory molecule (*e.g.,* a TCR/CD3 complex) with its cognate ligand thereby mediating a signal transduction event, such as, but not limited to, signal transduction via the TCR/CD3 complex. Stimulation can mediate altered expression of certain molecules, such as downregulation of TGF-beta, and/or reorganization of cytoskeletal structures, and the like.

The term "subject" is intended to include living organisms in which an immune response can be elicited (e.g., mammals). A "subject" or "patient," as used therein, may be a human or non-human mammal. Non-human mammals include, for example, livestock and pets, such as sheep, cattle, pigs, cats, dogs, mice, and rats. Preferably, the subject is human.

As used herein, the term "T cell receptor" or "TCR" refers to a heterodimeric membrane protein that participates in the activation of T cells in response to the presentation of antigen. The TCR is responsible for recognizing antigens bound to major histocompatibility complex molecules. The TCR cannot transduce signals through the cell membrane, instead relying on the activity of CD3, another heterodimeric protein complexed with the TCR to form a TCR/CD3 complex. TCR is composed of a heterodimer of an alpha (α) and beta (β) chain, although in some cells the TCR consists of gamma (γ) and delta (δ) chains. TCRs may exist in alpha/beta and gamma/delta forms, which are structurally similar but have distinct anatomical locations and functions. Each chain is composed of two extracellular domains, a variable and constant domain. In some embodiments, the TCR may be modified on any cell comprising a TCR, including, for example, a helper T cell, a cytotoxic T cell, a memory T cell, regulatory T cell, natural killer T cell, and gamma delta T cell.

The term "therapeutic" as used herein describes a type of treatment and/or prophylaxis. A therapeutic effect is obtained by suppression, remission, or eradication of a disease state.

The terms "transfected" or "transformed" or "transduced" are used interchangeably herein to refer to a process by which exogenous nucleic acid is transferred or introduced into the host cell. A "transfected" or "transformed" or "transduced" cell is one which has been transfected, transformed or transduced with exogenous nucleic acid (*e.g.,* a lentiviral vector comprising a CAR, a plasmid, etc.). The cell includes the primary subject cell actually transfected, transformed, or transduced and its progeny.

To "treat" a disease as the term is used herein, means to reduce the frequency or severity of at least one sign or symptom of a disease or disorder experienced by a subject.

The phrase "under transcriptional control" or "operatively linked" as used herein means that the promoter is in the correct location and orientation in relation to a polynucleotide to control the initiation of transcription by RNA polymerase and expression of the polynucleotide.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, Sendai viral vectors, adenoviral vectors, adeno-associated virus vectors, retroviral vectors, lentiviral vectors, and the like.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

The present invention relates to methods for engineering immune cells by introducing genetic circuits that endow them with additional functionalities such as autonomous antigen-induced expression of immunomodulatory molecules limited to the tumor microenvironment. The present invention relates to a broadly applicable expression platform that combines a constitutive immune receptor or receptor subunit, receptor fusion protein or fluorescent marker and an inducible effector in a clinically relevant setting. This platform provides a potentially field-changing approach to managing severe side effects associated with adoptive cell therapy, including cytokine release syndrome and neurological toxicities, by autonomously regulated and in-situ expression of an effector that blocks the IL-6 or another inflammatory signaling axis. To extend adoptive cell therapy from hematologic malignancies into the treatment of solid tumors, various immune enhancing molecules were integrated into the developed system in combination with clinically validated immune receptors.

Currently no vector system exists in the field of immunotherapy that enables simultaneous expression of constitutive and inducible modules, in part due to complexities of engineering lentiviral-derived constructs, where a fine balance between desired functionalities, low basal leakiness, maintaining high levels of maximal production and prevention of immunogenicity is required, while maintaining the titer of the virus high enough to enable transduction of primary immune cells. Limitations of two-vector systems include non-homogenous cell populations in which only a part of the population receives both components. Consequently, laborious and often therapeutically non-feasible selection procedures are needed.

A disadvantage of having inducible and constitutive modules on two separate vectors is that it may result in non-homogeneous modification of engineered cells in which only a part of the population receives both components, resulting in a large part of modified cells being non-functional. Furthermore, even in double transduced cells, system behavior is unpredictable since different amounts of the inducible and constitutive modules may be integrated into the different genomic loci in the genome. Consequently, laborious and often therapeutically non-feasible selection procedures are often needed to obtain something that works as expected with a two-vector system. Thus, the all-in-one system of the invention has several advantages in that it enables the manufacture of a well-defined cell product with a more homogeneous transgene distribution.

Another advantage of the all-in-one system is its simplified modular assembly, where only one virus need be produced to achieve constitutive expression of a transgene, for example a CAR or TCR or TCR subunit, and inducible expression of an effector.

An important aspect for the use of intracellular effectors to modify functionalities of T cells, such as transcriptional factors or regulatory element or a DNA binding domain of a transcription factor, for example, T-bet, TCF7, EOMES, a Runx family member, BLIMP1, Bcl2, Bcl6, FoxP3, FoxO1, FoxO1-3A, or a portion thereof, or any other transcription factor providing benefit to immune cell function when overexpressed, is that in order to turn on expression of these effectors and modify functionality of the engineered cell, an inducible part is needed that carries genetic information for expression of such regulatory element in the same cell as constitutively expressed immune receptor, that drives endogenous signaling to activate inducible part. So, if the constitutive and inducible parts were on separate plasmids, a large majority of the transduced population would be non-functional. This is of course also true for secreted effectors, however since they are secreted, they can still have an effect, because they influence all the cells in the vicinity.

A further advantage of the all-in-one system is that it can be readily used, along with the functionalities that it has, as a donor template delivered with, for example, an adeno associated virus such as AAV6, for use with a CRISPR system, for example a CRISPR/Cas9 system, for homologous recombination-mediated knock-in into a desired locus, as described below. In some embodiments, the locus is the endogenous TCR of the cell, or an HLA or immune checkpoint or other molecule that negatively affects the immune cell function of interest of the engineered cell. This application would not be possible with a two-component system.

The present disclosure relates to a broadly applicable all-in-one lentiviral system with highly optimized transcriptional elements. This will improve the safety of adoptive cell therapy by treating CRS and neurological toxicities in-situ by therapeutic cells themselves. Alternatively, the modular system enables the engineering of T cells to augment efficacy of treating solid tumors using CAR or TCR modified immune cells in adoptive cancer immunotherapy.

Disclosed herein, but not part of the invention, is a viral vector comprising: a first polynucleotide comprising a constitutive promoter operably linked to a nucleic acid encoding at least one transgene, wherein one of the at least one transgenes encodes a receptor or receptor subunit, a receptor fusion protein, of a fluorescent marker; and a second polynucleotide comprising an inducible promoter operably linked to a nucleic acid encoding an effector.

The at least one transgene may encode a receptor fusion protein. The receptor fusion protein may comprise an antigen-specific synNotch receptor and an optional reporter. The receptor fusion protein further comprise a 2A peptide between the antigen-specific synNotch receptor and the reporter. The reporter may be BFP2. The antigen-specific synNotch receptor fusion protein may be a HER2-specific synNotch receptor fused to Gal4-VP64. Binding of the synNotch receptor to its target antigen HER2 may induce Notch1 cleavage which releases the transcription factor Gal4-VP64 from the fusion protein.

The transgene may encode a receptor or receptor subunit. The receptor or receptor subunit may be a TCR or TCR subunit. The receptor may be a TCR, wherein the TCR comprises an extracellular antigen binding domain capable of specifically binding a cognate antigen and an intracellular domain. The intracellular domain may drive expression of the effector when the antigen-binding domain of the TCR binds its cognate antigen by activating the inducible promoter operably linked to the effector.

The receptor or receptor subunit is-may be a cytokine receptor or cytokine receptor subunit. The cytokine receptor subunit may be IL-6Ra.

The receptor fusion protein may be a chimeric antigen receptor (CAR) comprising an extracellular antigen-binding domain capable of specifically binding a cognate antigen and an intracellular signaling domain, wherein the inducible promoter may be capable of driving expression of the effector when the antigen binding domain binds its target.

The receptor fusion protein may be a CAR comprising an extracellular antigen-binding domain capable of specifically binding a cognate antigen and an intracellular signaling domain. The intracellular signaling domain may drive expression of the effector when the antigen-binding domain of the CAR binds its cognate antigen by activating the inducible promoter operably linked to the effector. The inducible promoter may be capable of driving expression of the effector when the receptor binds its ligand.

The transgene may encode a fluorescent marker. The fluorescent marker may be mCherry.

The first polynucleotide, the second polynucleotide or both the first and the second polynucleotides may comprise an insulator sequence or a linker sequence.

The second polynucleotide may encode more than one effector, wherein each effector may be under the control of at least one promoter. The at least one promoter may be at least one inducible promoter.

The viral vector may be a retroviral vector, an adenoviral vector, or an adeno-associated viral vector. The retroviral vector may be a lentiviral vector.

The inducible promoter and the constitutive promoter may drive expression in the same direction, or in different directions.

The CAR may comprise an antigen binding domain, a transmembrane domain, and an intracellular signaling domain. The intracellular signaling domain may comprise one or more costimulatory signaling domains and an immunoreceptor tyrosine activation motif-containing signaling domain. The one or more costimulatory signaling domains may comprise the intracellular domain of a costimulatory molecule selected from the group consisting of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, and a ligand that specifically binds with CD83. The immunoreceptor tyrosine activation motif-containing signaling domain may comprise a CD3 zeta signaling domain.

Also provided is an engineered cell comprising the viral vector disclosed herein. In some embodiments, the engineered cell is an immune cell or precursor cell thereof. In some embodiments, an endogenous TCR or TCR subunit or HLA (Human leukocyte antigen) or immune checkpoint molecule (such as PD-1, TIM3, LAG3, CTLA4, 2B4, CD160, CD5) of the engineered cell has been knocked out using a CRISPR-related system, for example a CRISPR/Cas9 system, with non-homologous end joining or homologous recombination where the viral vector serves as a donor template molecule. In some embodiments, at least one TCR subunit is knocked out using a CRISPR-related system, a TALEN or a Zinc finger nuclease. In some embodiments, the CRISPR-related system is CRISPR/Cas9. In further embodiments, both subunits of the endogenous TCR are knocked out. In some embodiments, transgenes of interest may be introduced into the genome using CRISPR-related knock-in systems to place the specific transgenes downstream of naturally occurring promoters.

Also provided is a method for generating an engineered cell, comprising (1) obtaining a sample comprising immune cells or precursors of immune cells; (2) stimulating/activating the cells; (3) introducing the viral vector; (4) expanding the cells and (5) resting the cells. In some embodiments, the engineered cell is an immune cell or precursor cell thereof. In some embodiments, the method further comprises the step of knocking out the endogenous TCR or HLA (Human leukocyte antigen) or immune checkpoint molecule (such as PD-1, TIM3, LAG3, CTLA4, 2B4, CD160, CD5) or other molecule that negatively affects immune cell function of interest of the engineered cell has been knocked out using a CRISPR-related system, for example a CRISPR/Cas9 system with non-homologous end joining or homologous recombination where the viral vector serves as a donor template molecule.

The present invention provides for treating a patient having a disease, disorder or condition associated with expression of an antigen, the method comprising administering to the patient an effective amount of a composition comprising the engineered cell of any one of the previous embodiments. In some embodiments, the expression of the antigen is abnormal. In some embodiments, the engineered cell is an immune cell or precursor cell thereof. In further embodiments, the engineered cell is an NK cell, a B cell, a dendritic cell or a macrophage. In some embodiments, the composition further comprises a pharmaceutically acceptable carrier or adjuvant.

Disclosed herein is an engineered immune cell or engineered immune cell precursor cell, comprising: an insertion in a gene locus, wherein the insertion comprises a first polynucleotide comprising a constitutive promoter operably linked to a nucleic acid encoding at least one transgene, wherein one of the at least one transgenes encodes a receptor or receptor subunit, a receptor fusion protein or a fluorescent marker; and a second polynucleotide comprising an inducible promoter operably linked to a nucleic acid encoding an effector. The transgene may encode a receptor fusion protein. The receptor fusion protein may comprise an antigen-specific synNotch receptor and an optional reporter. The receptor fusion protein may further comprise a 2A peptide between the antigen-specific synNotch receptor and the reporter.

### Improved functional properties of cells

Described herein is the discovery that inducible expression of transcription factors improves functional qualities of CAR T cells in preclinical *in vivo* models. Also described herein are the development and validated cellular products that comprise inducible transcription factor expression in CAR T cells (iTF-CAR T). These transcription factors are TCF7 and an engineered variant of FOXO1 referred to as FOXO1-3A (Moffett, et al. Nat. Commun. 2017 Aug 30; 8(1):389; Kodama et al., Mol. Cell. Biol. 24, 7931-7940 (2004)). that is resistant to AKT mediated phosphorylation and hence insensitive to nuclear export, unlike the parental FOXO1 molecule. The results of experiments examining both examples demonstrate enhanced antigen-dependent proliferation and a less differentiated T cell state following repeated stimulations with cancer cells *in vitro.* CyTOF analysis of iTF-CAR T cells showed that antigen-inducible expression of a single TF favorably affected T cell markers of efficacy, including increased expression of IFNγ and T-bet, and reduced exhaustion marker TIGIT. iTF-CAR T cell activity was then tested *in vivo* in mice bearing tumor xenografts. CAR T cells that expressed a transcription factor FOXO1-3A in an antigen-dependent manner demonstrated drastically increased peak expansion in the blood when compared to control CAR T cells. Importantly, expansion was transient and iFOXO1-3A-CAR T cell numbers ultimately contracted to the normal levels after tumor was cleared. This finding underscores the importance of inducible TF expression, unlike constitutive expression, to reduce the risk of malignant transformation of the cellular product. It is reasonable to infer that improved expansion may enable the use of a lower CAR T cell dose while favorable phenotype and differentiation states may translate into enhanced anti-tumor responses and better persistence of the infusion product.

Thus, the methods using inducible FOXO1-3A described herein can potentially prolong or strengthen CAR T cells that would ultimately become exhausted. Activation induced expression of the transcription factor is safe and effective. Increased proliferation means fewer CAR T cells are needed to be infused into a patient, which will in term lower the cost and time to manufacture the cells.

### FOXO1

The present invention is based, at least in part, on the discovery that chimeric antigen receptor (CAR) T cells with induced FOXO1-3A expression upon binding of the CAR to its target exhibited properties making the cells favorable for use in adoptive cell therapy, such less differentiated status, increased proliferation, and increased activity. In particular, as described elsewhere herein, CAR T cells that inducibly expressed FOXO1-3A upon activation exhibited unparalleled in vivo proliferation. The cells contracted by day 50 and are therefore not leukemic.

The present invention relates to an engineered immune cell comprising a chimeric antigen receptor (CAR), wherein the immune cell is genetically modified to be capable of increased FOXO1 expression relative to an unmodified cell, and wherein the cell is genetically modified to decrease nuclear export of FOXO1 relative to an unmodified cell. Disclosed herein is a cell for use in adoptive cell therapy, wherein the cell comprises a CAR, wherein the cell is genetically modified to be capable of increased FOXO1 expression relative to an unmodified cell, and wherein the cell is genetically modified to decrease nuclear export of FOXO1 relative to an unmodified cell. Also disclosed herein is a cell for use in treating a disease, such as cancer, wherein the cell comprises a CAR, wherein the cell is genetically modified to be capable of increased FOXO1 expression relative to an unmodified cell, and wherein the cell is genetically modified to decrease nuclear export of FOXO1 relative to an unmodified cell. In some embodiments, the cell is a T cell. In some embodiments, the cell is a human T cell. In some embodiments, the cell is an autologous cell.

In some embodiments, the cell is a cell comprising a receptor capable of receiving an activating signal. For example the cell can be an immune cell (e.g., T cell, a TIL, NK cell, macrophage) and the receptor can be, for example, a T cell receptor or a chimeric antigen receptor (CAR).

The present invention relates to methods of programming differentiation status, increasing proliferation, and increasing activity of a cell (e.g., an immune cell, a T cell). The present invention also relates to methods of generating cells (e.g., immune cells, T cells) for use in adoptive cell therapy. The methods may include genetically modifying a cell to increase FOXO1 expression relative to an unmodified cell and genetically modifying a cell to decrease FOXO1 nuclear export relative to an unmodified cell.

The present invention provides an engineered cell comprising (a) a nucleic acid encoding a receptor capable of receiving an activating signal and (b) an inducible promoter operably linked to a nucleic acid encoding FOXO1-3A, wherein the inducible promoter is induced when the receptor receives an activating signal.

The present invention also provides a cell comprising a receptor capable of receiving an activating signal according to the present invention for use in methods of treating cancer in a subject in need thereof, wherein the cell comprises an inducible promoter operably linked to a nucleic acid encoding FOXO1-3A, and wherein the inducible promoter is induced when the receptor receives an activating signal. The method may include administering to a subject in need thereof a cell comprising a chimeric antigen receptor (CAR) that specifically binds a disease-associated antigen (e.g., a tumor antigen), wherein the cell is genetically modified to be capable of increased FOXO1 expression relative to an unmodified cell, and wherein the cell is genetically modified to decrease nuclear export of FOXO1 relative to an unmodified cell.

The cell can be genetically modified to increase or be capable of increased FOXO1 expression by a variety ways. For example, the cell can be modified to place FOXO1 expression under the control of a strong promoter (e.g., a constitutive promoter), the native FOXO1 promoter can be modified to increase its strength, multiple copies of FOXO1 can be introduced into the cell, or the cell can be modified to put FOXO1 expression under the control of an inducible promoter.

In some embodiments, the cell is modified to inducibly express FOXO1. In some embodiments, the cell is modified to transiently express FOXO1. In a particular embodiment, the cell is genetically modified to be capable of increased FOXO1 expression by modifying the cell so that expression of FOXO1 is under the control of an inducible promoter.

A nucleic acid encoding an inducible promoter operably linked to a nucleic acid encoding FOXO1 is introduced into a cell. The nucleic acid encoding an inducible promoter operably linked to a nucleic acid encoding FOXO1 can be introduced into the cell in a variety of ways. For example, an nucleic acid encoding an inducible promoter can be introduced into the FOXO1 genomic locus of the cell such that the expression of FOXO1 is under the control of the inducible promoter. As another example, a vector (e.g., a lentiviral vector) that contains nucleic acid encoding an inducible promoter operably linked to a nucleic acid encoding FOXO1 is introduced into the cell. In some embodiments, the genetic modifications are made to the cell using a CRISPR-related system. For example, a nucleic acid encoding an inducible promoter can be introduced to the FOXO1 locus such that FOXO1 expression is under the control of the inducible promoter via a CRISPR knock-in. In some embodiments, the CRISPR-related system is a CRISPR/Cas9 system. In some embodiments, the inducible promoter is an NFAT promoter. In some embodiments, the inducible promoter can be any promoter induced by activation of the cell (e.g., an NFAT promoter, a synthetic NFAT promoter, a NF-kB promoter, a CD69 promoter, a CD137 promoter, synthetic hypoxia-responsive element (HRE) promoter, or a PD-1 promoter).

The cell can be genetically modified to decrease nuclear export of FOXO1 in a variety of ways. FOXO1 is a transcription factor that is a target of insulin signaling and regulates metabolic homeostasis in response to oxidative stress. A forkhead transcription factor, FOXO1 is an activator of gluconeogenic genes such as PEPCK1, G6P, and insulin-like growth factor-binding protein 1. Insulin treatment triggers phosphorylation of FOXO1 via a phosphatidylinositol 3-kinase (PI3K)-Akt pathway. Phosphorylation inactivates FOXO1 by exporting FOXO1 from the nucleus to the cytoplasm. Thus, in some embodiments, nuclear export of FOXO1 is decreased by mutating one or more Akt phosphorylation sites on FOXO1 to decrease or abolish Akt phosphorylation of FOXO1. In some embodiments, nuclear export of FOXO1 is decreased by mutating threonine at position 24 to alanine (T24-A24). In some embodiments, nuclear export of FOXO1 is decreased by mutating serine at position 256 to alanine (S256-A256). In some other embodiments, nuclear export of FOXO1 is decreased by mutating serine at position 319 to alanine (S319-A319). In some other embodiments, nuclear export of FOXO1 is decreased by mutating threonine at position 24 (T24) to alanine (T24-A24), mutating serine at position 256 to alanine (S256-A256), and mutating serine at position 319 to alanine (S319-A319). In a particular embodiment, the cell is genetically modified to decrease nuclear export of FOXO1 by introducing to the cell a nucleic acid encoding FOXO1-3A. In one embodiment, the cell is genetically modified to decrease nuclear export of FOXO1 by introducing to the cell a nucleic acid encoding the amino acid sequence set forth in SEQ ID NO: 31.

A nucleic acid encoding FOXO1-3A or encoding an amino acid sequence of SEQ ID NO: 31 can be introduced into a cell in a variety of ways. For example, the native FOXO1 sequence in the genome of the cell can be mutated to introduce S319-A319, T24-A24, and S256-A256 mutations, using for example, a CRISPR-related system. As another example, a vector (e.g., a lentiviral vector)containing a nucleic acid encoding FOXO1-3A or the amino acid sequence of SEQ ID NO: 31 can be introduced into the cell.

In some embodiments, nucleic acid encoding FOXO1 in a cell is modified by introducing mutations via a CRISPR-related system. In one embodiment, a cell is modified to express FOXO1-3A using a CRISPR-related system. In one embodiment, a FOXO1 locus in a cell is modified to introduce one or more mutations to the nucleic acid encoding FOXO1, such that at least one amino acid residue in the FOXO1 protein is mutated. In some embodiment, a mutation to the amino acid sequence of FOXO1 is introduced at one or more positions selected from: position 24, 256, and 319. In one embodiment, a mutation is introduced at positions 24, 256, and 319. In another embodiment, a FOXO1 locus in a cell is modified to introduce a T24-A24 mutation in the amino acid sequence of FOXO1. In another embodiment, a FOXO1 locus in a cell is modified to introduce a S256-A256 mutation in the amino acid sequence of FOXO1. In another embodiment, a FOXO1 locus in a cell is modified to introduce a S319-A319 in the amino acid sequence of FOXO1. The mutation(s) can be introduced using a CRISPR-related system. In some embodiments, the CRISPR-related system is a CRISPR/Cas9 system.

The cell can also be genetically modified to knock out an endogenous receptor (e.g., a T cell receptor (TCR)). In some embodiments of the invention, an endogenous TCR of the engineered cell has been knocked out using a CRISPR-related system. In some embodiments, the CRISPR-related system is a CRISPR/Cas9 system. Also provided are some embodiments with a CRISPR-related system-mediated knock-in of the designed expression cassette into the TCR or HLA (or other relevant loci) to make the introduced receptor-mediated signaling construct the exclusive activator of the system and to make engineered cells universal, respectively. In some embodiments, the designed expression cassette is encoded in a donor template. In some embodiments, the donor template is encoded in a viral vector. In some embodiments, the viral vector is an adeno-associated viral vector. In further embodiments, the adeno-associated viral vector is AAV6. In some embodiments, the CRISPR-related system is a CRISPR/Cas9 system. In some embodiments, such a system may prevent, decrease or treat recombinant TCR-mediated graft-versus-host disease.

Thus, in some embodiments, the method further comprises the step of knocking out the endogenous TCR or HLA (Human leukocyte antigen) or immune checkpoint molecule (such as PD-1, TIM3, LAG3, CTLA4, 2B4, CD160, CD5) or other molecule that negatively affects immune cell function of interest of the engineered cell has been knocked out using a CRISPR/Cas9 system with non-homologous end joining or homologous recombination where the viral vector serves as a donor template molecule.

In order to render response of the system exclusive to the specific antigen triggered signaling through introduced CAR or TCR, endogenous TCR may be knocked out using a CRISPR/Cas9 system. This may greatly increase safety of engineered T cells over existing NFAT-inducible systems, which will not be able to respond to unpredicted potential antigens through native TCRs.

### TCF7

Further, the present invention is based, at least in part, on the discovery that chimeric antigen receptor (CAR) T cells with induced TCF7 expression upon binding of the CAR to its target exhibited properties making the cells favorable for use in adoptive cell therapy, such less differentiated status, increased proliferation, and increased activity

The present invention relates to an engineered immune cell comprising a chimeric antigen receptor (CAR), wherein the immune cell is genetically modified to be capable of increased TCF7 expression relative to an unmodified cell. Disclosed herein is a cell for use in adoptive cell therapy, wherein the cell comprises a CAR, wherein the cell is genetically modified to be capable of increased TCF7 expression relative to an unmodified cell. Also disclosed herein is a cell for use in treating cancer, wherein the cell comprises a CAR, wherein the cell is genetically modified to be capable of increased TCF7 expression relative to an unmodified cell. In some embodiments, the cell is a T cell. In some embodiments, the cell is a human T cell. In some embodiments, the cell is an autologous cell.

In some embodiments, the cell is a cell comprising a receptor capable of receiving an activating signal. For example the cell can be an immune cell (e.g., T cell, a TIL, NK cell, macrophage) and the receptor can be, for example, a T cell receptor or a chimeric antigen receptor (CAR).

The present invention provides an engineered cell comprising (a) a nucleic acid encoding a receptor capable of receiving an activating signal and (b) an inducible promoter operably linked to a nucleic acid encoding TCF7, wherein the inducible promoter is induced when the receptor receives an activating signal.

The present invention relates to methods of programming differentiation status, increasing proliferation, and increasing activity of a cell (e.g., an immune cell, a T cell). The present invention also relates to methods of generating cells (e.g., immune cells, T cells) for use in adoptive cell therapy. The methods include genetically modifying a cell to increase TCF7 expression relative to an unmodified cell.

The present invention also provides a cell comprising a receptor capable of receiving an activating signal for use in a method of treating cancer in a subject in need thereof, wherein the cell comprises an inc ucible promoter operably linked to a nucleic acid encoding TCF7, and wherein the inducible promoter is induced when the receptor receives an activating signal. The method may include administering to a subject in need thereof a cell comprising a chimeric antigen receptor (CAR) that specifically binds a disease-associated antigen (e.g., a tumor antigen), wherein the cell is genetically modified to be capable of increased TCF7 expression relative to an unmodified cell.

The cell can be genetically modified to increase or be capable of increased TCF7 expression by a variety ways. For example, the cell can be modified to put TCF7 expression under the control of a strong promoter (e.g., a constitutive promoter), the native TCF7 promoter can be modified to increase its strength, multiple copies of TCF7 can be introduced into the cell, or the cell can be modified to put TCF7 expression under the control of an inducible promoter. In some embodiments, the inducible promoter can be any promoter induced by activation of the cell (e.g., an NFAT promoter, a synthetic NFAT promoter, a NF-kB promoter, a CD69 promoter, a CD137 promoter, synthetic hypoxia-responsive element (HRE) promoter, or a PD-1 promoter).

In a particular embodiment, the cell is genetically modified to be capable of increased TCF7 expression by introducing to the cell a nucleic acid encoding an inducible promoter operably linked to a nucleic acid encoding TCF7. In one embodiment, the nucleic acid encoding TCF7 is the amino acid sequence set forth in SEQ ID NO: 31. The nucleic acid encoding an inducible promoter operably linked to a nucleic acid encoding TCF7 can be introduced into the cell in a variety of ways. For example, an nucleic acid encoding an inducible promoter can be introduced into the TCF7 genomic locus of the cell such that the expression of FOXO1 is under the control of the inducible promoter. As another example, a vector (e.g., a lentiviral vector) that contains nucleic acid encoding an inducible promoter operably linked to a nucleic acid encoding TCF7 is introduced into the cell. In some embodiments, the genetic modifications are made to the cell using a CRISPR-related system. For example, a nucleic acid encoding an inducible promoter can be introduced to the TCF7 locus such that TCF7 expression is under the control of the inducible promoter via a CRISPR knock-in. In some embodiments, the CRISPR-related system is a CRISPR/Cas9 system. In some embodiments, the inducible promoter is an NFAT promoter.

The cell can also be genetically modified to knock out an endogenous receptor (e.g., a T cell receptor (TCR)). In some embodiments of the invention, an endogenous TCR of the engineered cell has been knocked out using a CRISPR-related system. In some embodiments, the CRISPR-related system is a CRISPR/Cas9 system. Also provided are some embodiments with a CRISPR-related system-mediated knock-in of the designed expression cassette into the TCR or HLA (or other relevant loci) to make the introduced receptor-mediated signaling construct the exclusive activator of the system and to make engineered cells universal, respectively. In some embodiments, the designed expression cassette is encoded in a donor template. In some embodiments, the donor template is encoded in a viral vector. In some embodiments, the viral vector is an adeno-associated viral vector. In further embodiments, the adeno-associated viral vector is AAV6. In some embodiments, the CRISPR-related system is a CRISPR/Cas9 system. In some embodiments, such a system may prevent, decrease or treat recombinant TCR-mediated graft-versus-host disease.

Thus, in some embodiments, the method further comprises the step of knocking out the endogenous TCR or HLA (Human leukocyte antigen) or immune checkpoint molecule (such as PD-1, TIM3, LAG3, CTLA4, 2B4, CD160, CD5) or other molecule that negatively affects immune cell function of interest of the engineered cell has been knocked out using a CRISPR/Cas9 system with non-homologous end joining or homologous recombination where the viral vector serves as a donor template molecule.

To make a response of the system exclusive to the specific antigen triggered signaling through introduced CAR or TCR, endogenous TCR may be knocked out using a CRISPR/Cas9 system. This may greatly increase safety of engineered T cells over existing NFAT-inducible systems, which will not be able to respond to unpredicted potential antigens through native TCRs.

### Methods of screening

Described elsewhere herein are results demonstrating that the single vector system (or Uni-Vect system) can be used as a robust tool to develop and characterize immune receptors such as CARs or TCRs. Specifically it enables characterization of expression levels, function, specificity and functional avidity.

Accordingly, disclosed herein, but not part of the invention, are methods of screening or identifying a receptor (e.g., an immune receptor) specific to an antigen. The methods include (a) contacting a cell (e.g., a T cell) with an antigen, wherein the cell comprises a single viral vector comprising a first polynucleotide comprising a constitutive promoter operably linked to a nucleic acid encoding a candidate receptor or subunit thereof; and a second polynucleotide comprising an inducible promoter operably linked to a nucleic acid encoding a reporter protein; and (b) determining the level of expression of the reporter protein in the cell; wherein the candidate receptor is identified as a receptor specific to the antigen if the level of expression of the reporter protein is increased relative to a reference level.

Also disclosed herein but not part of the invention, is a method of screening and identifying an antigen that specifically binds a receptor. The method includes (a) contacting a cell with a candidate antigen, wherein the cell comprises a single viral vector comprising a first polynucleotide comprising a constitutive promoter operably linked to a nucleic acid encoding a receptor or subunit thereof; and a second polynucleotide comprising an inducible promoter operably linked to a nucleic acid encoding a reporter protein; and (b) determining the level of expression of the reporter protein in the cell; wherein the candidate antigen is identified as an antigen that specifically binds the receptor if the level of expression of the reporter protein is increased relative to a reference level.

Also disclosed herein but not part of the invention, is a method of screening and identifying an antigen that specifically binds a receptor, the method comprising
(a) contacting a cell with a candidate antigen, wherein the cell comprises a receptor capable of receiving an activating signal, and wherein the cell comprises a single viral vector comprising a first polynucleotide comprising a constitutive promoter operably linked to a nucleic acid encoding a first reporter protein; and a second polynucleotide comprising an inducible promoter operably linked to a nucleic acid encoding a second reporter protein; and
(b) determining the level of expression of the second reporter protein in the cell; wherein the candidate antigen is identified as an antigen that specifically binds the receptor if the level of expression of the second reporter protein is increased relative to a reference level. The first and second reporter proteins may be different. The expression of the first reporter protein may be used to select for cells that have been transduced with the vector.

Also disclosed herein but not part of the invention, is a method of identifying a receptor that specifically binds an antigen, the method comprising
(a) contacting a cell with an antigen, wherein the cell comprises a candidate receptor capable of receiving an activating signal, and wherein the cell comprises a single viral vector comprising a first polynucleotide comprising a constitutive promoter operably linked to a nucleic acid encoding a first reporter protein; and a second polynucleotide comprising an inducible promoter operably linked to a nucleic acid encoding a second reporter protein; and
(b) determining the level of expression of the second reporter protein in the cell;
wherein the candidate receptor is identified as a receptor that specifically binds the antigen if the level of expression of the second reporter protein is increased relative to a reference level. The first and second reporter proteins may be different. The expression of the first reporter protein may be used to select for cells that have been transduced with the vector.

The reference level can be a control or pre-determined level. For example, the reference level can be the level of expression of the reporter protein in the cell when the cell is not contacted with any antigen. The reporter protein can be any detectable protein, for example, a fluorescent protein such as mCherry or eGFP. The inducible promoter can be any promoter capable of driving expression of the reporter protein when the cell (e.g., T cell) is activated. The inducible promoter may be an NFAT promoter.

The method may further comprise characterizing the specificity and/or functionality such as functional avidity of the receptor.

The cell may further be genetically modified to knockdown or knockout an endogenous receptor, e.g., an endogenous T cell receptor. In order to render the response of the system exclusive to the specific antigen triggered signaling through introduced CAR or TCR, endogenous TCR may be knocked out using a CRISPR/Cas9 system. An endogenous TCR of the engineered cell may have been knocked out using a CRISPR-related system. The CRISPR-related system may be a CRISPR/Cas9 system. Also disclosed herein is a CRISPR-related system-mediated knock-in of the designed expression cassette into the TCR or HLA (or other relevant loci) to make the introduced receptor-mediated signaling construct the exclusive activator of the system and to make engineered cells universal, respectively. The designed expression cassette may be encoded in a donor template. The donor template may be encoded in a viral vector. The viral vector may be an adeno-associated viral vector. The adeno-associated viral vector may be AAV6. The donor template may be a synthetic double stranded DNA that is introduced through electroporation of such DNA template and Cas9 RNP (Cas9 protein complexed with specific sgRNA guides)

In some embodiments, the candidate receptor is a chimeric antigen receptor (CAR). For example, the single-vector system can be utilized to combine constitutive expression of antigen-targeting CARs with inducible expression of a reporter protein (e.g., NFAT-inducible eGFP expression) (Figure 28). CAR function, specificity and functional avidity can be assessed. In some other embodiments, the candidate receptor is a T cell receptor (TCR). For example, the system can be utilized to combine constitutive expression of TCRs with inducible expression of a reporter protein.

In some embodiments, the antigen is a tumor antigen. In some other embodiments, the antigen is a peptide ligand (e.g., a peptide ligand presented by HLA, such as peptide ligands presented by HLA on tumor cells). In some embodiments, the antigen is expressed on the surface of a cell.

The single vector system may also be used to screen for immunomodulatory molecules. Also disclosed herein, but not part of the invention, are methods of identifying immunomodulators. The methods include (a) contacting a cell (e.g., a T cell) with an antigen, wherein the T cell comprises a single viral vector comprising a first polynucleotide comprising a constitutive promoter operably linked to a nucleic acid encoding a chimeric antigen receptor (CAR); and a second polynucleotide comprising an inducible promoter operably linked to a nucleic acid encoding a candidate immunomodulatory molecule; and (b) determining the level of activity the cell; wherein the candidate molecule is identified as an immunomodulatory molecule if the level of activity is increased relative to a reference level. The activity measured can be any activity indicative of functionalities of CAR T cells, for example, the capacity to lyse target cells (e.g., tumor cells). The reference level can be a control or pre-determined level. For example, when measuring lytic activity against a target cell line, a control can be the level of activity of the CAR T cell when contacted with a cell line that does not express the antigen that is targeted by the CAR.

### Promoters

In some embodiments, the constitutive promoter that is operably linked to the transgene is an EF-1alpha promoter or any other constitutive promoter that drives constitutive expression in immune cells (such as PGK-1, UBC, CMV, CAGG, SV40 or pan-hematopoietic promoter, such as vav). In some embodiments, the promoter is a human promoter.

In some embodiments, the inducible promoter that is operably linked to the polynucleotide encoding the effector is any promoter the activation of which is responsive to a transcriptional factor that is increased when immune cells are specifically activated or localized to a given microenvironment (could be tumor microenvironment), such as an NFAT promoter, a STAT3-sensing promoter, a CD69 promoter, a CD137 promoter, or a hypoxia-responsive element promoter. In some embodiments, the inducible promoter further comprises a minimal promoter operably linked to an inducible enhancer. In some embodiments, the promoter is a human promoter. NFAT-inducible promoter can be exchanged with any inducible promoter that specifically binds specific transcriptional factors. Hypoxia responsive element, for example, instead of NFAT responsive element, would bind HIF transcriptional factors and activate the system in hypoxic conditions. Without wishing to be bound by theory, if NFAT responsive element is present, it needs a signal from TCR/CAR or other immune receptor that induces NFAT signaling.

In further embodiments, the inducible promoter is linked to a minimal promoter (P_{MIN}). In some embodiments the minimal promoter is derived from the pGL4.23[luc2/minP]; Promega and validated in (Smole, A., Lainšček, D., Bezeljak, U., Horvat, S. & Jerala, R. A Synthetic Mammalian Therapeutic Gene Circuit for Sensing and Suppressing Inflammation. *Mol. Ther.* **25,** 102-119 (2017)). Alternative minimal promoters can be used, such as minimal TATA box promoter, minimal CMV promoter or minimal IL-2 promoter. In some embodiments, the minimal promoter is optimized for a desired level or rate of transcription. In some embodiments, an inducible promoter linked to a minimal promoter enables a medium ON state while remaining silent in the absence of a trigger signal. This configuration is of high value when no leakiness is acceptable due to potential toxicities, for example when using immune modulators such as cytokines. In some embodiments, an inducible promoter is linked to a minimal promoter that enables a high ON state. In some embodiments the system's properties are tailored and are optimized to the specific therapeutic needs. For the inducible expression of an immune augmenting molecule, a system with minimal leakage is required to prevent toxicities. Without wishing to be bound by theory, the level of maximal production might not be that important in the case of an immune augmenting molecule, or might even be deleterious if too high, given the potent immune-enhancing effect. For inducible expression of blocking antibodies, the system is optimized for high maximal expression (ON state).

### Effector

The effector may be a cytokine, an interleukin, an interferon, a chemokine, a receptor, a ligand, an antibody or antibody fragment, a bispecific antibody, a BiTE, a checkpoint inhibitor antagonist, an agonist, an enzyme, a regulatory element, a transcription factor, or a DNA binding domain of a transcription factor. Transcriptional factors usually comprise DNA binding domain and the domain that modifies transcription (activates or represses). Part of transcriptional factor means that the DNA binding domain of the specific transcriptional factor may be used, which would act as a competitive inhibitor of endogenous transcriptional factor - for example, to suppress activity of transcriptional factor that leads to the exhaustion of engineered cells. On the other hand, the portion of NFAT that has transcriptional activation activity and the domain that enables entry into the nucleus only upon Ca-mediated signaling may be fused (meaning the DNA binding domains that enables binding to NFAT DNA binding site is removed) and then this part is fused with a different DNA binding domain (which could be also designable DNA binding domain such as ZFN, TALEN or Cas9) to rewire endogenous NFAT signaling to activation or repression (if we add repression domain such as KRAB) of any gene.

In some embodiments, the cytokine is an interleukin. In some embodiments, the interleukin is IL-2, IL-7, IL-12, IL-15, IL-18, or IL-21.

In some embodiments, the interferon is IFNα or IFNβ.

In some embodiments, the antibody or antibody fragment or bispecific antibody is anti-IL-6, anti-IL-6R, anti-IL-6Ra, anti-TNFalpha, anti-IL-1, anti-PD1, anti-CD25, anti-CD3, anti-CD20, anti-CD40 agonistic antibody, anti-IL-8, anti-MCP1, anti-MIP-1, anti-TGFβ, anti-CD47, anti-CSF1R, anti-CD28, anti-TIGIT, anti-VEGFR, anti-FAP or any antibody that blocks cytokines, depletes or alters a particular cell subset, or alters immunity, survival, trafficking or signaling or increases efficacy of immune cells.

In some embodiments, the checkpoint inhibitor antagonist is anti-PD-L1, anti-PD-1, anti-CTLA4, anti-LAG3, anti-TIM3, anti-2B4, or anti-CD160, anti-CD5.

In some embodiments, the bispecific antibody comprises functional domains of any of but not limited to anti-IL-6, anti-IL-6R, anti-IL-6Ra, anti-TNFalpha, anti-IL-1, anti-PD1, anti-CD25, anti-CD3, anti-CD20, anti-CD40 agonistic antibody, anti-IL-8, anti-MCP1, anti-MIP-1, anti-TGFβ, anti-CD47, anti-CSF1R, anti-CD28, anti-TIGIT, anti-VEGFR, anti-FAP or any antibody that blocks cytokines, depletes or alters a particular cell subset, or alters immunity, survival, trafficking or signaling or increases efficacy of immune cells or anti-PD-L1, anti-PD-1, anti-CTLA4, anti-LAG3, anti-TIM3, anti-2B4, or anti-CD160, anti-CD5 or any other immune checkpoint molecule.

In some embodiments, the chemokine is CCL5 (RANTES), XCL-1, XCL-2, CCR-7, CCL-19, CCL-21.

In some embodiments, a transcription factor or regulatory element or a part of transcription factor (derived from the transcription factor) is T-bet, TCF7, EOMES, a Runx family member, BLIMP1, Bcl2, Bcl6, FoxP3, FoxO1, FoxO1-3A, or a portion thereof, or any other transcription factor providing benefit to immune cell function when overexpressed. In some embodiments, the enzyme is heparinase, collagenase, or a metalloproteinase. In further embodiments, the Runx family member is Runx1 or Runx3.

In some embodiments, the effectors are used in combinations of at least two effectors from all the above mentioned groups linked through 2A peptide or IRES.

### Dead Cas9 as an effector

In some embodiments, the effector is dead Cas9 (dCas9), a version of Cas9 that does not have catalytic activity but retains target-specific binding to DNA-based transcriptional modulators. For example, dCas9-repressor domain fusion protein represses a targeted gene, whereas dCas9-activation domain fusion protein activates a targeted gene. In this system, dCas9 fusion protein would be expressed in an inducible manner, to limit immunogenicity, while sgRNA or multiplex sgRNAs targeting multiple genes may be expressed in a constitutive manner through RNA polymerase III promoter or from the LTR of a lentiviral vector. Examples of transcriptional repression domains are KRAB or DNMT3A, while examples of transcriptional activation domains are VP16, VP64, VPR or SunTag-based activators. This system allows shifting of T cell phenotype and function to a desired state based on targeted upregulation and/or downregulation of genes involved in the function of engineered immune cells. Such a system is feasible only in an all-in-one system.

### Chimeric Antigen Receptors

The invention relates to compositions and methods for treating a disease. In some embodiments, the disease is a cancer, including but not limited to hematologic malignancies and solid tumors. The invention also encompasses methods of treating and preventing a disease, such as certain types of cancer, including primary and metastatic cancer, as well as cancers that are refractory or resistant to conventional chemotherapy. The methods comprise administering to a patient in need of such treatment or prevention a therapeutically or prophylactically effective amount of a T cell transduced to express a chimeric antigen receptor (CAR). Provided are compositions and methods comprising a CAR. CARs are molecules that combine antibody-based specificity for a desired antigen (e.g., tumor antigen) with a T cell receptor-activating intracellular domain to generate a chimeric protein that exhibits a specific anti-tumor cellular immune activity.

In some embodiments of any of the methods above, the methods result in a measurable reduction in tumor size or evidence of disease or disease progression, complete response, partial response, stable disease, increase or elongation of progression free survival, increase or elongation of overall survival, or reduction in toxicity.

In one embodiment, the CAR of the invention comprises an antigen binding domain, a transmembrane domain, and an intracellular signaling domain. In some embodiments, the intracellular signaling domain comprises one or more costimulatory signaling domains and an immunoreceptor tyrosine activation motif-containing signaling domain. In further embodiments, the immunoreceptor tyrosine activation motif-containing signaling domain comprises a CD3 zeta signaling domain.

In some embodiments, the one or more costimulatory signaling domains comprise the intracellular domain of a costimulatory molecule selected from the group consisting of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, and a ligand that specifically binds with CD83.

The CAR of the invention can be engineered to comprise an extracellular domain having an antigen binding domain that targets tumor antigen fused to an intracellular signaling domain comprising the T cell antigen receptor complex zeta chain (e.g., CD3 zeta). An exemplary tumor antigen B cell antigen is CD19 because this antigen is expressed on malignant B cells. However, the invention is not limited to targeting CD19. Rather, the invention includes any tumor antigen binding moiety. In some aspects, CARs comprise: (1) a single-chain variable fragment (scFv) targeting a clinically-relevant antigen derived from a monoclonal antibody, (2) a transmembrane domain, (3) one or more costimulatory domains, and (4) an ITAM-containing signaling domain such as CD3-zeta.

In one embodiment, the CAR of the invention comprises a CD137 (4-1BB) signaling domain. For example, inclusion of the CD137 (4-1BB) signaling domain significantly increased CAR mediated activity and *in vivo* persistence of CAR T cells compared to an otherwise identical CAR T cell not engineered to express CD137 (4-1BB). However, the invention is not limited to a specific CAR. Rather, any CAR that targets a desired antigen, for example a tumor antigen, can be used in the present invention. Compositions and methods of making and using CARs have been described in PCT/US11/64191.

The present invention provides a T cell genetically modified to express a chimeric antigen receptor (CAR) comprising an extracellular and intracellular domain. Compositions and methods of making CARs have been described in PCT/US11/64191.

The extracellular domain comprises a target-specific binding element otherwise referred to as an antigen binding domain. In some embodiments, the extracellular domain also comprises a hinge domain. In one embodiment, the intracellular domain comprises a costimulatory signaling domain and a CD3 zeta chain portion. The costimulatory signaling domain refers to a portion of the CAR comprising the intracellular domain of a costimulatory molecule. Costimulatory molecules are cell surface molecules other than antigen receptors or their ligands that are required for an efficient response of lymphocytes to antigen.

Between the extracellular domain and the transmembrane domain of the CAR, or between the cytoplasmic domain and the transmembrane domain of the CAR, there may be incorporated a spacer domain. In this context, the term "spacer domain" generally means any oligo- or polypeptide that functions to link the transmembrane domain to, either the extracellular domain or, the cytoplasmic domain in the polypeptide chain. A spacer domain may comprise up to 300 amino acids, preferably 10 to 100 amino acids and most preferably 25 to 50 amino acids.

The present invention includes retroviral and lentiviral vector constructs expressing a CAR that can be directly transduced into a cell. The present invention also includes an RNA construct that can be directly transfected into a cell. A method for generating mRNA for use in transfection involves in vitro transcription (IVT) of a template with specially designed primers, followed by polyA addition, to produce a construct containing 3' and 5' untranslated sequence ("UTR"), a 5' cap and/or Internal Ribosome Entry Site (IRES), the gene to be expressed, and a polyA tail, typically 50-2000 bases in length. RNA so produced can efficiently transfect different kinds of cells. In one embodiment, the template includes sequences for the CAR.

Preferably, the CAR comprises an extracellular domain, a transmembrane domain and a cytoplasmic domain. The extracellular domain and transmembrane domain can be derived from any desired source of such domains. In some instances, the hinge domain of the CAR of the invention comprises the CD8α hinge domain.

In one embodiment, the CAR of the invention comprises a target-specific binding element otherwise referred to as an antigen binding domain. The choice of moiety depends upon the type and number of ligands that define the surface of a target cell. For example, the antigen binding domain may be chosen to recognize a ligand that acts as a cell surface marker on target cells associated with a particular disease state. Thus examples of cell surface markers that may act as ligands for the antigen moiety domain in the CAR of the invention include those associated with viral, bacterial and parasitic infections, autoimmune disease and cancer cells.

In one embodiment, the CAR of the invention can be engineered to target a tumor antigen of interest by way of engineering a desired antigen binding domain that specifically binds to an antigen on a tumor cell.

Tumor antigens are proteins that are produced by tumor cells that elicit an immune response, particularly T-cell mediated immune responses. The selection of the antigen binding domain of the invention will depend on the particular type of cancer to be treated. Tumor antigens are well known in the art and include, for example, a glioma-associated antigen, carcinoembryonic antigen (CEA), β-human chorionic gonadotropin, alphafetoprotein (AFP), lectin-reactive AFP, thyroglobulin, RAGE-1, MN-CA IX, human telomerase reverse transcriptase, RU1, RU2 (AS), intestinal carboxyl esterase, mut hsp70-2, M-CSF, prostase, prostate-specific antigen (PSA), PAP, NY-ESO-1, LAGE-1a, p53, prostein, PSMA, Her2/neu, survivin and telomerase, prostate-carcinoma tumor antigen-1 (PCTA-1), MAGE, ELF2M, neutrophil elastase, ephrinB2, CD22, insulin growth factor (IGF)-I, IGF-II, IGF-I receptor, IL13Rα2 and mesothelin. The antigens discussed herein are merely included by way of example. The list is not intended to be exclusive and further examples will be readily apparent to those of skill in the art.

In one embodiment, the tumor antigen comprises one or more antigenic cancer epitopes associated with a malignant tumor. Malignant tumors express a number of proteins that can serve as target antigens for an immune attack. These molecules include but are not limited to tissue-specific antigens such as MART-1, tyrosinase and GP100 in melanoma and prostatic acid phosphatase (PAP) and prostate-specific antigen (PSA) in prostate cancer. Other target molecules belong to the group of transformation-related molecules such as the oncogene HER-2/Neu/ErbB-2. Yet another group of target antigens are onco-fetal antigens such as carcinoembryonic antigen (CEA). In B-cell lymphoma the tumor-specific idiotype immunoglobulin constitutes a truly tumor-specific immunoglobulin antigen that is unique to the individual tumor. B-cell differentiation antigens such as CD19, CD20 and CD37 are other candidates for target antigens in B-cell lymphoma. Some of these antigens (CEA, HER-2, CD19, CD20) have been used as targets for passive immunotherapy with monoclonal antibodies with limited success.

In some embodiments, the antigen is CD22, CD123, CD33, CD79b, ROR-1, CAIX, mesothelin, CMET, CD70, CLL-1, IL1RA, CD38, BCMA, CS-1, MUC1, CD2, CD5, CD7, CD30, CCR4, CD4, CD8, CD3, CD37, NKIG2D or FLT-3.

The type of tumor antigen referred to in the invention may also be a tumor-specific antigen (TSA) or a tumor-associated antigen (TAA). A TSA is unique to tumor cells and does not occur on other cells in the body. A TAA associated antigen is not unique to a tumor cell and instead is also expressed on a normal cell under conditions that fail to induce a state of immunologic tolerance to the antigen. The expression of the antigen on the tumor may occur under conditions that enable the immune system to respond to the antigen. TAAs may be antigens that are expressed on normal cells during fetal development when the immune system is immature and unable to respond or they may be antigens that are normally present at extremely low levels on normal cells but which are expressed at much higher levels on tumor cells.

Non-limiting examples of TSA or TAA antigens include the following: Differentiation antigens such as MART-1/MelanA (MART-I), gp100 (Pmel 17), tyrosinase, TRP-1, TRP-2 and tumor-specific multilineage antigens such as MAGE-1, MAGE-3, BAGE, GAGE-1, GAGE-2, p15; overexpressed embryonic antigens such as CEA; overexpressed oncogenes and mutated tumor-suppressor genes such as p53, Ras, HER-2/neu; unique tumor antigens resulting from chromosomal translocations; such as BCR-ABL, E2A-PRL, H4-RET, IGH-IGK, MYL-RAR; and viral antigens, such as the Epstein Barr virus antigens EBVA and the human papillomavirus (HPV) antigens E6 and E7. Other large, protein-based antigens include TSP-180, MAGE-4, MAGE-5, MAGE-6, RAGE, NY-ESO, p185erbB2, p180erbB-3, c-met, nm-23H1, PSA, TAG-72, CA 19-9, CA 72-4, CAM 17.1, NuMa, K-ras, beta-Catenin, CDK4, Mum-1, p 15, p 16, 43-9F, 5T4, 791Tgp72, alpha-fetoprotein, beta-HCG, BCA225, BTAA, CA 125, CA 15-3\CA 27.29\BCAA, CA 195, CA 242, CA-50, CAM43, CD68\P1, CO-029, FGF-5, G250, Ga733\EpCAM, HTgp-175, M344, MA-50, MG7-Ag, MOV18, NB/70K, NY-CO-1, RCAS1, SDCCAG16, TA-90\Mac-2 binding protein\cyclophilin C-associated protein, TAAL6, TAG72, TLP, and TPS.

In a preferred embodiment, the antigen binding domain portion of the CAR targets an antigen that includes but is not limited to CD19, CD20, CD22, ROR1, Mesothelin, CD33/IL3Ra, c-Met, PSMA, Glycolipid F77, EGFRvIII, GD-2, NY-ESO-1 TCR, MAGE A3 TCR, IL13Rα2 and the like.

Depending on the desired antigen to be targeted, the CAR of the invention can be engineered to include an appropriate antigen-binding moiety specific to the desired target.

The antigen-binding domain can be any domain that specifically binds to the antigen including but not limited to monoclonal antibodies, polyclonal antibodies, synthetic antibodies, human antibodies, humanized antibodies, and fragments thereof. In some instances, it is beneficial for the antigen-binding domain to be derived from the same species in which the CAR will ultimately be used. For example, for use in humans, it may be beneficial for the antigen-binding domain of the CAR to comprise a human antibody or fragment thereof. Thus, in one embodiment, the antigen binding domain portion comprises a human antibody or a fragment thereof. Alternatively, in some embodiments, a non-human antibody is humanized, where specific sequences or regions of the antibody are modified to increase similarity to an antibody naturally produced in a human.

In one embodiment of the present invention, a plurality of types of CARs is expressed on the surface of a T cell. The different types of CAR may differ in their antigen-binding domain. That is, in one embodiment, the different types of CARs each bind a different antigen. In one embodiment, the different antigens are markers for a specific tumor. For example, in one embodiment, the different types of CARs each bind to a different antigen, where each antigen is expressed on a specific type of tumor. Examples of such antigens are discussed elsewhere herein.

In some embodiments, the CAR comprises a transmembrane domain that is fused to the extracellular domain of the CAR. In one embodiment, the transmembrane domain that naturally is associated with one of the domains in the CAR is used. In some instances, the transmembrane domain can be selected or modified by amino acid substitution to avoid binding of such domains to the transmembrane domains of the same or different surface membrane proteins to minimize interactions with other members of the receptor complex.

The transmembrane domain may be derived either from a natural or a synthetic source. Where the source is natural, the domain may be derived from any membrane-bound or transmembrane protein. Transmembrane regions of particular use in this invention may be derived from (i.e. comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154, ICOS. Alternatively the transmembrane domain may be synthetic, in which case it will comprise predominantly hydrophobic residues such as leucine and valine. Preferably a triplet of phenylalanine, tryptophan and valine will be found at each end of a synthetic transmembrane domain. Optionally, a short oligo- or polypeptide linker, preferably between 2 and 10 amino acids in length may form the linkage between the transmembrane domain and the cytoplasmic signaling domain of the CAR. A glycine-serine doublet provides a particularly suitable linker.

The cytoplasmic domain or otherwise the intracellular signaling domain of the CAR of the invention is responsible for activation of at least one of the normal effector functions of the immune cell in which the CAR has been placed in. The term "effector function" refers to a specialized function of a cell. Effector function of a T cell, for example, may be cytolytic activity or helper activity including the secretion of cytokines. Thus the term "intracellular signaling domain" refers to the portion of a protein which transduces the effector function signal and directs the cell to perform a specialized function. While usually the entire intracellular signaling domain can be employed, in many cases it is not necessary to use the entire domain. To the extent that a truncated portion of the intracellular signaling domain is used, such truncated portion may be used in place of the intact signaling domain as long as it transduces the effector function signal. The term intracellular signaling domain is thus meant to include any truncated portion of the intracellular signaling domain sufficient to transduce the effector function signal.

In one embodiment, the effector function of the cell is dependent upon the binding of a plurality of types of CARs to their targeted antigen. For example, in one embodiment, binding of one type of CAR to its target is not sufficient to induce the effector function of the cell.

Primary cytoplasmic signaling sequences regulate activation of the TCR/CD3 complex either in a stimulatory way, or in an inhibitory way. Primary cytoplasmic signaling sequences that act in a stimulatory manner may contain signaling motifs which are known as immunoreceptor tyrosine-based activation motifs or ITAMs.

Examples of ITAM containing primary cytoplasmic signaling sequences that are of particular use in the invention include those derived from TCR zeta, FcR gamma, FcR beta, CD3 gamma, CD3 delta, CD3 epsilon, CD5, CD22, CD79a, CD79b, and CD66d. It is particularly preferred that cytoplasmic signaling molecule in the CAR of the invention comprises a cytoplasmic signaling sequence derived from CD3 zeta.

In one embodiment, the cytoplasmic domain of the CAR comprises the CD3-zeta signaling domain by itself or combined with any other desired cytoplasmic domain(s) useful in the context of the CAR of the invention. For example, the cytoplasmic domain of the CAR can comprise a CD3-zeta chain portion and a costimulatory signaling domain. The costimulatory signaling domain refers to a portion of the CAR comprising the intracellular domain of a costimulatory molecule.

The cytoplasmic signaling sequences within the cytoplasmic signaling portion of the CAR of the invention may be linked to each other in a random or specified order. Optionally, a short oligo- or polypeptide linker, preferably between 2 and 10 amino acids in length may form the linkage. A glycine-serine doublet provides a particularly suitable linker.

In one embodiment, the cytoplasmic domain comprises the signaling domain of CD3-zeta. In another embodiment, the cytoplasmic domain comprises the signaling domain of CD3-zeta and the signaling domain of 4-1BB. In one embodiment of the present invention, a plurality of types of CARs is expressed on a cell, where the different types of CAR may vary in their cytoplasmic domain. In one embodiment, at least one type of CAR comprises the CD3 zeta domain, while at least one type of CAR comprises a costimulatory domain, for example the 4-1BB domain. However, the different types of CARs are not limited by any particular cytoplasmic domain.

In one embodiment, the T cell genetically modified to express a CAR is further engineered or edited using CRISPR/Cas.

### Extracellular Domain

In another embodiment, the extracellular domain can include any portion of an antibody that binds to antigen including, but not limited to, the antigen binding domain of a synthetic antibody, human antibody, humanized antibody, single domain antibody, single chain variable fragments, and fragments thereof. In some instances, it is beneficial for the extracellular domain to be derived from the same species in which the chimeric membrane protein will ultimately be used in. For example, for use in humans, it may be beneficial for the extracellular domain of the chimeric membrane protein to comprise a human antibody or fragment thereof. Thus, in one embodiment, the extracellular domain portion comprises a human antibody or a fragment thereof.

For in vivo use of antibodies in humans, it may be preferable to use human antibodies. Completely human antibodies are particularly desirable for therapeutic treatment of human subjects. Human antibodies can be made by a variety of methods known in the art including phage display methods using antibody libraries derived from human immunoglobulin sequences, including improvements to these techniques. See, also, U.S. Pat. Nos. 4,444,887 and 4,716,111; and PCT publications WO 98/46645, WO 98/50433, WO 98/24893, WO98/16654, WO 96/34096, WO 96/33735, and WO 91/10741. A human antibody can also be an antibody wherein the heavy and light chains are encoded by a nucleotide sequence derived from one or more sources of human DNA.

Alternatively, in some embodiments, a non-human antibody is humanized, where specific sequences or regions of the antibody are modified to increase similarity to an antibody naturally produced in a human. In one embodiment, the antigen binding domain portion is humanized.

A humanized antibody has one or more amino acid residues introduced into it from a source which is nonhuman. These nonhuman amino acid residues are often referred to as "import" residues, which are typically taken from an "import" variable domain. Thus, humanized antibodies comprise one or more CDRs from nonhuman immunoglobulin molecules and framework regions from human. Humanization of antibodies is well-known in the art and can essentially be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody, i.e., CDR-grafting (EP 239,400; PCT Publication No. WO 91/09967; and U.S. Pat. Nos. 4,816,567; 6,331,415; 5,225,539; 5,530,101; 5,585,089; 6,548,640. In such humanized chimeric antibodies, substantially less than an intact human variable domain has been substituted by the corresponding sequence from a nonhuman species. In practice, humanized antibodies are typically human antibodies in which some CDR residues and possibly some FR residues are substituted by residues from analogous sites in rodent antibodies. Humanization of antibodies can also be achieved by veneering or resurfacing (EP 592,106; EP 519,596; Padlan, 1991, Molecular Immunology, 28(4/5):489-498; Studnicka et al., Protein Engineering, 7(6):805-814 (1994); and Roguska et al., PNAS, 91:969-973 (1994)) or chain shuffling (U.S. Pat. No. 5,565,332).

The choice of human variable domains, both light and heavy, to be used in making the humanized antibodies is to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987). Another method uses a particular framework derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993).

Antibodies can be humanized with retention of high affinity for the target antigen and other favorable biological properties. Humanized antibodies can be prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind the target antigen. In this way, FR residues can be selected and combined from the recipient and import sequences so that the desired antibody characteristic, such as increased affinity for the target antigen, is achieved. In general, the CDR residues are directly and most substantially involved in influencing antigen binding.

A "humanized" antibody retains a similar antigenic specificity as the original antibody. However, using certain methods of humanization, the affinity and/or specificity of binding of the antibody for human CD3 antigen may be increased using methods of "directed evolution," as described by Wu et al., J. Mol. Biol., 294:151 (1999).

In one embodiment, the antibody is a synthetic antibody, human antibody, a humanized antibody, single chain variable fragment, single domain antibody, an antigen binding fragment thereof, and any combination thereof.

### Intracellular Domain

The intracellular domain or cytoplasmic domain comprises a costimulatory signaling domain. The costimulatory signaling domain refers to an intracellular domain of a costimulatory molecule. Costimulatory molecules are cell surface molecules other than antigen receptors or their ligands that are required for an efficient response of lymphocytes to antigen.

### Other Domains of the Chimeric Antigen Receptor

Between the extracellular domain and the transmembrane domain of the chimeric antigen receptor, or between the cytoplasmic domain and the transmembrane domain of the chimeric antigen receptor, there may be incorporated a spacer domain.

In some embodiments, the chimeric membrane protein further comprises a transmembrane domain. In some embodiments, the chimeric membrane protein further comprises a hinge domain. In one embodiment, the RNA encoding the chimeric membrane protein further comprises a transmembrane and hinge domain, such as a CD28 transmembrane domain and a CD8alpha hinge domain.

### Therapy

As a first form of gene transfer therapy to gain approval by FDA, immunotherapy with chimeric antigen receptor (CAR) T cells specific for the CD19 B lymphocyte molecule revolutionized treatment of certain blood cancers. CAR T cells kill large amounts of tumor cells in a short time. This antigen-specific killing can be associated with an uncontrolled release of inflammatory mediators and general disruption of homeostasis referred to as cytokine release syndrome (CRS). This can cause life-threatening adverse events and if CRS is manifested in the central nervous system (CNS), this can lead to fatal neurological toxicities. Systemic administration of Tocilizumab (a monoclonal antibody that blocks the interleukin-6 receptor (IL-6R)) that blocks the IL-6 signaling axis is currently used to manage such adverse events. Early detection and administration are needed to prevent damage, but controlling neurological toxicities is inefficient because the blood-brain barrier prevents entry of the drug into the CNS. The use of CD19 CARs targeting hematological malignancies (or CAR T cells targeting other antigens) in combination with in-situ secretion of an engineered antibody that blocks IL-6 signaling and/or additional inflammatory mediators such as TNFalpha or IL-1beta will likely increase the safety of cancer immunotherapy in which CRS triggered by adoptive cell therapy is a major problem. Without wishing to be bound by theory, autonomous and prompt delivery of the drug by therapeutic cells themselves might suppress CRS even before it becomes clinically detectable. Since therapeutic cells that enter CNS are responsible for neurological toxicities, this could solve the issue of poor CNS penetrance with Tocilizumab (or any other antibody that blocks IL-6 signaling or signaling by other inflammatory cyto- and chemokines), which represents a paradigm changing approach to control toxicities related to successful immunotherapy.

The engineered cells described herein may be included in a composition for therapy either separately or in combination. The composition may include a pharmaceutical composition and further include a pharmaceutically acceptable carrier. A therapeutically effective amount of the pharmaceutical composition comprising the engineered cells described herein may be administered.

In some embodiments, the engineered cell may be administered with a second agent. In some embodiments, the engineered cell and the second agent are administered to the patient simultaneously or sequentially. In some embodiments, the engineered cell and the second agent are administered to the patient in the same composition. In some embodiments, the engineered cell and the second agent are administered to the patient as separate compositions.

The present disclosure further relates to stimulating a T cell-mediated immune response to a target cell or tissue in a subject comprising administering to a subject an effective amount of an engineered cell. The engineered cells described herein may be administered to induce lysis of the target cell or tissue, such as where the induced lysis results from antibody-dependent cell-mediated cytotoxicity (ADCC).

The present disclosure further relates to adoptive cell transfer therapy comprising administering a population of engineered cells described herein to a subject in need thereof to prevent or treat an immune reaction that is adverse to the subject.

Also described herein is treating a disease or condition associated with enhanced immunity in a subject comprising administering a population of engineered cells described herein to a subject in need thereof.

The modified T cells generated as described herein possess T cell function. Further, the engineered cells described herein can be administered to an animal, preferably a mammal, even more preferably a human, to suppress an immune reaction, such as those common to autoimmune diseases such as diabetes, psoriasis, rheumatoid arthritis, multiple sclerosis, graft versus host disease (GVHD), enhancing allograft tolerance induction, transplant rejection, and the like. In addition, the cells of the present invention can be used for the treatment of any condition in which a diminished or otherwise inhibited immune response, especially a cell-mediated immune response, is desirable to treat or alleviate the disease. The disclosure further relates to treating a condition, such as an autoimmune disease, in a subject, comprising administering to the subject a therapeutically effective amount of a pharmaceutical composition comprising a population of modified T cells.

In some embodiments for the treatment of an autoimmune disease, or any condition in which a diminished or otherwise inhibited immune response, especially a cell-mediated immune response, is desirable to treat or alleviate the disease, the modified T cell or the engineered immune cell or precursor cell thereof may be a Treg or a myeloid derived suppressor cell (MDSC). In some embodiments, the effector is an immunosuppressive molecule, such as IL-10, or an anti-inflammatory blocker, such as anti-TNF, anti-IL1 or anti-IL6.

Examples of autoimmune disease include but are not limited to, Acquired Immunodeficiency Syndrome (AIDS, which is a viral disease with an autoimmune component), alopecia areata, ankylosing spondylitis, antiphospholipid syndrome, autoimmune Addison's disease, autoimmune hemolytic anemia, autoimmune hepatitis, autoimmune inner ear disease (AIED), autoimmune lymphoproliferative syndrome (ALPS), autoimmune thrombocytopenic purpura (ATP), Behcet's disease, cardiomyopathy, celiac sprue-dermatitis hepetiformis; chronic fatigue immune dysfunction syndrome (CFIDS), chronic inflammatory demyelinating polyneuropathy (CIPD), cicatricial pemphigold, cold agglutinin disease, crest syndrome, Crohn's disease, Degos' disease, dermatomyositis-juvenile, discoid lupus, essential mixed cryoglobulinemia, fibromyalgia-fibromyositis, Graves' disease, Guillain-Barre syndrome, Hashimoto's thyroiditis, idiopathic pulmonary fibrosis, idiopathic thrombocytopenia purpura (ITP), IgA nephropathy, insulin-dependent diabetes mellitus, juvenile chronic arthritis (Still's disease), juvenile rheumatoid arthritis, Meniere's disease, mixed connective tissue disease, multiple sclerosis, myasthenia gravis, pernacious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomena, Reiter's syndrome, rheumatic fever, rheumatoid arthritis, sarcoidosis, scleroderma (progressive systemic sclerosis (PSS), also known as systemic sclerosis (SS)), Sjogren's syndrome, stiff-man syndrome, systemic lupus erythematosus, Takayasu arteritis, temporal arteritis/giant cell arteritis, ulcerative colitis, uveitis, vitiligo and Wegener's granulomatosis.

The engineered cells generated as described herein can also be modified and used to treat inflammatory disorders. Examples of inflammatory disorders include but are not limited to, chronic and acute inflammatory disorders. Examples of inflammatory disorders include Alzheimer's disease, asthma, atopic allergy, allergy, atherosclerosis, bronchial asthma, eczema, glomerulonephritis, graft vs. host disease, hemolytic anemias, osteoarthritis, sepsis, stroke, transplantation of tissue and organs, vasculitis, diabetic retinopathy and ventilator induced lung injury.

In another embodiment, the engineered cells described herein may be used for the manufacture of a medicament for the treatment of an immune response in a subject in need thereof.

Cells of the invention can be administered in dosages and routes and at times to be determined in appropriate pre-clinical and clinical experimentation and trials. Cell compositions may be administered multiple times at dosages within these ranges. Administration of the cells of the invention may be combined with other methods useful to treat the desired disease or condition as determined by those of skill in the art.

The cells of the invention to be administered may be autologous or allogenic with respect to the subject undergoing therapy.

The administration of the cells or compositions of the invention may be carried out in any convenient manner known to those of skill in the art. The cells or compositions of the present invention may be administered to a subject by injection, transfusion, implantation or transplantation. The compositions described herein may be administered to a patient transarterially, subcutaneously, intradermally, intratumorally, intranodally, intramedullary, intramuscularly, by intravenous (i.v.) injection, or intraperitoneally. In other instances, the cells of the invention are injected directly into a site of inflammation in the subject, a local disease site in the subject, a lymph node, an organ, a tumor, and the like.

The cells or compositions described herein can also be administered using any number of matrices. The present invention utilizes such matrices within the novel context of acting as an artificial lymphoid organ to support, maintain, or modulate the immune system, typically through modulation of T cells. Accordingly, the present invention can utilize those matrix compositions and formulations which have demonstrated utility in tissue engineering. Accordingly, the type of matrix that may be used in the compositions, devices and methods of the invention is virtually limitless and may include both biological and synthetic matrices. In one particular example, the compositions and devices set forth by U.S. Pat. Nos. 5,980,889; 5,913,998; 5,902,745; 5,843,069; 5,787,900; or 5,626,561 are utilized. Matrices comprise features commonly associated with being biocompatible when administered to a mammalian host. Matrices may be formed from natural and/or synthetic materials. The matrices may be non-biodegradable in instances where it is desirable to leave permanent structures or removable structures in the body of an animal, such as an implant; or biodegradable. The matrices may take the form of sponges, implants, tubes, telfa pads, fibers, hollow fibers, lyophilized components, gels, powders, porous compositions, or nanoparticles. In addition, matrices can be designed to allow for sustained release of seeded cells or produced cytokine or other active agent. In certain embodiments, the matrix of the present invention is flexible and elastic, and may be described as a semisolid scaffold that is permeable to substances such as inorganic salts, aqueous fluids and dissolved gaseous agents including oxygen.

A matrix is used herein as an example of a biocompatible substance. However, the current invention is not limited to matrices and thus, wherever the term matrix or matrices appears these terms should be read to include devices and other substances which allow for cellular retention or cellular traversal, are biocompatible, and are capable of allowing traversal of macromolecules either directly through the substance such that the substance itself is a semi-permeable membrane or used in conjunction with a particular semi-permeable substance.

### CRISPR

In some embodiments of the invention, an endogenous TCR of the engineered cell has been knocked out using a CRISPR-related system. In some embodiments, the CRISPR-related system is a CRISPR/Cas9 system.

Also provided are some embodiments with a CRISPR-related system-mediated knock-in of the designed expression cassette into the TCR or HLA (or other relevant loci) to make the introduced receptor-mediated signaling construct the exclusive activator of the system and to make engineered cells universal, respectively. In some embodiments, the designed expression cassette is encoded in a donor template. In some embodiments, the donor template is encoded in a viral vector. In some embodiments the viral vector is an adeno-associated viral vector. In further embodiments, the adeno-associated viral vector is AAV6. In some embodiments, the CRISPR-related system is a CRISPR/Cas9 system.

In some embodiments, such a system may prevent, decrease or treat recombinant TCR-mediated graft-versus-host disease.

Also disclosed herein is an engineered immune cell or engineered immune cell precursor cell, comprising: an insertion in a gene locus, wherein the insertion comprises a first polynucleotide comprising a constitutive promoter operably linked to a nucleic acid encoding at least one transgene, wherein one of the at least one transgenes encodes a receptor or receptor subunit, a receptor fusion protein or a fluorescent marker; and a second polynucleotide comprising an inducible promoter operably linked to a nucleic acid encoding an effector. The transgene may encode a receptor fusion protein.

The receptor fusion protein may comprise an antigen-specific synNotch receptor fusion protein and an optional reporter. The receptor fusion protein may further comprise a 2A peptide between the antigen-specific synNotch receptor and the reporter. The reporter may be BFP2. The antigen-specific synNotch receptor fusion protein may be a HER2-specific synNotch receptor fused to Gal4-VP64. Binding of the synNotch receptor to its target antigen HER2 may induce Notch1 cleavage which releases the transcription factor Gal4-VP64 from the fusion protein.

The transgene may be a receptor or receptor subunit. The receptor or receptor subunit may be a cytokine receptor or a cytokine receptor subunit. The cytokine receptor subunit may be IL-6Ra.

The receptor fusion protein may be a CAR comprising an extracellular antigen-binding domain capable of specifically binding a cognate antigen and an intracellular signaling domain.

The transgene may be a fluorescent marker. The fluorescent marker may be mCherry.

The first polynucleotide, the second polynucleotide or both the first and the second polynucleotides may comprise an insulator sequence or a linker sequence.

The insertion may be encoded in a donor template. The donor template may be encoded in a viral vector. The gene locus may encode a TCR subunit, an HLA or an immune checkpoint molecule. The immune checkpoint molecule may PD-1, TIM3, LAG3, CTLA4, 2B4, CD160 or CD5.

The viral vector may be an adeno-associated viral vector. The adeno-associated viral vector may be AAV6. Also disclosed herein is an endogenous TCR or HLA (Human leukocyte antigen) or immune checkpoint molecule (such as PD-1, TIM3, LAG3, CTLA4, 2B4, CD160, CD5) or other molecule that negatively affects immune cell function of interest of the engineered cell has been knocked out using a CRISPR system, for example a CRISPR/Cas9 system, with non-homologous end joining or homologous recombination where designed construct serves a donor template molecule.

The gene locus may encode TCR or HLA (Human leukocyte antigen) or immune checkpoint molecule (such as PD-1, TIM3, LAG3, CTLA4, 2B4, CD160, CD5) or other molecule that negatively affects immune cell function of interest of the engineered cell.

The insertion in a gene locus is may be mediated by a CRISPR-related system. The CRISPR-related system may be CRISPR/Cas9.

### Pharmaceutical compositions

Pharmaceutical compositions of the present invention may comprise an engineered immune cell as described herein, in combination with one or more pharmaceutically or physiologically acceptable carriers, diluents, adjuvants or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an immunologically effective amount", "an anti-immune response effective amount", "an immune response-inhibiting effective amount", or "therapeutic amount" is indicated, the precise amount of the compositions of the present invention to be administered can be determined by a physician with consideration of individual differences in age, weight, immune response, and condition of the patient (subject). It can generally be stated that a pharmaceutical composition comprising the modified T cells described herein may be administered at a dosage of 10⁴ to 10⁹ cells/kg body weight, preferably 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988). The optimal dosage and treatment regime for a particular patient can readily be determined by one skilled in the art of medicine by monitoring the patient for signs of disease and adjusting the treatment accordingly.

In certain embodiments, it may be desired to administer activated T cells to a subject and then subsequently redraw blood (or have an apheresis performed), activate T cells therefrom according to the present invention, and reinfuse the patient with these activated and expanded T cells. This process can be carried out multiple times every few weeks. In certain embodiments, T cells can be activated from blood draws of from 10 ml to 400 ml. In certain embodiments, T cells are activated from blood draws of 20 ml, 30 ml, 40 ml, 50 ml, 60 ml, 70 ml, 80 ml, 90 ml, or 100 ml. Not to be bound by theory, using this multiple blood draw/multiple reinfusion protocol, may select out certain populations of T cells.

In certain embodiments of the present invention, cells expanded and modified using the methods described herein, or other methods known in the art where T cells are expanded to therapeutic levels, are administered to a patient in conjunction with (e.g., before, simultaneously or following) any number of relevant treatment modalities, including but not limited to treatment with agents such as antiviral therapy, cidofovir and interleukin-2, Cytarabine (also known as ARA-C) or natalizumab treatment for MS patients or efalizumab treatment for psoriasis patients or other treatments for PML patients. In further embodiments, the T cells of the invention may be used in combination with chemotherapy, radiation, immunosuppressive agents, such as cyclosporin, azathioprine, methotrexate, mycophenolate, and FK506, antibodies, or other immunoablative agents such as CAM PATH, anti-CD3 antibodies or other antibody therapies, cytoxin, fludaribine, cyclosporin, FK506, rapamycin, mycophenolic acid, steroids, FR901228, cytokines, and irradiation. These drugs inhibit either the calcium dependent phosphatase calcineurin (cyclosporine and FK506) or inhibit the p70S6 kinase that is important for growth factor induced signaling (rapamycin). (Liu et al., Cell 66:807-815, 1991; Henderson et al., Immun. 73:316-321, 1991; Bierer et al., Curr. Opin. Immun. 5:763-773, 1993). In a further embodiment, the cell compositions of the present invention are administered to a patient in conjunction with *(e.g.,* before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH. In another embodiment, the cell compositions of the present invention are administered following B-cell ablative therapy such as agents that react with CD20, e.g., Rituxan. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

The dosage of the above treatments to be administered to a patient will vary with the precise nature of the condition being treated and the recipient of the treatment. The scaling of dosages for human administration can be performed according to art-accepted practices. The dose for CAMPATH, for example, will generally be in the range 1 to about 100 mg for an adult patient, usually administered daily for a period between 1 and 30 days. The preferred daily dose is 1 to 10 mg per day although in some instances larger doses of up to 40 mg per day may be used (described in U.S. Patent No. 6,120,766).

Human dosage amounts can initially be determined by extrapolating from the amount of compound used in mice, as a skilled artisan recognizes it is routine in the art to modify the dosage for humans compared to animal models. In certain embodiments it is envisioned that the dosage may include an effective amount from between about 0.001 mg compound/Kg body weight to about 100 mg compound/Kg body weight; or from about 0.05 mg/Kg body weight to about 75 mg/Kg body weight or from about 0.1 mg/Kg body weight to about 50 mg/Kg body weight; or from about 0.5 mg/Kg body weight to about 40 mg/Kg body weight; or from about 0.1 mg/Kg body weight to about 30 mg/Kg body weight; or from about 1 mg/Kg body weight to about 20 mg/Kg body weight. In other embodiments, the effective amount may be about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9, 10, 15, 20, 25, 30, 35, 40, 45, 50, 55, 60, 65, 70, 75, 80, 85, 90, 95, or about 100 mg/Kg body weight. In other embodiments, it is envisaged that effective amounts may be in the range of about 2 mg compound to about 100 mg compound. In other embodiments, the effective amount may be about 1, 2, 3, 4, 5, 6, 7, 8, 9, 10, 11, 12, 13, 14, 15, 16, 17, 18, 19, or 20 mg per single dose. In another embodiment, the effective amount comprises less than about 2, 3, 4, 5, 6, 7, 8, 9, 10, 15, 20, 25, 30, 35, 40, 45, 50, 55, 60, 65, 70, 75, 80, 85, 90, 95 mg daily. In an exemplary embodiment, the effective amount comprises less than about 50 mg daily. Of course, the single dosage amount or daily dosage amount may be adjusted upward or downward, as is routinely done in such treatment protocols, depending on the results of the initial clinical trials and the needs of a particular patient.

The precise determination of what would be considered an effective dose is based on factors individual to each subject, including their size, age, sex, weight, and condition of the particular subject. Dosages can be readily ascertained by those skilled in the art from this disclosure and the knowledge in the art.

Optionally, the methods of the invention provide for the administration of a composition of the invention to a suitable animal model to identify the dosage of the composition(s), concentration of components therein and timing of administering the composition(s), which elicit tissue repair, reduce cell death, or induce another desirable biological response. Such determinations do not require undue experimentation, but are routine and can be ascertained without undue experimentation.

The biologically active agents can be conveniently provided to a subject as sterile liquid preparations, e.g., isotonic aqueous solutions, suspensions, emulsions, dispersions, or viscous compositions, which may be buffered to a selected pH. Cells and agents of the invention may be provided as liquid or viscous formulations. For some applications, liquid formations are desirable because they are convenient to administer, especially by injection. Where prolonged contact with a tissue is desired, a viscous composition may be preferred. Such compositions are formulated within the appropriate viscosity range. Liquid or viscous compositions can comprise carriers, which can be a solvent or dispersing medium containing, for example, water, saline, phosphate buffered saline, polyol (for example, glycerol, propylene glycol, liquid polyethylene glycol, and the like) and suitable mixtures thereof.

Sterile injectable solutions are prepared by suspending talampanel and/or perampanel in the required amount of the appropriate solvent with various amounts of the other ingredients, as desired. Such compositions may be in admixture with a suitable carrier, diluent, or excipient, such as sterile water, physiological saline, glucose, dextrose, or the like. The compositions can also be lyophilized. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts, such as "REMINGTON'S PHARMACEUTICAL SCIENCE", 17th edition, 1985, may be consulted to prepare suitable preparations, without undue experimentation.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin. According to the present invention, however, any vehicle, diluent, or additive used would have to be compatible with the cells or agents present in their conditioned media.

The compositions can be isotonic, i.e., they can have the same osmotic pressure as blood and lacrimal fluid. The desired isotonicity of the compositions of this invention may be accomplished using sodium chloride, or other pharmaceutically acceptable agents such as dextrose, boric acid, sodium tartrate, propylene glycol or other inorganic or organic solutes. Sodium chloride is preferred particularly for buffers containing sodium ions.

Viscosity of the compositions, if desired, can be maintained at the selected level using a pharmaceutically acceptable thickening agent, such as methylcellulose. Other suitable thickening agents include, for example, xanthan gum, carboxymethyl cellulose, hydroxypropyl cellulose, carbomer, and the like. The choice of suitable carriers and other additives will depend on the exact route of administration and the nature of the particular dosage form, e.g., liquid dosage form (e.g., whether the composition is to be formulated into a solution, a suspension, gel or another liquid form, such as a time release form or liquid-filled form). Those skilled in the art will recognize that the components of the compositions should be selected to be chemically inert.

It should be understood that the method and compositions that would be useful in the present invention are not limited to the particular formulations set forth in the examples. The following examples are put forth so as to provide those of ordinary skill in the art with a complete disclosure and description of how to make and use the cells, expansion and culture methods, and therapeutic methods of the invention, and are not intended to limit the scope of what the inventors regard as their invention.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", fourth edition (Sambrook, 2012); "Oligonucleotide Synthesis" (Gait, 1984); "Culture of Animal Cells" (Freshney, 2010); "Methods in Enzymology" "Handbook of Experimental Immunology" (Weir, 1997); "Gene Transfer Vectors for Mammalian Cells" (Miller and Calos, 1987); "Short Protocols in Molecular Biology" (Ausubel, 2002); "Polymerase Chain Reaction: Principles, Applications and Troubleshooting", (Babar, 2011); "Current Protocols in Immunology" (Coligan, 2002). These techniques are applicable to the production of the polynucleotides and polypeptides of the invention, and, as such, may be considered in making and practicing the invention. Particularly useful techniques for particular embodiments will be discussed in the sections that follow.

### EXAMPLES

The invention is further described in detail by reference to the following experimental examples. These examples are provided for purposes of illustration only, and are not intended to be limiting unless otherwise specified. Thus, the invention should in no way be construed as being limited to the following examples.

Without further description, it is believed that one of ordinary skill in the art can, using the preceding description and the following illustrative examples, make and utilize the compounds of the present invention and practice the claimed methods. The following working examples therefore, specifically point out the preferred embodiments of the present invention, and are not to be construed as limiting in any way the remainder of the disclosure.

### Example 1-System Design

In the presented invention the designing and testing of a novel first-of-its-kind lentiviral vector system with unique properties that allowed for a simultaneous constitutive and inducible expression within a single lentiviral vector was demonstrated (Figure 1). A constitutive part of the system enabled strong constitutive promoter driven expression of immune receptor such as CAR or TCR, while inducible part or the system provided autonomous expression of effector immunomodulatory molecules triggered by introduced CAR/TCR-specific or other tumor microenvironment characteristic signaling. More specifically, the system presented in this disclosure sensed endogenous CAR/TCR or other tumor microenvironment characteristic signaling through the activation of specific promoters introduced with the regulatory part of the lentiviral vector and rewired this signaling to the expression of the selected immunomodulatory molecules, which should in principle be restricted to the local tumor microenvironment.

For this purpose different architectures of unique lentiviral plasmids were prepared, which comprised different inducible promoters including a synthetic NFAT promoter, CD69 promoter, CD137 promoter or synthetic hypoxia responsive element (HRE) promoter linked with best in class minimal promoter (P_{MIN}) validated in our previous studies (Smole, A., Lainšček, D., Bezeljak, U., Horvat, S. & Jerala, R. A Synthetic Mammalian Therapeutic Gene Circuit for Sensing and Suppressing Inflammation. *Mol. Ther.* **25,** 102-119 (2017)), which enabled low basal leakage and high expression when induced through NFAT promoter. Inducible part was connected with the constitutively expressed module in the same lentiviral vector in different architectures whether both driving expression in the same (pASP4 architecture) or in the opposite (pASP5 architecture) directions (Figure 2).

### Example 2- Autonomous NFAT-driven expression of eGFP and constitutive expression of mCherry from a single lentiviral vector in Jurkat cell line.

As a proof of principle, the lentiviral system described herein enabled constitutive production of mCherry and inducible expression of eGFP in transduced Jurkat cells as measured by fluorescence microscopy and flow cytometry (Figure 3). Cells were nonspecifically activated with CSC (ionomycin and phorbol myristate acetate) which short-circuits Ca²⁺ TCR signaling in T cells. Different promotor elements that are active specifically in the activated T cells were tested. Architectures pASP8 and pASP9 comprised CD69 and CD137 promoters respectively. ON/OFF ratio in both examples was low (from 1.5-3) and additionally, CD69 promoter shown substantial leakiness. Architecture pASP4.2, comprising synthetic NFAT promoter linked to the optimized minimal promoter (P_{MIN}) enabled medium ON state while it remained silent in the absence of the trigger signal, which is attributed to the careful selection of NFAT binding sites and P_{MIN}, resulting in 65-fold induction. This configuration is of high value when no leakiness is acceptable due to potential toxicities, for example when using immune modulators such as cytokines. Architecture pASP5 differs from pASP4.2 in the orientation of the inducible part, which is placed in the reverse orientation, back-to-back with the constitutively expressed part of the vector. This architecture resulted in substantially higher ON state but also higher leakiness (fold induction of around 25), while virus titer was also significantly better. The latter is attributed to the orientation of consecutive NFAT binding sites which likely interfered with virus mRNA production and was less adverse when placed in the opposite direction to the virus mRNA transcription. pASP5 configuration is suitable for the systems where some basal leakiness is not expected to pose serious safety concerns and high ON state production is required, which would be the case when using therapeutic antibodies such as inflammatory cytokines blockers or checkpoint inhibitors. pASP7 architecture is the same as pASP5 with the only difference of SV40 poly adenylation (pA) signal added to the 3'end of eGFP to increase its expression. While increasing eGFP expression, this configuration also decreased virus titer significantly, since SV40 pA even if placed in the reverse orientation severely interferes with virus mRNA production.

Interestingly, back-to-back orientation of inducible and constitutive promoters influenced expression in both directions as observed in constructs pASP5 and pASP7, where constitutively expressed mCherry increased after stimulation and leakage of inducible eGFP was higher than expected in non-stimulated cells. This phenomenon and its use to our advantage are discussed in more detail below.

### Example 3- Autonomous NFAT-driven expression of eGFP and constitutive expression of mCherry from a single lentiviral vector in primary human T cells

To demonstrate that the developed system works also in therapeutically used primary T cells with more relevant inducers resampling TCR signalling, primary donor-derived T cells were activated, transduced with constructs pASP8, pASP9, pASP4.2, pASP7 and pASP5 and expanded according to the standard protocol. Engineered cells were rested for 14 days to return activation state to the basal levels and then stimulated with CSC, anti-CD3/CD28 beads and anti-CD3/CD28 antibodies-coated plates. Results recapitulated our findings from Jurkat cell lines, showing reliable constitutive expression of mCherry and roboust inducibility of eGFP in constructs pASP4.2 and pASP5 as measured by fluorescence microscopy and flow cytometry (Figure 4). Imprtantly, physiologicaly relevant TCR-mediated signaling induced by anti-CD3/CD28 beads and anti-CD3/CD28 antibodies-coated plates resulted in similar activation amplitude as in the case of CSC that represented maximal system's activity. System showed tight regulation with no detectable (pASP4.2) or very low (pASP5) background activity in the absence of activation signal.

### Example 4- Capacity of the activated system

In one aspect, the purpose of the developed system is to produce various effectors in sufficient amounts to mediate therapeutic effect. To validate the capacity of the developed system, median fluorescence intensity was determined as a measure of the amount of eGFP produced in an inducible manner. pASP5 as a higher expressing architecture produced comparable levels to constitutively expressed eGFP, meaning that the system's capacity was high. pASP4.2 version, as expected, expressed at lower amounts, which recapitulates findings shown in previous figures and supports the rationale for using these two architectures according to the specific needs of the respective therapeutic molecule (Figure 5).

### Example 5- Reversibility of the system

The reversibility of the presented system is evidenced by the data presented herein and its capability of responding to multiple rounds of stimulation, which is how a native immune system works. This is relevant if the cancer resides in different sites of the body and same cell encounters target cells many times or if the engineered cells are exposed to the same target multiple times (for example in reoccurring disease). Using IncuCyte technology to monitor fluorescence in a real-time manner, pASP4.2, pASP5 and pASP7 transduced Jurkat cells were stimulated with CSC for 24 h and observed rapid eGFP expression, which was detectable already after 4 h revealing rapid kinetics of the developed inducible system (Figure 6A). After washing step, eGFP decreased gradually over 4 days, which is limited to the molecule half-life, and after re-stimulation at day 5, it increased again, demonstrating a reversible nature of the system. This was shown also later with a secreted molecule to more accurately capture the kinetics and to show that the system resets quickly after stimulus removal and returns to the initial maximum activation levels after second stimulation (section "On demand tailored and reversible expression of hIL-12 to increase efficacy of engineered primary T cells"). mCherry as a constitutive part remained expressed regardless of the stimulation, however in the pASP5 and pASP7 back-to-back orientation its expression was increased in stimulated cells, which is in agreement with results shown in previous figures and also with published studies showing different levels of bidirectional activity of certain promoter elements (Amendola, M., Venneri, M. A., Biffi, A., Vigna, E. & Naldini, L. Coordinate dual-gene transgenesis by lentiviral vectors carrying synthetic bidirectional promoters. Nat. Biotechnol. 23, 108-116 (2005)). This phenomenon probably increased basal expression of inducible part in pASP5 orientation as well, since EF-1alpha promoter influences NFAT-P_{MIN} inducible part. On the other hand, NFAT transcriptional factors, recruited to consecutive NFAT binding sites in synthetic promoter in stimulated T cells increased the strength of already strong EF-1alpha promoter even further as shown by increase in mCherry expression after stimulation. This is useful feature because the system behaves in a feed-forward loop manner, meaning that CAR expression would be increased in a positive-feedback loop after initial recognition but in an antigen-dependent manner. This could increase sensitivity of the system in the tumor microenvironment where cancer cells downregulate target antigen, whereas sensitivity would return to normal levels when antigen in no more present.

In this Example, a functionally connected regulatory unit has been shown in the forward orientation but the same configuration is used when inducible and constitutive modules are placed back to back in the opposite direction, where inducible module is placed in reverse and constitutive module in forward orientation. Also provided herein is the 5'-3' sequence and annotation of this exact part, because this orientation is often used because it confers better virus titer and hence higher clinical relevance:
Preceded in reverse orientation by immune modulator starting with a start codon - 
GGTGGCTTTACCAACAGTACCGGAATGCCCCCGGGCTGGAAGTCGAGCTTCCA
TTATATACCCTCTAACTAGTACGCCTTCTGTATGAAACAGTTTTTCCTCCACGC
 
CTTCTGTATGAAACAGTTTTTCCTCCACGCCTTCTGTATGAAACAGTTTTTCCT
CCACGCCTTCTGTATGAAACAGTTTTTCCTCCACGCCTTCTGTATGAAACAGTT
TTTCCTCCACGCCTTCTGTATGAAACAGTTTTTCCTCCAACAGTACCGG (SEQ
ID NO: 3)
- Followed in forward orientation by strong constitutive promoter such as EF1alpha - followed in forward by immune receptor such as CAR or TCR starting with start codon.
   **Kozak sequence: bold**
   Spacer for optimal distance between transcription and translation start site: *italics*
   Minimal promoter: underlined
   Synthetic NFAT promoter: wave underline
   Spacer for cloning purposes: double underline

A schematic representation is shown in Figure 26 where orientation of the arrow means direction (forward or reverse) and entire sequence as exemplified by FOXO1-3A as immune modulator and 4D5 Her2-targeting CAR (4-1BB+CD3Z). The functionally connected regulatory unit from above is marked in bold in the sequence below (SEQ ID NO: 4):

TTAGCCGGAAACCCAGCTGTGTGTGGTGGTTTTCACGCTGTGAGGGAAGCTCT
GGTTGGGCAGCACGTTATCGAAGTTGAAGTCCAGGGTATCGCCGTCCATCAG
GTCGTTGCGGATGATGGATTCCATGTCGCAGTCCAGTCTCTCGATGAACATGC
CGTCCAGATCAGAGGGCAGCTTCTCTTGGTGCAGCAGGCCCATTCTGCCGTAG
CCATTACAGCTGGACACGCTGGAGTAGCCGCCGAGGGCAGACATCTGCATAG
GATGAGGCAGTGGGACCTGCACAGGGGTTTTCACTTGGGTCAGCCGGTTCAT
GCCGCTGGTATGAGGCATGGTGGACACGGTGTGAGGCAGAGGTCTGCCATTA
ACGGCGCTTGTCTGCTGGGCATGTCCAGGGTGTGTGTGGCTGCTGGGGTTCAT
CATCTTGTTGTGGCTGGCCTGAGAGCCGTATGTGCTCATCACGCTATTGGGGC
CCATCATCACGTTCTGGCCCAGCACTCTACTATTAGGCTGGGCCACGCCAGGG
TCCACAGGTGTCATGATGTCGTTGTGAGGAGGGCTGTCAGAGGTCAGCAGCT
CTTTCAGCAGTCCAGGGGCACAATTGTACTGGCTCATGCCGCCGTAGGAGGA
CTTGTTGTCCTGCAGGGTCTGAATGGGCATCTGTGGCAGAGGGCTCATGCTGC
TCTGGCCGTAGGTGTACTTCTGGTAGTTAGGAGATGGGCTGTTCAGGCTTGTG
TTAGGAGGGGCGAAGCTGTAGCAAGGGGTCTGCTGCATCATGGTGCCGGGGC
TGCTTTGTGTAGACACGGTCAGGCTGGTAGGGCTGCTCAGCAGATTCAGGTTG
TCCAGCAGGTTTTCCATGTTCTCGGGGTTGCTGATCTCGCTCAGGCTTGGCAG
TGTGCTGGCCATTTTGGCGGCGCTTGGAGGATACACCATGCTGTGCACGTCGC
CTTCTCCGAGATCATCCTGCTCGGTCATGATGGGGCTCAGTCTGCCAGAGATG
GTGCTGGCATTGGCGCTGGTTCTGGGTCTGAAGGTGGACCAGTTGTCGAAGTC
GTCGTTGCTGTGAGAGCCAGGAGAGGCGGGCCACTTAGAAAACTGGCTGCCA
GGAGAATCGCCAGCGCCTTCTTGTCCAGACTGCAGAGAGGCCTTCTTCTTGGC
GGCTCTGCTTCTGCTCTTGGCGAACTTGGAGTTGTTGTCCATGGCAGCGGCCC
TTCTACGAGGAGACTTGCCGCTCTTGCCGCCTTCGGGATTCAGCATCCACCAA
GAGGACTTGCCGGTGCCTTCATTCTGCACCCGGATGAACTTGCTGTGCAGGGA
CAGATTGTGCCGGATGCTGTTCTTCCAGCCGGCGGAGCTATTGCTGTCGCCCT
TGTCCTTGAAGTAGGGCACGCTCTTGACCATCCACTCGTAGATCTGGCTCAGG
GTCAGTCTCTTCTCGGCGCTGCTCTCAATGGCCTTGGTGATCAGGTCGGCGTA

GCTCAGATTGCCCCAGGCGTTTCTTCTGCTGGAGCTGCTCTTTCTAGGCTGTCC
AGCAAGTGGTCCAGCGGCAGCTGGAGGAACAGGAGGATGCTGAGACAGAGG
TCCAGGTGGAGGTGGTTGAGGTGGAGCAGGGTGCAGACATCCGGCCTCAGGT
CCCTGAAAATCGCCGCAAAGTCCACCTGTAGCAGCAGCTGCGGCTGCGGCGG
CAACAGCTGCAGCCACAGATCCAGGAGCCTGAGGGAAGTCCTCGCTCTCTTC
CAGCAGGCTCAGGTTGCTCATGAAGTCGGCGGACACTGCAGCAGCAGAGGCA
GAAGGCAGTCCAGCAGCAGCATCAGGATTAGCGGCGGCAGATCCGCTAGGA
GCAGGGCTAGATGTGGCGCTGTTGCTCTGGCTGAACTCAGGTCTAGGCAGAG
GCCAAGCACAAGATCTAGGCCGAGGCAGGGGCTCGAAATCAGGGTCGATTTC
CACCACCTGGGGAGCCTCGGCCATGGTGGCTTTACCAACAGTACCGGAATG
CCCCCGGGCTGGAAGTCGAGCTTCCATTATATACCCTCTAACTAGTACGC
CTTCTGTATGAAACAGTTTTTCCTCCACGCCTTCTGTATGAAACAGTTTTT
CCTCCACGCCTTCTGTATGAAACAGTTTTTCCTCCACGCCTTCTGTATGA
AACAGTTTTTCCTCCACGCCTTCTGTATGAAACAGTTTTTCCTCCACGCC
TTCTGTATGAAACAGTTTTTCCTCCAACAGTACCGGATGCATCGTGAGGCT
CCGGTGCCCGTCAGTGGGCAGAGCGCACATCGCCCACAGTCCCCGAGAAGTT
GGGGGGAGGGGTCGGCAATTGAACCGGTGCCTAGAGAAGGTGGCGCGGGGT
AAACTGGGAAAGTGATGTCGTGTACTGGCTCCGCCTTTTTCCCGAGGGTGGGG
GAGAACCGTATATAAGTGCAGTAGTCGCCGTGAACGTTCTTTTTCGCAACGGG
TTTGCCGCCAGAACACAGGTAAGTGCCGTGTGTGGTTCCCGCGGGCCTGGCCT
CTTTACGGGTTATGGCCCTTGCGTGCCTTGAATTACTTCCACCTGGCTGCAGT
ACGTGATTCTTGATCCCGAGCTTCGGGTTGGAAGTGGGTGGGAGAGTTCGAG
GCCTTGCGCTTAAGGAGCCCCTTCGCCTCGTGCTTGAGTTGAGGCCTGGCCTG
GGCGCTGGGGCCGCCGCGTGCGAATCTGGTGGCACCTTCGCGCCTGTCTCGCT
GCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTAT
TTCGGTTTTTGGGGCCGCGGGCGGCGACGGGGCCCGTGCGTCCCAGCGCACA
TGTTCGGCGAGGCGGGGCCTGCGAGCGCGGCCACCGAGAATCGGACGGGGGT
AGTCTCAAGCTGGCCGGCCTGCTCTGGTGCCTGGCCTCGCGCCGCCGTGTATC
GCCCCGCCCTGGGCGGCAAGGCTGGCCCGGTCGGCACCAGTTGCGTGAGCGG
AAAGATGGCCGCTTCCCGGCCCTGCTGCAGGGAGCTCAAAATGGAGGACGCG
GCGCTCGGGAGAGCGGGCGGGTGAGTCACCCACACAAAGGAAAAGGGCCTT
TCCGTCCTCAGCCGTCGCTTCATGTGACTCCACTGAGTACCGGGCGCCGTCCA
GGCACCTCGATTAGTTCTCGTGCTTTTGGAGTACGTCGTCTTTAGGTTGGGGG
GAGGGGTTTTATGCGATGGAGTTTCCCCACACTGAGTGGGTGGAGACTGAAG
TTAGGCCAGCTTGGCACTTGATGTAATTCTCCTTGGAATTTGCCCTTTTTGAGT
TTGGATCTTGGTTCATTCTCAAGCCTCAGACAGTGGTTCAAAGTTTTTTTCTTC
CATTTCAGGTGTCGTGATTCTAGAGCCACCATGGCCTTACCAGTGACCGCCTT
GCTCCTGCCGCTGGCCTTGCTGCTCCACGCCGCCAGGCCGGACATTCAAATGA
CACAGAGTCCCTCATCCCTCAGTGCCAGCGTGGGCGATCGGGTGACTATAAC
CTGCAGAGCTTCTCAGGACGTGAATACCGCTGTGGCGTGGTACCAGCAGAAG
CCAGGCAAAGCGCCTAAGCTTCTCATTTATAGTGCCAGCTTCCTGTACTCAGG
TGTTCCGTCTCGCTTTTCTGGAAGTAGAAGTGGGACCGATTTCACATTGACGA
TCAGCAGCTTGCAGCCCGAAGATTTCGCCACCTACTACTGTCAGCAGCACTAC
ACTACCCCACCGACATTTGGTCAAGGCACAAAAGTAGAGATTAAACGCACTG
GTTCCACCAGCGGGAGCGGGAAACCCGGCTCTGGGGAGGGGAGCGAGGTCC
AGCTGGTGGAATCCGGGGGTGGTCTTGTGCAGCCAGGAGGATCCTTGAGGTT
GTCCTGCGCCGCAAGCGGCTTTAACATCAAAGATACATACATCCATTGGGTCC
GACAGGCCCCTGGAAAGGGCCTGGAGTGGGTCGCCCGGATCTACCCAACTAA
CGGGTACACTCGCTACGCTGATAGCGTCAAGGGTCGGTTTACTATTTCTGCCG

ACACCTCAAAAAACACAGCCTACCTCCAGATGAACTCTCTCAGAGCTGAGGA
TACAGCCGTGTACTATTGCAGCCGGTGGGGAGGCGACGGGTTCTACGCTATG
GATGTGTGGGGGCAGGGCACACTGGTCACCGTGAGCTCATCCGGAACCACGA
CGCCAGCGCCGCGACCACCAACACCGGCGCCCACCATCGCGTCGCAGCCCCT
GTCCCTGCGCCCAGAGGCGTGCCGGCCAGCGGCGGGGGGCGCAGTGCACACG
AGGGGGCTGGACTTCGCCTGTGATATCTACATCTGGGCGCCCTTGGCCGGGAC
TTGTGGGGTCCTTCTCCTGTCACTGGTTATCACCCTTTACTGCAAACGGGGCA
GAAAGAAACTCCTGTATATATTCAAACAACCATTTATGAGACCAGTACAAAC
TACTCAAGAGGAAGATGGCTGTAGCTGCCGATTTCCAGAAGAAGAAGAAGGA
GGATGTGAACTGAGAGTGAAGTTCAGCAGGAGCGCAGACGCCCCCGCGTACA
AGCAGGGCCAGAACCAGCTCTATAACGAGCTCAATCTAGGACGAAGAGAGG
AGTACGATGTTTTGGACAAGAGACGTGGCCGGGACCCTGAGATGGGGGGAAA
GCCGAGAAGGAAGAACCCTCAGGAAGGCCTGTACAATGAACTGCAGAAAGA
TAAGATGGCGGAGGCCTACAGTGAGATTGGGATGAAAGGCGAGCGCCGGAG
GGGCAAGGGGCACGATGGCCTTTACCAGGGTCTCAGTACAGCCACCAAGGAC
ACCTACGACGCCCTTCACATGCAGGCCCTGCCCCCTCGCTAA

### Example 6- Designer T cells respond specifically to antigen-positive cancer cell line and retain CAR mediated killing

After system optimization, constitutively expressed mCherry was exchanged with CAR to access killing capacity and simultaneous eGFP upregulation to demonstrate a proof of principle. As an example, 4D5 (anti-Her2) scFv in a combination with 4-1BB or CD28 costimulatory domains was used, designated here as pASP30 and pASP31, respectively. Primary T cells were activated, transduced, expanded and rested according to the standard protocol and then co-cultured with SKOV3 cancer cell line that expresses high levels of Her2 at various effector to target (E:T) ratios. As a control MDA468 cell line that does not express significant levels of Her2 was used. Using fluorescence microscopy and flow cytometry, robust increase in eGFP was observed only when pASP30-engineered cells were co-cultured with antigen-positive cell line, whereas the system remained insensitive to the antigen-negative cell lines at all ratios (Figure 7A). As measured by MFI derived from flow cytometry data (Figure 7A, bottom left), Her2 positive cell line activated system to the similar levels as CSC which is surprising since CSC shortcuts TCR signaling and hence maximizes T cell activation. Interestingly, surface CAR in engineered T cells dramatically decreased when co-cultured with antigen-positive cell lines, probably because of the known phenomena of CAR internalization (Figure 7A, flow cytometry and MFI). The higher the E:T ratio, the lower was downregulation of surface CAR, which can be explained by the fraction of CAR molecules engaged with an antigen, which is the highest when there are least T cells in the co-culture. Concomitant with NFAT-regulated eGFP upregulation, pASP30-transduced T cells also specifically killed antigen-positive cancer cells measured by standard luciferase-based assay showing preserved functionalities of T cells when engineered with the developed all-in-one lentiviral vector systems (Figure 7A, bottom right).

Similar results were observed with pASP31, where the 4-1BB costimulatory domain was exchanged with CD28, meaning that different costimulatory domains did not significantly influence activity of NFAT promoter. Interestingly basal activity of NFAT signaling was also similar suggesting similar tonic activity of CD28 and 4-1BB in the system. However, less downregulation of surface CAR expression and slightly better killing was observed when using CD28 domain compared to 4-1BB, in a co-culture with antigen-positive cell line (Figure 7B).

In order to determine functionality of the system in the context of hematological malignancies, Her2 CAR in pASP30 was replaced with anti-CD20 CAR to obtain pASP28 and CAR expression and NFAT-driven eGFP upregulation was verified after CSC stimulation (Figure 8A). Activated, transduced and rested primary T cells were then co-cultured with cell lines, which endogenously expressed (NALM6) or overexpressed (GL-1 CD20 positive and K562 CD20 positive) CD20 antigen to investigate antigen-dependent response. Robust eGFP expression as measured by MFI was demonstrated when system was stimulated with antigen-positive cell lines K562 and NALM6, reaching 60 % of maximal activity achieved by CSC as a positive control (Figure 8B). Simultaneously with NFAT-driven eGFP upregulation, T cells engineered with pASP28 retained anti-CD20 CAR-mediated lysis capacity as observed by clearance of CD20 positive, but not negative target cell lines determined by flow cytometry. Untransduced T cells did not possess capacity to kill target cell lines (Figure 8C). Co-culture with CD20 positive GL1 cell line resulted in a very weak expression of inducible part. Without wishing to be bound by theory, different target cell lines may elicit different responses in engineered effector T cells resulting in different kinetics and intensity of NFAT signaling. For example, GL1 cell line is known to be easy-to-lyse by CAR T cells and hence its lysis might be rapid and underlying NFAT-signaling lost quickly. This is important because it demonstrates that activity of the system is operational only when antigen is present and its activity rapidly decreases with distance from antigen, making it local and tumor restricted. Importantly, the system's activity remained at the basal levels in the absence of stimulation signal or when co-cultured with parental antigen-negative cell lines, showing reliable antigen-dependent NFAT-inducible signaling (Figures 8A-C).

Taken together, the all-in-one lentiviral system enables antigen-specific lysis and NFAT-driven expression of an inducible molecule when in contact with adherent or suspension cell lines. This enabled the advancement with implementations in the context of solid as well as hematological malignancies.

### Example 7- All-in-one lentiviral system has superior functionalities compared to two-component lentiviral system

To compare properties and performance of all-in-one with two-component lentiviral systems, the pASP30 was benchmarked, where inducible eGFP and constitutive 4D5 anti-Her2 CAR are encoded in the same lentiviral vector with the system, where the components were broken into two lentiviral vectors, one encoding NFAT-inducible eGFP (pASP5) and other constitutive 4D5 anti-Her2 CAR (pP7). Primary T cells were transcribed with either pASP30 derived virus at MOI3 or a combination of pASP5 and pP7 derived viruses, each added at MOI3. In principle, the number of integrated inducible and constitutive portions should be similar in all-in-one and two-component system; however, the total amount of virus added to the cells was two times higher when two separate viruses were added. In the case of all-in-one lentiviral system, both inducible and constitutive parts were integrated into the same genomic locus at equimolar ratios, since they integrate as a single DNA molecule. To support that, it was demonstrated herein that in the CSC-stimulated primary T cells, the CAR-positive population homogenously shifted up in NFAT-induced eGFP (Figure 9A). In contrast, it was anticipated that the two-component system would lead to separate integration events of inducible and constitutive parts leading to the non-homogenous distribution, where only a fraction of the cells receives (possibly variable amounts) of both parts. This was demonstrated in Figure 9B, where three distinct populations (CAR-only, eGFP-only and double positive) were observed after non-specific, CAR-independent stimulation, each at around 20% of the parental population. In contrast, when the cells were activated with target cell lines expressing Her2, one would expect that only CAR-positive cells would express NFAT-inducible eGFP. However, it has already been shown herein that CAR surface expression is downregulated after recognizing cognate ligand, possibly because of internalization, and hence CAR-low and GFP-high populations are observed although they express both the CAR and eGFP (Figures 7A-B). Nevertheless, clearly lower expression of eGFP was observed in the CAR-negative portion of the two-component system compared to the CSC regime, which was not the case in all-in-one lentiviral system where every cell that expresses CAR, expresses inducible part as well (Figures 9A-B). MFI as a measure of system's capacity showed superior function of all-in-one over the two-component lentiviral system resulting in higher fold induction when stimulated with either non-specific CSC or specific, antigen-driven target cell stimulation (Figures 9A-B). Without wishing to be bound by theory, the strong constitutive EF-1alpha promoter may open chromatin and make the NFAT inducible part in the immediate vicinity more accessible for binding of NFAT transcriptional factors and hence may lead to improved performance. Taken together, apart from a simplified modular assembly of lentiviral transfer plasmid and virus production in the all-in-one lentiviral system, where only one virus needs to be produced to achieve constitutive and inducible expression, this system also functionally outperforms two-component system even at lower absolute amount of virus added. Of clinical importance, the all-in-one lentiviral system enables the manufacture of well-defined cell product with homogenous transgene distribution.

Figures 14A-14B further illustrate that the single lentiviral system has superior functionalities compared to a two-component lentiviral system. Figures 14A-14B show the results of the same experiments as shown in Figures 9A-9B, but the data is represented side-by-side, and quantification data is shown in the bar graphs in Figure 14B.

### Example 8- On demand tailored and reversible expression of hIL-12 to increase efficacy of engineered primary T cells

After successful demonstration of constitutive CAR expression that, in a response to a specific antigen, activates the inducible part of the system, the feasibility of expressing an immune modulator to increase efficacy of engineered T cells in an inducible manner was tested. To that end, a pASP18 construct was designed by exchanging eGFP in a pASP4.2 with human IL-12 consisting of p40 and p35 subunits connected with a flexible linker according to the previously published design (Zhang, L. et al. Improving adoptive T cell therapy by targeting and controlling IL-12 expression to the tumor environment. Mol. Ther. 19, 751-9 (2011)). Primary human T cells were activated, transduced with pASP18, expanded and rested according to the standard protocol and then subjected to an activation regime to show expression and reversibility. First, cells were stimulated with CSC or anti-CD3/CD28 beads for 2 days, then washed and left non-stimulated for 2 days and then re-stimulated for another 2 days. Supernatant was collected daily and hIL-12 levels were measured by ELISA. Robust expression was demonstrated when system was stimulated, while there was no detectable IL-12 expression in the absence of stimulation signal, showing reliable performance with a very tight regulation of the effector expression that is needed for safe therapeutic applications. Additionally, the system was fully reversible and as such able to respond to the multiple rounds of antigen encounter (Figure 10A).

After showing that individual components work as intended, inducible hIL-12 was combined with 4D5 (anti-Her2) scFv CAR with 4-1BB costimulatory domain to obtain construct pASP38. To investigate the effect of inducible hIL-12, a control construct pASP26 was used, where hIL-12 was exchanged by eGFP. Engineered primary T cells were then co-cultured with target cell lines and similar lysis was observed with both constructs after 24 h, demonstrating functionality of engineered cells (Figure 10B).

When hIL-12 levels were measured in the supernatant of the 24 h co-culture, antigen-dependent and inducible expression of hIL-12 was observed only when co-cultured with Her2 positive SKOV3 cell line but not with Her2 negative MDA468 cell line (Figure 10C, left). Inducible hIL-12 expression, in turn, increased INF-gamma expression in pASP38-transduced primary T cells compared to pASP26, when co-cultured with target cell lines (Figure 10C, middle). Importantly, IL-2 remained at similar levels (Figure 10C, right), demonstrating that inducible hIL-12 specifically shifts engineered cell into the preferable Tₕ1 phenotype, which should in principle increase antitumor efficacy of adoptively transferred CAR/TCR-engineered T cells.

### Example 9- Antigen-dependent expression of engineered antibody that blocks IL-6 signaling to increase safety of engineered immune cells

After demonstrating feasibility of increasing efficacy of engineered immune cells, the goal was to advance safety of adoptive cancer immunotherapy. The all-in-one lentiviral system developed in this study enabled the coupling of specific tumor antigen recognition with an inducible expression of an immune modulator to control adverse events associated with immunotherapy such as CRS and neurotoxicity. Currently CRS is most effectively treated nonspecifically with corticosteroids or specifically by administration of Tocilizumab, clinically approved monoclonal antibody that binds interleukin-6 receptor (IL-6R). This blocks interleukin-6 signaling axis, which was shown to be one of the main players in CRS, produced by various immune cells (Neelapu, S. S. et al. Chimeric antigen receptor T-cell therapy - assessment and management of toxicities. Nat. Rev. Clin. Oncol. (2017). doi:10.1038/nrclinonc.2017.148). Physicians apply Tocilizumab only after clinical symptoms appear and often that do not completely prevent adverse processes. Additionally, therapeutic effect of systemic Tocilizumab administration is limited by blood-brain barrier and would require intra-thecal delivery to control neurological toxicities related to immunotherapy (Johnson, L. A. & June, C. H. Driving gene-engineered T cell immunotherapy of cancer. Cell Res. 27, 38-58 (2016)). Similarly it is expected for other biological drugs that would be used to block various inflammatory mediators in central nervous system.

It was hypothesized that autonomous and early in-situ expression and secretion of interleukin-6 receptor (IL-6R) blocking molecule or other inflammatory blockers (such as neutralizers of tumor-necrosis factor alpha and interleukin beta) by therapeutic immune cells themselves, would prevent CRS-related damage even before it clinically commenced and amplified. Additionally, engineered immune cells that non-intentionally or intentionally (when targeting brain tumors) enter central nervous system would be equipped with build-in features to control accompanying toxicities by local production of inflammatory-blocking molecule, solving the inability of systemically delivered antibodies to cross BBB.

IL-6R blocking antibody was designed by linking Tocilizumab derived heavy and light chains (Tsuchiya, M., Koh, S., Bendig, M. M., Jones, S. T. & Saldanha, J. W. Reshaped human antibody to human interleukin-6 receptor, U.S. Patent No. 5,817,790) in a scFv format and fused that with human IgG1 Fc to obtain anti-hIL6R scFv-Fc. This designed molecule was cloned as an inducible component combined in reverse back-to-back orientation of the all-in-one lentiviral system with constitutively expressed anti-CD20 CAR to obtain construct pASP52. This construct enabled the targeting of hematological malignancies where CRS and neurological toxicities were demonstrated in clinics.

Primary human T cells were activated, transduced with pASP52, expanded and rested according to the standard protocol. Expression of hCD20 CAR was demonstrated and CSC stimulation further increased its level (Figure 11A), which is in agreement with our previous data showing bi-directional influence in back-to-back orientation that confers increased sensitivity of engineered cells in the direct vicinity of tumor (Figure 6). Rested effector cells were then co-cultured with cell lines, which endogenously expressed (NALM6) or overexpressed (GL-1 CD20 positive and K562 CD20 positive) CD20 antigen to investigate antigen-dependent response. Supernatants were collected after 72 h and anti-hIL6R scFv-Fc levels were measured by ELISA. Robust expression was demonstrated when system was stimulated with antigen-positive cell lines K562 and NALM6, reaching 60 % of maximal activity achieved by CSC as a positive control (Figure 11B). Activation with CD20 positive GL1 cell line resulted in a lower expression of inducible antibody. Without wishing to be bound by theory, different target cell lines may elicit different responses in engineered effector T cells resulting in different kinetics and intensity of NFAT signaling. For example, GL1 cell line is known to be easy-to-lyse by CAR T cells and hence its lysis might be rapid and underlying NFAT-signaling lost quickly. This is important since it demonstrates that activity of our system occurs only when antigen is present and rapidly decreases making it local and tumor restricted. Importantly, the system's activity remained at the basal levels in the absence of stimulation signal or when co-cultured with parental antigen-negative cell lines, showing reliable antigen-dependent NFAT-inducible signaling (Figure 11B).

T cells engineered with inducible anti-hIL6R scFv-Fc expression retained anti-CD20 CAR-mediated killing as observed by clearance of CD20 positive, but not negative target cell lines determined by flow cytometry. Untransduced T cells did not possess capacity to kill target cell lines (Figure 11C). Retaining CAR-mediated killing presents important aspect of our system since it demonstrates that blocking IL-6 signaling axis doesn't prevent cytotoxic activity of engineered cells, while potentially enabling CRS and neurological toxicities management.

An aim of this study was to advance safety and efficacy of adoptive immunotherapy for cancer and to this end engineered immune cells were endowed with a capacity to produce tumor-localized immunomodulatory effector. Specifically, antigen-dependent secretion of engineered anti-hIL6R scFv-Fc was introduced, to address management of CRS and neurological toxicities that accompany successful immunotherapy to address safety aspect. Alternatively, T cells were engineered to express IL-12 to advance efficacy in treating solid tumors. However, since there was no available expression system that could be readily used to engineer T cells with desired features and in clinically relevant and feasible manner, a novel universal all-in-one lentiviral system was developed to meet these tasks.

Provided is an all-in-one lentiviral system for simultaneous constitutive immune receptor and inducible immune modulator expression and its use in a combination with different immune receptors and immune modulators. Two main implementations constitute inducible expression of anti-hIL6R scFv-Fc, a completely novel approach to address safety and IL-12 as an example to address efficacy of adoptive immunotherapy. Supporting data show reliable performance and full functionality according to the design.

Various CARs and TCRs were combined with various immune modulators using the all-in-one lentiviral system provided herein (Figure 2). To increase efficacy in solid tumors by recruiting endogenous immune responses, increasing persistence, overcoming T cell exhaustion and enabling resistance to checkpoints of implanted T cells, one aspect of the invention combines Her2, mesothelin, folic receptor alpha and EGFRvIII CAR with IL-12, type I interferons, IL-15, IL-18, IL-21, anti-CD25, anti-CD3, anti-CD20, anti-CD40 and anti-PD1 and/or anti-PDL1. Also provided is tumor localized depletion of regulatory T cells using the present system. To increase safety, CD20 and CD19 CARs are combined with anti-inflammatory antibodies that block IL-6, anti-TNFalpha and IL-1 signaling axis to early detect and autonomously suppress cytokine release syndrome and neurological toxicities. To increase penetrance tumor microenvironment restricted expression of tumor stroma degrading enzymes may be tested.

To make a response of the system exclusive to the specific antigen triggered signaling through introduced CAR or TCR, endogenous TCR may be knocked out using a CRISPR/Cas9 system. This may greatly increase safety of engineered T cells over existing NFAT-inducible systems, which will not be able to respond to unpredicted potential antigens through native TCRs.

### Example 10- CRISPR knock-in

Provided is a vector system and gene construct for dual constitutive and inducible expression in the single (lentiviral) vector. Inducible promoters that function exclusively upon TCR/CAR-signaling are identified. A combination of NFAT responsive element with an optimized minimal promoter P_{MIN} may be used in some embodiments. Various effectors may be used in this configuration, and the system permits knock-in of the effectors under the control of endogenous promoters that are induced in activated immune cells after specific antigen recognition. Also provided is a CRISPR-related system-mediated knock-in of the designed expression system into the TCR or HLA (or other relevant loci) to make introduced receptor-mediated signaling the exclusive activator of the system (and simultaneously prevent TCR-mediated graft-versus-host disease) and to make engineered cells universal, respectively. In some embodiments, the designed expression cassette is encoded in a donor template. In some embodiments, the donor template is encoded in a viral vector. In some embodiments the viral vector is an adeno-associated viral vector. In further embodiments, the adeno-associated viral vector is AAV6. In some embodiments, the CRISPR-related system is a CRISPR/Cas9 system.

### Example 11- Effect on Engineered T cells of Blocking IL-6 Signaling

Construct pASP52 is compared with the same construct where anti-hIL-6R scFv-Fc is replaced by eGFP to benchmark and analyze the effect of interfering with IL-6 signaling axis on the functionality of engineered T cells *in vitro* and *in vivo.*

The biological activity and capacity of a designed effector to block IL-6 mediated signaling is also determined.

### Example 12- Further Constructs

Constructs as described in Example 8 are generated and tested, where IL-12 effector may be replaced with IL-18, type I interferons, and other immunomodulatory molecules which are disclosed herein for use in the system.

### Nucleotide sequences - all are listed in 5' - 3' direction

### Building elements

1. Synthetic NFAT promoter (6 x consensus NFAT binding sites) (SEQ ID NO: 5) 
GGAGGAAAAACTGTTTCATACAGAAGGCGTGGAGGAAAAACTGTTTCATACA
GAAGGCGTGGAGGAAAAACTGTTTCATACAGAAGGCGTGGAGGAAAAACTG
TTTCATACAGAAGGCGTGGAGGAAAAACTGTTTCATACAGAAGGCGTGGAGG
AAAAACTGTTTCATACAGAAGGCGT
2. Minimal promoter (SEQ ID NO: 6) TAGAGGGTATATAATGGAAGCTCGACTTCCAG
3. EF1α promoter (SEQ ID NO: 7) 
CGTGAGGCTCCGGTGCCCGTCAGTGGGCAGAGCGCACATCGCCCACAGTCCC
CGAGAAGTTGGGGGGAGGGGTCGGCAATTGAACCGGTGCCTAGAGAAGGTG
GCGCGGGGTAAACTGGGAAAGTGATGTCGTGTACTGGCTCCGCCTTTTTCCCG
AGGGTGGGGGAGAACCGTATATAAGTGCAGTAGTCGCCGTGAACGTTCTTTT
TCGCAACGGGTTTGCCGCCAGAACACAGGTAAGTGCCGTGTGTGGTTCCCGC
GGGCCTGGCCTCTTTACGGGTTATGGCCCTTGCGTGCCTTGAATTACTTCCAC
CTGGCTGCAGTACGTGATTCTTGATCCCGAGCTTCGGGTTGGAAGTGGGTGGG
AGAGTTCGAGGCCTTGCGCTTAAGGAGCCCCTTCGCCTCGTGCTTGAGTTGAG
GCCTGGCCTGGGCGCTGGGGCCGCCGCGTGCGAATCTGGTGGCACCTTCGCG
CCTGTCTCGCTGCTTTCGATAAGTCTCTAGCCATTTAAAATTTTTGATGACCTG
CTGCGACGCTTTTTTTCTGGCAAGATAGTCTTGTAAATGCGGGCCAAGATCTG
CACACTGGTATTTCGGTTTTTGGGGCCGCGGGCGGCGACGGGGCCCGTGCGTC
CCAGCGCACATGTTCGGCGAGGCGGGGCCTGCGAGCGCGGCCACCGAGAATC
GGACGGGGGTAGTCTCAAGCTGGCCGGCCTGCTCTGGTGCCTGGCCTCGCGC
CGCCGTGTATCGCCCCGCCCTGGGCGGCAAGGCTGGCCCGGTCGGCACCAGT
TGCGTGAGCGGAAAGATGGCCGCTTCCCGGCCCTGCTGCAGGGAGCTCAAAA
TGGAGGACGCGGCGCTCGGGAGAGCGGGCGGGTGAGTCACCCACACAAAGG
AAAAGGGCCTTTCCGTCCTCAGCCGTCGCTTCATGTGACTCCACTGAGTACCG
GGCGCCGTCCAGGCACCTCGATTAGTTCTCGTGCTTTTGGAGTACGTCGTCTT
TAGGTTGGGGGGAGGGGTTTTATGCGATGGAGTTTCCCCACACTGAGTGGGT
GGAGACTGAAGTTAGGCCAGCTTGGCACTTGATGTAATTCTCCTTGGAATTTG
CCCTTTTTGAGTTTGGATCTTGGTTCATTCTCAAGCCTCAGACAGTGGTTCAAA
GTTTTTTTCTTCCATTTCAGGTGTCGTGA
4. Spacer + Kozak sequence (this is the sequence between 3' end of the minimal promoter or any inducible promoter and ATG start codon of constitutively expressed module and enables functional spacing between transcription start site and translational start site) (SEQ ID NO: 8) GGCATTCCGGTACTGTTGGTAAAGCCACC
5. Example of a functionally connected regulatory unit for an inducible module followed by constitutive module in the same direction. Same configuration is used when inducible and constitutive modules are placed back to back in the opposite direction, where inducible module is placed in reverse and constitutive module in forward orientation. (SEQ ID NO: 9) 
GGAGGAAAAACTGTTTCATACAGAAGGCGTGGAGGAAAAACTGTTTCATACA
GAAGGCGTGGAGGAAAAACTGTTTCATACAGAAGGCGTGGAGGAAAAACTG
 
TTTCATACAGAAGGCGTGGAGGAAAAACTGTTTCATACAGAAGGCGTGGAGG
AAAAACTGTTTCATACAGAAGGCGTACTAGTTAGAGGGTATATAATGGAAGC
TCGACTTCCAGCCCGGGGGCATTCCGGTACTGTTGGTAAAGCCACC – followed
 by immune modulator starting with a start codon and ending with stop codon - followed by strong constitutive promoter such as EF1α - followed by immune receptor such as CAR or TCR starting with start codon and ending with stop codon.
   Synthetic NFAT promoter: underline
   Minimal promoter: wavy underline
   Spacer: double underline

### Constructs used for experiments:

1. pASP4.2 (orientation of inducible and constitutive modules: same direction) (SEQ ID NO: 10) 
TCGAGTTAATTAAGCTAGCCCGGTACTGTTGGAGGAAAAACTGTTTCATACAGAAGGCGTGGAGG
AAAAACTGTTTCATACAGAAGGCGTGGAGGAAAAACTGTTTCATACAGAAGGCGTGGAGGAAAA
ACTGTTTCATACAGAAGGCGTGGAGGAAAAACTGTTTCATACAGAAGGCGTGGAGGAAAAACTGT
TTCATACAGAAGGCGTACTAGTTAGAGGGTATATAATGGAAGCTCGACTTCCAGCCCGGGGGCAT
TCCGGTACTGTTGGTAAAGCCACCATGGTGAGCAAGGGCGAGGAGCTGTTCACCGGGGTGGTGCC
CATCCTGGTCGAGCTGGACGGCGACGTAAACGGCCACAAGTTCAGCGTGTCCGGCGAGGGCGAGG
GCGATGCCACCTACGGCAAGCTGACCCTGAAGTTCATCTGCACCACCGGCAAGCTGCCCGTGCCCT
GGCCCACCCTCGTGACCACCCTGACCTACGGCGTGCAGTGCTTCAGCCGCTACCCCGACCACATGA
AGCAGCACGACTTCTTCAAGTCCGCCATGCCCGAAGGCTACGTCCAGGAGCGCACCATCTTCTTCA
AGGACGACGGCAACTACAAGACCCGCGCCGAGGTGAAGTTCGAGGGCGACACCCTGGTGAACCG
CATCGAGCTGAAGGGCATCGACTTCAAGGAGGACGGCAACATCCTGGGGCACAAGCTGGAGTACA
ACTACAACAGCCACAACGTCTATATCATGGCCGACAAGCAGAAGAACGGCATCAAGGTGAACTTC
AAGATCCGCCACAACATCGAGGACGGCAGCGTGCAGCTCGCCGACCACTACCAGCAGAACACCCC
CATCGGCGACGGCCCCGTGCTGCTGCCCGACAACCACTACCTGAGCACCCAGTCCGCCCTGAGCA
AAGACCCCAACGAGAAGCGCGATCACATGGTCCTGCTGGAGTTCGTGACCGCCGCCGGGATCACT
CTCGGCATGGACGAGCTGTACAAGTAAATGCATCGTGAGGCTCCGGTGCCCGTCAGTGGGCAGAG
CGCACATCGCCCACAGTCCCCGAGAAGTTGGGGGGAGGGGTCGGCAATTGAACCGGTGCCTAGAG
AAGGTGGCGCGGGGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAACGGGTTTGCCGCCA
GAACACAGGTAAGTGCCGTGTGTGGTTCCCGCGGGCCTGGCCTCTTTACGGGTTATGGCCCTTGCG
TGCCTTGAATTACTTCCACCTGGCTGCAGTACGTGATTCTTGATCCCGAGCTTCGGGTTGGAAGTG
GGTGGGAGAGTTCGAGGCCTTGCGCTTAAGGAGCCCCTTCGCCTCGTGCTTGAGTTGAGGCCTGGC
CTGGGCGCTGGGGCCGCCGCGTGCGAATCTGGTGGCACCTTCGCGCCTGTCTCGCTGCTTTCGATA
AGTCTCTAGCCATTTAAAATTTTTGATGACCTGCTGCGACGCTTTTTTTCTGGCAAGATAGTCTTGT
AAATGCGGGCCAAGATCTGCACACTGGTATTTCGGTTTTTGGGGCCGCGGGCGGCGACGGGGCCC
GTGCGTCCCAGCGCACATGTTCGGCGAGGCGGGGCCTGCGAGCGCGGCCACCGAGAATCGGACGG
GGGTAGTCTCAAGCTGGCCGGCCTGCTCTGGTGCCTGGCCTCGCGCCGCCGTGTATCGCCCCGCCC
TGGGCGGCAAGGCTGGCCCGGTCGGCACCAGTTGCGTGAGCGGAAAGATGGCCGCTTCCCGGCCC
TGCTGCAGGGAGCTCAAAATGGAGGACGCGGCGCTCGGGAGAGCGGGCGGGTGAGTCACCCACA
CAAAGGAAAAGGGCCTTTCCGTCCTCAGCCGTCGCTTCATGTGACTCCACTGAGTACCGGGCGCCG
TCCAGGCACCTCGATTAGTTCTCGTGCTTTTGGAGTACGTCGTCTTTAGGTTGGGGGGAGGGGTTTT
ATGCGATGGAGTTTCCCCACACTGAGTGGGTGGAGACTGAAGTTAGGCCAGCTTGGCACTTGATGT
AATTCTCCTTGGAATTTGCCCTTTTTGAGTTTGGATCTTGGTTCATTCTCAAGCCTCAGACAGTGGT
TCAAAGTTTTTTTCTTCCATTTCAGGTGTCGTGATTCTAGAGCCACCATGGTGAGCAAGGGCGAGG
AGGATAACATGGCCATCATCAAGGAGTTCATGCGCTTCAAGGTGCACATGGAGGGCTCCGTGAAC
GGCCACGAGTTCGAGATCGAGGGCGAGGGCGAGGGCCGCCCCTACGAGGGCACCCAGACCGCCA
AGCTGAAGGTGACCAAGGGTGGCCCCCTGCCCTTCGCCTGGGACATCCTGTCCCCTCAGTTCATGT
ACGGCTCCAAGGCCTACGTGAAGCACCCCGCCGACATCCCCGACTACTTGAAGCTGTCCTTCCCCG
AGGGCTTCAAGTGGGAGCGCGTGATGAACTTCGAGGACGGCGGCGTGGTGACCGTGACCCAGGAC
TCCTCCCTGCAGGACGGCGAGTTCATCTACAAGGTGAAGCTGCGCGGCACCAACTTCCCCTCCGAC
GGCCCCGTAATGCAGAAGAAGACCATGGGCTGGGAGGCCTCCTCCGAGCGGATGTACCCCGAGGA
CGGCGCCCTGAAGGGCGAGATCAAGCAGAGGCTGAAGCTGAAGGACGGCGGCCACTACGACGCT
 
GAGGTCAAGACCACCTACAAGGCCAAGAAGCCCGTGCAGCTGCCCGGCGCCTACAACGTCAACAT
CAAGTTGGACATCACCTCCCACAACGAGGACTACACCATCGTGGAACAGTACGAACGCGCCGAGG
GCCGCCACTCCACCGGCGGCATGGACGAGCTGTACAAGAGGGCCAAGAGGCGTACGTAAG
2. pASPS (orientation of inducible and constitutive modules: opposite direction) (SEQ ID NO: 11) 
TCGAGTTAATTAAGCTAGCTTACTTGTACAGCTCGTCCATGCCGAGAGTGATCCCGGCGGCGGTCA
CGAACTCCAGCAGGACCATGTGATCGCGCTTCTCGTTGGGGTCTTTGCTCAGGGCGGACTGGGTGC
TCAGGTAGTGGTTGTCGGGCAGCAGCACGGGGCCGTCGCCGATGGGGGTGTTCTGCTGGTAGTGG
TCGGCGAGCTGCACGCTGCCGTCCTCGATGTTGTGGCGGATCTTGAAGTTCACCTTGATGCCGTTCT
TCTGCTTGTCGGCCATGATATAGACGTTGTGGCTGTTGTAGTTGTACTCCAGCTTGTGCCCCAGGAT
GTTGCCGTCCTCCTTGAAGTCGATGCCCTTCAGCTCGATGCGGTTCACCAGGGTGTCGCCCTCGAA
CTTCACCTCGGCGCGGGTCTTGTAGTTGCCGTCGTCCTTGAAGAAGATGGTGCGCTCCTGGACGTA
GCCTTCGGGCATGGCGGACTTGAAGAAGTCGTGCTGCTTCATGTGGTCGGGGTAGCGGCTGAAGC
ACTGCACGCCGTAGGTCAGGGTGGTCACGAGGGTGGGCCAGGGCACGGGCAGCTTGCCGGTGGTG
CAGATGAACTTCAGGGTCAGCTTGCCGTAGGTGGCATCGCCCTCGCCCTCGCCGGACACGCTGAAC
TTGTGGCCGTTTACGTCGCCGTCCAGCTCGACCAGGATGGGCACCACCCCGGTGAACAGCTCCTCG
CCCTTGCTCACCATGGTGGCTTTACCAACAGTACCGGAATGCCCCCGGGCTGGAAGTCGAGCTTCC
ATTATATACCCTCTAACTAGTACGCCTTCTGTATGAAACAGTTTTTCCTCCACGCCTTCTGTATGAA
ACAGTTTTTCCTCCACGCCTTCTGTATGAAACAGTTTTTCCTCCACGCCTTCTGTATGAAACAGTTT
TTCCTCCACGCCTTCTGTATGAAACAGTTTTTCCTCCACGCCTTCTGTATGAAACAGTTTTTCCTCCA
ACAGTACCGGATGCATCGTGAGGCTCCGGTGCCCGTCAGTGGGCAGAGCGCACATCGCCCACAGT
CCCCGAGAAGTTGGGGGGAGGGGTCGGCAATTGAACCGGTGCCTAGAGAAGGTGGCGCGGGGTA
AACTGGGAAAGTGATGTCGTGTACTGGCTCCGCCTTTTTCCCGAGGGTGGGGGAGAACCGTATATA
AGTGCAGTAGTCGCCGTGAACGTTCTTTTTCGCAACGGGTTTGCCGCCAGAACACAGGTAAGTGCC
GTGTGTGGTTCCCGCGGGCCTGGCCTCTTTACGGGTTATGGCCCTTGCGTGCCTTGAATTACTTCCA
CCTGGCTGCAGTACGTGATTCTTGATCCCGAGCTTCGGGTTGGAAGTGGGTGGGAGAGTTCGAGGC
CTTGCGCTTAAGGAGCCCCTTCGCCTCGTGCTTGAGTTGAGGCCTGGCCTGGGCGCTGGGGCCGCC
GCGTGCGAATCTGGTGGCACCTTCGCGCCTGTCTCGCTGCTTTCGATAAGTCTCTAGCCATTTAAAA
TTTTTGATGACCTGCTGCGACGCTTTTTTTCTGGCAAGATAGTCTTGTAAATGCGGGCCAAGATCTG
CACACTGGTATTTCGGTTTTTGGGGCCGCGGGCGGCGACGGGGCCCGTGCGTCCCAGCGCACATGT
TCGGCGAGGCGGGGCCTGCGAGCGCGGCCACCGAGAATCGGACGGGGGTAGTCTCAAGCTGGCCG
GCCTGCTCTGGTGCCTGGCCTCGCGCCGCCGTGTATCGCCCCGCCCTGGGCGGCAAGGCTGGCCCG
GTCGGCACCAGTTGCGTGAGCGGAAAGATGGCCGCTTCCCGGCCCTGCTGCAGGGAGCTCAAAAT
GGAGGACGCGGCGCTCGGGAGAGCGGGCGGGTGAGTCACCCACACAAAGGAAAAGGGCCTTTCC
GTCCTCAGCCGTCGCTTCATGTGACTCCACTGAGTACCGGGCGCCGTCCAGGCACCTCGATTAGTT
CTCGTGCTTTTGGAGTACGTCGTCTTTAGGTTGGGGGGAGGGGTTTTATGCGATGGAGTTTCCCCA
CACTGAGTGGGTGGAGACTGAAGTTAGGCCAGCTTGGCACTTGATGTAATTCTCCTTGGAATTTGC
CCTTTTTGAGTTTGGATCTTGGTTCATTCTCAAGCCTCAGACAGTGGTTCAAAGTTTTTTTCTTCCAT
TTCAGGTGTCGTGATTCTAGAGCCACCATGGTGAGCAAGGGCGAGGAGGATAACATGGCCATCAT
CAAGGAGTTCATGCGCTTCAAGGTGCACATGGAGGGCTCCGTGAACGGCCACGAGTTCGAGATCG
AGGGCGAGGGCGAGGGCCGCCCCTACGAGGGCACCCAGACCGCCAAGCTGAAGGTGACCAAGGG
TGGCCCCCTGCCCTTCGCCTGGGACATCCTGTCCCCTCAGTTCATGTACGGCTCCAAGGCCTACGTG
AAGCACCCCGCCGACATCCCCGACTACTTGAAGCTGTCCTTCCCCGAGGGCTTCAAGTGGGAGCGC
GTGATGAACTTCGAGGACGGCGGCGTGGTGACCGTGACCCAGGACTCCTCCCTGCAGGACGGCGA
GTTCATCTACAAGGTGAAGCTGCGCGGCACCAACTTCCCCTCCGACGGCCCCGTAATGCAGAAGA
AGACCATGGGCTGGGAGGCCTCCTCCGAGCGGATGTACCCCGAGGACGGCGCCCTGAAGGGCGAG
ATCAAGCAGAGGCTGAAGCTGAAGGACGGCGGCCACTACGACGCTGAGGTCAAGACCACCTACA
AGGCCAAGAAGCCCGTGCAGCTGCCCGGCGCCTACAACGTCAACATCAAGTTGGACATCACCTCC
CACAACGAGGACTACACCATCGTGGAACAGTACGAACGCGCCGAGGGCCGCCACTCCACCGGCGG
CATGGACGAGCTGTACAAGAGGGCCAAGAGGCGTACGTAAG
3. pASP7 (orientation of inducible and constitutive modules: opposite direction) (SEQ ID NO: 12) 
TCGAGTACCACATTTGTAGAGGTTTTACTTGCTTTAAAAAACCTCCCACACCTCCCCCTGAACCTGA
AACATAAAATGAATGCAATTGTTGTTGTTAACTTGTTTATTGCAGCTTATAATGGTTACAAATAAA
GCAATAGCATCACAAATTTCACAAATAAAGCATTTTTTTCACTGCATTCTAGTTGTGGTTTGTCCAA
ACTCATCAATGTATCTTATCATGTCTGTTAATTAAGCTAGCTTACTTGTACAGCTCGTCCATGCCGA
GAGTGATCCCGGCGGCGGTCACGAACTCCAGCAGGACCATGTGATCGCGCTTCTCGTTGGGGTCTT
 
TGCTCAGGGCGGACTGGGTGCTCAGGTAGTGGTTGTCGGGCAGCAGCACGGGGCCGTCGCCGATG
GGGGTGTTCTGCTGGTAGTGGTCGGCGAGCTGCACGCTGCCGTCCTCGATGTTGTGGCGGATCTTG
AAGTTCACCTTGATGCCGTTCTTCTGCTTGTCGGCCATGATATAGACGTTGTGGCTGTTGTAGTTGT
ACTCCAGCTTGTGCCCCAGGATGTTGCCGTCCTCCTTGAAGTCGATGCCCTTCAGCTCGATGCGGTT
CACCAGGGTGTCGCCCTCGAACTTCACCTCGGCGCGGGTCTTGTAGTTGCCGTCGTCCTTGAAGAA
GATGGTGCGCTCCTGGACGTAGCCTTCGGGCATGGCGGACTTGAAGAAGTCGTGCTGCTTCATGTG
GTCGGGGTAGCGGCTGAAGCACTGCACGCCGTAGGTCAGGGTGGTCACGAGGGTGGGCCAGGGCA
CGGGCAGCTTGCCGGTGGTGCAGATGAACTTCAGGGTCAGCTTGCCGTAGGTGGCATCGCCCTCGC
CCTCGCCGGACACGCTGAACTTGTGGCCGTTTACGTCGCCGTCCAGCTCGACCAGGATGGGCACCA
CCCCGGTGAACAGCTCCTCGCCCTTGCTCACCATGGTGGCTTTACCAACAGTACCGGAATGCCCCC
GGGCTGGAAGTCGAGCTTCCATTATATACCCTCTAACTAGTACGCCTTCTGTATGAAACAGTTTTTC
CTCCACGCCTTCTGTATGAAACAGTTTTTCCTCCACGCCTTCTGTATGAAACAGTTTTTCCTCCACG
CCTTCTGTATGAAACAGTTTTTCCTCCACGCCTTCTGTATGAAACAGTTTTTCCTCCACGCCTTCTGT
ATGAAACAGTTTTTCCTCCAACAGTACCGGATGCATCGTGAGGCTCCGGTGCCCGTCAGTGGGCAG
AGCGCACATCGCCCACAGTCCCCGAGAAGTTGGGGGGAGGGGTCGGCAATTGAACCGGTGCCTAG
AGAAGGTGGCGCGGGGTAAACTGGGAAAGTGATGTCGTGTACTGGCTCCGCCTTTTTCCCGAGGG
TGGGGGAGAACCGTATATAAGTGCAGTAGTCGCCGTGAACGTTCTTTTTCGCAACGGGTTTGCCGC
CAGAACACAGGTAAGTGCCGTGTGTGGTTCCCGCGGGCCTGGCCTCTTTACGGGTTATGGCCCTTG
CGTGCCTTGAATTACTTCCACCTGGCTGCAGTACGTGATTCTTGATCCCGAGCTTCGGGTTGGAAGT
GGGTGGGAGAGTTCGAGGCCTTGCGCTTAAGGAGCCCCTTCGCCTCGTGCTTGAGTTGAGGCCTGG
CCTGGGCGCTGGGGCCGCCGCGTGCGAATCTGGTGGCACCTTCGCGCCTGTCTCGCTGCTTTCGAT
AAGTCTCTAGCCATTTAAAATTTTTGATGACCTGCTGCGACGCTTTTTTTCTGGCAAGATAGTCTTG
TAAATGCGGGCCAAGATCTGCACACTGGTATTTCGGTTTTTGGGGCCGCGGGCGGCGACGGGGCC
CGTGCGTCCCAGCGCACATGTTCGGCGAGGCGGGGCCTGCGAGCGCGGCCACCGAGAATCGGACG
GGGGTAGTCTCAAGCTGGCCGGCCTGCTCTGGTGCCTGGCCTCGCGCCGCCGTGTATCGCCCCGCC
CTGGGCGGCAAGGCTGGCCCGGTCGGCACCAGTTGCGTGAGCGGAAAGATGGCCGCTTCCCGGCC
CTGCTGCAGGGAGCTCAAAATGGAGGACGCGGCGCTCGGGAGAGCGGGCGGGTGAGTCACCCAC
ACAAAGGAAAAGGGCCTTTCCGTCCTCAGCCGTCGCTTCATGTGACTCCACTGAGTACCGGGCGCC
GTCCAGGCACCTCGATTAGTTCTCGTGCTTTTGGAGTACGTCGTCTTTAGGTTGGGGGGAGGGGTT
TTATGCGATGGAGTTTCCCCACACTGAGTGGGTGGAGACTGAAGTTAGGCCAGCTTGGCACTTGAT
GTAATTCTCCTTGGAATTTGCCCTTTTTGAGTTTGGATCTTGGTTCATTCTCAAGCCTCAGACAGTG
GTTCAAAGTTTTTTTCTTCCATTTCAGGTGTCGTGATTCTAGAGCCACCATGGTGAGCAAGGGCGA
GGAGGATAACATGGCCATCATCAAGGAGTTCATGCGCTTCAAGGTGCACATGGAGGGCTCCGTGA
ACGGCCACGAGTTCGAGATCGAGGGCGAGGGCGAGGGCCGCCCCTACGAGGGCACCCAGACCGC
CAAGCTGAAGGTGACCAAGGGTGGCCCCCTGCCCTTCGCCTGGGACATCCTGTCCCCTCAGTTCAT
GTACGGCTCCAAGGCCTACGTGAAGCACCCCGCCGACATCCCCGACTACTTGAAGCTGTCCTTCCC
CGAGGGCTTCAAGTGGGAGCGCGTGATGAACTTCGAGGACGGCGGCGTGGTGACCGTGACCCAGG
ACTCCTCCCTGCAGGACGGCGAGTTCATCTACAAGGTGAAGCTGCGCGGCACCAACTTCCCCTCCG
ACGGCCCCGTAATGCAGAAGAAGACCATGGGCTGGGAGGCCTCCTCCGAGCGGATGTACCCCGAG
GACGGCGCCCTGAAGGGCGAGATCAAGCAGAGGCTGAAGCTGAAGGACGGCGGCCACTACGACG
CTGAGGTCAAGACCACCTACAAGGCCAAGAAGCCCGTGCAGCTGCCCGGCGCCTACAACGTCAAC
ATCAAGTTGGACATCACCTCCCACAACGAGGACTACACCATCGTGGAACAGTACGAACGCGCCGA
GGGCCGCCACTCCACCGGCGGCATGGACGAGCTGTACAAGAGGGCCAAGAGGCGTACGTAAG
4. pASP8 (orientation of inducible and constitutive modules: same direction) (SEQ ID NO: 13) 
TCGAGTTAATTAAGCTAGCAAGGCTTTCTGTTTCCTGCATTCTTACTTCTTACTACGTGATACATCT
AGTCACCAGGGAAGAAGCGAATGACACACTTCCAAAAACCAATTCGTAGCTTTCTAAATAAAACC
CTTTCTAGCTGGAGAGAGATCCATGAGCATAGAGATCTTAAAATTCATGTTCAGCAATAAATCCTG
GGGCCCCAGACAGTGTCAGGTGCATAGGGGGTGTTCAGTAAATATCAGTTAAATGTTGCATAAAT
CCGATAAACGGGATTCCTGGAAAATACTACACTCTCCTTCTCCAAATTATCTTCATCTCAAAGACA
GGAACCTCTAACTTTTAATTCTTTACTTAGATTATGCTGTCTCCTAAACTGTTTATGTTTTCTGAAAT
TTAAGGCAGGATGTCTCAGAGTCTGGGAAAATCCCACTTTCCTCCTGCTACACCTTACAGTTGTGA
GAAAGCACATTTCAGACAACAGGGAAAACCCATACTTCACCACAACAACACACTATACATTGTCT
GGTCCACTGGAGCATAAATTAAAGAGAAACAATGTAGTCAAGCAAGTAGGCGGCAAGAGGAAGG
GGGCGGAGACATCATCAGGGAGTATAAACTCTGAGATGCCTCAGAGCCTCACAGACTCAACAAGA
GCTCCAGCAAAGACTTTCACTGTAGCTTGACTTGACCTGAGATTAACTAGGGAATCTTGAGAATAA
AGCCCGGGGGCATTCCGGTACTGTTGGTAAAGCCACCATGGTGAGCAAGGGCGAGGAGCTGTTCA
CCGGGGTGGTGCCCATCCTGGTCGAGCTGGACGGCGACGTAAACGGCCACAAGTTCAGCGTGTCC
GGCGAGGGCGAGGGCGATGCCACCTACGGCAAGCTGACCCTGAAGTTCATCTGCACCACCGGCAA
 
GCTGCCCGTGCCCTGGCCCACCCTCGTGACCACCCTGACCTACGGCGTGCAGTGCTTCAGCCGCTA
CCCCGACCACATGAAGCAGCACGACTTCTTCAAGTCCGCCATGCCCGAAGGCTACGTCCAGGAGC
GCACCATCTTCTTCAAGGACGACGGCAACTACAAGACCCGCGCCGAGGTGAAGTTCGAGGGCGAC
ACCCTGGTGAACCGCATCGAGCTGAAGGGCATCGACTTCAAGGAGGACGGCAACATCCTGGGGCA
CAAGCTGGAGTACAACTACAACAGCCACAACGTCTATATCATGGCCGACAAGCAGAAGAACGGCA
TCAAGGTGAACTTCAAGATCCGCCACAACATCGAGGACGGCAGCGTGCAGCTCGCCGACCACTAC
CAGCAGAACACCCCCATCGGCGACGGCCCCGTGCTGCTGCCCGACAACCACTACCTGAGCACCCA
GTCCGCCCTGAGCAAAGACCCCAACGAGAAGCGCGATCACATGGTCCTGCTGGAGTTCGTGACCG
CCGCCGGGATCACTCTCGGCATGGACGAGCTGTACAAGTAAATGCATCGTGAGGCTCCGGTGCCC
GTCAGTGGGCAGAGCGCACATCGCCCACAGTCCCCGAGAAGTTGGGGGGAGGGGTCGGCAATTGA
ACCGGTGCCTAGAGAAGGTGGCGCGGGGTAAACTGGGAAAGTGATGTCGTGTACTGGCTCCGCCT
TTTTCCCGAGGGTGGGGGAGAACCGTATATAAGTGCAGTAGTCGCCGTGAACGTTCTTTTTCGCAA
CGGGTTTGCCGCCAGAACACAGGTAAGTGCCGTGTGTGGTTCCCGCGGGCCTGGCCTCTTTACGGG
TTATGGCCCTTGCGTGCCTTGAATTACTTCCACCTGGCTGCAGTACGTGATTCTTGATCCCGAGCTT
CGGGTTGGAAGTGGGTGGGAGAGTTCGAGGCCTTGCGCTTAAGGAGCCCCTTCGCCTCGTGCTTGA
GTTGAGGCCTGGCCTGGGCGCTGGGGCCGCCGCGTGCGAATCTGGTGGCACCTTCGCGCCTGTCTC
GCTGCTTTCGATAAGTCTCTAGCCATTTAAAATTTTTGATGACCTGCTGCGACGCTTTTTTTCTGGC
AAGATAGTCTTGTAAATGCGGGCCAAGATCTGCACACTGGTATTTCGGTTTTTGGGGCCGCGGGCG
GCGACGGGGCCCGTGCGTCCCAGCGCACATGTTCGGCGAGGCGGGGCCTGCGAGCGCGGCCACCG
AGAATCGGACGGGGGTAGTCTCAAGCTGGCCGGCCTGCTCTGGTGCCTGGCCTCGCGCCGCCGTGT
ATCGCCCCGCCCTGGGCGGCAAGGCTGGCCCGGTCGGCACCAGTTGCGTGAGCGGAAAGATGGCC
GCTTCCCGGCCCTGCTGCAGGGAGCTCAAAATGGAGGACGCGGCGCTCGGGAGAGCGGGCGGGTG
AGTCACCCACACAAAGGAAAAGGGCCTTTCCGTCCTCAGCCGTCGCTTCATGTGACTCCACTGAGT
ACCGGGCGCCGTCCAGGCACCTCGATTAGTTCTCGTGCTTTTGGAGTACGTCGTCTTTAGGTTGGG
GGGAGGGGTTTTATGCGATGGAGTTTCCCCACACTGAGTGGGTGGAGACTGAAGTTAGGCCAGCT
TGGCACTTGATGTAATTCTCCTTGGAATTTGCCCTTTTTGAGTTTGGATCTTGGTTCATTCTCAAGCC
TCAGACAGTGGTTCAAAGTTTTTTTCTTCCATTTCAGGTGTCGTGATTCTAGAGCCACCATGGTGAG
CAAGGGCGAGGAGGATAACATGGCCATCATCAAGGAGTTCATGCGCTTCAAGGTGCACATGGAGG
GCTCCGTGAACGGCCACGAGTTCGAGATCGAGGGCGAGGGCGAGGGCCGCCCCTACGAGGGCACC
CAGACCGCCAAGCTGAAGGTGACCAAGGGTGGCCCCCTGCCCTTCGCCTGGGACATCCTGTCCCCT
CAGTTCATGTACGGCTCCAAGGCCTACGTGAAGCACCCCGCCGACATCCCCGACTACTTGAAGCTG
TCCTTCCCCGAGGGCTTCAAGTGGGAGCGCGTGATGAACTTCGAGGACGGCGGCGTGGTGACCGT
GACCCAGGACTCCTCCCTGCAGGACGGCGAGTTCATCTACAAGGTGAAGCTGCGCGGCACCAACT
TCCCCTCCGACGGCCCCGTAATGCAGAAGAAGACCATGGGCTGGGAGGCCTCCTCCGAGCGGATG
TACCCCGAGGACGGCGCCCTGAAGGGCGAGATCAAGCAGAGGCTGAAGCTGAAGGACGGCGGCC
ACTACGACGCTGAGGTCAAGACCACCTACAAGGCCAAGAAGCCCGTGCAGCTGCCCGGCGCCTAC
AACGTCAACATCAAGTTGGACATCACCTCCCACAACGAGGACTACACCATCGTGGAACAGTACGA
ACGCGCCGAGGGCCGCCACTCCACCGGCGGCATGGACGAGCTGTACAAGAGGGCCAAGAGGCGT
ACGTAAG
5. pASP9 (orientation of inducible and constitutive modules: same direction) (SEQ ID NO: 14) 
TCGAGTTAATTAAGCTAGCAATGCTTTGTCCTGGAGAGCTATCTTAAGGGACAAAATCGTTTTCCC
AGCGTCATCTGTGACACATCCTGACAGTAGAGAGCTGCTTCCAAGAAGCAATTTGAAGTGCCATTA
TCAGGCAGGGACGGGGGCTCTAGGGGATTTCGGGGTCAGCAGATATGAAATGAATGATTTCATAG
GGCTGTCACAGAGCTGTGGTGGGAATTTCCCATGAGACCCCGCCCCTGGCTGAGTCACCGCACTCC
TGTGTTTGACCTGAAGTCCTCTCGTGCTGCAGAAGCCTGAAGACCAAGGAGTGGAAAGTTCTCCGG
CAGCCCTGAGATCTCCCGGGGGCATTCCGGTACTGTTGGTAAAGCCACCATGGTGAGCAAGGGCG
AGGAGCTGTTCACCGGGGTGGTGCCCATCCTGGTCGAGCTGGACGGCGACGTAAACGGCCACAAG
TTCAGCGTGTCCGGCGAGGGCGAGGGCGATGCCACCTACGGCAAGCTGACCCTGAAGTTCATCTG
CACCACCGGCAAGCTGCCCGTGCCCTGGCCCACCCTCGTGACCACCCTGACCTACGGCGTGCAGTG
CTTCAGCCGCTACCCCGACCACATGAAGCAGCACGACTTCTTCAAGTCCGCCATGCCCGAAGGCTA
CGTCCAGGAGCGCACCATCTTCTTCAAGGACGACGGCAACTACAAGACCCGCGCCGAGGTGAAGT
TCGAGGGCGACACCCTGGTGAACCGCATCGAGCTGAAGGGCATCGACTTCAAGGAGGACGGCAAC
ATCCTGGGGCACAAGCTGGAGTACAACTACAACAGCCACAACGTCTATATCATGGCCGACAAGCA
GAAGAACGGCATCAAGGTGAACTTCAAGATCCGCCACAACATCGAGGACGGCAGCGTGCAGCTCG
CCGACCACTACCAGCAGAACACCCCCATCGGCGACGGCCCCGTGCTGCTGCCCGACAACCACTAC
CTGAGCACCCAGTCCGCCCTGAGCAAAGACCCCAACGAGAAGCGCGATCACATGGTCCTGCTGGA
GTTCGTGACCGCCGCCGGGATCACTCTCGGCATGGACGAGCTGTACAAGTAAATGCATCGTGAGG
CTCCGGTGCCCGTCAGTGGGCAGAGCGCACATCGCCCACAGTCCCCGAGAAGTTGGGGGGAGGGG
 
TCGGCAATTGAACCGGTGCCTAGAGAAGGTGGCGCGGGGTAAACTGGGAAAGTGATGTCGTGTAC
TGGCTCCGCCTTTTTCCCGAGGGTGGGGGAGAACCGTATATAAGTGCAGTAGTCGCCGTGAACGTT
CTTTTTCGCAACGGGTTTGCCGCCAGAACACAGGTAAGTGCCGTGTGTGGTTCCCGCGGGCCTGGC
CTCTTTACGGGTTATGGCCCTTGCGTGCCTTGAATTACTTCCACCTGGCTGCAGTACGTGATTCTTG
ATCCCGAGCTTCGGGTTGGAAGTGGGTGGGAGAGTTCGAGGCCTTGCGCTTAAGGAGCCCCTTCGC
CTCGTGCTTGAGTTGAGGCCTGGCCTGGGCGCTGGGGCCGCCGCGTGCGAATCTGGTGGCACCTTC
GCGCCTGTCTCGCTGCTTTCGATAAGTCTCTAGCCATTTAAAATTTTTGATGACCTGCTGCGACGCT
TTTTTTCTGGCAAGATAGTCTTGTAAATGCGGGCCAAGATCTGCACACTGGTATTTCGGTTTTTGGG
GCCGCGGGCGGCGACGGGGCCCGTGCGTCCCAGCGCACATGTTCGGCGAGGCGGGGCCTGCGAGC
GCGGCCACCGAGAATCGGACGGGGGTAGTCTCAAGCTGGCCGGCCTGCTCTGGTGCCTGGCCTCG
CGCCGCCGTGTATCGCCCCGCCCTGGGCGGCAAGGCTGGCCCGGTCGGCACCAGTTGCGTGAGCG
GAAAGATGGCCGCTTCCCGGCCCTGCTGCAGGGAGCTCAAAATGGAGGACGCGGCGCTCGGGAGA
GCGGGCGGGTGAGTCACCCACACAAAGGAAAAGGGCCTTTCCGTCCTCAGCCGTCGCTTCATGTG
ACTCCACTGAGTACCGGGCGCCGTCCAGGCACCTCGATTAGTTCTCGTGCTTTTGGAGTACGTCGT
CTTTAGGTTGGGGGGAGGGGTTTTATGCGATGGAGTTTCCCCACACTGAGTGGGTGGAGACTGAA
GTTAGGCCAGCTTGGCACTTGATGTAATTCTCCTTGGAATTTGCCCTTTTTGAGTTTGGATCTTGGT
TCATTCTCAAGCCTCAGACAGTGGTTCAAAGTTTTTTTCTTCCATTTCAGGTGTCGTGATTCTAGAG
CCACCATGGTGAGCAAGGGCGAGGAGGATAACATGGCCATCATCAAGGAGTTCATGCGCTTCAAG
GTGCACATGGAGGGCTCCGTGAACGGCCACGAGTTCGAGATCGAGGGCGAGGGCGAGGGCCGCC
CCTACGAGGGCACCCAGACCGCCAAGCTGAAGGTGACCAAGGGTGGCCCCCTGCCCTTCGCCTGG
GACATCCTGTCCCCTCAGTTCATGTACGGCTCCAAGGCCTACGTGAAGCACCCCGCCGACATCCCC
GACTACTTGAAGCTGTCCTTCCCCGAGGGCTTCAAGTGGGAGCGCGTGATGAACTTCGAGGACGG
CGGCGTGGTGACCGTGACCCAGGACTCCTCCCTGCAGGACGGCGAGTTCATCTACAAGGTGAAGC
TGCGCGGCACCAACTTCCCCTCCGACGGCCCCGTAATGCAGAAGAAGACCATGGGCTGGGAGGCC
TCCTCCGAGCGGATGTACCCCGAGGACGGCGCCCTGAAGGGCGAGATCAAGCAGAGGCTGAAGCT
GAAGGACGGCGGCCACTACGACGCTGAGGTCAAGACCACCTACAAGGCCAAGAAGCCCGTGCAG
CTGCCCGGCGCCTACAACGTCAACATCAAGTTGGACATCACCTCCCACAACGAGGACTACACCATC
GTGGAACAGTACGAACGCGCCGAGGGCCGCCACTCCACCGGCGGCATGGACGAGCTGTACAAGA
GGGCCAAGAGGCGTACGTAAG
6. pASP18 (orientation of inducible and constitutive modules: same direction) (SEQ ID NO: 15) 
TCGAGTTAATTAAGCTAGCCCGGTACTGTTGGAGGAAAAACTGTTTCATACAGAAGGCGTGGAGG
AAAAACTGTTTCATACAGAAGGCGTGGAGGAAAAACTGTTTCATACAGAAGGCGTGGAGGAAAA
ACTGTTTCATACAGAAGGCGTGGAGGAAAAACTGTTTCATACAGAAGGCGTGGAGGAAAAACTGT
TTCATACAGAAGGCGTACTAGTTAGAGGGTATATAATGGAAGCTCGACTTCCAGCCCGGGGGCAT
TCCGGTACTGTTGGTAAAGCCACCATGTGCCACCAGCAGCTGGTCATCAGCTGGTTCAGCCTGGTG
TTCCTGGCCAGCCCTCTGGTGGCCATCTGGGAGCTGAAGAAAGACGTGTACGTGGTGGAACTGGA
CTGGTATCCCGACGCCCCTGGCGAGATGGTGGTGCTGACCTGCGACACCCCTGAAGAGGACGGCA
TCACCTGGACCCTGGACCAGTCTAGCGAGGTGCTGGGCAGCGGCAAGACCCTGACCATCCAGGTC
AAAGAGTTCGGCGACGCCGGCCAGTACACCTGTCACAAGGGCGGAGAGGTGCTGAGCCACAGCCT
GCTGCTGCTCCACAAGAAAGAGGATGGCATTTGGAGCACCGACATCCTGAAGGACCAGAAAGAGC
CCAAGAACAAGACCTTCCTGAGATGCGAGGCCAAGAACTACAGCGGCCGGTTCACCTGTTGGTGG
CTGACCACCATCAGCACCGACCTGACCTTCAGCGTGAAGTCCAGCAGAGGCAGCAGCGACCCTCA
GGGCGTGACATGTGGCGCCGCTACACTGTCTGCCGAGAGAGTGCGGGGCGACAACAAAGAGTACG
AGTACAGCGTCGAGTGCCAGGAAGATAGCGCCTGCCCTGCCGCCGAGGAAAGCCTGCCTATCGAA
GTGATGGTGGACGCCGTGCACAAGCTGAAGTACGAGAACTACACCTCCAGCTTTTTCATCCGGGAC
ATCATCAAGCCCGACCCTCCCAAGAACCTGCAGCTGAAGCCTCTGAAGAACAGCAGACAGGTGGA
AGTGTCCTGGGAGTACCCCGACACCTGGTCCACCCCTCACAGCTACTTCAGCCTGACATTCTGTGT
GCAAGTCCAGGGCAAGTCCAAGCGCGAGAAAAAGGACCGGGTGTTCACCGACAAGACCAGCGCC
ACCGTGATCTGCCGGAAGAACGCCTCTATCAGCGTGCGGGCCCAGGACCGGTACTACAGCAGCTC
TTGGAGCGAGTGGGCCAGCGTGCCATGTTCTGGTGGCGGAGGCGGCGGAAGCAGAAATCTGCCAG
TGGCCACCCCTGACCCCGGCATGTTTCCTTGTCTGCACCACAGCCAGAACCTGCTGCGGGCCGTGT
CCAACATGCTGCAGAAGGCCCGGCAGACCCTGGAATTCTACCCCTGCACCAGCGAGGAAATCGAC
CACGAGGACATCACCAAGGATAAGACCAGCACCGTGGAAGCCTGTCTGCCCCTGGAACTGACCAA
GAACGAGAGCTGCCTGAACAGCCGGGAAACCAGCTTCATCACCAACGGCAGCTGTCTGGCCAGCA
GAAAGACCTCCTTCATGATGGCCCTGTGCCTGAGCAGCATCTACGAGGACCTGAAGATGTACCAG
GTCGAGTTCAAGACCATGAACGCCAAGCTGCTGATGGACCCCAAGCGGCAGATCTTTCTGGACCA
GAATATGCTGGCCGTGATCGACGAGCTGATGCAGGCCCTGAACTTCAACAGCGAGACAGTGCCCC
AGAAGTCCTCCCTGGAAGAACCCGACTTCTACAAGACCAAGATCAAGCTGTGCATCCTGCTGCAC
 
GCCTTCCGGATCAGAGCCGTGACCATCGACAGAGTGATGAGCTACCTGAACGCCTCCTAAATGCAT
CGTGAGGCTCCGGTGCCCGTCAGTGGGCAGAGCGCACATCGCCCACAGTCCCCGAGAAGTTGGGG
GGAGGGGTCGGCAATTGAACCGGTGCCTAGAGAAGGTGGCGCGGGGTAAACTGGGAAAGTGATG
TCGTGTACTGGCTCCGCCTTTTTCCCGAGGGTGGGGGAGAACCGTATATAAGTGCAGTAGTCGCCG
TGAACGTTCTTTTTCGCAACGGGTTTGCCGCCAGAACACAGGTAAGTGCCGTGTGTGGTTCCCGCG
GGCCTGGCCTCTTTACGGGTTATGGCCCTTGCGTGCCTTGAATTACTTCCACCTGGCTGCAGTACGT
GATTCTTGATCCCGAGCTTCGGGTTGGAAGTGGGTGGGAGAGTTCGAGGCCTTGCGCTTAAGGAGC
CCCTTCGCCTCGTGCTTGAGTTGAGGCCTGGCCTGGGCGCTGGGGCCGCCGCGTGCGAATCTGGTG
GCACCTTCGCGCCTGTCTCGCTGCTTTCGATAAGTCTCTAGCCATTTAAAATTTTTGATGACCTGCT
GCGACGCTTTTTTTCTGGCAAGATAGTCTTGTAAATGCGGGCCAAGATCTGCACACTGGTATTTCG
GTTTTTGGGGCCGCGGGCGGCGACGGGGCCCGTGCGTCCCAGCGCACATGTTCGGCGAGGCGGGG
CCTGCGAGCGCGGCCACCGAGAATCGGACGGGGGTAGTCTCAAGCTGGCCGGCCTGCTCTGGTGC
CTGGCCTCGCGCCGCCGTGTATCGCCCCGCCCTGGGCGGCAAGGCTGGCCCGGTCGGCACCAGTTG
CGTGAGCGGAAAGATGGCCGCTTCCCGGCCCTGCTGCAGGGAGCTCAAAATGGAGGACGCGGCGC
TCGGGAGAGCGGGCGGGTGAGTCACCCACACAAAGGAAAAGGGCCTTTCCGTCCTCAGCCGTCGC
TTCATGTGACTCCACTGAGTACCGGGCGCCGTCCAGGCACCTCGATTAGTTCTCGTGCTTTTGGAGT
ACGTCGTCTTTAGGTTGGGGGGAGGGGTTTTATGCGATGGAGTTTCCCCACACTGAGTGGGTGGAG
ACTGAAGTTAGGCCAGCTTGGCACTTGATGTAATTCTCCTTGGAATTTGCCCTTTTTGAGTTTGGAT
CTTGGTTCATTCTCAAGCCTCAGACAGTGGTTCAAAGTTTTTTTCTTCCATTTCAGGTGTCGTGATT
CTAGAGCCACCATGGTGAGCAAGGGCGAGGAGGATAACATGGCCATCATCAAGGAGTTCATGCGC
TTCAAGGTGCACATGGAGGGCTCCGTGAACGGCCACGAGTTCGAGATCGAGGGCGAGGGCGAGG
GCCGCCCCTACGAGGGCACCCAGACCGCCAAGCTGAAGGTGACCAAGGGTGGCCCCCTGCCCTTC
GCCTGGGACATCCTGTCCCCTCAGTTCATGTACGGCTCCAAGGCCTACGTGAAGCACCCCGCCGAC
ATCCCCGACTACTTGAAGCTGTCCTTCCCCGAGGGCTTCAAGTGGGAGCGCGTGATGAACTTCGAG
GACGGCGGCGTGGTGACCGTGACCCAGGACTCCTCCCTGCAGGACGGCGAGTTCATCTACAAGGT
GAAGCTGCGCGGCACCAACTTCCCCTCCGACGGCCCCGTAATGCAGAAGAAGACCATGGGCTGGG
AGGCCTCCTCCGAGCGGATGTACCCCGAGGACGGCGCCCTGAAGGGCGAGATCAAGCAGAGGCTG
AAGCTGAAGGACGGCGGCCACTACGACGCTGAGGTCAAGACCACCTACAAGGCCAAGAAGCCCG
TGCAGCTGCCCGGCGCCTACAACGTCAACATCAAGTTGGACATCACCTCCCACAACGAGGACTAC
ACCATCGTGGAACAGTACGAACGCGCCGAGGGCCGCCACTCCACCGGCGGCATGGACGAGCTGTA
CAAGAGGGCCAAGAGGCGTACGTAAG
7. pASP26 (orientation of inducible and constitutive modules: same direction) (SEQ ID NO: 16) 
TCGAGTTAATTAAGCTAGCCCGGTACTGTTGGAGGAAAAACTGTTTCATACAGAAGGCGTGGAGG
AAAAACTGTTTCATACAGAAGGCGTGGAGGAAAAACTGTTTCATACAGAAGGCGTGGAGGAAAA
ACTGTTTCATACAGAAGGCGTGGAGGAAAAACTGTTTCATACAGAAGGCGTGGAGGAAAAACTGT
TTCATACAGAAGGCGTACTAGTTAGAGGGTATATAATGGAAGCTCGACTTCCAGCCCGGGGGCAT
TCCGGTACTGTTGGTAAAGCCACCATGGTGAGCAAGGGCGAGGAGCTGTTCACCGGGGTGGTGCC
CATCCTGGTCGAGCTGGACGGCGACGTAAACGGCCACAAGTTCAGCGTGTCCGGCGAGGGCGAGG
GCGATGCCACCTACGGCAAGCTGACCCTGAAGTTCATCTGCACCACCGGCAAGCTGCCCGTGCCCT
GGCCCACCCTCGTGACCACCCTGACCTACGGCGTGCAGTGCTTCAGCCGCTACCCCGACCACATGA
AGCAGCACGACTTCTTCAAGTCCGCCATGCCCGAAGGCTACGTCCAGGAGCGCACCATCTTCTTCA
AGGACGACGGCAACTACAAGACCCGCGCCGAGGTGAAGTTCGAGGGCGACACCCTGGTGAACCG
CATCGAGCTGAAGGGCATCGACTTCAAGGAGGACGGCAACATCCTGGGGCACAAGCTGGAGTACA
ACTACAACAGCCACAACGTCTATATCATGGCCGACAAGCAGAAGAACGGCATCAAGGTGAACTTC
AAGATCCGCCACAACATCGAGGACGGCAGCGTGCAGCTCGCCGACCACTACCAGCAGAACACCCC
CATCGGCGACGGCCCCGTGCTGCTGCCCGACAACCACTACCTGAGCACCCAGTCCGCCCTGAGCA
AAGACCCCAACGAGAAGCGCGATCACATGGTCCTGCTGGAGTTCGTGACCGCCGCCGGGATCACT
CTCGGCATGGACGAGCTGTACAAGTAAATGCATCGTGAGGCTCCGGTGCCCGTCAGTGGGCAGAG
CGCACATCGCCCACAGTCCCCGAGAAGTTGGGGGGAGGGGTCGGCAATTGAACCGGTGCCTAGAG
AAGGTGGCGCGGGGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAACGGGTTTGCCGCCA
GAACACAGGTAAGTGCCGTGTGTGGTTCCCGCGGGCCTGGCCTCTTTACGGGTTATGGCCCTTGCG
TGCCTTGAATTACTTCCACCTGGCTGCAGTACGTGATTCTTGATCCCGAGCTTCGGGTTGGAAGTG
GGTGGGAGAGTTCGAGGCCTTGCGCTTAAGGAGCCCCTTCGCCTCGTGCTTGAGTTGAGGCCTGGC
CTGGGCGCTGGGGCCGCCGCGTGCGAATCTGGTGGCACCTTCGCGCCTGTCTCGCTGCTTTCGATA
AGTCTCTAGCCATTTAAAATTTTTGATGACCTGCTGCGACGCTTTTTTTCTGGCAAGATAGTCTTGT
 
AAATGCGGGCCAAGATCTGCACACTGGTATTTCGGTTTTTGGGGCCGCGGGCGGCGACGGGGCCC
GTGCGTCCCAGCGCACATGTTCGGCGAGGCGGGGCCTGCGAGCGCGGCCACCGAGAATCGGACGG
GGGTAGTCTCAAGCTGGCCGGCCTGCTCTGGTGCCTGGCCTCGCGCCGCCGTGTATCGCCCCGCCC
TGGGCGGCAAGGCTGGCCCGGTCGGCACCAGTTGCGTGAGCGGAAAGATGGCCGCTTCCCGGCCC
TGCTGCAGGGAGCTCAAAATGGAGGACGCGGCGCTCGGGAGAGCGGGCGGGTGAGTCACCCACA
CAAAGGAAAAGGGCCTTTCCGTCCTCAGCCGTCGCTTCATGTGACTCCACTGAGTACCGGGCGCCG
TCCAGGCACCTCGATTAGTTCTCGTGCTTTTGGAGTACGTCGTCTTTAGGTTGGGGGGAGGGGTTTT
ATGCGATGGAGTTTCCCCACACTGAGTGGGTGGAGACTGAAGTTAGGCCAGCTTGGCACTTGATGT
AATTCTCCTTGGAATTTGCCCTTTTTGAGTTTGGATCTTGGTTCATTCTCAAGCCTCAGACAGTGGT
TCAAAGTTTTTTTCTTCCATTTCAGGTGTCGTGATTCTAGAGCCACCATGGCCTTACCAGTGACCGC
CTTGCTCCTGCCGCTGGCCTTGCTGCTCCACGCCGCCAGGCCGGACATTCAAATGACACAGAGTCC
CTCATCCCTCAGTGCCAGCGTGGGCGATCGGGTGACTATAACCTGCAGAGCTTCTCAGGACGTGAA
TACCGCTGTGGCGTGGTACCAGCAGAAGCCAGGCAAAGCGCCTAAGCTTCTCATTTATAGTGCCAG
CTTCCTGTACTCAGGTGTTCCGTCTCGCTTTTCTGGAAGTAGAAGTGGGACCGATTTCACATTGACG
ATCAGCAGCTTGCAGCCCGAAGATTTCGCCACCTACTACTGTCAGCAGCACTACACTACCCCACCG
ACATTTGGTCAAGGCACAAAAGTAGAGATTAAACGCACTGGTTCCACCAGCGGGAGCGGGAAACC
CGGCTCTGGGGAGGGGAGCGAGGTCCAGCTGGTGGAATCCGGGGGTGGTCTTGTGCAGCCAGGAG
GATCCTTGAGGTTGTCCTGCGCCGCAAGCGGCTTTAACATCAAAGATACATACATCCATTGGGTCC
GACAGGCCCCTGGAAAGGGCCTGGAGTGGGTCGCCCGGATCTACCCAACTAACGGGTACACTCGC
TACGCTGATAGCGTCAAGGGTCGGTTTACTATTTCTGCCGACACCTCAAAAAACACAGCCTACCTC
CAGATGAACTCTCTCAGAGCTGAGGATACAGCCGTGTACTATTGCAGCCGGTGGGGAGGCGACGG
GTTCTACGCTATGGATGTGTGGGGGCAGGGCACACTGGTCACCGTGAGCTCATCCGGAACCACGA
CGCCAGCGCCGCGACCACCAACACCGGCGCCCACCATCGCGTCGCAGCCCCTGTCCCTGCGCCCA
GAGGCGTGCCGGCCAGCGGCGGGGGGCGCAGTGCACACGAGGGGGCTGGACTTCGCCTGTGATAT
CTACATCTGGGCGCCCTTGGCCGGGACTTGTGGGGTCCTTCTCCTGTCACTGGTTATCACCCTTTAC
TGCAAACGGGGCAGAAAGAAACTCCTGTATATATTCAAACAACCATTTATGAGACCAGTACAAAC
TACTCAAGAGGAAGATGGCTGTAGCTGCCGATTTCCAGAAGAAGAAGAAGGAGGATGTGAACTGA
GAGTGAAGTTCAGCAGGAGCGCAGACGCCCCCGCGTACAAGCAGGGCCAGAACCAGCTCTATAAC
GAGCTCAATCTAGGACGAAGAGAGGAGTACGATGTTTTGGACAAGAGACGTGGCCGGGACCCTGA
GATGGGGGGAAAGCCGAGAAGGAAGAACCCTCAGGAAGGCCTGTACAATGAACTGCAGAAAGAT
AAGATGGCGGAGGCCTACAGTGAGATTGGGATGAAAGGCGAGCGCCGGAGGGGCAAGGGGCACG
ATGGCCTTTACCAGGGTCTCAGTACAGCCACCAAGGACACCTACGACGCCCTTCACATGCAGGCCC
TGCCCCCTCGCTAAG
8. pASP28 (orientation of inducible and constitutive modules: opposite direction) (SEQ ID NO: 17) 
TCGAGTTAATTAAGCTAGCTTACTTGTACAGCTCGTCCATGCCGAGAGTGATCCCGGCGGCGGTCA
CGAACTCCAGCAGGACCATGTGATCGCGCTTCTCGTTGGGGTCTTTGCTCAGGGCGGACTGGGTGC
TCAGGTAGTGGTTGTCGGGCAGCAGCACGGGGCCGTCGCCGATGGGGGTGTTCTGCTGGTAGTGG
TCGGCGAGCTGCACGCTGCCGTCCTCGATGTTGTGGCGGATCTTGAAGTTCACCTTGATGCCGTTCT
TCTGCTTGTCGGCCATGATATAGACGTTGTGGCTGTTGTAGTTGTACTCCAGCTTGTGCCCCAGGAT
GTTGCCGTCCTCCTTGAAGTCGATGCCCTTCAGCTCGATGCGGTTCACCAGGGTGTCGCCCTCGAA
CTTCACCTCGGCGCGGGTCTTGTAGTTGCCGTCGTCCTTGAAGAAGATGGTGCGCTCCTGGACGTA
GCCTTCGGGCATGGCGGACTTGAAGAAGTCGTGCTGCTTCATGTGGTCGGGGTAGCGGCTGAAGC
ACTGCACGCCGTAGGTCAGGGTGGTCACGAGGGTGGGCCAGGGCACGGGCAGCTTGCCGGTGGTG
CAGATGAACTTCAGGGTCAGCTTGCCGTAGGTGGCATCGCCCTCGCCCTCGCCGGACACGCTGAAC
TTGTGGCCGTTTACGTCGCCGTCCAGCTCGACCAGGATGGGCACCACCCCGGTGAACAGCTCCTCG
CCCTTGCTCACCATGGTGGCTTTACCAACAGTACCGGAATGCCCCCGGGCTGGAAGTCGAGCTTCC
ATTATATACCCTCTAACTAGTACGCCTTCTGTATGAAACAGTTTTTCCTCCACGCCTTCTGTATGAA
ACAGTTTTTCCTCCACGCCTTCTGTATGAAACAGTTTTTCCTCCACGCCTTCTGTATGAAACAGTTT
TTCCTCCACGCCTTCTGTATGAAACAGTTTTTCCTCCACGCCTTCTGTATGAAACAGTTTTTCCTCCA
ACAGTACCGGATGCATCGTGAGGCTCCGGTGCCCGTCAGTGGGCAGAGCGCACATCGCCCACAGT
CCCCGAGAAGTTGGGGGGAGGGGTCGGCAATTGAACCGGTGCCTAGAGAAGGTGGCGCGGGGTA
AACTGGGAAAGTGATGTCGTGTACTGGCTCCGCCTTTTTCCCGAGGGTGGGGGAGAACCGTATATA
AGTGCAGTAGTCGCCGTGAACGTTCTTTTTCGCAACGGGTTTGCCGCCAGAACACAGGTAAGTGCC
GTGTGTGGTTCCCGCGGGCCTGGCCTCTTTACGGGTTATGGCCCTTGCGTGCCTTGAATTACTTCCA
CCTGGCTGCAGTACGTGATTCTTGATCCCGAGCTTCGGGTTGGAAGTGGGTGGGAGAGTTCGAGGC
CTTGCGCTTAAGGAGCCCCTTCGCCTCGTGCTTGAGTTGAGGCCTGGCCTGGGCGCTGGGGCCGCC
GCGTGCGAATCTGGTGGCACCTTCGCGCCTGTCTCGCTGCTTTCGATAAGTCTCTAGCCATTTAAAA
TTTTTGATGACCTGCTGCGACGCTTTTTTTCTGGCAAGATAGTCTTGTAAATGCGGGCCAAGATCTG
 
CACACTGGTATTTCGGTTTTTGGGGCCGCGGGCGGCGACGGGGCCCGTGCGTCCCAGCGCACATGT
TCGGCGAGGCGGGGCCTGCGAGCGCGGCCACCGAGAATCGGACGGGGGTAGTCTCAAGCTGGCCG
GCCTGCTCTGGTGCCTGGCCTCGCGCCGCCGTGTATCGCCCCGCCCTGGGCGGCAAGGCTGGCCCG
GTCGGCACCAGTTGCGTGAGCGGAAAGATGGCCGCTTCCCGGCCCTGCTGCAGGGAGCTCAAAAT
GGAGGACGCGGCGCTCGGGAGAGCGGGCGGGTGAGTCACCCACACAAAGGAAAAGGGCCTTTCC
GTCCTCAGCCGTCGCTTCATGTGACTCCACTGAGTACCGGGCGCCGTCCAGGCACCTCGATTAGTT
CTCGTGCTTTTGGAGTACGTCGTCTTTAGGTTGGGGGGAGGGGTTTTATGCGATGGAGTTTCCCCA
CACTGAGTGGGTGGAGACTGAAGTTAGGCCAGCTTGGCACTTGATGTAATTCTCCTTGGAATTTGC
CCTTTTTGAGTTTGGATCTTGGTTCATTCTCAAGCCTCAGACAGTGGTTCAAAGTTTTTTTCTTCCAT
TTCAGGTGTCGTGATTCTAGAATGGCCTTACCAGTGACCGCCTTGCTCCTGCCGCTGGCCTTGCTGC
TCCACGCCGCCAGGCCGGGATCCGACATCGTTCTGAGTCAGAGCCCCGCCATACTGAGCGCCTCCC
CCGGGGAGAAGGTGACCATGACCTGTAGAGCCAGCTCCAGCCTTTCATTTATGCACTGGTACCAGC
AGAAACCCGGTAGTAGTCCAAAGCCGTGGATTTACGCTACATCCAACCTGGCAAGCGGAGTACCA
GCCCGCTTCTCCGGCAGCGGATCAGGAACCTCCTACTCCCTGACAATCAGCCGCGTCGAAGCCGAG
GATGCAGCCACATACTTTTGCCACCAGTGGTCCTCTAATCCCCTGACATTTGGCGCAGGCACCAAG
CTCGAACTGAAGCGGGGATCTACCAGCGGCAGTGGAAAACCTGGTAGCGGAGAGGGGTCTACCAA
GGGGCAAGTGCAACTCAGACAGCCTGGCGCAGAACTCGTGAAGCCCGGCGCATCCGTCAAGATGT
CTTGTAAAGCCAGTGGCTACACCTTCACTAGCTATAATATGCACTGGGTTAAACAGACTCCCGGTC
AGGGCCTCGAGTGGATTGGCGCAATCTACCCGGGTAATGGTGATACTTCTTACAATCAGAAGTTTA
AGGGGAAAGCTACCCTGACCGCCGACAAGTCTTCATCAACCGCCTACATGCAGCTGAGCTCTCTGA
CGTCTGAGGATTCCGCCGTGTATTACTGCGCCCGGAGCCACTACGGGAGTAATTATGTCGATTATT
TTGACTACTGGGGTCAGGGAACAACCCTGACTGTCTCCAGCTCCGGAACCACGACGCCAGCGCCG
CGACCACCAACACCGGCGCCCACCATCGCGTCGCAGCCCCTGTCCCTGCGCCCAGAGGCGTGCCG
GCCAGCGGCGGGGGGCGCAGTGCACACGAGGGGGCTGGACTTCGCCTGTGATATCTACATCTGGG
CGCCCTTGGCCGGGACTTGTGGGGTCCTTCTCCTGTCACTGGTTATCACCCTTTACTGCAAACGGGG
CAGAAAGAAACTCCTGTATATATTCAAACAACCATTTATGAGACCAGTACAAACTACTCAAGAGG
AAGATGGCTGTAGCTGCCGATTTCCAGAAGAAGAAGAAGGAGGATGTGAACTGAGAGTGAAGTTC
AGCAGGAGCGCAGACGCCCCCGCGTACAAGCAGGGCCAGAACCAGCTCTATAACGAGCTCAATCT
AGGACGAAGAGAGGAGTACGATGTTTTGGACAAGAGACGTGGCCGGGACCCTGAGATGGGGGGA
AAGCCGAGAAGGAAGAACCCTCAGGAAGGCCTGTACAATGAACTGCAGAAAGATAAGATGGCGG
AGGCCTACAGTGAGATTGGGATGAAAGGCGAGCGCCGGAGGGGCAAGGGGCACGATGGCCTTTA
CCAGGGTCTCAGTACAGCCACCAAGGACACCTACGACGCCCTTCACATGCAGGCCCTGCCCCCTCG
CTAAG
9. pASP30 (orientation of inducible and constitutive modules: opposite direction) (SEQ ID NO: 18) 
TCGAGTTAATTAAGCTAGCTTACTTGTACAGCTCGTCCATGCCGAGAGTGATCCCGGCGGCGGTCA
CGAACTCCAGCAGGACCATGTGATCGCGCTTCTCGTTGGGGTCTTTGCTCAGGGCGGACTGGGTGC
TCAGGTAGTGGTTGTCGGGCAGCAGCACGGGGCCGTCGCCGATGGGGGTGTTCTGCTGGTAGTGG
TCGGCGAGCTGCACGCTGCCGTCCTCGATGTTGTGGCGGATCTTGAAGTTCACCTTGATGCCGTTCT
TCTGCTTGTCGGCCATGATATAGACGTTGTGGCTGTTGTAGTTGTACTCCAGCTTGTGCCCCAGGAT
GTTGCCGTCCTCCTTGAAGTCGATGCCCTTCAGCTCGATGCGGTTCACCAGGGTGTCGCCCTCGAA
CTTCACCTCGGCGCGGGTCTTGTAGTTGCCGTCGTCCTTGAAGAAGATGGTGCGCTCCTGGACGTA
GCCTTCGGGCATGGCGGACTTGAAGAAGTCGTGCTGCTTCATGTGGTCGGGGTAGCGGCTGAAGC
ACTGCACGCCGTAGGTCAGGGTGGTCACGAGGGTGGGCCAGGGCACGGGCAGCTTGCCGGTGGTG
CAGATGAACTTCAGGGTCAGCTTGCCGTAGGTGGCATCGCCCTCGCCCTCGCCGGACACGCTGAAC
TTGTGGCCGTTTACGTCGCCGTCCAGCTCGACCAGGATGGGCACCACCCCGGTGAACAGCTCCTCG
CCCTTGCTCACCATGGTGGCTTTACCAACAGTACCGGAATGCCCCCGGGCTGGAAGTCGAGCTTCC
ATTATATACCCTCTAACTAGTACGCCTTCTGTATGAAACAGTTTTTCCTCCACGCCTTCTGTATGAA
ACAGTTTTTCCTCCACGCCTTCTGTATGAAACAGTTTTTCCTCCACGCCTTCTGTATGAAACAGTTT
TTCCTCCACGCCTTCTGTATGAAACAGTTTTTCCTCCACGCCTTCTGTATGAAACAGTTTTTCCTCCA
ACAGTACCGGATGCATCGTGAGGCTCCGGTGCCCGTCAGTGGGCAGAGCGCACATCGCCCACAGT
CCCCGAGAAGTTGGGGGGAGGGGTCGGCAATTGAACCGGTGCCTAGAGAAGGTGGCGCGGGGTA
AACTGGGAAAGTGATGTCGTGTACTGGCTCCGCCTTTTTCCCGAGGGTGGGGGAGAACCGTATATA
AGTGCAGTAGTCGCCGTGAACGTTCTTTTTCGCAACGGGTTTGCCGCCAGAACACAGGTAAGTGCC
GTGTGTGGTTCCCGCGGGCCTGGCCTCTTTACGGGTTATGGCCCTTGCGTGCCTTGAATTACTTCCA
CCTGGCTGCAGTACGTGATTCTTGATCCCGAGCTTCGGGTTGGAAGTGGGTGGGAGAGTTCGAGGC
CTTGCGCTTAAGGAGCCCCTTCGCCTCGTGCTTGAGTTGAGGCCTGGCCTGGGCGCTGGGGCCGCC
GCGTGCGAATCTGGTGGCACCTTCGCGCCTGTCTCGCTGCTTTCGATAAGTCTCTAGCCATTTAAAA
TTTTTGATGACCTGCTGCGACGCTTTTTTTCTGGCAAGATAGTCTTGTAAATGCGGGCCAAGATCTG
 
CACACTGGTATTTCGGTTTTTGGGGCCGCGGGCGGCGACGGGGCCCGTGCGTCCCAGCGCACATGT
TCGGCGAGGCGGGGCCTGCGAGCGCGGCCACCGAGAATCGGACGGGGGTAGTCTCAAGCTGGCCG
GCCTGCTCTGGTGCCTGGCCTCGCGCCGCCGTGTATCGCCCCGCCCTGGGCGGCAAGGCTGGCCCG
GTCGGCACCAGTTGCGTGAGCGGAAAGATGGCCGCTTCCCGGCCCTGCTGCAGGGAGCTCAAAAT
GGAGGACGCGGCGCTCGGGAGAGCGGGCGGGTGAGTCACCCACACAAAGGAAAAGGGCCTTTCC
GTCCTCAGCCGTCGCTTCATGTGACTCCACTGAGTACCGGGCGCCGTCCAGGCACCTCGATTAGTT
CTCGTGCTTTTGGAGTACGTCGTCTTTAGGTTGGGGGGAGGGGTTTTATGCGATGGAGTTTCCCCA
CACTGAGTGGGTGGAGACTGAAGTTAGGCCAGCTTGGCACTTGATGTAATTCTCCTTGGAATTTGC
CCTTTTTGAGTTTGGATCTTGGTTCATTCTCAAGCCTCAGACAGTGGTTCAAAGTTTTTTTCTTCCAT
TTCAGGTGTCGTGATTCTAGAGCCACCATGGCCTTACCAGTGACCGCCTTGCTCCTGCCGCTGGCCT
TGCTGCTCCACGCCGCCAGGCCGGACATTCAAATGACACAGAGTCCCTCATCCCTCAGTGCCAGCG
TGGGCGATCGGGTGACTATAACCTGCAGAGCTTCTCAGGACGTGAATACCGCTGTGGCGTGGTACC
AGCAGAAGCCAGGCAAAGCGCCTAAGCTTCTCATTTATAGTGCCAGCTTCCTGTACTCAGGTGTTC
CGTCTCGCTTTTCTGGAAGTAGAAGTGGGACCGATTTCACATTGACGATCAGCAGCTTGCAGCCCG
AAGATTTCGCCACCTACTACTGTCAGCAGCACTACACTACCCCACCGACATTTGGTCAAGGCACAA
AAGTAGAGATTAAACGCACTGGTTCCACCAGCGGGAGCGGGAAACCCGGCTCTGGGGAGGGGAG
CGAGGTCCAGCTGGTGGAATCCGGGGGTGGTCTTGTGCAGCCAGGAGGATCCTTGAGGTTGTCCTG
CGCCGCAAGCGGCTTTAACATCAAAGATACATACATCCATTGGGTCCGACAGGCCCCTGGAAAGG
GCCTGGAGTGGGTCGCCCGGATCTACCCAACTAACGGGTACACTCGCTACGCTGATAGCGTCAAG
GGTCGGTTTACTATTTCTGCCGACACCTCAAAAAACACAGCCTACCTCCAGATGAACTCTCTCAGA
GCTGAGGATACAGCCGTGTACTATTGCAGCCGGTGGGGAGGCGACGGGTTCTACGCTATGGATGT
GTGGGGGCAGGGCACACTGGTCACCGTGAGCTCATCCGGAACCACGACGCCAGCGCCGCGACCAC
CAACACCGGCGCCCACCATCGCGTCGCAGCCCCTGTCCCTGCGCCCAGAGGCGTGCCGGCCAGCG
GCGGGGGGCGCAGTGCACACGAGGGGGCTGGACTTCGCCTGTGATATCTACATCTGGGCGCCCTT
GGCCGGGACTTGTGGGGTCCTTCTCCTGTCACTGGTTATCACCCTTTACTGCAAACGGGGCAGAAA
GAAACTCCTGTATATATTCAAACAACCATTTATGAGACCAGTACAAACTACTCAAGAGGAAGATG
GCTGTAGCTGCCGATTTCCAGAAGAAGAAGAAGGAGGATGTGAACTGAGAGTGAAGTTCAGCAGG
AGCGCAGACGCCCCCGCGTACAAGCAGGGCCAGAACCAGCTCTATAACGAGCTCAATCTAGGACG
AAGAGAGGAGTACGATGTTTTGGACAAGAGACGTGGCCGGGACCCTGAGATGGGGGGAAAGCCG
AGAAGGAAGAACCCTCAGGAAGGCCTGTACAATGAACTGCAGAAAGATAAGATGGCGGAGGCCT
ACAGTGAGATTGGGATGAAAGGCGAGCGCCGGAGGGGCAAGGGGCACGATGGCCTTTACCAGGG
TCTCAGTACAGCCACCAAGGACACCTACGACGCCCTTCACATGCAGGCCCTGCCCCCTCGCTAAG
10. pASP31 (orientation of inducible and constitutive modules: opposite direction) (SEQ ID NO: 19) 
TCGAGTTAATTAAGCTAGCTTACTTGTACAGCTCGTCCATGCCGAGAGTGATCCCGGCGGCGGTCA
CGAACTCCAGCAGGACCATGTGATCGCGCTTCTCGTTGGGGTCTTTGCTCAGGGCGGACTGGGTGC
TCAGGTAGTGGTTGTCGGGCAGCAGCACGGGGCCGTCGCCGATGGGGGTGTTCTGCTGGTAGTGG
TCGGCGAGCTGCACGCTGCCGTCCTCGATGTTGTGGCGGATCTTGAAGTTCACCTTGATGCCGTTCT
TCTGCTTGTCGGCCATGATATAGACGTTGTGGCTGTTGTAGTTGTACTCCAGCTTGTGCCCCAGGAT
GTTGCCGTCCTCCTTGAAGTCGATGCCCTTCAGCTCGATGCGGTTCACCAGGGTGTCGCCCTCGAA
CTTCACCTCGGCGCGGGTCTTGTAGTTGCCGTCGTCCTTGAAGAAGATGGTGCGCTCCTGGACGTA
GCCTTCGGGCATGGCGGACTTGAAGAAGTCGTGCTGCTTCATGTGGTCGGGGTAGCGGCTGAAGC
ACTGCACGCCGTAGGTCAGGGTGGTCACGAGGGTGGGCCAGGGCACGGGCAGCTTGCCGGTGGTG
CAGATGAACTTCAGGGTCAGCTTGCCGTAGGTGGCATCGCCCTCGCCCTCGCCGGACACGCTGAAC
TTGTGGCCGTTTACGTCGCCGTCCAGCTCGACCAGGATGGGCACCACCCCGGTGAACAGCTCCTCG
CCCTTGCTCACCATGGTGGCTTTACCAACAGTACCGGAATGCCCCCGGGCTGGAAGTCGAGCTTCC
ATTATATACCCTCTAACTAGTACGCCTTCTGTATGAAACAGTTTTTCCTCCACGCCTTCTGTATGAA
ACAGTTTTTCCTCCACGCCTTCTGTATGAAACAGTTTTTCCTCCACGCCTTCTGTATGAAACAGTTT
TTCCTCCACGCCTTCTGTATGAAACAGTTTTTCCTCCACGCCTTCTGTATGAAACAGTTTTTCCTCCA
ACAGTACCGGATGCATCGTGAGGCTCCGGTGCCCGTCAGTGGGCAGAGCGCACATCGCCCACAGT
CCCCGAGAAGTTGGGGGGAGGGGTCGGCAATTGAACCGGTGCCTAGAGAAGGTGGCGCGGGGTA
AACTGGGAAAGTGATGTCGTGTACTGGCTCCGCCTTTTTCCCGAGGGTGGGGGAGAACCGTATATA
AGTGCAGTAGTCGCCGTGAACGTTCTTTTTCGCAACGGGTTTGCCGCCAGAACACAGGTAAGTGCC
GTGTGTGGTTCCCGCGGGCCTGGCCTCTTTACGGGTTATGGCCCTTGCGTGCCTTGAATTACTTCCA
CCTGGCTGCAGTACGTGATTCTTGATCCCGAGCTTCGGGTTGGAAGTGGGTGGGAGAGTTCGAGGC
CTTGCGCTTAAGGAGCCCCTTCGCCTCGTGCTTGAGTTGAGGCCTGGCCTGGGCGCTGGGGCCGCC
GCGTGCGAATCTGGTGGCACCTTCGCGCCTGTCTCGCTGCTTTCGATAAGTCTCTAGCCATTTAAAA
TTTTTGATGACCTGCTGCGACGCTTTTTTTCTGGCAAGATAGTCTTGTAAATGCGGGCCAAGATCTG
CACACTGGTATTTCGGTTTTTGGGGCCGCGGGCGGCGACGGGGCCCGTGCGTCCCAGCGCACATGT
 
TCGGCGAGGCGGGGCCTGCGAGCGCGGCCACCGAGAATCGGACGGGGGTAGTCTCAAGCTGGCCG
GCCTGCTCTGGTGCCTGGCCTCGCGCCGCCGTGTATCGCCCCGCCCTGGGCGGCAAGGCTGGCCCG
GTCGGCACCAGTTGCGTGAGCGGAAAGATGGCCGCTTCCCGGCCCTGCTGCAGGGAGCTCAAAAT
GGAGGACGCGGCGCTCGGGAGAGCGGGCGGGTGAGTCACCCACACAAAGGAAAAGGGCCTTTCC
GTCCTCAGCCGTCGCTTCATGTGACTCCACTGAGTACCGGGCGCCGTCCAGGCACCTCGATTAGTT
CTCGTGCTTTTGGAGTACGTCGTCTTTAGGTTGGGGGGAGGGGTTTTATGCGATGGAGTTTCCCCA
CACTGAGTGGGTGGAGACTGAAGTTAGGCCAGCTTGGCACTTGATGTAATTCTCCTTGGAATTTGC
CCTTTTTGAGTTTGGATCTTGGTTCATTCTCAAGCCTCAGACAGTGGTTCAAAGTTTTTTTCTTCCAT
TTCAGGTGTCGTGATTCTAGAGCCACCATGGCCTTACCAGTGACCGCCTTGCTCCTGCCGCTGGCCT
TGCTGCTCCACGCCGCCAGGCCGGACATTCAAATGACACAGAGTCCCTCATCCCTCAGTGCCAGCG
TGGGCGATCGGGTGACTATAACCTGCAGAGCTTCTCAGGACGTGAATACCGCTGTGGCGTGGTACC
AGCAGAAGCCAGGCAAAGCGCCTAAGCTTCTCATTTATAGTGCCAGCTTCCTGTACTCAGGTGTTC
CGTCTCGCTTTTCTGGAAGTAGAAGTGGGACCGATTTCACATTGACGATCAGCAGCTTGCAGCCCG
AAGATTTCGCCACCTACTACTGTCAGCAGCACTACACTACCCCACCGACATTTGGTCAAGGCACAA
AAGTAGAGATTAAACGCACTGGTTCCACCAGCGGGAGCGGGAAACCCGGCTCTGGGGAGGGGAG
CGAGGTCCAGCTGGTGGAATCCGGGGGTGGTCTTGTGCAGCCAGGAGGATCCTTGAGGTTGTCCTG
CGCCGCAAGCGGCTTTAACATCAAAGATACATACATCCATTGGGTCCGACAGGCCCCTGGAAAGG
GCCTGGAGTGGGTCGCCCGGATCTACCCAACTAACGGGTACACTCGCTACGCTGATAGCGTCAAG
GGTCGGTTTACTATTTCTGCCGACACCTCAAAAAACACAGCCTACCTCCAGATGAACTCTCTCAGA
GCTGAGGATACAGCCGTGTACTATTGCAGCCGGTGGGGAGGCGACGGGTTCTACGCTATGGATGT
GTGGGGGCAGGGCACACTGGTCACCGTGAGCTCATCCGGAACCACGACGCCAGCGCCGCGACCAC
CAACACCGGCGCCCACCATCGCGTCGCAGCCCCTGTCCCTGCGCCCAGAGGCGTGCCGGCCAGCG
GCGGGGGGCGCAGTGCACACGAGGGGGCTGGACTTCGCCTGTGATTTTTGGGTGCTGGTGGTGGTT
GGTGGAGTCCTGGCTTGCTATAGCTTGCTAGTAACAGTGGCCTTTATTATTTTCTGGGTGAGGAGT
AAGAGGAGCAGGCTCCTGCACAGTGACTACATGAACATGACTCCCCGCCGCCCCGGGCCCACCCG
CAAGCATTACCAGCCCTATGCCCCACCACGCGACTTCGCAGCCTATCGCTCCATCGATAGAGTGAA
GTTCAGCAGGAGCGCAGACGCCCCCGCGTACCAGCAGGGCCAGAACCAGCTCTATAACGAGCTCA
ATCTAGGACGAAGAGAGGAGTACGATGTTTTGGACAAGAGACGTGGCCGGGACCCTGAGATGGG
GGGAAAGCCGAGAAGGAAGAACCCTCAGGAAGGCCTGTACAATGAACTGCAGAAAGATAAGATG
GCGGAGGCCTACAGTGAGATTGGGATGAAAGGCGAGCGCCGGAGGGGCAAGGGGCACGATGGCC
TTTACCAGGGTCTCAGTACAGCCACCAAGGACACCTACGACGCCCTTCACATGCAGGCCCTGCCCC
CTCGCTAAG
11. pASP38 (orientation of inducible and constitutive modules: same direction) (SEQ ID NO: 20) 
TCGAGTTAATTAAGCTAGCCCGGTACTGTTGGAGGAAAAACTGTTTCATACAGAAGGCGTGGAGG
AAAAACTGTTTCATACAGAAGGCGTGGAGGAAAAACTGTTTCATACAGAAGGCGTGGAGGAAAA
ACTGTTTCATACAGAAGGCGTGGAGGAAAAACTGTTTCATACAGAAGGCGTGGAGGAAAAACTGT
TTCATACAGAAGGCGTACTAGTTAGAGGGTATATAATGGAAGCTCGACTTCCAGCCCGGGGGCAT
TCCGGTACTGTTGGTAAAGCCACCATGTGCCACCAGCAGCTGGTCATCAGCTGGTTCAGCCTGGTG
TTCCTGGCCAGCCCTCTGGTGGCCATCTGGGAGCTGAAGAAAGACGTGTACGTGGTGGAACTGGA
CTGGTATCCCGACGCCCCTGGCGAGATGGTGGTGCTGACCTGCGACACCCCTGAAGAGGACGGCA
TCACCTGGACCCTGGACCAGTCTAGCGAGGTGCTGGGCAGCGGCAAGACCCTGACCATCCAGGTC
AAAGAGTTCGGCGACGCCGGCCAGTACACCTGTCACAAGGGCGGAGAGGTGCTGAGCCACAGCCT
GCTGCTGCTCCACAAGAAAGAGGATGGCATTTGGAGCACCGACATCCTGAAGGACCAGAAAGAGC
CCAAGAACAAGACCTTCCTGAGATGCGAGGCCAAGAACTACAGCGGCCGGTTCACCTGTTGGTGG
CTGACCACCATCAGCACCGACCTGACCTTCAGCGTGAAGTCCAGCAGAGGCAGCAGCGACCCTCA
GGGCGTGACATGTGGCGCCGCTACACTGTCTGCCGAGAGAGTGCGGGGCGACAACAAAGAGTACG
AGTACAGCGTCGAGTGCCAGGAAGATAGCGCCTGCCCTGCCGCCGAGGAAAGCCTGCCTATCGAA
GTGATGGTGGACGCCGTGCACAAGCTGAAGTACGAGAACTACACCTCCAGCTTTTTCATCCGGGAC
ATCATCAAGCCCGACCCTCCCAAGAACCTGCAGCTGAAGCCTCTGAAGAACAGCAGACAGGTGGA
AGTGTCCTGGGAGTACCCCGACACCTGGTCCACCCCTCACAGCTACTTCAGCCTGACATTCTGTGT
GCAAGTCCAGGGCAAGTCCAAGCGCGAGAAAAAGGACCGGGTGTTCACCGACAAGACCAGCGCC
ACCGTGATCTGCCGGAAGAACGCCTCTATCAGCGTGCGGGCCCAGGACCGGTACTACAGCAGCTC
TTGGAGCGAGTGGGCCAGCGTGCCATGTTCTGGTGGCGGAGGCGGCGGAAGCAGAAATCTGCCAG
TGGCCACCCCTGACCCCGGCATGTTTCCTTGTCTGCACCACAGCCAGAACCTGCTGCGGGCCGTGT
CCAACATGCTGCAGAAGGCCCGGCAGACCCTGGAATTCTACCCCTGCACCAGCGAGGAAATCGAC
CACGAGGACATCACCAAGGATAAGACCAGCACCGTGGAAGCCTGTCTGCCCCTGGAACTGACCAA
GAACGAGAGCTGCCTGAACAGCCGGGAAACCAGCTTCATCACCAACGGCAGCTGTCTGGCCAGCA
GAAAGACCTCCTTCATGATGGCCCTGTGCCTGAGCAGCATCTACGAGGACCTGAAGATGTACCAG
 
GTCGAGTTCAAGACCATGAACGCCAAGCTGCTGATGGACCCCAAGCGGCAGATCTTTCTGGACCA
GAATATGCTGGCCGTGATCGACGAGCTGATGCAGGCCCTGAACTTCAACAGCGAGACAGTGCCCC
AGAAGTCCTCCCTGGAAGAACCCGACTTCTACAAGACCAAGATCAAGCTGTGCATCCTGCTGCAC
GCCTTCCGGATCAGAGCCGTGACCATCGACAGAGTGATGAGCTACCTGAACGCCTCCTAAATGCAT
CGTGAGGCTCCGGTGCCCGTCAGTGGGCAGAGCGCACATCGCCCACAGTCCCCGAGAAGTTGGGG
GGAGGGGTCGGCAATTGAACCGGTGCCTAGAGAAGGTGGCGCGGGGTAAACTGGGAAAGTGATG
TCGTGTACTGGCTCCGCCTTTTTCCCGAGGGTGGGGGAGAACCGTATATAAGTGCAGTAGTCGCCG
TGAACGTTCTTTTTCGCAACGGGTTTGCCGCCAGAACACAGGTAAGTGCCGTGTGTGGTTCCCGCG
GGCCTGGCCTCTTTACGGGTTATGGCCCTTGCGTGCCTTGAATTACTTCCACCTGGCTGCAGTACGT
GATTCTTGATCCCGAGCTTCGGGTTGGAAGTGGGTGGGAGAGTTCGAGGCCTTGCGCTTAAGGAGC
CCCTTCGCCTCGTGCTTGAGTTGAGGCCTGGCCTGGGCGCTGGGGCCGCCGCGTGCGAATCTGGTG
GCACCTTCGCGCCTGTCTCGCTGCTTTCGATAAGTCTCTAGCCATTTAAAATTTTTGATGACCTGCT
GCGACGCTTTTTTTCTGGCAAGATAGTCTTGTAAATGCGGGCCAAGATCTGCACACTGGTATTTCG
GTTTTTGGGGCCGCGGGCGGCGACGGGGCCCGTGCGTCCCAGCGCACATGTTCGGCGAGGCGGGG
CCTGCGAGCGCGGCCACCGAGAATCGGACGGGGGTAGTCTCAAGCTGGCCGGCCTGCTCTGGTGC
CTGGCCTCGCGCCGCCGTGTATCGCCCCGCCCTGGGCGGCAAGGCTGGCCCGGTCGGCACCAGTTG
CGTGAGCGGAAAGATGGCCGCTTCCCGGCCCTGCTGCAGGGAGCTCAAAATGGAGGACGCGGCGC
TCGGGAGAGCGGGCGGGTGAGTCACCCACACAAAGGAAAAGGGCCTTTCCGTCCTCAGCCGTCGC
TTCATGTGACTCCACTGAGTACCGGGCGCCGTCCAGGCACCTCGATTAGTTCTCGTGCTTTTGGAGT
ACGTCGTCTTTAGGTTGGGGGGAGGGGTTTTATGCGATGGAGTTTCCCCACACTGAGTGGGTGGAG
ACTGAAGTTAGGCCAGCTTGGCACTTGATGTAATTCTCCTTGGAATTTGCCCTTTTTGAGTTTGGAT
CTTGGTTCATTCTCAAGCCTCAGACAGTGGTTCAAAGTTTTTTTCTTCCATTTCAGGTGTCGTGATT
CTAGAGCCACCATGGCCTTACCAGTGACCGCCTTGCTCCTGCCGCTGGCCTTGCTGCTCCACGCCG
CCAGGCCGGACATTCAAATGACACAGAGTCCCTCATCCCTCAGTGCCAGCGTGGGCGATCGGGTG
ACTATAACCTGCAGAGCTTCTCAGGACGTGAATACCGCTGTGGCGTGGTACCAGCAGAAGCCAGG
CAAAGCGCCTAAGCTTCTCATTTATAGTGCCAGCTTCCTGTACTCAGGTGTTCCGTCTCGCTTTTCT
GGAAGTAGAAGTGGGACCGATTTCACATTGACGATCAGCAGCTTGCAGCCCGAAGATTTCGCCAC
CTACTACTGTCAGCAGCACTACACTACCCCACCGACATTTGGTCAAGGCACAAAAGTAGAGATTA
AACGCACTGGTTCCACCAGCGGGAGCGGGAAACCCGGCTCTGGGGAGGGGAGCGAGGTCCAGCT
GGTGGAATCCGGGGGTGGTCTTGTGCAGCCAGGAGGATCCTTGAGGTTGTCCTGCGCCGCAAGCG
GCTTTAACATCAAAGATACATACATCCATTGGGTCCGACAGGCCCCTGGAAAGGGCCTGGAGTGG
GTCGCCCGGATCTACCCAACTAACGGGTACACTCGCTACGCTGATAGCGTCAAGGGTCGGTTTACT
ATTTCTGCCGACACCTCAAAAAACACAGCCTACCTCCAGATGAACTCTCTCAGAGCTGAGGATACA
GCCGTGTACTATTGCAGCCGGTGGGGAGGCGACGGGTTCTACGCTATGGATGTGTGGGGGCAGGG
CACACTGGTCACCGTGAGCTCATCCGGAACCACGACGCCAGCGCCGCGACCACCAACACCGGCGC
CCACCATCGCGTCGCAGCCCCTGTCCCTGCGCCCAGAGGCGTGCCGGCCAGCGGCGGGGGGCGCA
GTGCACACGAGGGGGCTGGACTTCGCCTGTGATATCTACATCTGGGCGCCCTTGGCCGGGACTTGT
GGGGTCCTTCTCCTGTCACTGGTTATCACCCTTTACTGCAAACGGGGCAGAAAGAAACTCCTGTAT
ATATTCAAACAACCATTTATGAGACCAGTACAAACTACTCAAGAGGAAGATGGCTGTAGCTGCCG
ATTTCCAGAAGAAGAAGAAGGAGGATGTGAACTGAGAGTGAAGTTCAGCAGGAGCGCAGACGCC
CCCGCGTACAAGCAGGGCCAGAACCAGCTCTATAACGAGCTCAATCTAGGACGAAGAGAGGAGTA
CGATGTTTTGGACAAGAGACGTGGCCGGGACCCTGAGATGGGGGGAAAGCCGAGAAGGAAGAAC
CCTCAGGAAGGCCTGTACAATGAACTGCAGAAAGATAAGATGGCGGAGGCCTACAGTGAGATTGG
GATGAAAGGCGAGCGCCGGAGGGGCAAGGGGCACGATGGCCTTTACCAGGGTCTCAGTACAGCC
ACCAAGGACACCTACGACGCCCTTCACATGCAGGCCCTGCCCCCTCGCTAAG
12. pASP52 (orientation of inducible and constitutive modules: opposite direction) (SEQ ID NO: 21

TCGAGTTAATTAAGCTAGCTTACATATGTCTCTTGGCCCGCAGGTCCTCTTCGGAGATCAGCTTCTG
CTCGCTTCCCTTGCCAGGAGACAGGCTCAGGGACTTCTGGGTGTAGTGGTTGTGCAGGGCCTCGTG
CATCACGCTGCAGGAGAACACGTTTCCCTGCTGCCATCTGCTCTTGTCCACTGTCAGCTTGCTGTAC
AGGAAGAATGAGCCGTCGCTGTCCAGCACAGGAGGGGTTGTCTTGTAGTTGTTCTCAGGCTGGCCA
TTGCTCTCCCATTCCACGGCAATGTCGCTGGGGTAAAAGCCCTTGACCAGGCAGGTCAGGGACACC
TGATTCTTGGTCAGCTCGTCCCTGCTTGGAGGCAGTGTGTAAACCTGGGGTTCCCTAGGCTGGCCC
TTGGCCTTGCTGATGGTTTTCTCGATAGGAGCAGGCAGGGCCTTGTTGGACACCTTGCACTTGTACT
CTTTGCCGTTCAGCCAATCCTGGTGCAGCACGGTCAGCACGGACACCACTCTGTAGGTGCTGTTGT
ACTGTTCCTCTCTAGGCTTGGTCTTGGCGTTGTGCACTTCCACGCCGTCCACGTACCAATTGAACTT
CACTTCGGGGTCCTCGTGAGACACATCCACCACCACGCAGGTCACTTCAGGGGTTCTGCTGATCAT
CAGGGTGTCCTTAGGCTTTGGAGGAAACAGGAACACGGAAGGTCCGCCGAGCAGTTCTGGAGCAG
GACATGGAGGACAGGTGTGGGTCTTGTCGCTGCTCTTGGGTTCCACTTTGATTTCCACCTTGGTGCC

CTGGCCAAATGTGTAAGGCAGGGTGTTGCCCTGCTGGCAGTAGTAGGTGGCAATGTCCTCAGGCTG
CAGGCTAGATATGGTGAAGGTGAAGTCGGTGCCGCTGCCAGAGCCAGAAAATCTGCTGGGCACGC
CGCTGTGCAGTCTGCTGGTGTAGTAGATCAGCAGTTTAGGGGCCTTGCCGGGCTTTTGCTGATACC
AATTCAGGTAGCTGCTGATGTCCTGGCTGGCCCGACAGGTGATTGTCACTCTGTCTCCCACAGAGG
CAGACAGGCTGCTGGGGCTCTGTGTCATCTGAATATCGCTACCGCCTCCGCCACTTCCTCCGCCAC
CAGATCCTCCGCCTCCGCTAGAAACGGTGACCAGAGAGCCCTGGCCCCAATAATCCATGGCGGTG
GTTCTGGCCAGGCTTCTGGCGCAGTAGTACACGGCTGTATCAGCAGCGGTCACGCTAGACAGCCGC
AGGCTGAACTGGTTCTTGCTGGTGTCTCTCAGCATGGTCACTCTGGACTTCAGGCTGGGATTGTAG
GTGGTGATGCCGCTGTAGCTGATGTAGCCGATCCATTCCAGGCCTCTTCCAGGAGGTTGTCGGACC
CAGGACCAGGCATGATCGCTGGTGATGCTGTAGCCGGACACGGTACAGGTCAGGCTCAGTGTTTG
GCTAGGCCGCACGAGTCCAGGTCCAGACTCTTGCAGCTGAACCTCGCACTGCACGCCTCTAAGCAG
AGCCACCAGGAACACCCAGCTCAGGCCGAATTCCATGGTGGCTTTACCAACAGTACCGGAATGCC
CCCGGGCTGGAAGTCGAGCTTCCATTATATACCCTCTAACTAGTACGCCTTCTGTATGAAACAGTT
TTTCCTCCACGCCTTCTGTATGAAACAGTTTTTCCTCCACGCCTTCTGTATGAAACAGTTTTTCCTCC
ACGCCTTCTGTATGAAACAGTTTTTCCTCCACGCCTTCTGTATGAAACAGTTTTTCCTCCACGCCTT
CTGTATGAAACAGTTTTTCCTCCAACAGTACCGGATGCATCGTGAGGCTCCGGTGCCCGTCAGTGG
GCAGAGCGCACATCGCCCACAGTCCCCGAGAAGTTGGGGGGAGGGGTCGGCAATTGAACCGGTGC
CTAGAGAAGGTGGCGCGGGGTAAACTGGGAAAGTGATGTCGTGTACTGGCTCCGCCTTTTTCCCGA
GGGTGGGGGAGAACCGTATATAAGTGCAGTAGTCGCCGTGAACGTTCTTTTTCGCAACGGGTTTGC
CGCCAGAACACAGGTAAGTGCCGTGTGTGGTTCCCGCGGGCCTGGCCTCTTTACGGGTTATGGCCC
TTGCGTGCCTTGAATTACTTCCACCTGGCTGCAGTACGTGATTCTTGATCCCGAGCTTCGGGTTGGA
AGTGGGTGGGAGAGTTCGAGGCCTTGCGCTTAAGGAGCCCCTTCGCCTCGTGCTTGAGTTGAGGCC
TGGCCTGGGCGCTGGGGCCGCCGCGTGCGAATCTGGTGGCACCTTCGCGCCTGTCTCGCTGCTTTC
GATAAGTCTCTAGCCATTTAAAATTTTTGATGACCTGCTGCGACGCTTTTTTTCTGGCAAGATAGTC
TTGTAAATGCGGGCCAAGATCTGCACACTGGTATTTCGGTTTTTGGGGCCGCGGGCGGCGACGGGG
CCCGTGCGTCCCAGCGCACATGTTCGGCGAGGCGGGGCCTGCGAGCGCGGCCACCGAGAATCGGA
CGGGGGTAGTCTCAAGCTGGCCGGCCTGCTCTGGTGCCTGGCCTCGCGCCGCCGTGTATCGCCCCG
CCCTGGGCGGCAAGGCTGGCCCGGTCGGCACCAGTTGCGTGAGCGGAAAGATGGCCGCTTCCCGG
CCCTGCTGCAGGGAGCTCAAAATGGAGGACGCGGCGCTCGGGAGAGCGGGCGGGTGAGTCACCC
ACACAAAGGAAAAGGGCCTTTCCGTCCTCAGCCGTCGCTTCATGTGACTCCACTGAGTACCGGGCG
CCGTCCAGGCACCTCGATTAGTTCTCGTGCTTTTGGAGTACGTCGTCTTTAGGTTGGGGGGAGGGG
TTTTATGCGATGGAGTTTCCCCACACTGAGTGGGTGGAGACTGAAGTTAGGCCAGCTTGGCACTTG
ATGTAATTCTCCTTGGAATTTGCCCTTTTTGAGTTTGGATCTTGGTTCATTCTCAAGCCTCAGACAG
TGGTTCAAAGTTTTTTTCTTCCATTTCAGGTGTCGTGATTCTAGAATGGCCTTACCAGTGACCGCCT
TGCTCCTGCCGCTGGCCTTGCTGCTCCACGCCGCCAGGCCGGGATCCGACATCGTTCTGAGTCAGA
GCCCCGCCATACTGAGCGCCTCCCCCGGGGAGAAGGTGACCATGACCTGTAGAGCCAGCTCCAGC
CTTTCATTTATGCACTGGTACCAGCAGAAACCCGGTAGTAGTCCAAAGCCGTGGATTTACGCTACA
TCCAACCTGGCAAGCGGAGTACCAGCCCGCTTCTCCGGCAGCGGATCAGGAACCTCCTACTCCCTG
ACAATCAGCCGCGTCGAAGCCGAGGATGCAGCCACATACTTTTGCCACCAGTGGTCCTCTAATCCC
CTGACATTTGGCGCAGGCACCAAGCTCGAACTGAAGCGGGGATCTACCAGCGGCAGTGGAAAACC
TGGTAGCGGAGAGGGGTCTACCAAGGGGCAAGTGCAACTCAGACAGCCTGGCGCAGAACTCGTGA
AGCCCGGCGCATCCGTCAAGATGTCTTGTAAAGCCAGTGGCTACACCTTCACTAGCTATAATATGC
ACTGGGTTAAACAGACTCCCGGTCAGGGCCTCGAGTGGATTGGCGCAATCTACCCGGGTAATGGT
GATACTTCTTACAATCAGAAGTTTAAGGGGAAAGCTACCCTGACCGCCGACAAGTCTTCATCAACC
GCCTACATGCAGCTGAGCTCTCTGACGTCTGAGGATTCCGCCGTGTATTACTGCGCCCGGAGCCAC
TACGGGAGTAATTATGTCGATTATTTTGACTACTGGGGTCAGGGAACAACCCTGACTGTCTCCAGC
TCCGGAACCACGACGCCAGCGCCGCGACCACCAACACCGGCGCCCACCATCGCGTCGCAGCCCCT
GTCCCTGCGCCCAGAGGCGTGCCGGCCAGCGGCGGGGGGCGCAGTGCACACGAGGGGGCTGGACT
TCGCCTGTGATATCTACATCTGGGCGCCCTTGGCCGGGACTTGTGGGGTCCTTCTCCTGTCACTGGT
TATCACCCTTTACTGCAAACGGGGCAGAAAGAAACTCCTGTATATATTCAAACAACCATTTATGAG
ACCAGTACAAACTACTCAAGAGGAAGATGGCTGTAGCTGCCGATTTCCAGAAGAAGAAGAAGGA
GGATGTGAACTGAGAGTGAAGTTCAGCAGGAGCGCAGACGCCCCCGCGTACAAGCAGGGCCAGA
ACCAGCTCTATAACGAGCTCAATCTAGGACGAAGAGAGGAGTACGATGTTTTGGACAAGAGACGT
GGCCGGGACCCTGAGATGGGGGGAAAGCCGAGAAGGAAGAACCCTCAGGAAGGCCTGTACAATG
AACTGCAGAAAGATAAGATGGCGGAGGCCTACAGTGAGATTGGGATGAAAGGCGAGCGCCGGAG
GGGCAAGGGGCACGATGGCCTTTACCAGGGTCTCAGTACAGCCACCAAGGACACCTACGACGCCC
TTCACATGCAGGCCCTGCCCCCTCGCTAAG

### Amino acid sequences

1. Anti-human IL-6 receptor single-chain variable fragment crystallizable region (anti-hIL-6R scFv-Fc) (SEQ ID NO: 22) 
MEFGLSWVFLVALLRGVQCeVQLQESGPGLVRPSQTLSLTCTVSGYsiTSDHAWS
WVRQPPGRGLEWIGYISYSGITTYNPSLKSRVTMLrDTSKNQFSLRLSSVTAADTA
VYYCARSLARTTAMDYWGQGSLVTVSSGGGGSGGGGSGGGGSDIQMTQSPSS
LSASVGDRVTITCRASQDISSYLNWYQQKPGKAPKLLIYYTSRLHSGVPSRFSGSG
SGTDFTFTISSLQPEDIATYYCQQGNTLPYTFGQGTKVEIKVEPKSSDKTHTCPPC
PAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESN
GQPENNYKTTPPVLDSDGSFFLYSKLTVDKSRWQQGNVFSCSVMHEALHNH
YTQKSLSLSPGKGSEQKLISEEDLrakr
   Signal peptide derived from IgG variable heavy chain: underline
   Variable heavy chain: wavy underline
   Linker: **bold**
   Variable light chain: double underline
   IgG1 Fc with C103S mutation: **bold and underline**
   Myc tag: *bold italics*
   Furin cleavage site to enable linking multiple effectors via 2A peptide: *italics*
   *What is not in capital but bold is different from the patent that we are referring to in the text (4 amino acid difference). This sequence was derived from combining sequence of tocilizumab from this patent and this article: Physicochemical and Biological Characterization of the Proposed Biosimilar Tocilizumab. Shiwei Miao,1 Li Fan,1 Liang Zhao,1 Ding Ding, 2 Xiaohui Liu,2 Haibin Wang,2 and Wen-Song Tan1 1State Key Laboratory of Bioreactor Engineering, East China University of Science and Technology, Shanghai 200237, China 2Hisun Pharmaceutical (Hangzhou) Co., Ltd, Fuyang, Hangzhou, Zhejiang 311404, China
2. Anti-CD20 CAR human (hCD8 leader-CD20 scFv-hCD8 hinge-hCD8 transmembrane-h4-1BB-hCD3 zeta);h refers to human sequence (SEQ ID NO: 23) 
MALPVTALLLPLALLLHAARPGSDIVLSQSPAILSASPGEKVTMTCRASSSLSFMH
WYQQKPGSSPKPWIYATSNLASGVPARFSGSGSGTSYSLTISRVEAEDAATYFCH
QWSSNPLTFGAGTKLELKRGSTSGSGKPGSGEGSTKGQVQLRQPGAELVKPGAS
VKMSCKASGYTFTSYNMHWVKQTPGQGLEWIGAIYPGNGDTSYNQKFKGKATL
TADKSSSTAYMQLSSLTSEDSAVYYCARSHYGSNYVDYFDYWGQGTTLTVSSSG
TTTPAPRPPTPAPTIASQPLSLRPEACRPAAGGAVHTRGLDFACDIYIWAPLAGTC
GVLLLSLVITLYCKRGRKKLLYIFKQPFMRPVQTTQEEDGCSCRFPEEEEGGCELR
VKFSRSADAPAYKQGQNQLYNELNLGRREEYDVLDKRRGRDPEMGGKPRRKNP
QEGLYNELQKDKMAEAYSEIGMKGERRRGKGHDGLYQGLSTATKDTYDALHM
QALPPR
3. Anti-CD20 CAR canine (cCD8 leader-CD20 scFv-cCD8 hinge-cCD8 transmembrane-c4-1BB-cCD3 zeta);c refers to canine sequence (SEQ ID NO: 24) 
MASRVTALLLPLALLLRAAAASDIVLSQSPAILSASPGEKVTMTCRASSSLSFMH
WYQQKPGSSPKPWIYATSNLASGVPARFSGSGSGTSYSLTISRVEAEDAATYFCH
QWSSNPLTFGAGTKLELKRGSTSGSGKPGSGEGSTKGQVQLRQPGAELVKPGAS
VKMSCKASGYTFTSYNMHWVKQTPGQGLEWIGAIYPGNGDTSYNQKFKGKATL
TADKSSSTAYMQLSSLTSEDSAVYYCARSHYGSNYVDYFDYWGQGTTLTVSSAS
 
PTTPAPRPPTRAPTNASKPVSPRGETCRPAAGSAVKTSGLDFACEIYIWAPLAGTC
AVLLLSLVITIICHGRKKLLYLFKQPFMRPVQTAQEEDACSCRFPEEEEGECDLRA
KFGRSAAAPEHQQGPNQLYNELNLRGREEYEVLDKRRGLDPEMGGKQRKRNPQ
EVVYNALQKDKMAEAYSEIGIKSENQRRRGKGHDGLYQGLSTATKDTYDALHM
QALPPR
4. Anti-Her2 human CAR with 4-1BB costimulatory domain (hCD8 leader-4D5 scFv-hCD8 hinge-hCD8 transmembrane-h4-1BB-hCD3 zeta);h refers to human sequence (SEQ ID NO: 25) 
MALPVTALLLPLALLLHAARPDIQMTQSPSSLSASVGDRVTITCRASQDVNTAVA
WYQQKPGKAPKLLIYSASFLYSGVPSRFSGSRSGTDFTLTISSLQPEDFATYYCQQ
HYTTPPTFGQGTKVEIKRTGSTSGSGKPGSGEGSEVQLVESGGGLVQPGGSLRLS
CAASGFNIKDTYIHWVRQAPGKGLEWVARIYPTNGYTRYADSVKGRFTISADTS
KNTAYLQMNSLRAEDTAVYYCSRWGGDGFYAMDVWGQGTLVTVSSSGTTTPA
PRPPTPAPTIASQPLSLRPEACRPAAGGAVHTRGLDFACDIYIWAPLAGTCGVLLL
SLVITLYCKRGRKKLLYIFKQPFMRPVQTTQEEDGCSCRFPEEEEGGCELRVKFSR
SADAPAYKQGQNQLYNELNLGRREEYDVLDKRRGRDPEMGGKPRRKNPQEGLY
NELQKDKMAEAYSEIGMKGERRRGKGHDGLYQGLSTATKDTYDALHMQALPP
R
5. Anti-Her2 human CAR with CD28 costimulatory domain (hCD8 leader-4D5 scFv-hCD8 hinge-hCD8 transmembrane-hCD28-hCD3 zeta);h refers to human sequence (SEQ ID NO: 26) 
MALPVTALLLPLALLLHAARPDIQMTQSPSSLSASVGDRVTITCRASQDVNTAVA
WYQQKPGKAPKLLIYSASFLYSGVPSRFSGSRSGTDFTLTISSLQPEDFATYYCQQ
HYTTPPTFGQGTKVEIKRTGSTSGSGKPGSGEGSEVQLVESGGGLVQPGGSLRLS
CAASGFNIKDTYIHWVRQAPGKGLEWVARIYPTNGYTRYADSVKGRFTISADTS
KNTAYLQMNSLRAEDTAVYYCSRWGGDGFYAMDVWGQGTLVTVSSSGTTTPA
PRPPTPAPTIASQPLSLRPEACRPAAGGAVHTRGLDFACDFWVLVVVGGVLACYS
LLVTVAFIIFWVRSKRSRLLHSDYMNMTPRRPGPTRKHYQPYAPPRDFAAYRSID
RVKFSRSADAPAYQQGQNQLYNELNLGRREEYDVLDKRRGRDPEMGGKPRRKN
PQEGLYNELQKDKMAEAYSEIGMKGERRRGKGHDGLYQGLSTATKDTYDALH
MQALPPR
6. hIL-12 (p40-linker-p35);h refers to human (SEQ ID NO: 27) 
MCHQQLVISWFSLVFLASPLVAIWELKKDVYVVELDWYPDAPGEMVVLTCDTP
EEDGITWTLDQSSEVLGSGKTLTIQVKEFGDAGQYTCHKGGEVLSHSLLLLHKKE
DGIWSTDILKDQKEPKNKTFLRCEAKNYSGRFTCWWLTTISTDLTFSVKSSRGSS
DPQGVTCGAATLSAERVRGDNKEYEYSVECQEDSACPAAEESLPIEVMVDAVHK
LKYENYTSSFFIRDIIKPDPPKNLQLKPLKNSRQVEVSWEYPDTWSTPHSYFSLTF
CVQVQGKSKREKKDRVFTDKTSATVICRKNASISVRAQDRYYSSSWSEWASVPC
SGGGGGGSRNLPVATPDPGMFPCLHHSQNLLRAVSNMLQKARQTLEFYPCTSEEI
DHEDITKDKTSTVEACLPLELTKNESCLNSRETSFITNGSCLASRKTSFMMALCLS
SIYEDLKMYQVEFKTMNAKLLMDPKRQIFLDQNMLAVIDELMQALNFNSETVPQ
KSSLEEPDFYKTKIKLCILLHAFRIRAVTIDRVMSYLNAS
7. cIL-12 (p40-linker-p35);c refers to canine (SEQ ID NO: 28)

MHPQQLVISWFSLVLLASSLMTIWELEKDVYVVELDWHPDAPGEMVVLTCHTPE
EDDITWTSAQSSEVLGSGKTLTIQVKEFGDAGQYTCHKGGKVLSRSLLLIHKKED
GIWSTDILKEQKESKNKIFLKCEAKNYSGRFTCWWLTAISTDLKFSVKSSRGFSDP

QGVTCGAVTLSAERVRVDNRDYKKYTVECQEGSACPSAEESLPIEVVVDAIHKL
KYENYTSSFFIRDIIKPDPPTNLQLKPLKNSRHVEVSWEYPDTWSTPHSYFSLTFC
VQAQGKNNREKKDRLCVDKTSAKVVCHKDAKIRVQARDRYYSSSWSDWASVS
CSGGGGGGSRSLPTASPSPGIFQCLNHSQNLLRAVSNTLQKARQTLELYSCTSEEI
DHEDITKDKTSTVEACLPLELTMNESCLASREISLITNGSCLASGKASFMTVLCLS
SIYEDLKMYQMEFKAMNAKLLMDPKRQIFLDQNMLTAIDELLQALNFNSVTVPQ
KSSLEEPDFYKTKIKLCILLHAFRIRAVTIDRMMSYLNSS

### Example 13- Effect of ATG sequence between the minimal promoter and the spacer

The effect of 6-nucleotide sequences placed between the minimal promoter and the spacer in the inducible module was investigated. How the presence of ATG as a potential upstream start codon influences the performance of the system was studied. It was demonstrated herein that a CAT**ATG** 6-nucleotide sequence (SEQ ID NO: 1) and a CCCGGG 6-nucleotide sequence (SEQ ID NO: 2) perform similarly in terms of transduction efficacy, leakage and maximal activity of the system (Figures 12A-12D). Thus, a potential ATG start codon was found not to interfere with translation from the intended and optimized start codon with an optimal Kozak sequence. Without wishing to be bound by theory, the position of this ATG is likely too close downstream to the TATA box (21 nt) in the minimal promoter and hence was not transcribed and present in the mRNA.

The sequence CCCGGG (SEQ ID NO: 2), without an upstream ATG sequence between the minimal promoter and the spacer, may be found in constructs pASP52 (anti-hIL-6R scFv-Fc reverse + CD20-targeting CAR forward), pASP72 (TCF7 reverse + Her2-targeting CAR forward), pASP73 (FOXO1-3A reverse + Her2-targeting CAR forward), pASP79 (c225-OKT3 BiTE reverse + IL-13Ra2-targeting CAR forward), pASP83 (806-OKT3 BiTE reverse + IL-13Ra2-targeting CAR forward), lentiviral vectors from 12A and 12B (eGFP forward-mCherry forward; pASP4.2.1) where functionality was investigated without this non-intentional upstream ATG.

Any DNA sequence that modulates distance between inducible and constitutive part when in the back-to-back orientation may be integrated. In some embodiments, DNA sequences without known function and without unfavorable secondary structures are used. In some embodiments, a spacer used for cloning purposes may be present.

The following sequence, or any amount of repetitions thereof, may be used as a spacer:
5' - GGCATTCCGGTACTGTTGGTAAA - 3'(SEQ ID NO: 29)

### Example 14- CAR downregulation and re-expression on cell surface

The surface downregulation of CAR after ligating cognate antigen on target cells, which was observed in Figures 7A-7B, was further characterized. Primary T cells were activated, transduced with a single-vector system pASP30 (inducible eGFP + constitutive 4D5 anti-Her2 scFv 4-1BB+CD3-zeta CAR) at MOI5, expanded and rested according to the standard protocol. At the end of the expansion they were sorted for CAR+ cell population (Day 0). Cells were then co-cultured with SKOV3 cancer cell line that expresses high levels of Her2 at 0.3:1 effector to target ratio. Two days later cell were stained for CAR expression and profound downregulation of CAR was observed in cells that recognized target antigen as evidenced by upregulated NFAT inducible eGFP module in this population. Five days after the co-culture was set up, CAR T cells lysed a large majority of SKOV3 target cells which led to the re-expression of CAR. This demonstrated target mediated downregulation and re-expression of CAR on the cell surface (Figures 13A-13B).

Thus, by starting with a sorted CAR positive population, it was demonstrated that the CAR is downregulated on the cell surface after binding antigen-positive cells, but because of its constitutive expression, it is re-expressed after the target cells are lysed. (Figures 13A-13B). Additionally, in this way, CAR T cells that normally downregulate surface CAR expression after activation may now be detected via induced intracellular GFP expression.

### Example 15-In vivo validation of the system

The *in vivo* functionality of the system was shown by testing an example of the single-vector system (pASP26; inducible eGFP + constitutive 4D5 anti-Her2 scFv 4-1BB+CD3-zeta CAR; forward orientation) in a tumor xenograft model. One million CAR+ primary T cells controlled established SKOV3 tumor growth as evidenced by no detectible tumor measured by imaging (Fig. 15A), quantification of luciferase activity (Fig. 15B), caliper measurement of tumor growth (Fig. 15C) and survival (Fig. 15D). Blood counts of human primary T cells in mice correlated with tumor control where persistence was only observed in the highest dose of CAR+ T cells (Fig. 15E). Although a dose of 1 million CAR+ primary T cells per mice is depicted here as the group "High", this is actually low dose of CAR T cells compared to other studies. This data shows that CAR T cells with activation induced transgene (GFP) expression have the capacity to mediate tumor control *in vivo.*

### Example 16-CAR T cells designed to secrete an activation inducible anti-hIL-6R scFv-Fc secrete biologically active scFv-Fc after antigen-specific CAR T cell stimulation as a method to reduce cytokine release syndrome

To demonstrate functionality of autonomously secreted engineered human IL-6Ra-blocking antibody from primary T cells, its binding capacity to human IL-6Ra expressed on the cell surface was investigated. Primary T cells were activated, transduced with pASP52 (inducible anti-hIL-6R-Myc tag + constitutive anti-CD20 scFv 4-1BB+CD3-zeta CAR; back-to-back orientation) or control pP2 construct (constitutive anti-CD20 scFv 4-1BB+CD3-zeta CAR) at MOI5, expanded and rested according to the standard protocol. Engineered T cells were co-cultured with CD20 positive cell line NALM6 at 3: 1 ratio for 72 h to induce antigen-dependent expression and secretion of hIL-6R blocking antibody. Supernatant from these co-cultures was added to the HEK 293T cells engineered to express hIL-6Ra or control parental cells (with no detectable IL-6Ra expression) and incubated for 1 h. Binding anti-hIL-6R scFv-Fc-Myc tag was detected by secondary stain against Myc tag. Only supernatant from anti-hIL-6R scFv-Fc producing, but not control engineered T cells stained target IL-6Ra expressing cells in a dose dependent manner. The same supernatant did not stain parental HEK 293 T cells demonstrating specific binding. Commercial recombinant anti-hIL-6R antibody was used as a positive control. SN means supernatant.

Only supernatant from anti-hIL-6R scFv-Fc producing, but not control engineered T cells (both activated specifically though CAR), stained IL-6Ra-expressing cells in a dose dependent manner. The same supernatant did not stain parental HEK 293 T cells that do not express human IL-6Ra, demonstrating specificity of the binding (Fig. 16).

To further demonstrate biological activity of secreted engineered human IL-6Ra-blocking antibody, whether its binding to human IL-6Ra expressed on the cell surface would block subsequent binding of commercial anti-hIL-6Ra antibody was investigated. Only supernatant from anti-hIL-6R scFv-Fc producing HEK 293T cells, but not control cells secreting irrelevant antibody, blocked staining of HEK 293T cells engineered to express hIL-6Ra with recombinant anti-IL-6Ra antibody. The blocking effect was found to be dose-dependent. This demonstrated that as designed, the engineered anti-hIL-6R scFv-Fc specifically interacts with hIL-6Ra and has capacity to block binding of other molecules to human IL-6Ra. *See* Figures 19A-19B.

### Example 17- Antigen-inducible transcriptional factors TCF-7 and FOXO1-3A mediate less differentiated status and improved antigen-dependent proliferation in CAR T cells

Emerging concepts show that particular phenotypes of engineered immune cells, such as *memory component* and *less differentiated status,* are beneficial for therapeutic effect in cell based immunotherapies. By introducing transcriptional factors that are known to regulate memory phenotype and stemness as an activation inducible module in the single-vector system, CAR T cell products with a less differentiated phenotype and improved function were developed. As an example, NFAT-inducible TCF-7 and FOXO1 transcriptional factors were implemented in a combination with a Her2-targeting CAR. Since T cell stimulation activates signaling pathways that phosphorylate human wild type FOXO1 which leads to its nuclear export and inactivation of transcriptional activity, key residues were mutated (T24-A24, S256-A256 and S319-A319) according to the published work of Kodama et al. Mol Cell Biol. 2004 Sep;24(18):7931-40, and according to the human FOXO1 sequence in UniProt (www.uniprot.org/uniprot/Q12778). The human FOXO1 sequence as shown in UniProt (Q12778) is reproduced below (SEQ ID NO: 38):

MAEAPQVVEIDPDFEPLPRPRSCTWPLPRPEFSQSNSATSSPAPSGSAAANPDAAAGL
PSASAAAVSADFMSNLSLLEESEDFPQAPGSVAAAVAAAAAAATGGLCGDFQGPE
AGCLHPAPPQPPPPGPLSQHPPVPPAAAGPLAGQPRKSSSSRRNAWGNLSYADLITKA
IESSAEKRLTLSQIYEWMVKSVPYFKDKGDSNSSAGWKNSIRHNLSLHSKFIRVQNE
GTGKSSWWMLNPEGGKSGKSPRRRAASMDNNSKFAKSRSRAAKKKASLQSGQEGA
GDSPGSQFSKWPASPGSHSNDDFDNWSTFRPRTSSNASTISGRLSPIMTEQDDLGEGD
VHSMVYPPSAAKMASTLPSLSEISNPENMENLLDNLNLLSSPTSLTVSTQSSPGTMMQ
QTPCYSFAPPNTSLNSPSPNYQKYTYGQSSMSPLPQMPIQTLQDNKSSYGGMSQYNC
APGLLKELLTSDSPPHNDIMTPVDPGVAQPNSRVLGQNVMMGPNSVMSTYGSQASH
NKMMNPSSHTHPGHAQQTSAVNGRPLPHTVSTMPHTSGMNRLTQVKTPVQVPLPHP
MQMSALGGYSSVSSCNGYGRMGLLHQEKLPSDLDGMFIERLDCDMESIIRNDLMDG
DTLDFNFDNVLPNQSFPHSVKTTTHSWVSG

With this intervention, a FOXO1-3A that is resistant to AKT mediated phosphorylation and is hence insensitive to nuclear export was generated. In CAR T cells exposed to repeated stimulations with target cancer cells, FOXO1-3A was shown to preserve a less differentiated status as determined by increased frequency of CD62L positive, Naïve (CD62L⁺, CD45RA⁺, CD95⁻) and stem memory T cell ***T_{SCM}***(CD62L⁺, CD45RA⁺, CD95⁺) populations compared to control cells that express inducible eGFP (Fig. 19A). Inducible expression of both TCF-7 and FOXO1-3A increased antigen-dependent proliferation of CAR T cells upon repeated stimulations, demonstrating improved functionalities instilled by the single-vector system (Fig. 19B). Antigen-independent proliferation was not observed. Finally, decreased expression of exhaustion marker Lag3 was observed in TCF-7 and FOXO1-3A engineered CAR T cells compared to eGFP control (Fig. 19C). At the same time, incorporation of the inducible TCF-7 and FOXO1-3A did not impair lysis capacity of the engineered Her2-targeting CAR T cells even after several rounds of antigen exposure (Fig. 20).

Together, this data suggests that antigen-dependent expression of transcriptional factors that modify epigenetic profile and phenotype of CAR T cells can improve CAR T cell functions when integrated in the single-vector system. Importantly, the single-vector system ensures that the genetic information for a CAR and transcriptional factor is delivered together, which enables coordinated expression of transcriptional factor induced by CAR signaling in the same T cell. This is in contrast with two-component vector systems where only 30% of the transduced population integrates both constitutive and inducible modules, as demonstrated in Figures 14A-14B.
Amino Acid Sequence of TCF-7 (SEQ ID NO: 30): 
MPQLDSGGGGAGGGDDLGAPDELLAFQDEGEEQDDKSRDSAAGPERDLAELKSSLV
NESEGAAGGAGIPGVPGAGAGARGEAEALGREHAAQRLFPDKLPEPLEDGLKAPEC
TSGMYKETVYSAFNLLMHYPPPSGAGQHPQPQPPLHKANQPPHGVPQLSLYEHFNSP
HPTPAPADISQKQVHRPLQTPDLSGFYSLTSGSMGQLPHTVSWFTHPSLMLGSGVPG
HPAAIPHPAIVPPSGKQELQPFDRNLKTQAESKAEKEAKKPTIKKPLNAFMLYMKEM
RAKVIAECTLKESAAINQILGRRWHALSREEQAKYYELARKERQLHMQLYPGWSAR
DNYGKKKRRSREKHQESTTETNWPRELKDGNGQESLSMSSSSSPA
Amino Acid Sequence of FOXO1-3A (SEQ ID NO: 31):

MAEAPQVVEIDPDFEPLPRPRSCAWPLPRPEFSQSNSATSSPAPSGSAAANPDAAAGL
PSASAAAVSADFMSNLSLLEESEDFPQAPGSVAAAVAAAAAAAATGGLCGDFQGPE
AGCLHPAPPQPPPPGPLSQHPPVPPAAAGPLAGQPRKSSSSRRNAWGNLSYADLITKA
IESSAEKRLTLSQIYEWMVKSVPYFKDKGDSNSSAGWKNSIRHNLSLHSKFIRVQNE
GTGKSSWWMLNPEGGKSGKSPRRRAAAMDNNSKFAKSRSRAAKKKASLQSGQEG
AGDSPGSQFSKWPASPGSHSNDDFDNWSTFRPRTSANASTISGRLSPIMTEQDDLGEG
DVHSMVYPPSAAKMASTLPSLSEISNPENMENLLDNLNLLSSPTSLTVSTQSSPGTMM
QQTPCYSFAPPNTSLNSPSPNYQKYTYGQSSMSPLPQMPIQTLQDNKSSYGGMSQYN
CAPGLLKELLTSDSPPHNDIMTPVDPGVAQPNSRVLGQNVMMGPNSVMSTYGSQAS
HNKMMNPSSHTHPGHAQQTSAVNGRPLPHTVSTMPHTSGMNRLTQVKTPVQVPLP
HPMQMSALGGYSSVSSCNGYGRMGLLHQEKLPSDLDGMFIERLDCDMESIIRNDLM
DGDTLDFNFDNVLPNQSFPHSVKTTTHSWVSG

### Example 18- Adaptation of the single-vector system to sense and respond to IL-6

The single-vector system is modularly designed which makes it amenable for customized design in regards to selection of the immune receptor (here we show use of Her2, CD20 and IL-13Ra2 targeting CARs) and inducible effectors (here we show fluorescent proteins, IL-12, IL-6Ra blocking antibody, TCF-7 and FOXO1-3A transcriptional factors and bispecific T cell engagers). Additionally, by exchanging the DNA response element (promoter region), this system can be engineered to sense customized inputs. As an example, NFAT-sensing promoter was exchanged with the STAT3-sensing promoter and the CAR was exchanged with IL-6Ra in the single-vector system to generate pASP59 (STAT3-eGFP + hIL-6Ra). HEK 293 T cells were transduced with pASP59 and stimulated with increasing amounts of human IL-6. Specific and dose dependent response was observed, demonstrating modular design of the single-vector system where elements (receptors and promoters) can be easily exchanged to detect customized signals and respond by a relevant therapeutic output (Fig. 21). IL-6 sense-response system is useful to generate sensor cell lines to monitor IL-6 signaling pathways, for example detecting IL-6 in the samples or to monitor inhibition of IL-6 by IL-6 blocking antibodies. Therapeutically, sensing IL-6 and responding by producing IL-6 blocking antibody by T cells or other engineered cells in the context of cytokine release syndrome is an alternative to the NFAT-responding system.
Sequence of STATS responsive element (SEQ ID NO: 32): 
[?]
CCGGTACTGTTAGCTTCATTTCCCGTAAATCGTCGAAGCTTCATTTCCCGTAAATC
GTCGAAGCTTCATTTCCCGTAAATCGTCGAAGCTTCATTTCCCGTAAATCGTCGA
Sequence of hIL-6Ra (SEQ ID NO: 33):

ATGCTGGCCGTGGGATGTGCTTTGCTTGCTGCTCTGTTGGCTGCTCCTGGTGCTGC
ACTGGCCCCTAGAAGATGTCCTGCTCAAGAGGTGGCAAGAGGCGTGCTGACATC
TCTGCCTGGCGATAGCGTGACCCTGACATGTCCTGGCGTGGAACCCGAGGATAAT
GCCACCGTGCATTGGGTGCTGAGAAAGCCTGCCGCCGGATCTCATCCTAGCAGAT
GGGCTGGCATGGGCCGTAGACTGCTGCTGAGATCTGTCCAGCTGCACGACAGCG
GCAACTACAGCTGTTACAGAGCCGGCAGACCTGCCGGAACAGTGCATCTGCTGG
TGGATGTGCCTCCTGAGGAACCCCAGCTGAGCTGCTTCAGAAAGTCCCCTCTGAG
CAACGTCGTGTGCGAGTGGGGCCCTAGAAGCACACCTAGCCTGACAACAAAGGC
CGTGCTGCTCGTGCGGAAGTTCCAGAATTCTCCCGCCGAGGACTTCCAAGAGCCT
TGCCAGTACAGCCAAGAGAGCCAGAAGTTCAGCTGTCAGCTGGCTGTGCCTGAG
GGCGACAGCAGCTTCTACATCGTGTCTATGTGCGTGGCCAGCAGCGTGGGCAGC
AAGTTTAGCAAGACCCAGACCTTCCAAGGCTGCGGCATCCTGCAACCTGATCCTC
CAGCCAACATCACCGTGACCGCCGTGGCCAGAAATCCCAGATGGCTGTCTGTGA
CCTGGCAGGACCCTCACAGCTGGAACTCCAGCTTTTACCGGCTGAGATTCGAGCT
GCGGTACAGGGCCGAGAGAAGCAAGACCTTCACCACCTGGATGGTCAAGGACCT
GCAGCATCACTGCGTGATCCACGATGCTTGGAGCGGCCTGAGACATGTGGTGCA
GCTGAGAGCCCAAGAGGAATTCGGCCAAGGCGAGTGGAGTGAATGGTCCCCTGA
AGCCATGGGCACCCCTTGGACAGAGAGCAGATCTCCACCAGCCGAGAACGAGGT
GTCCACACCTATGCAGGCCCTGACCACCAACAAGGACGACGACAACATCCTGTT
CCGGGACAGCGCCAATGCCACAAGTCTGCCTGTGCAGGATAGCTCCTCTGTGCCC
CTGCCTACCTTTCTGGTTGCTGGCGGATCTCTGGCCTTTGGCACCCTGCTGTGTAT
CGCCATCGTGCTGCGGTTCAAGAAAACCTGGAAGCTGCGGGCCCTGAAAGAGGG
CAAGACCTCTATGCACCCTCCTTACAGCCTGGGCCAGCTGGTGCCTGAAAGACCT
AGACCTACACCTGTGCTGGTGCCCCTGATCAGCCCTCCTGTGTCTCCATCTAGCCT
GGGCAGCGACAATACCAGCAGCCACAACAGACCCGACGCTCGGGACCCTAGATC
TCCCTACGACATCAGCAATACCGACTACTTCTTCCCACGC

### Example 19- Use of the single-vector system to screen for antigen-specific immune cells

The single-vector platform can be used as a high-throughput and quantitative screening approach for identification of antigen specific immune cells, such as CAR T cells, TCR modified cells or endogenous TILs. Here successful transduction of patient Tumor-Infiltrating Lymphocytes (TILs) with pASP5 (inducible eGFP + constitutive mCherry) was demonstrated. The inducible module was upregulated in chemically stimulated TILs demonstrating a proof-of-principle to monitor NFAT-mediated signaling in patient derived TILs. When TILs were co-cultured with matching autologous tumor sample, inducible eGFP signature was increased (Fig. 22A). Correlation between standard activation markers used in determining antigen-specific immune cells and NFAT-inducible eGFP was also investigated. Correlation was observed with CD39, CD137 and CD103 but not PD-1 (Fig. 22B).

### Example 20- IL13Rα2-targeting CAR-inducible secretion of functional bispecific T cell engaster pASP79 (BiTE)

To demonstrate implementation of the single-vector system using bispecific T cell engagers (BiTEs), we tested constructs combining constitutive expression of IL13Rα2-targeting CAR with 2 different inducible BiTEs (pASP79 and pASP83; both BiTEs can target both EGFR wild type (EGFRwt) and EGFR variant III (EGFRvIII) proteins). When engineered cells were co-cultured with an IL13Rα2 expressing cell line, inducible expression of soluble BiTEs was observed (Fig. 23A, Fig. 23B and Fig. 24A-24B). Functionality of the CAR T cell-secreted BiTEs was demonstrated by specific lysis of EGFRwt and EGFRvIII expressing target cells when supernatants from antigen activated CAR T cells were added to co-culture of un-transduced primary T cells and target cells (Fig. 23C and Fig. 24C). Thus, pASP79 and pASP83 CAR T cells secrete biologically active EGFR-specific BiTEs in response to antigen activation, and the secreted BiTEs can redirect the activity of un-transduced T cells against EGFR-expressing cancer cells.

### Example 21- Antigen-inducible transcriptional factors TCF-7 and FOXO1-3A mediate less differentiated status and improved antigen-dependent proliferation in CAR T cells

CAR T cells are prepared as follows: Primary T cells are activated, transduced with the control construct pASP30 (inducible eGFP + constitutive 4D5 anti-Her2 scFv 4-1BB+CD3-zeta CAR; back-to-back orientation), and constructs pASP72 (inducible hTCF-7 + constitutive 4D5 anti-Her2 scFv 4-1BB+CD3-zeta CAR; back-to-back orientation) and pASP73 (inducible human FOXO1-3A + constitutive 4D5 anti-Her2 scFv 4-1BB+CD3-zeta CAR; back-to-back orientation) at MOI5, expanded and rested according to the standard protocol. Engineered primary T cells will be injected intravenously (i.v.) at 3 different doses (0.15/0.3/1 million of CAR+ cells per mouse) into NSG mice bearing subcutaneous (s.c.) xenograft SKOV3 tumors. 1 million SKOV3 cells (engineered to express luciferase) will be injected s.c. 4 days prior to the administration of CAR T cells to establish tumors.

Tumor growth is monitored by imaging, quantification of luciferase activity, caliper measurements and survival. Blood counts of human primary T cells in mice will be monitored. Using multicolor flow cytometry, phenotype of engineered cells is investigated.

In Her2-targeting CAR T cells engineered with inducible transcriptional factors, improved tumor control and less differentiated and less exhausted phenotype of CAR T cells is expected to be observed. In the FOXO1-3A example, increased memory component is also expected to be observed. Improved persistence of CAR T cells is expected. Tumor rechallenge model is performed, where better response when transcriptional factor will be engineered into primary T cells along with a CAR, using single vector system is observed.

Along with inducible transcriptional factors, constitutive expression of the same genes as a control is observed. More profound effects on the phenotype are expected to be observed in this case but also increased risk of malignant transformations of engineered cells with certain transcriptional factors.

### Example 22- Use of the single-vector system to screen for immunomodulatory molecules

The single-vector system is utilized to combine solid tumor-targeting CARs with inducible expression of a candidate immunomodulatory molecule. Individual constructs are prepared comprising a CAR, for example a Her2-specific CAR, in combination with a candidate immunomodulatory molecule, and primary T cells are transduced. The functionalities of CAR T cells so generated is determined ex vivo in normal and immunosuppressive conditions. Inducible, antigen-driven expression of integrated molecules is validated on the transcriptional (RT-qPCR) and protein level (ELISA and Flow Cytometry). Their biological activity is further validated. For example, the capacity to lyse Her2 positive target cell line SKOV3 is measured for Her2-specific CAR T cells. Cytokine profiles released by activated CAR T cells are examined using for example the Luminex system. Her2 negative cell line MDA468 may serve as a negative control. The phenotype of CAR T cells is measured by multi-color flow cytometry.

### Example 23-synNotch Single-Vector System

The synNotch receptor is comprised of a portion of Notch wherein both the extracellular and intracellular domains have been completely replaced, leaving only the small central regulatory region (Morsut et al., Cell 164, 780-791, February 11, 2016). The extracellular and intracellular domains from Notch can be swapped with diverse domains. On the extracellular side, different antigen binding domains can be linked to Notch (e.g. scFvs). On the intracellular side, different effectors can be used (e.g. transcriptional activators or transcriptional repressors). Examples of transcriptional activators and repressors that can be used with the synNotch system include but are not limited to Gal4-VP64, tetR-VP64 (tTA), ZFHD1-VP64, and Gal4-KRAB. Upon binding of the extracellular antigen binding domain to its target molecule, Notch is cleaved which releases the intracellular domain containing the transcriptional activator/repressor which leads to the activation/repression of downstream genes.

Primary human T cells were transduced with the synNotch single-vector construct shown in Figure 25A. The constitutive EF1alpha promoter leads to the continuous expression of a HER2-specific synNotch receptor (4D5-Notch1-Gal4-VP64) and a blue fluorescent BFP2 reporter protein. Binding of the synNotch receptor to its target antigen HER2 induces Notch1 cleavage which releases the transcription factor Gal4-VP64 from the receptor. Gal4-VP64 binds to the Gal4 enhancer sequence (Gal4-UAS) thereby activating the minimal CMV promoter (mCMV) and leading to the production of a red fluorescent mCherry reporter protein.

Transduced T cells were cultured in the absence or in the presence of HER2-expressing SKOV3 tumor cells for 24 hours and analyzed with a LSRFortessa flow-cytometer (BD Biosciences). Figure 25B shows experimental validation of the synNotch single-vector system. Representative flow-cytometry plots were generated showing expression of BFP2 and mCherry in primary human T cells transduced with the synNotch single-vector construct.

### Sequences

**Regulatory unit (DNA sequence)** (SEQ ID NO: 34) 
AGGCTGGATCGGTCCCGGTGTCTTCTATGGAGGTCAAAACAGCGTGGATGG
CGTCTCCAGGCGATCTGACGGTTCACTAAACGAGCTCTGCTTATATAGGCCT
CCCACCGTACACGCCTACGGAGGACAGTACTCCGTGCGGAGGACAGTACTCCGTC
CGGAGGACAGTACTCCGATCGGAGGACAGTACTCCGGTCGGAGGACAGTACTCCGCT
CGGAGGACAGTACTCCGACGCGTCGTGAGGCTCCGGTGCCCGTCAGTGGGCAGAG
CGCACATCGCCCACAGTCCCCGAGAAGTTGGGGGGAGGGGTCGGCAATTGAACC
GGTGCCTAGAGAAGGTGGCGCGGGGTAAACTGGGAAAGTGATGTCGTGTACTGG
CTCCGCCTTTTTCCCGAGGGTGGGGGAGAACCGTATATAAGTGCAGTAGTCGCCG
TGAACGTTCTTTTTCGCAACGGGTTTGCCGCCAGAACACAGGTAAGTGCCGTGTG
TGGTTCCCGCGGGCCTGGCCTCTTTACGGGTTATGGCCCTTGCGTGCCTTGAATTA
CTTCCACCTGGCTGCAGTACGTGATTCTTGATCCCGAGCTTCGGGTTGGAAGTGG
GTGGGAGAGTTCGAGGCCTTGCGCTTAAGGAGCCCCTTCGCCTCGTGCTTGAGTT
GAGGCCTGGCCTGGGCGCTGGGGCCGCCGCGTGCGAATCTGGTGGCACCTTCGC
GCCTGTCTCGCTGCTTTCGATAAGTCTCTAGCCATTTAAAATTTTTGATGACCTGC
TGCGACGCTTTTTTTCTGGCAAGATAGTCTTGTAAATGCGGGCCAAGATCTGCAC
ACTGGTATTTCGGTTTTTGGGGCCGCGGGCGGCGACGGGGCCCGTGCGTCCCAGC
GCACATGTTCGGCGAGGCGGGGCCTGCGAGCGCGGCCACCGAGAATCGGACGGG
GGTAGTCTCAAGCTGGCCGGCCTGCTCTGGTGCCTGGCCTCGCGCCGCCGTGTAT
CGCCCCGCCCTGGGCGGCAAGGCTGGCCCGGTCGGCACCAGTTGCGTGAGCGGA
AAGATGGCCGCTTCCCGGCCCTGCTGCAGGGAGCTCAAAATGGAGGACGCGGCG
 
CTCGGGAGAGCGGGCGGGTGAGTCACCCACACAAAGGAAAAGGGCCTTTCCGTC
CTCAGCCGTCGCTTCATGTGACTCCACTGAGTACCGGGCGCCGTCCAGGCACCTC
GATTAGTTCTCGTGCTTTTGGAGTACGTCGTCTTTAGGTTGGGGGGAGGGGTTTT
ATGCGATGGAGTTTCCCCACACTGAGTGGGTGGAGACTGAAGTTAGGCCAGCTT
GGCACTTGATGTAATTCTCCTTGGAATTTGCCCTTTTTGAGTTTGGATCTTGGTTC
ATTCTCAAGCCTCAGACAGTGGTTCAAAGTTTTTTTCTTCCATTTCAGGTGTCGTG
A
   mCMV (rev): **bold**
   Gal4-UAS: *italics*
   EF1alpha (fwd): wave underline
**mCherry reporter (protein sequence)** (SEQ ID NO: 35) 
MVSKGEEDNMAIIKEFMRFKVHMEGSVNGHEFEIEGEGEGRPYEGTQTAKLKVTKG
GPLPFAWDILSPQFMYGSKAYVKHPADIPDYLKLSFPEGFKWERVMNFEDGGVVTV
TQDSSLQDGEFIYKVKLRGTNFPSDGPVMQKKTMGWEASSERMYPEDGALKGEIKQ
RLKLKDGGHYDAEVKTTYKAKKPVQLPGAYNVNIKLDITSHNEDYTIVEQYERAEG
RHSTGGMDELYK
**4D5-Notch1-Gal4-VP64 receptor (protein sequence)** (SEQ ID NO: 36) 
4D5 Notch-Gal4-VP64 receptor (protein sequence) (SEQ ID NO: 60)
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTGSTSGSG
KPGSGEGSEVQLVESGGGLVQPGGSLRLSCAASGFNIKDTYIHWVRQAPGKGLEWV
ARIYPTNGYTRYADSVKGRFTISADTSKNTAYLQMNSLRAEDTAVYYCSRWGGDGF
YAMDVWGQGTLVTVSSILDYSFTGGAGRDIPPPQIEEACELPECQVDAGNKVCNLQC
NNHACGWDGGDCSLNFNDPWKNCTQSLQCWKYFSDGHCDSQCNSAGCLFDGFDC
QLTEGQCNPLYDQYCKDHFSDGHCDQGCNSAECEWDGLDCAEHVPERLAAGTLVL
VVLLPPDQLRNNSFHFLRELSHVLHTNVVFKRDAQGQQMIFPYYGHEEELRKHPIKR
STVGWATSSLLPGTSGGRQRRELDPMDIRGSIVYLEIDNRQCVQSSSQCFQSATDVA
AFLGALASLGSLNIPYKIEAVKSEPVEPPLPSQLHLMYVAAAAFVLLFFVGCGVLLSR
KRRRMKLLSSIEQACDICRLKKLKCSKEKPKCAKCLKNNWECRYSPKTKRSPLTRAH
LTEVESRLERLEQLFLLIFPREDLDMILKMDSLQDIKALLTGLFVQDNVNKDAVTDRL
ASVETDMPLTLRQHRISATSSSEESSNKGQRQLTVSAAAGGSGGSGGSDALDDFDLD
MLGSDALDDFDLDMLGSDALDDFDLDMLGSDALDDFDLDMLGS
**BFP2 reporter (protein sequence)** (SEQ ID NO: 37)

MSELIKENMHMKLYMEGTVDNHHFKCTSEGEGKPYEGTQTMRIKVVEGGPLPFAFD
ILATSFLYGSKTFINHTQGIPDFFKQSFPEGFTWERVTTYEDGGVLTATQDTSLQDGC
LIYNVKIRGVNFTSNGPVMQKKTLGWEAFTETLYPADGGLEGRNDMALKLVGGSHL
IANAKTTYRSKKPAKNLKMPGVYYVDYRLERIKEANNETYVEQHEVAVARYCDLPS
KLGHKLN

### Example 24

Constructs are made that express a CAR and a transcription factor under control of a single constitutive promoter. Some constructs include a 2A peptide spacer between the CAR and the transcription factor. Immune cells, for example T cells, are transduced with the constructs.

Figure 27 illustrates constructs comprising a CAR and a transcription factor under control of a single constitutive promoter with a 2A peptide spacer between the CAR and the transcription factor.

Constructs are made that express a CAR under control of a first constitutive promoter and a transcription factor under control of a second constitutive promoter. Immune cells, for example T cells, are transduced with the constructs.

### Example 25: Inducible Transcription Factors Program CAR T Cell Differentiation and Enhance In vivo Expansion

Genetically integrated systems that combine autonomous antigen-induced production of a molecule of choice were developed, along with constitutive CAR expression in a single lentiviral vector - Uni-Vect (Figure 28). By knocking out the endogenous TCR, CAR signaling became an exclusive activator of the system (Figure 29) while retaining specific lysis through a CAR (Figure 30). To enhance therapeutically relevant T cell states, Uni-Vect was used to modulate CAR T cell-intrinsic features via transient, activation-inducible transcription factor expression (iTF-CAR T cells). Phenotype, expansion, and anti-tumor activity of "upgraded" CAR T cells were analyzed *in vitro* and *in vivo.*

Using Uni-Vect, NFAT-inducible TCF7 or FOXO1-3A transcription factors were combined with a Her2-targeting CAR. Since T cell stimulation activates signaling pathways that phosphorylate human wild type FOXO1 which leads to its nuclear export and inactivation of transcriptional activity, key residues were mutated (T24-A24, S256-A256 and S319-A319) according to the published work (Moffett, et al. Nat. Commun. 2017 Aug 30; 8(1):389; Kodama et al., Mol. Cell. Biol. 24, 7931-7940 (2004)). With this intervention a FOXO1-3A that is resistant to AKT mediated phosphorylation and hence insensitive to nuclear export, was generated. In CAR T cells exposed to repeated stimulations with target cancer cells *in vitro* (Figure 31), it was demonstrated that antigen-dependent FOXO1-3A expression preserved less differentiated phenotype as determined by increased frequency of CD62L⁺, CD45RA⁺ compared to control cells that express inducible eGFP. This population was also CD95⁺, indicating T memory stem cell phenotype (T_{SCM}; CD62L⁺, CD45RA⁺, CD95⁺). These effects were only observed in CAR positive but not in CAR negative population, demonstrating causal role of the inducible transcription factor expression that was necessary and sufficient for differences in phenotype.

Next a similar experiment was conducted in that iTF-CAR T cells were subjected to repeated rounds of stimulations with target cancer cells *in vitro.,* However, CAR positive populations were sorted during CAR T cell product manufacturing (Figure 33). Inducible expression of both TCF7 and FOXO1-3A increased antigen-dependent proliferation of CAR T cells upon repeated stimulations with Her2 positive target cell line *in vitro* (Figure 34). This demonstrated improved expansion instilled by the single-vector system. Antigen-independent proliferation was not observed. Further, decreased expression of exhaustion marker Lag3 was observed in TCF7 and FOXO1-3A engineered CAR T cells compared to eGFP control after repeated exposures to target cells (Figure 35). Finally, CyTOF analysis of iTF-CAR T cells revealed that antigen-inducible expression of a single TF FOXO1-3A favorably affected T cell markers of efficacy, including increased expression of IFNy and T-Bet, and reduced exhaustion marker TIGIT (Figure 36). At the same time, incorporation of the inducible TCF7 and FOXO1-3A did not impair lysis capacity of the engineered Her2-targeting CAR T cells even after several rounds of antigen exposure (Figure 37). Together, these data suggest that antigen-dependent expression of transcription factors that modify differentiation state and phenotype of CAR T cells can improve function when integrated in the single-vector system. Importantly, single-vector system ensures that the genetic information for a CAR and transcription factor is delivered together, which enables coordinated expression of transcription factor induced by CAR signaling in the same T cell, which is in contrast to two component systems.

iTF-CAR T cell activity was then tested *in vivo* in mice bearing tumor xenografts. Two iTF-CAR T cell products and control CAR T cells (iTF1 represents TCF7, iTF2 represents FOXO1-3A and control construct expresses inducible ieGFP) all targeting Her2 were injected in NSG mice with established Her2 positive solid tumors. 1 million of CAR positive primary T cells engineered with inducible eGFP, TCF7 or FOXO1-3A were all able to clear established SKOV3 tumors 3 weeks after infusion as evidenced by no detectible tumor measured by quantification of luciferase activity in tumor cells (Figure 38 upper) and caliper measurement of tumor growth (Figure 38, lower). Further all three iTF-CAR T cell product prevented tumor growth after two re-challenges with the same tumor cells. Because this dose of CAR T cells in this *in vivo* model is optimized to enable effective tumor control even with a control CAR, improved tumor control in iTF-CAR T cells was not observed (Figure 39). Instead the purpose of this study was to investigate how iTF-CAR T cells expand compared to control CAR T cells in this optimal setting. CAR T cells that expressed a transcription factor FOXO1-3A (referred to as iTF2 in the figure) demonstrated significantly increased peak expansion in the blood when compared to control CAR T cells (Figure 40). Importantly, iTF2-CAR T cells expansion was transient and ultimately contracted to the normal levels after tumor was cleared. This finding underscores the importance of inducible TF expression, unlike constitutive, to reduce the risk of malignant transformation of the cellular product. It is reasonable to infer that improved expansion may enable a use of lower CAR T cell dose while favorable phenotype profiles may translate into enhanced anti-tumor responses and better persistence of the infusion product.

Improved *in vitro* expansion (Figure 34) and accompanying differences in phenotype such as such as less differentiated T cell state and increased T_{SCM} pool (Figure 32), decreased inhibitory marker (Figure 35) and increased makers that favor efficacy (Figure 36), led to investigation of what differentiation states and phenotypes might be associated with drastically increased *in vivo* expansion (Figure 40). First the phenotype of iTF-CAR T cell infusion product and a control CAR were analyzed. Similarly as in repeated *in vitro* stimulation tests (Figure 32), a less differentiated T cell state was observed, as determined by increased frequency of CD62L⁺, CD45RA⁺ population in iTF-CAR T featuring FOXO1-3A compared to control cells that express inducible eGFP (Figure 41). This population was also CD95⁺, indicating T memory stem cell phenotype (T_{SCM}; CD62L⁺, CD45RA⁺, CD95⁺) populations. Interestingly, this difference was observed already at the end of a standard 14-day manufacturing process. This is due to inducible expression of FOXO1-3A during 6-days activation phase with anti-CD3/CD28 beads that was driven by the NFAT signaling. Interestingly, since iTF-CAR T cells are fully rested at day 14, differences in phenotype demonstrated programming of CAR T cell differentiation states during temporal inducible expression of the designed transcription factor FOXO1-3A.

Along with T cell blood counts, we analyzed CAR expression on day 24 and demonstrated that a large frequency of CAR T cells were CAR positive (Figure 42). That is expected since T cells recognizing tumor through CAR, specifically and preferentially expand. This is also supported by the fact that in the infusion product, CAR expression was normalized to 13% CAR positive. On day 24, estimated frequency of T cells expressing CAR were in the range of 80% in most mice.

Next, the phenotype of iTF was analyzed against and control CAR T cells. Interestingly, a marked increase in frequency of CD62L⁻, CD45RA⁺ population in iTF-CAR T featuring FOXO1-3A (average 50%) compared to control cells (average 25%) that expressed inducible eGFP at a peak expansion on a day 24 was observed (Figure 43). This phenotype and differentiation state is associated with terminally differentiated antigen experienced effector cells capable of mounting effective anti-tumor responses. This trend was similar also at day 50 which is after 2 subsequent re-challenges with tumor cells (Figure 44). This result can be interpreted as a productive immune response that resamples effective antiviral responses where antigen-specific cells are capable of productive differentiation into effector cells that clear infection. Supporting this hypothesis are the results of *in vitro* rechallenge model where increased expression of molecules that favor efficacy was observed using CyTOF (Figure 36). Additionally, since CAR T cells contracted to normal levels after tumor clearance this further marks features of effective endogenous immune response against infections.

Finally, survival of mice with infused iTF and control CAR T cells was comparable (Figure 45) with an interesting observation that iFOXO-3A conferred apparently delayed GVHD responses.

In summary, the results described herein demonstrated that inducible TF expression equips CAR T cells with improved therapeutically relevant T cell states including enhanced antigen-dependent proliferation, less differentiated phenotype, and decreased expression of the inhibitory marker following repeated stimulations with antigen-expressing cancer cells *in vitro.* This translated into improved *in vivo* T cell expansion which infers enhanced anti-tumor responses and potentially reduced CAR T cell dose required for therapeutic responses, because of the improved fitness of the cellular product. With these contributions, a foundation for more effective next-generation cellular immunotherapies is established.

### Example 26: Screening of mKRAS-specific TCR avidity using a Jurkat cell reporter system

### Materials and Methods:

*Jurkat Reporter System and TCR Antigen Specificity and Avidity:* Using Cas9 protein and riboprobes as guides against TRAC (TGTGCTAGACATGAGGTCTA) (SEQ ID NO: 39) and TRBC (GGAGAATGACGAGTGGACCC) (SEQ ID NO: 40), a Jurkat 76 TCR negative cell line was generated. To generate J90 _NFAT reporter cells, TCR negative Jurkat 76 cells were subsequently transduced with lentiviral particles encoding human CD8ab as well as a the Asp90 reporter construct. The Asp90 reporter construct encodes for constitutive mCherry expression driven by the EF1a promoter and inducible eGFP expression driven by the NFAT promoter as a means to assess activation of TCR signaling. J90_NFAT reporter cells were flow cytometrically sorted to purity based on TCR- (anti-human TCRa/b antibody clone IP26, Biolegend, San Diego, CA), CD8+ (anti-CD8 antibody, ThermoFisher Scientific, Waltham, MA) and mCherry+ expression. The functionality of J90_NFAT reporter cells was tested for low background expression of reporter gene and strong upregulation of eGFP upon stimulation with PMA (50 ng/mL) and ionomycin (750 ng/mL). The TCRa and TCRb chains of TCRA3V, TCRA11V and TCRB7R were expressed via lentivirus in J90 _NFAT using the pTRPE vector to generate the J-TCRA3V, J-TCRA11V and J-TCRB7R cell lines. These cell lines were sorted to yield a unimodal dextramer positive cell population and expanded for use in functional assays. Sorted J-TCRA3V, J-TCRA11V and J-TCRB7R cell lines were mixed 1:1 with HLA-SCD-expressing K562 cells pulsed with titrated concentrations of target peptides ranging from 10 mM - 1 pM. After 16-20 h of incubation at 37 °C, cells were analyzed by flow cytometry to determine percentage of eGFP positive cells in each sample. Cells activated with PMA and Ionomycin were included as positive controls and data was fitted to a dose response curve by linear non-regression analysis using GraphPad Prism version 7.0.

*TCRs as Probes for Recognition of Endogenously Processed and Presented mKRAS Epitopes:* Tumor cell lines (2.5 X 10^5 cells/well) were cocultured with TCR transduced J90_NFAT cells at a 1:1 ratio in 48-well flat bottom plates in a final volume of 500 ml of media (RPMI, 10% FBS, L-Glutamine, Penicillin/Streptomycin). Each tumor cell lines was tested as parental (non-HLA matched, no modification) or expressing HLA-SCD (HLA class I matched). After 16-20h of incubation at 37 °C, cells were analyzed by flow cytometry to determine the percentage of live, CD8+mCherry+eGFP+ cells in each sample. Cells activated with PMA and Ionomycin were included as positive controls.

### Results:

The expression and function of mKRAS-TCRs were characterized using J90_NFAT reporter cells derived from Jurkat E6.1 cells gene-edited using CRISPR-Cas9 to delete endogenous TCRa and TCRb and transgenically modified to express the CD8ab heterodimer as well as an NFAT-inducible eGFP reporter system. mKRAS-TCRa/b chain pairs were cloned into lentiviral constructs then transgenically expressed in J90 _NFAT reporter cells. p/MHC multimer staining were performed on transduced J90 _NFAT reporter cells to validate TCRA3V, TCRA11V and TCRB7R expression and antigen specificity (Figure 46A). mKRAS-specific TCR function, specificity and avidity were assessed. TCRa/b-transduced J90_NFAT reporter cells (J-TCR) were co-cultured with HLA class I monoallelic K562 cells pulsed with WT or mKRAS synthetic peptides. J-TCRA3V, J-TCRA11V and J-TCRB7R cells demonstrated specific reactivity to target mKRAS epitopes without cross reactivity to the equivalent WT KRAS peptides (Figure 46B). Furthermore, J-TCRA3V and J-TCRB7R cells did not demonstrate cross-reactivity to alternative mKRAS epitopes, while J-TCRA11V cells demonstrated cross-reactivity to KRAS G12C (VVV_C) albeit at decreased avidity as compared to target KRAS G12V (VVV_V). Using log10 peptide titration, the functional avidities of each TCR were determined based on the mean peptide concentrations to achieve 50% NFAT activation of transgenic TCR J90 _NFAT reporter cells. The avidities of TCRA3V, TCRA11V and TCRB7R were determined. These TCR-transfectants constituted high affinity probes for characterization of naturally processed and presented (bone fide) mKRAS epitopes by human tumor cells.

### mKRAS-specific TCRs recognize endogenous mKRAS/HLA class I complexes on tumor cells

Effective anti-tumor immunity conferred by TCR engineered T cells requires the recognition of antigen-MHC complexes on the surface of cancer cells. To this end, a panel of KRAS G12V-mutated (QGP-1, YAPC, SK-CO-1, SW620, COR-L23, NCI-H441) or G12R-mutated (CAL-62, HuP-T3, KP-2, PSN1) cancer cell lines of various tissue origins were assembled to be used as targets in functional assays. Aside from NCI-H441 cells, all G12V-mutated cell lines were modified to express either HLA-A*03:01 or HLA-A*11:01 by lentiviral transduction with HLA-SCD constructs. NCI-H441 cells which are HLA-A*03:01 positive were only modified to express HLA-A*11:01. Likewise, all G12R-mutated cell lines were modified to express HLA-B*07:02. The ability of J-TCRA3V and J-TCRA11V cells to recognize mKRAS G12V-expressing cancer cell lines was evaluated. J-TCRA3V and J-TCRA11V cells were cocultured with KRAS G12V-mutated cell lines expressing HLA-A*03:01 or BLA-A*11:01, respectively. Following 24-hour coculture, J-TCRA3V and J-TCRA11V demonstrated NFAT-induced GFP expression indicative of TCR sensing of mKRAS G12V (VVV_V)/BLA complexes presented on the surface of tumor cells (Figure 46C). The graded pattern of NFAT activation induced in J90_NFAT reporter cells suggests varied surface expression of VVV_V peptide/HLA complexes by cancer cells. Similarly, J - TCRB7R cells were cocultured for 24 hours with KRAS.

### Other Embodiments

The recitation of a listing of elements in any definition of a variable herein includes definitions of that variable as any single element or combination (or subcombination) of listed elements. The recitation of an embodiment herein includes that embodiment as any single embodiment or in combination with any other embodiment or portions thereof.

## Claims

1. An engineered cell comprising (a) a nucleic acid encoding a receptor capable of receiving an activating signal and (b) an inducible promoter operably linked to a nucleic acid encoding FOXO1-3A or TCF7, wherein the inducible promoter is induced when the receptor receives an activating signal.

2. A method of:
(i) programming differentiation status and/or increasing proliferation of a cell comprising a receptor capable of receiving an activating signal;
(ii) inducing therapeutically relevant cell states associated with improved anti-tumor activity, efficacy, and functionality in a cell comprising a receptor capable of receiving an activating signal; or
(iii) generating a cell for use in adoptive cell therapy, wherein the cell comprises a receptor capable of receiving an activating signal;
the method comprising genetically modifying the cell to operably link an inducible promoter to a nucleic acid encoding FOXO1-3A or TCF7, wherein the inducible promoter is induced when the receptor receives an activating signal.

3. A cell comprising a receptor capable of receiving an activating signal for use in a method of treating cancer in a subject in need thereof, wherein the cell comprises an inducible promoter operably linked to a nucleic acid encoding FOXO1-3A or TCF7, and wherein the inducible promoter is induced when the receptor receives an activating signal.

4. The engineered cell of claim 1, or method of claim 2, or the cell for use according to claim 3, wherein the cell is a T cell, NK cell, macrophage, or a regulatory T cell.

5. The engineered cell of claim 1 or 4, or the method of claim 2 or 4, or the cell for use according to claim 3 or 4, wherein the receptor is a chimeric antigen receptor (CAR) or a T cell receptor (TCR).

6. The engineered cell of claim 5, or the method of claim 5, or the cell for use according to claim 5, wherein the CAR comprises an antigen binding domain, a transmembrane domain, and an intracellular signaling domain.

7. The engineered cell of claim 6, or the method of claim 6, or the cell for use according to claim 6, wherein the antigen binding domain specifically binds a tumor antigen.

8. The engineered cell of claim 6 or 7, or the method of claim 6 or 7, or the cell for use according to claim 6 or 7, wherein the intracellular signaling domain comprises an intracellular domain of a costimulatory molecule selected from: CD27, CD28, 4-1BB(CD137), OX40, CD30, CD40, PD-1, ICOS, lymphocyte function-associated antigen-1 (LFA-1), CD2, CD7, LIGHT, NKG2C, B7-H3, and a ligand that specifically binds with CD83.

9. The engineered cell of claim 5, or the method of claim 5, or the cell for use according to claim 5, wherein the TCR comprises TCR alpha and beta chains, or TCR gamma and delta chains.

10. The engineered cell of any one of claims 1 and 4 to 9, or the method of any one of claims 2 and 4 to 9, or the cell for use according to any one of claims 3 to 9, wherein the inducible promoter is an NFAT promoter, a synthetic NFAT promoter, a NF-kB promoter, a CD69 promoter, a CD137 promoter, synthetic hypoxia-responsive element (HRE) promoter, or a PD-1 promoter.

11. The engineered cell of any one of claims 1 and 4 to 10, or the method of any one of claims 2 and 4 to 10, or the cell for use according to any one of claims 3 to 10, wherein:
(i) the nucleic acid encoding FOXO1-3A encodes the amino acid sequence set forth in SEQ ID NO: 31; and/or
(ii) the nucleic acid encoding TCF7 encodes the amino acid sequence set forth in SEQ ID NO: 30.

## Patentansprüche

1. Eine konstruierte Zelle, umfassend (a) eine Nukleinsäure, die einen Rezeptor codiert, der fähig ist, ein Aktivierungssignal zu empfangen, und (b) einen induzierbaren Promotor, der funktionsfähig mit einer Nukleinsäure verbunden ist, die FOXO1-3A oder TCF7 codiert, wobei der induzierbare Promotor induziert wird, wenn der Rezeptor ein Aktivierungssignal empfängt.

2. Ein Verfahren zum:
(i) Programmieren des Differenzierungsstatus und/oder Erhöhen der Proliferation einer Zelle, die einen Rezeptor umfasst, der fähig ist ein Aktivierungssignal zu empfangen;
(ii) Induzieren therapeutisch relevanter Zellzustände, die mit einer verbesserten Antitumoraktivität, Wirksamkeit und Funktionalität in einer Zelle verbunden sind, die einen Rezeptor umfasst, der fähig ist ein Aktivierungssignal zu empfangen; oder
(iii) Erzeugen einer Zelle zur Verwendung in der adoptiven Zelltherapie, wobei die Zelle einen Rezeptor umfasst, der fähig ist, ein Aktivierungssignal zu empfangen;
wobei das Verfahren das genetische Modifizieren der Zelle umfasst, um einen induzierbaren Promotor funktionsfähig mit einer Nukleinsäure zu verbinden, die FOXO1-3A oder TCF7 codiert, wobei der induzierbare Promotor induziert wird, wenn der Rezeptor ein Aktivierungssignal empfängt.

3. Eine Zelle, die einen Rezeptor umfasst, der fähig ist, ein Aktivierungssignal zu empfangen, zur Verwendung in einem Verfahren zum Behandeln von Krebs bei einem Subjekt, das dessen bedarf, wobei die Zelle einen induzierbaren Promotor umfasst, der funktionsfähig mit einer Nukleinsäure verbunden ist, die FOXO1-3A oder TCF7 codiert, und wobei der induzierbare Promotor induziert wird, wenn der Rezeptor ein Aktivierungssignal empfängt.

4. Die konstruierte Zelle nach Anspruch 1, oder das Verfahren nach Anspruch 2, oder die Zelle zur Verwendung nach Anspruch 3, wobei die Zelle eine T-Zelle, eine NK-Zelle, ein Makrophage oder eine regulatorische T-Zelle ist.

5. Die konstruierte Zelle nach Anspruch 1 oder 4, oder das Verfahren nach Anspruch 2 oder 4, oder die Zelle zur Verwendung nach Anspruch 3 oder 4, wobei der Rezeptor ein chimärer Antigenrezeptor (CAR) oder ein T-Zell-Rezeptor (TCR) ist.

6. Die konstruierte Zelle nach Anspruch 5, oder das Verfahren nach Anspruch 5, oder die Zelle zur Verwendung nach Anspruch 5, wobei das CAR eine Antigenbindungsdomäne, eine Transmembrandomäne, und eine intrazelluläre Signaldomäne umfasst.

7. Die konstruierte Zelle nach Anspruch 6, oder das Verfahren nach Anspruch 6, oder die Zelle zur Verwendung nach Anspruch 6, wobei die Antigenbindungsdomäne spezifisch an ein Tumorantigen bindet.

8. Die konstruierte Zelle nach Anspruch 6 oder 7, oder das Verfahren nach Anspruch 6 oder 7, oder die Zelle zur Verwendung nach Anspruch 6 oder 7, wobei die intrazelluläre Signaldomäne eine intrazelluläre Domäne eines kostimulatorischen Moleküls umfasst, ausgewählt aus: CD27, CD28, 4-1BB (CD137), OX40, CD30, CD40, PD-1, ICOS, Lymphozytenfunktions-assoziiertes Antigen-1 (LFA-1), CD2, CD7, LIGHT, NKG2C, B7-H3 und ein Liganden, der spezifisch an CD83 bindet.

9. Die konstruierte Zelle nach Anspruch 5, oder das Verfahren nach Anspruch 5, oder die Zelle zur Verwendung nach Anspruch 5, wobei der TCR TCR-Alpha- und Beta-Ketten oder TCR-Gamma- und Delta-Ketten umfasst.

10. Die konstruierte Zelle nach einem der Ansprüche 1 und 4 bis 9, oder das Verfahren nach einem der Ansprüche 2 und 4 bis 9, oder die Zelle zur Verwendung nach einem der Ansprüche 3 bis 9, wobei der induzierbare Promotor ein NFAT-Promotor, ein synthetischer NFAT-Promotor, ein NF-kB-Promotor, ein CD69-Promotor, ein CD137-Promotor, ein synthetischer Hypoxie-reagierendes Element (HRE)-Promotor oder ein PD-1-Promotor ist.

11. Die konstruierte Zelle nach einem der Ansprüche 1 und 4 bis 10, oder das Verfahren nach einem der Ansprüche 2 und 4 bis 10, oder die Zelle zur Verwendung nach einem der Ansprüche 3 bis 10, wobei:
(i) die Nukleinsäure, die FOXO1-3A codiert, die in SEQ ID NO: 31 angegebene Aminosäuresequenz codiert; und/oder
(ii) die Nukleinsäure, die TCF7 codiert, die in SEQ ID NO: 30 angegebene Aminosäuresequenz codiert.

## Revendications

1. Cellule génétiquement modifiée comprenant (a) un acide nucléique codant pour un récepteur capable de recevoir un signal d'activation et (b) un promoteur inductible fonctionnellement lié à un acide nucléique codant pour FOXO1-3A ou TCF7, dans laquelle le promoteur inductible est induit lorsque le récepteur reçoit un signal d'activation.

2. Procédé consistant à :
(i) programmer un état de différenciation et/ou accroître une prolifération d'une cellule comprenant un récepteur capable de recevoir un signal d'activation ;
(ii) induire des états cellulaires thérapeutiquement pertinents associés à une activité, une efficacité, et une fonctionnalité antitumorales améliorées chez une cellule comprenant un récepteur capable de recevoir un signal d'activation ; ou
(iii) générer une cellule pour l'utilisation dans une thérapie cellulaire adoptive, dans lequel la cellule comprend un récepteur capable de recevoir un signal d'activation ;
le procédé comprenant la modification génétiquement de la cellule pour la liaison fonctionnelle d'un promoteur inductible à un acide nucléique codant pour FOXO1-3A ou TCF7, dans lequel le promoteur inductible est induit lorsque le récepteur reçoit un signal d'activation.

3. Cellule comprenant un récepteur capable de recevoir un signal d'activation pour l'utilisation dans un procédé de traitement du cancer chez un sujet en ayant besoin, dans laquelle la cellule comprend un promoteur inductible fonctionnellement lié à un acide nucléique codant pour FOXO1-3A ou TCF7, et dans laquelle le promoteur inductible est induit lorsque le récepteur reçoit un signal d'activation.

4. Cellule génétiquement modifiée selon la revendication 1, ou procédé selon la revendication 2, ou cellule pour l'utilisation selon la revendication 3, dans lequel·laquelle la cellule est une cellule T, une cellule NK, un macrophage, ou une cellule T de régulation.

5. Cellule génétiquement modifiée selon la revendication 1 ou 4, ou procédé selon la revendication 2 ou 4, ou cellule pour l'utilisation selon la revendication 3 ou 4, dans lequel·laquelle le récepteur est un récepteur d'antigène chimère (CAR) ou un récepteur de cellule T (TCR).

6. Cellule génétiquement modifiée selon la revendication 5, ou procédé selon la revendication 5, ou cellule pour l'utilisation selon la revendication 5, dans lequel·laquelle le CAR comprend un domaine de liaison à un antigène, un domaine transmembranaire, et un domaine de signalisation intracellulaire.

7. Cellule génétiquement modifiée selon la revendication 6, ou procédé selon la revendication 6, ou cellule pour l'utilisation selon la revendication 6, dans lequel·laquelle le domaine de liaison à l'antigène se lie spécifiquement à un antigène tumoral.

8. Cellule génétiquement modifiée selon la revendication 6 ou 7, ou procédé selon la revendication 6 ou 7, ou cellule pour l'utilisation selon la revendication 6 ou 7, dans lequel·laquelle le domaine de signalisation intracellulaire comprend un domaine intracellulaire d'une molécule de costimulation sélectionnée parmi : CD27, CD28, 4-1BB(CD137), OX40, CD30, CD40, PD-1, ICOS, un antigène associé à une fonction des lymphocytes-1 (LFA-1), CD2, CD7, LIGHT, NKG2C, B7-H3, et un ligand qui se lie spécifiquement à CD83.

9. Cellule génétiquement modifiée selon la revendication 5, ou procédé selon la revendication 5, ou cellule pour l'utilisation selon la revendication 5, dans lequel·laquelle le TCR comprend le TCR alpha et des chaînes bêtas, ou le TCR gamma et des chaînes deltas.

10. Cellule génétiquement modifiée selon l'une quelconque des revendications 1 et 4 à 9, ou procédé selon l'une quelconque des revendications 2 et 4 à 9, ou cellule pour l'utilisation selon l'une quelconque des revendications 3 à 9, dans lequel·laquelle le promoteur inductible est un promoteur NFAT, un promoteur NFAT synthétique, un promoteur NF-kB, un promoteur CD69, un promoteur CD137, un promoteur synthétique d'élément de réponse à l'hypoxie (HRE), ou un promoteur PD-1.

11. Cellule génétiquement modifiée selon l'une quelconque des revendications 1 et 4 à 10, ou procédé selon l'une quelconque des revendications 2 et 4 à 10, ou cellule pour l'utilisation selon l'une quelconque des revendications 3 à 10, dans lequel·laquelle :
(i) l'acide nucléique codant pour FOXO1-3A code pour la séquence d'acides aminés énoncée selon SEQ ID n° : 31 ; et/ou
(ii) l'acide nucléique codant pour TCF7 code pour la séquence d'acides aminés énoncée selon SEQ ID n° : 30.
